(19) Europäisches Patentamt
European Patent Office
Office européen des brevets

(11) EP 4 775 208 A1

(12) EUROPEAN PATENT APPLICATION
published in accordance with Art. 153(4) EPC

(43) Date of publication:
**15.07.2026 Bulletin 2026/29**

(21) Application number: **24876662.8**

(22) Date of filing: **12.10.2024**

(51) International Patent Classification (IPC):
***A61K 31/519*** (2006.01) ***A61K 31/536*** (2006.01)
***C07D 471/14*** (2006.01) ***C07D 487/04*** (2006.01)
***C07D 519/00*** (2006.01) ***A61P 35/00*** (2006.01)

(52) Cooperative Patent Classification (CPC):
**A61K 31/519; A61K 31/536; A61P 35/00; C07D 471/14; C07D 487/04; C07D 519/00**

(86) International application number:
**PCT/CN2024/124325**

(87) International publication number:
**WO 2025/077857 (17.04.2025 Gazette 2025/16)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.10.2023 CN 202311331756**

(71) Applicant: **Apeiron Therapeutics (Hong Kong) Ltd.**
**Central, Hong Kong (HK)**

(72) Inventors:
- **YAO, Bing**
  **Shanghai 201210 (CN)**
- **GU, Xiaohui**
  **Shanghai 201210 (CN)**
- **MAO, Weifeng**
  **Shanghai 201210 (CN)**
- **ASWAD, Fred Jullien**
  **Shanghai 201210 (CN)**
- **JOSEPH, James David**
  **Shanghai 201210 (CN)**
- **LI, Mingxi**
  **Shanghai 201210 (CN)**

(74) Representative: **Bayramoglu et al.**
**Mira Office**
**Kanuni Sultan Süleyman Boulevard 5387**
**Street Beytepe, floor 12, no:50**
**06800 Cankaya, Ankara (TR)**

# (54) USE OF PRMT5 INHIBITOR IN TREATMENT OF TUMORS OR CANCERS

(57) The present disclosure describes novel molecules having Protein Arginine Methyltransferase 5 (PRMT5) inhibitory activity, as well as methods for the preparation and use thereof. Specifically, the present disclosure describes compounds of Formula (I), or pharmaceutically acceptable salts, hydrates, or solvates thereof, and methods for the synthesis and use of said compounds.



Processed by Luminess, 75001 PARIS (FR)

(Cont. next page)

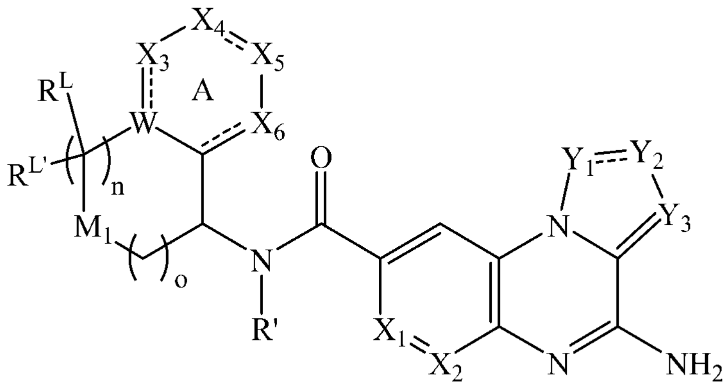

Formula (I)

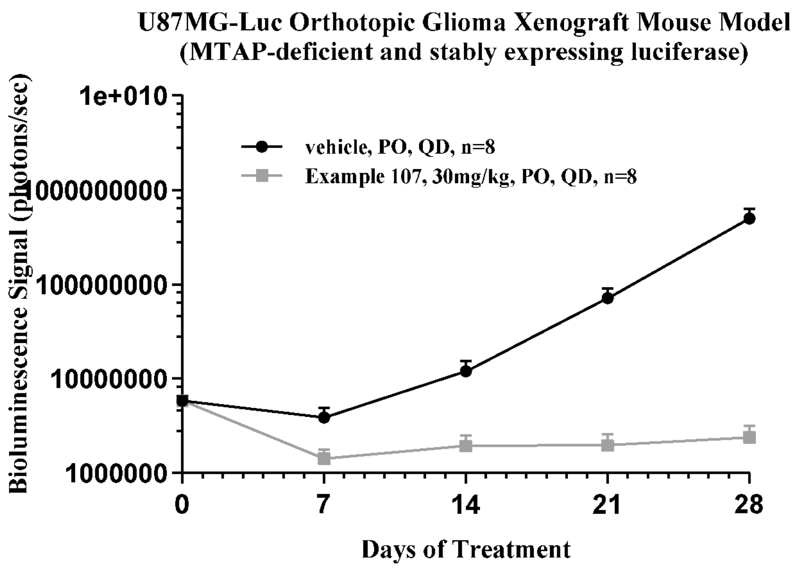


Figure 1

## Description

### TECHNICAL FIELD

The present invention relates to the pharmaceutical field. In particular, the present invention relates to PRMTS inhibitors and uses thereof.

### BACKGROUND TECHNOLOGY

Epigenetic changes play a crucial role in driving and sustaining the malignant phenotype of tumors. Processes such as DNA methylation, histone acetylation and methylation, non-coding RNA, and post-translational modifications are epigenetic drivers of cancer, independent of DNA sequence changes. Arginine methylation is a significant post-translational modification that, by regulating transcription and post-transcriptional RNA processing, affects cell growth, proliferation, apoptosis, angiogenesis, and metastasis. Methylated arginines include three types: ω-NG, N'G-asymmetric dimethylarginine (ADMA), and ω-NG, N'G-symmetric dimethylarginine (SDMA). This modification is catalyzed by the protein arginine methyltransferase (PRMT) family, transferring methyl groups from S-adenosyl methionine (AdoMet) to arginine side chains in both histone and non-histone proteins. The human genome annotates nine PRMT genes, categorized into Type I (PRMT1, 2, 3, 4, 6, and 8), Type II (PRMTS and PRMT9), and Type III enzymes (PRMT7). PRMT5, primarily a Type II enzyme, is responsible for catalyzing symmetric dimethylation of arginine. It was initially discovered through a yeast two-hybrid experiment screening for proteins interacting with Janus kinase 2 (Jak2).

PRMTS serves as a versatile transcriptional repressor, forming complexes with various transcription factors like BRG1, Hbrm, Blimp1, and Snail. Engaging in diverse cellular processes, PRMTS methylates substrates in both the cytoplasm and nucleus, including histone H4 residue Arg3 (H4R3) and histone H3 residue Arg8 (H3R8). H4R3 methylation is linked to transcriptional repression, while H3R8 methylation is associated with both transcriptional activation and repression. Beyond inducing repressive histone marks directly, PRMTS's role in gene silencing involves the formation of multi-repressive protein complexes, incorporating NuRD components, HDACs, MDB proteins, and DNA methyltransferases. PRMT5 influences substrate specificity through interactions with binding proteins within these complexes. At the core of this protein complex is MEP50, crucial for PRMTS's enzymatic activity. Studies reveal PRMTS's ability to methylate proteins involved in RNA splicing, such as SmD3, providing insight into monitoring PRMT5's chemical activity in cellular biology.

PRMT5 plays a crucial role in tumorigenesis. Studies have shown upregulation of PRMTS expression in various cancers, including lymphoma, lung cancer, breast cancer, and colorectal cancer. Additionally, PRMTS expression is elevated in Mantle Cell Lymphoma (MCL) patient samples, and PRMTS depletion inhibits MCL cell proliferation, highlighting its significance in MCL. PRMTS overexpression promotes cell proliferation, while PRMT5 knockout inhibits proliferation in melanoma, breast cancer, and lung cancer cell lines. Therefore, PRMTS stands as a potential target for cancer therapy.

The loss of Methylthioadenosine Phosphorylase (MTAP) imparts selective dependence on PRMT5 and its binding protein WDR77. MTAP is often lost due to its proximity to the frequently deleted tumor suppressor gene CDKN2A. Cells with MTAP loss exhibit increased intracellular Methylthioadenosine (MTA, a metabolite cleaved by MTAP). As MTA shares a similar structure with S-adenosyl methionine (SAM), an inherent selective inhibitor, elevated MTA concentration inhibits the binding of SAM to PRMT5, consequently suppressing PRMTS's methyltransferase activity.

The structural divergence between cancer cells with MTAP loss and those with wild-type MTAP primarily manifests in the PRMT5-MTA complex due to the accumulation of MTA in MTAP-deficient cells. Inhibitors designed to target the PRMT5-MTA complex demonstrate selective efficacy against cancer cells with MTAP loss, minimizing impact on normal cells and substantially enhancing the therapeutic index.

Hence, the identification and development of small molecules inhibiting PRMTS activity stand as methods for treating various PRMT5-associated diseases or conditions, particularly cancer.

### CONTENT OF THE INVENTION

To address the technical issues of the present invention, the invention provides a method for treating tumors or cancers, comprising administering to a subject in need thereof a compound represented by Formula (I), or a pharmaceutically acceptable salt, ester, prodrug, stereoisomer, or isotopic derivative thereof,

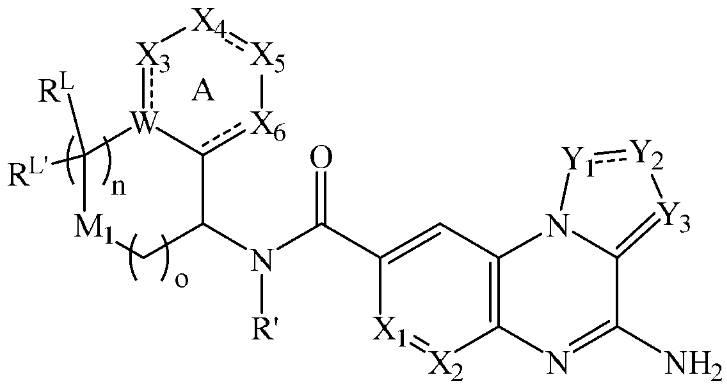

Formula (I)

Wherein, W is C;

Wherein, $X_3$ is N or $CR^{X3}$; $X_4$ is N or $CR^{X4}$; $X_5$ is N or $CR^{X5}$; $X_6$ is N or $CR^{X6}$;

Wherein, when $X_3$ is $CR^{X3}$, $R^{X3}$ is hydrogen, deuterium, $C_1$-$C_6$ alkyl, $C_1$-C6 haloalkyl, halogen, -$OR^a$, -$SR^a$, -P(O)$R^aR^b$, -CN, -$S(O)_2R^a$, -S(O)$R^a$, -$SF_5$, -$NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxyl($C_1$-$C_6$ alkyl), $NR^aR^b$, -CN, $C_1$-$C_6$ haloalkyl, $C_1$-$C_6$ haloalkoxy, -$SO_3R^a$, -$SR^a$, -$SF_5$, -C(O)$R^a$, -C(O)$OR^a$, -OC(O)$R^a$, -OC(O)$NR^aR^b$, -$NR^aCOR^b$ or -$CONR^aR^b$;

Wherein, when $X_4$ is $CR^{X4}$, $R^{X4}$ is hydrogen, deuterium, $C_1$-$C_6$ alkyl, $C_1$-C6 haloalkyl, halogen, -$OR^a$, -$SR^a$, -P(O)$R^aR^b$, -CN, -$S(O)_2R^a$, -S(O)$R^a$, -$SF_5$, -$NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxyl($C_1$-$C_6$ alkyl), $NR^aR^b$, -CN, $C_1$-$C_6$ haloalkyl, $C_1$-$C_6$ haloalkoxy, -$SO_3R^a$, -$SR^a$, -$SF_5$, -C(O)$R^a$, -C(O)$OR^a$, -OC(O)$R^a$, -OC(O)$NR^aR^b$, -$NR^aCOR^b$ or -$CONR^aR^b$;

Wherein, when $X_5$ is $CR^{X5}$, $R^{X5}$ is hydrogen, deuterium, $C_1$-$C_6$ alkyl, $C_1$-$C_6$ haloalkyl, halogen, -$OR^a$, -$SR^a$, -P(O)$R^aR^b$, -CN, -$S(O)_2R^a$, -S(O)$R^a$, -$SF_5$, -$NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxyl($C_1$-$C_6$ alkyl), $NR^aR^b$, -CN, $C_1$-$C_6$ haloalkyl, $C_1$-$C_6$ haloalkoxy, -$SO_3R^a$, -$SR^a$, -$SF_5$, -C(O)$R^a$, -C(O)$OR^a$, -OC(O)$R^a$, -OC(O)$NR^aR^b$, -$NR^aCOR^b$ or -$CONR^aR^b$;

Wherein, when $X_6$ is $CR^{X6}$, $R^{X6}$ is hydrogen, deuterium, $C_1$-$C_6$ alkyl, $C_1$-$C_6$ haloalkyl, halogen, -$OR^a$, -$SR^a$, -P(O)$R^aR^b$, -CN, -$S(O)_2R^a$, -S(O)$R^a$, -$SF_5$, -$NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxyl($C_1$-$C_6$ alkyl), $NR^aR^b$, -CN, $C_1$-$C_6$ haloalkyl, $C_1$-$C_6$ haloalkoxy, -$SO_3R^a$, -$SR^a$, -$SF_5$, -C(O)$R^a$, -C(O)$OR^a$, -OC(O)$R^a$, -OC(O)$NR^aR^b$, -$NR^aCOR^b$ or -$CONR^aR^b$;

Wherein, the ring A can optionally fuse at the chemical bond between $X_3$ and $X_4$ with a 5-6 membered saturated or unsaturated ring, which can contain 0-3 heteroatoms selected from O, N, S;

Wherein, the ring A can optionally fuse at the chemical bond between $X_4$ and $X_5$ with a 5-6 membered saturated or unsaturated ring, which can contain 0-3 heteroatoms selected from O, N, S;

Wherein, the ring A can optionally fuse at the chemical bond between $X_5$ and $X_6$ with a 5-6 membered saturated or unsaturated ring, which can contain 0-3 heteroatoms selected from O, N, S;

Wherein, the ring A also can be optionally substituted with 0, 1, 2, 3 groups selected from hydrogen, $C_1$-$C_6$ alkyl, deuterated $C_1$-$C_6$ alkyl, halogen, -$OR^a$, -$SR^a$, -P(O)$R^aR^b$, -CN, -$S(O)_2R^a$, -S(O)$R^a$, -$SF_5$, -$NR^aR^b$, $C_1$-$C_6$ haloalkyl, $C_1$-$C_6$ haloalkoxy, hydroxy($C_1$-$C_6$) alkyl or substituted groups which is optionally substituted with 0-4 substituted groups selected from deuterium, halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxy ($C_1$-$C_6$ alkyl), $NR^aR^b$, -CN, $C_1$-$C_6$ haloalkyl, $C_1$-$C_6$ haloalkoxy, -$SO_3R^a$, -$SR^a$, -$SF_5$, -C(O)$R^a$, -C(O)$OR^a$,

-OC(O)$R^a$, -OC(O)N$R^aR^b$, -N$R^a$CO$R^b$, or -CON$R^aR^b$.

Wherein, R' is $C_1$-$C_6$ alkyl, $C_3$-$C_{10}$ cycloalkyl, 4-10 membered heterocyclyl, $C_6$-$C_{10}$ aryl, 5-10 membered heteroaryl, which are independently optionally substituted with 0-3 groups selected from deuterium, halogen, $C_1$-$C_6$ alkyl, $C_2$-$C_6$ alkenyl, $C_2$-$C_6$ alkynyl, $C_3$-$C_6$ cycloalkyl, 3-6 membered saturated or unsaturated monocyclic heterocyclyl, -O$R^a$, oxo, hydroxyl($C_1$-$C_6$ alkyl), N$R^aR^b$, -CN, $C_1$-$C_6$ haloalkyl, $C_1$-$C_6$ haloalkoxy, -$SO_3R^a$, -S$R^a$, -S(O)$_2R^a$, -S(O)$R^a$, -$SF_5$, -C(O)$R^a$, -C(O)O$R^a$, -OC(O)$R^a$, -OC(O)N$R^aR^b$, -N$R^a$CO$R^b$ or -CON$R^aR^b$.

Preferably, R' is -CH$R^2R^3$ or -CD$R^2R^3$;

Wherein, $R^2$ and $R^3$ are independently hydrogen, deuterium, -O$R^a$, halogen, -CN, -$C_1$-$C_6$ alkyl, $C_1$-$C_6$ haloalkyl, $C_1$-$C_6$ haloalkoxy, hydroxy($C_1$-$C_6$ alkyl), -$C_3$-$C_{10}$ cycloalkyl or 4-10 membered heterocyclyl, $C_6$-$C_{10}$ aryl and 5-10 membered heteroaryl, the 4-10 membered heterocyclyl, $C_6$-$C_{10}$ aryl and 5-10 membered heteroaryl can be optionally substituted with 0-3 groups selected from deuterium, halogen, $C_1$-$C_6$ alkyl, $C_2$-$C_6$ alkenyl, $C_2$-$C_6$ alkynyl, $C_3$-$C_6$ cycloalkyl, -O$R^a$, oxo, hydroxyl($C_1$-$C_6$ alkyl), N$R^aR^b$, -CN, $C_1$-$C_6$ haloalkyl, $C_1$-$C_6$ haloalkoxy, -$SO_3R^a$, -S$R^a$, -S(O)$_2R^a$, -S(O)$R^a$, -$SF_5$, -C(O)$R^a$, -C(O)O$R^a$, -OC(O)$R^a$, -OC(O)N$R^aR^b$, -N$R^a$CO$R^b$ or -CON$R^aR^b$.

Wherein, $M^1$ is C$R^aR^b$, N$R^a$, O, S or Se;

Wherein, $R^L$ and $R^{L'}$ are independently hydrogen, deuterium, $C_1$-$C_6$ alkyl, or $R^L$ and $R^{L'}$ can be taken together with the atoms to which they are attached to form a $C_3$-$C_6$ carbocyclyl or heterocyclyl.

Wherein, n and o are independently 0, 1 or 2.

Wherein, $X_1$ is N or C$R^{X1}$;

Wherein, $X_2$ is N or C$R^{X2}$;

Wherein, $Y_1$ is C$R^{Y1}$R$Y^{1'}$, N$R^{Y1}$, O, S, Se;

Wherein, $Y_2$ is C$R^{Y2}$R$Y^{2'}$, N$R^{Y2}$, O, S, Se;

Wherein, $Y_3$ is C$R^{Y3}$R$Y^{3'}$, N$R^{Y3}$, O, S, Se;

Wherein, $R^{X1}$ and $R^{X2}$ are independently hydrogen, deuterium, $C_1$-$C_6$ alkyl, deuterated $C_1$-$C_6$ alkyl, $C_1$-$C_6$ haloalkyl, $C_1$-$C_6$ haloalkoxy, hydroxy($C_1$-$C_6$) alkyl, $C_2$-$C_6$ alkenyl, $C_2$-$C_6$ alkynl, -O$R^a$, -S$R^a$, -S(O)$_2R^a$, -S(O)$R^a$, -CN, -OC(O)$R^a$, -OCON$R^aR^b$, halogen, -$OSO_3R^a$, -N$R^aR^b$, -$SF_5$.

Wherein, $R^{Y1}$, $R^{Y1'}$ $R^{Y2}$, $R^{Y2'}$, $R^{Y3}$ and $R^{Y3'}$ are independtly absent, hydrogen, deuterium, $C_1$-$C_6$ alkyl, deuterated $C_1$-$C_6$ alkyl, $C_1$-$C_6$ haloalkyl, $C_1$-$C_6$ haloalkoxy, hydroxyl($C_1$-$C_6$) alkyl, $C_2$-$C_6$ alkenyl, $C_2$-$C_6$ alkynyl, $C_3$-$C_6$ cycloalkyl, -O$R^a$, -S$R^a$, -S(O)$_2R^a$, -S(O)$R^a$, -CN, -OC(O)$R^a$, -OCON$R^aR^b$, halogen, -$OSO_3R^a$, -N$R^aR^b$, -$SF_5$;

Wherein, - - - is single bond or double bond.

Wherein, $R^a$ and $R^b$ are independently hydrogen, deuterium, halogen, $C_1$-$C_6$ alkyl, $C_3$-$C_6$ cyclopropyl, $C_1$-$C_6$ haloalkyl, or $R^a$ and $R^b$ can be taken together with the atoms to which they are attached to form a 3-14 membered saturated or unsaturated cycle, which can optionally contain 0-2 heteroatoms selected from O, S and N.

In another embodiment, the invention provides a method for treating tumors or cancers, comprising administering to a subject in need thereof a compound represented by Formula (II), or a pharmaceutically acceptable salt, ester, prodrug, stereoisomer, or isotopic derivative thereof,

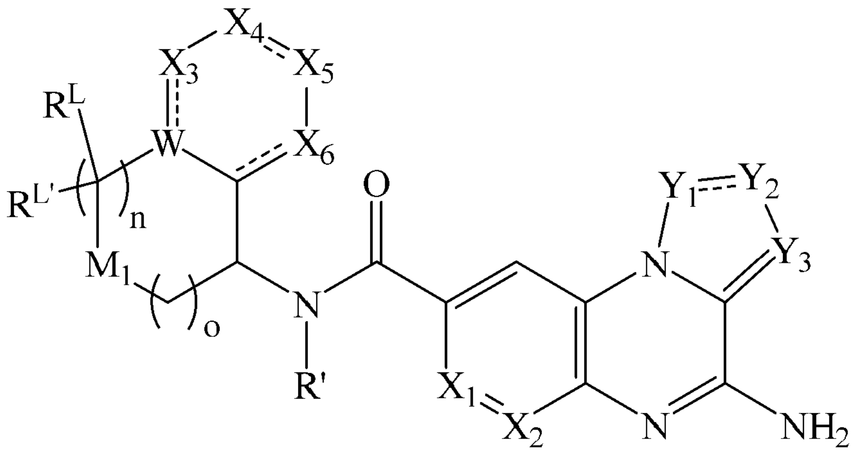

Formula (II)

Wherein, W is C;

Wherein, $X_3$ is N or C$R^{X3}$; $X_4$ is N or C$R^{X4}$; $X_5$ is N or C$R^{X5}$; $X_6$ is N or C$R^{X6}$;

Wherein, when $X_3$ is $CR^{X3}$, $R^{X3}$ is hydrogen, deuterium, $C_1$-$C_6$ alkyl, $C_1$-$C_6$ haloalkyl, halogen, $-OR^a$, $-SR^a$, -P(O)$R^aR^b$, -CN, $-S(O)_2R^a$, $-S(O)R^a$, $-SF_5$, $-NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, $-OR^a$, oxo, hydroxyl($C_1$-$C_6$ alkyl), $NR^aR^b$, -CN, $C_1$-$C_6$ haloalkyl, $C_1$-$C_6$ haloalkoxy, $-SO_3R^a$, $-SR^a$, $-SF_5$, $-C(O)R^a$, $-C(O)OR^a$, $-OC(O)R^a$, $-OC(O)NR^aR^b$, $-NR^aCOR^b$ or $-CONR^aR^b$;

Wherein, when $X_4$ is $CR^{X4}$, $R^{X4}$ is hydrogen, deuterium, $C_1$-$C_6$ alkyl, $C_1$-$C_6$ haloalkyl, halogen, $-OR^a$, $-SR^a$, -P(O)$R^aR^b$, -CN, $-S(O)_2R^a$, $-S(O)R^a$, $-SF_5$, $-NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, $-OR^a$, oxo, hydroxyl($C_1$-$C_6$ alkyl), $NR^aR^b$, -CN, $C_1$-$C_6$ haloalkyl, $C_1$-$C_6$ haloalkoxy, $-SO_3R^a$, $-SR^a$, $-SF_5$, $-C(O)R^a$, $-C(O)OR^a$, $-OC(O)R^a$, $-OC(O)NR^aR^b$, $-NR^aCOR^b$ or $-CONR^aR^b$;

Wherein, when $X_5$ is $CR^{X5}$, $R^{X5}$ is hydrogen, deuterium, $C_1$-$C_6$ alkyl, $C_1$-C6 haloalkyl, halogen, $-OR^a$, $-SR^a$, -P(O)$R^aR^b$, -CN, $-S(O)_2R^a$, $-S(O)R^a$, $-SF_5$, $-NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, $-OR^a$, oxo, hydroxyl($C_1$-$C_6$ alkyl), $NR^aR^b$, -CN, $C_1$-$C_6$ haloalkyl, $C_1$-$C_6$ haloalkoxy, $-SO_3R^a$, $-SR^a$, $-SF_5$, $-C(O)R^a$, $-C(O)OR^a$, $-OC(O)R^a$, $-OC(O)NR^aR^b$, $-NR^aCOR^b$ or $-CONR^aR^b$;

Wherein, when $X_6$ is $CR^{X6}$, $R^{X6}$ is hydrogen, deuterium, $C_1$-$C_6$ alkyl, $C_1$-C6 haloalkyl, halogen, $-OR^a$, $-SR^a$, -P(O)$R^aR^b$, -CN, $-S(O)_2R^a$, $-S(O)R^a$, $-SF_5$, $-NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, $-OR^a$, oxo, hydroxyl($C_1$-$C_6$ alkyl), $NR^aR^b$, -CN, $C_1$-$C_6$ haloalkyl, $C_1$-$C_6$ haloalkoxy, $-SO_3R^a$, $-SR^a$, $-SF_5$, $-C(O)R^a$, $-C(O)OR^a$, $-OC(O)R^a$, $-OC(O)NR^aR^b$, $-NR^aCOR^b$ or $-CONR^aR^b$;

Wherein, R' is $C_1$-$C_6$ alkyl, $C_3$-$C_{10}$ cycloalkyl, 4-10 membered heterocyclyl, $C_6$-$C_{10}$ aryl, 5-10 membered heteroaryl, which are independently optionally substituted with 0-3 groups selected from deuterium, halogen, $C_1$-$C_6$ alkyl, $C_2$-$C_6$ alkenyl, $C_2$-$C_6$ alkynyl, $C_3$-$C_6$ cycloalkyl, 3-6 membered saturated or unsaturated monocyclic heterocyclyl, $-OR^a$, oxo, hydroxyl($C_1$-$C_6$ alkyl), $NR^aR^b$, -CN, $C_1$-$C_6$ haloalkyl, $C_1$-$C_6$ haloalkoxy, $-SO_3R^a$, $-SR^a$, $-S(O)_2R^a$, $-S(O)R^a$, $-SF_5$, $-C(O)R^a$, $-C(O)OR^a$, $-OC(O)R^a$, $-OC(O)NR^aR^b$, $-NR^aCOR^b$ or $-CONR^aR^b$.

Preferably, R' is $-CHR^2R^3$ or $-CDR^2R^3$;

Wherein, $R^2$ and $R^3$ are independently hydrogen, deuterium, $-OR^a$, halogen, -CN, $-C_1$-$C_6$ alkyl, $C_1$-$C_6$ haloalkyl, $C_1$-$C_6$ haloalkoxy, hydroxy($C_1$-$C_6$ alkyl), $-C_3$-$C_{10}$ cycloalkyl or 4-10 membered heterocyclyl, $C_6$-$C_{10}$ aryl and 5-10 membered heteroaryl, the 4-10 membered heterocyclyl, $C_6$-$C_{10}$ aryl and 5-10 membered heteroaryl can be optionally substituted with 0-3 groups selected from deuterium, halogen, $C_1$-$C_6$ alkyl, $C_2$-$C_6$ alkenyl, $C_2$-$C_6$ alkynyl, $C_3$-$C_6$ cycloalkyl, $-OR^a$, oxo, hydroxyl($C_1$-$C_6$ alkyl), $NR^aR^b$, -CN, $C_1$-$C_6$ haloalkyl, $C_1$-$C_6$ haloalkoxy, $-SO_3R^a$, $-SR^a$, $-S(O)_2R^a$, $-S(O)R^a$, $-SF_5$, $-C(O)R^a$, $-C(O)OR^a$, $-OC(O)R^a$, $-OC(O)NR^aR^b$, $-NR^aCOR^b$ or $-CONR^aR^b$.

Wherein, $M^1$ is $CR^aR^b$, $NR^a$, O, S or Se;

Wherein, $R^L$ and $R^{L'}$ are independently hydrogen, deuterium, $C_1$-$C_6$ alkyl, or $R^L$ and $R^{L'}$ can be taken together with the atoms to which they are attached to form a $C_3$-$C_6$ carbocyclyl or heterocyclyl.

Wherein, n and o are independently 0, 1 or 2;

Wherein, $X_1$ is N or $CR^{X1}$;

Wherein, $X_2$ is N or $CR^{X2}$;

Wherein, $Y_1$ is $CR^{Y1}RY^{1'}$, $NR^{Y1}$, O, S, Se;

Wherein, $Y_2$ is $CR^{Y2}R^{Y2'}$, $NR^{Y2}$, O, S, Se;

Wherein, $Y_3$ is $CR^{Y3}R^{Y3'}$, $NR^{Y3}$, O, S, Se;

Wherein, $R^{X1}$ and $R^{X2}$ are independently hydrogen, deuterium, $C_1$-$C_6$ alkyl, deuterated $C_1$-$C_6$ alkyl, $C_1$-$C_6$ haloalkyl, $C_1$-$C_6$ haloalkoxy, hydroxy($C_1$-$C_6$) alkyl, $C_2$-$C_6$ alkenyl, $C_2$-$C_6$ alkynl, $-OR^a$, $-SR^a$, $-S(O)_2R^a$, $-S(O)R^a$, -CN, $-OC(O)R^a$, $-OCONR^aR^b$, halogen, $-OSO_3R^a$, $-NR^aR^b$, $-SF_5$.

Wherein, $R^{Y1}$, $R^{Y1'}$, $R^{Y2}$, $R^{Y2'}$, $R^{Y3}$ and $R^{Y3'}$ are independtly absent, hydrogen, deuterium, $C_1$-$C_6$ alkyl, deuterated $C_1$-$C_6$ alkyl, $C_1$-$C_6$ haloalkyl, $C_1$-$C_6$ haloalkoxy, hydroxy($C_1$-$C_6$) alkyl, $C_2$-$C_6$ alkenyl, $C_2$-$C_6$ alkynyl, $C_3$-$C_6$ cycloalkyl, $-OR^a$, $-SR^a$, $-S(O)_2R^a$, $-S(O)R^a$, -CN, $-OC(O)R^a$, $-OCONR^aR^b$, halogen, $-OSO_3R^a$, $-NR^aR^b$, $-SF_5$;

Wherein, $\overline{- - -}$ is single bond or double bond.

Wherein, $R^a$ and $R^b$ are independently hydrogen, deuterium, halogen, $C_1$-$C_6$ alkyl, $C_3$-$C_6$ cyclopropyl, $C_1$-$C_6$ haloalkyl, or $R^a$ and $R^b$ can be taken together with the atoms to which they are attached to form a 3-14 membered saturated or unsaturated cycle, which can optionally contain 0-2 heteroatoms selected from O, S and N.

In a preferred embodiment of the invention, wherein, $\overline{---}$ is double bond.

In a preferred embodiment of the invention, wherein, $X_1$ is $CR^{X1}$ or N, wherein, $R^{X1}$ is hydrogen, deuterium, halogen, -CN, $C_1$-$C_6$ alkyl, deuterated $C_1$-$C_6$ alkyl, $C_1$-$C_6$ alkoxy, $C_1$-$C_6$ haloalkyl.

In a preferred embodiment of the invention, wherein, $X_1$ is CH, CF or N.

In a preferred embodiment of the invention, wherein, $X_2$ is CH or CD.

In a preferred embodiment of the invention, wherein, $X_2$ is CH.

In a preferred embodiment of the invention, wherein, $X_3$ is CH, CD or N.

In a preferred embodiment of the invention, wherein, $X_3$ is CH.

In a preferred embodiment of the invention, wherein, $X_4$ is $CR^{X4}$ or N, wherein, $R^{X4}$ is hydrogen, deuterium, $C_1$-$C_6$ alkyl, deuterated $C_1$-$C_6$ alkyl, $C_1$-$C_6$ alkoxy, $C_1$-$C_6$ alkylthio, $C_1$-$C_6$ haloalkyl, $C_1$-$C_6$ haloalkoxy, $C_1$-$C_6$ haloalkylthio, halogen, $SF_5$, $-S(O)_2R^a$, -P(O) $R^aR^b$, CN or $C_3$-$C_{10}$ cycloalkyl, $C_6$-$C_{10}$ cycloalkenyl, 4-10 membered heterocyclyl, $C_6$-$C_{10}$ aryl and 5-10 membered heteroaryl, wherein, each ring is optionally substituted with 0-4 groups independently selected from deuterium, halogen, $C_1$-$C_6$ alkyl, $C_2$-$C_6$ alkenyl, $C_2$-$C_6$ alkynyl, $C_3$-$C_6$ cycloalkyl, $-OR^a$, oxo, hydroxy($C_1$-$C_6$) alkyl, $NR^aR^b$, -CN, $C_1$-$C_6$ haloalkyl, $C_1$-$C_6$ haloalkoxy, $-S(O)_2R^a$, $-SR^a$, $-SF_5$, $-C(O)R^a$, $-C(O)OR^a$, $-OC(O)R^a$, $-OC(O)NR^aR^b$, $-NR^aCOR^b$ or $-CONR^aR^b$.

In a preferred embodiment of the invention, wherein, $X_4$ is $CR^{X4}$; wherein, $R^{X4}$ is $C_1$-$C_6$ alkoxy, $C_1$-$C_6$ alkylthio, $C_1$-$C_6$ haloalkoxy, $C_1$-$C_6$ haloalkylthio, $C_1$-$C_6$ haloalkyl, $-SF_5$.

In a preferred embodiment of the invention, wherein, $X_4$ is $CR^{X4}$; wherein, $R^{X4}$ is $C_1$-$C_6$ haloalkoxy, $C_1$-$C_6$ haloalkylthio, $C_1$-$C_6$ haloalkyl, $-SF_5$.

In a preferred embodiment of the invention, wherein, $X_5$ is $CR^{X5}$ or N, wherein, $R^{X5}$ is hydrogen, deuterium, $C_1$-$C_6$ alkyl, $C_1$-$C_6$ alkoxy, $C_1$-$C_6$ haloalkyl, $C_1$-$C_6$ haloalkoxy, halogen, $-SF_5$ or CN.

In a preferred embodiment of the invention, wherein, $X_5$ is CH.

In a preferred embodiment of the invention, wherein, $X_6$ is CH, CD or N.

In a preferred embodiment of the invention, wherein, $X_6$ is CH.

In a preferred embodiment of the invention, wherein, the chemical bond between $Y_1$ and $Y_2$ is double bond.

In a preferred embodiment of the invention, wherein, $Y_1$ is CH, CD or $CCH_3$.

In a preferred embodiment of the invention, wherein, $Y_2$ is N.

In a preferred embodiment of the invention, wherein, $Y_3$ is CH, CD, $CCH_3$ or $CCH_2OH$.

In a preferred embodiment of the invention, wherein, R' is $-CHR^2R^3$ or $-CDR^2R^3$, wherein, $R^2$ and $R^3$ are independently hydrogen, deuterium, $C_1$-$C_6$ alkyl or $C_3$-$C_{10}$ cycloalkyl, 4-10 membered heterocyclyl, $C_6$-$C_{10}$ aryl, 5-10 membered heteroaryl, wherein, each ring is independently optionally substituted with 0-3 groups selected from halogen, $C_1$-$C_6$ alkyl, $C_2$-$C_6$ alkenyl, $C_2$-$C_6$ alkynyl, $C_3$-$C_6$ cycloalkyl, $-OR^a$, oxo, hydroxy($C_1$-$C_6$) alkyl, $NR^aR^b$, -CN, $C_1$-$C_6$ haloalkyl, $C_1$-$C_6$ haloalkoxy, $-SO_3R^a$, $-SR^a$, $-S(O)_2R^a$, $-S(O)R^a$, $-SF_5$, $-C(O)R^a$, $-C(O)OR^a$, $-OC(O)R^a$, $-OC(O)NR^aR^b$, $-NR^aCOR^b$ or $-CONR^aR^b$.

In a preferred embodiment of the invention, wherein, R' is $-CHR^2R^3$ or $-CDR^2R^3$, wherein, $R^2$ is hydrogen, deuterium, $C_1$-$C_6$ alkyl; $R^3$ is hydrogen, deuterium, $C_1$-$C_6$ alkyl or $C_3$-$C_{10}$ cycloalkyl, 4-10 membered heterocyclyl, $C_6$-$C_{10}$ aryl, 5-10 membered heteroaryl, wherein, each ring is independently optionally substituted with 0-3 groups selected from halogen, $C_1$-$C_6$ alkyl, $C_2$-$C_6$ alkenyl, $C_2$-$C_6$ alkynyl, $C_3$-$C_6$ cycloalkyl, $-OR^a$, oxo, hydroxy($C_1$-$C_6$) alkyl, $NR^aR^b$, -CN, $C_1$-$C_6$ haloalkyl, $C_1$-$C_6$ haloalkoxy, $-SO_3R^a$, $-SR^a$, $-S(O)_2R^a$, $-S(O)R^a$, $-SF_5$, $-C(O)R^a$, $-C(O)OR^a$, $-OC(O)R^a$, $-OC(O)NR^aR^b$, $-NR^aCOR^b$ or $-CONR^aR^b$.

In a preferred embodiment of the invention, wherein, R' is $C_3$-$C_{10}$ cycloalkyl which is optionally substituted with 0-3 groups independently selected from deuterium, halogen, $C_1$-$C_6$ alkyl, hydroxy($C_1$-$C_6$) alkyl, $-OR^a$, -CN, $NR^aR^b$, $C_1$-$C_6$ haloalkyl, $C_1$-$C_6$ haloalkoxy.

In a preferred embodiment of the invention, wherein, R' is $C_1$-$C_6$ alkyl (preferably methyl, ethyl) or deuterated $C_1$-$C_6$ alkyl (preferably deuterated methyl, deuterated ethyl) or $C_3$-$C_6$ cycloalkyl (preferably cyclopropyl).

In a preferred embodiment of the invention, wherein, $M_1$ is O or S.

In a preferred embodiment of the invention, wherein, o is 1 or 2.

In a preferred embodiment of the invention, wherein, o is 1.

In a preferred embodiment of the invention, wherein, n is 0 or 1.

In a preferred embodiment of the invention, wherein, n is 0.

In a preferred embodiment of the invention, wherein, n is 1.

In a preferred embodiment of the invention, wherein, $R^L$ and $R^{L'}$ are independently hydrogen or $C_1$-$C_6$ alkyl. Specifically, in an embodiment, the present invention provides a method for treating tumors or cancers, comprising administering to a subject in need thereof following compounds, or a pharmaceutically acceptable salt, ester, prodrug,

stereoisomer, or isotopic derivative thereof,

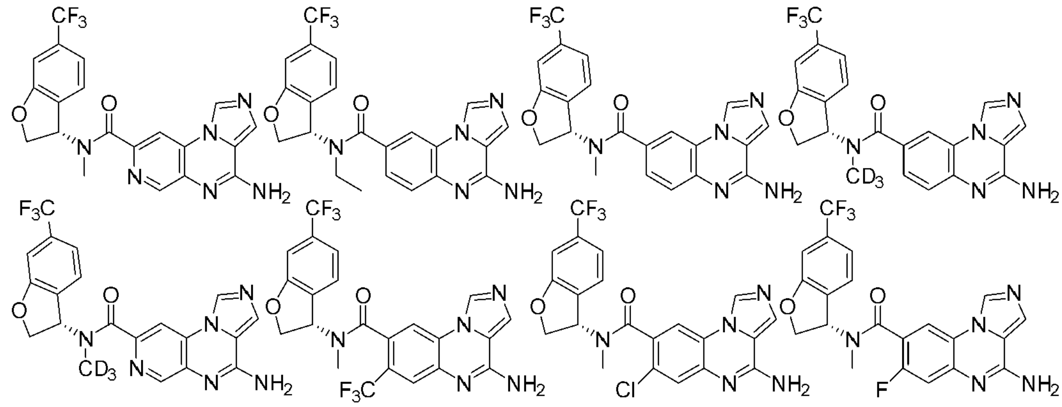

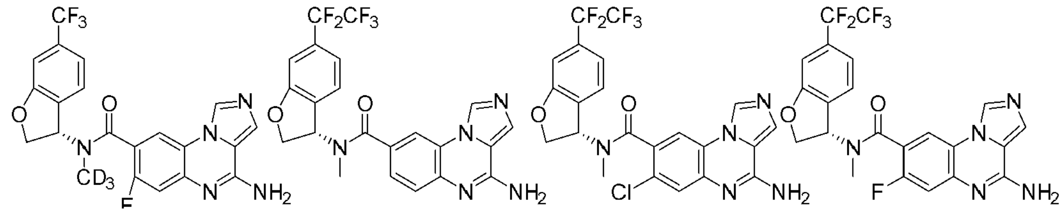

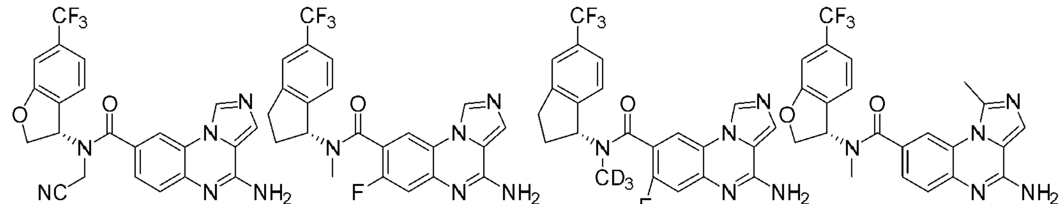

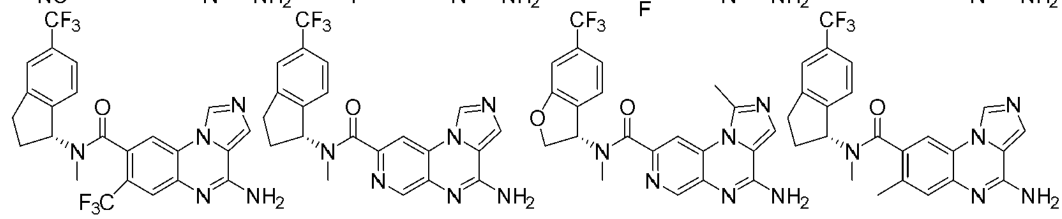

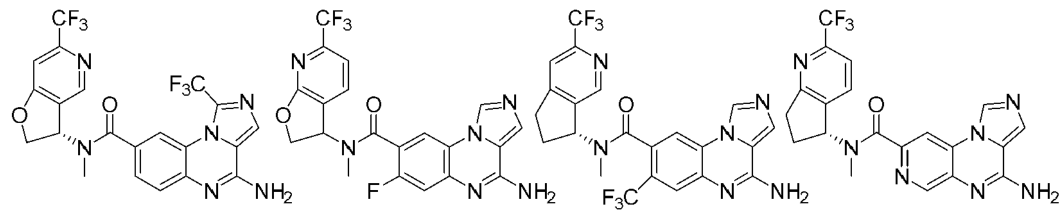

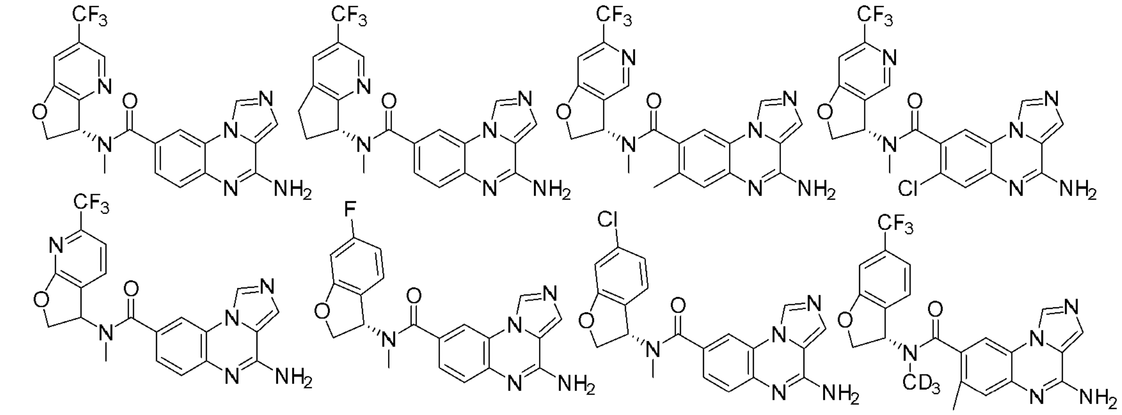

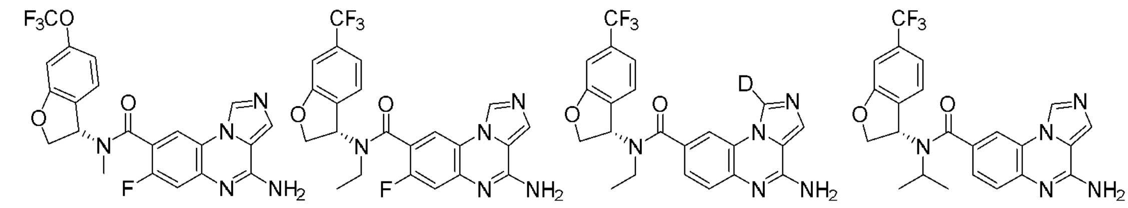

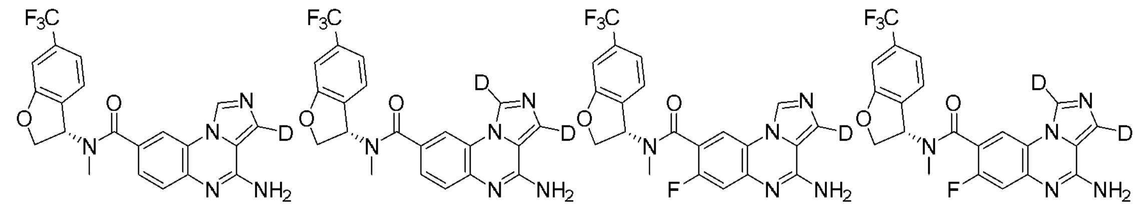

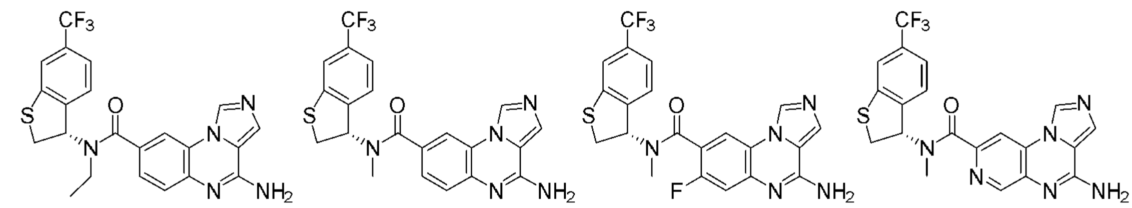

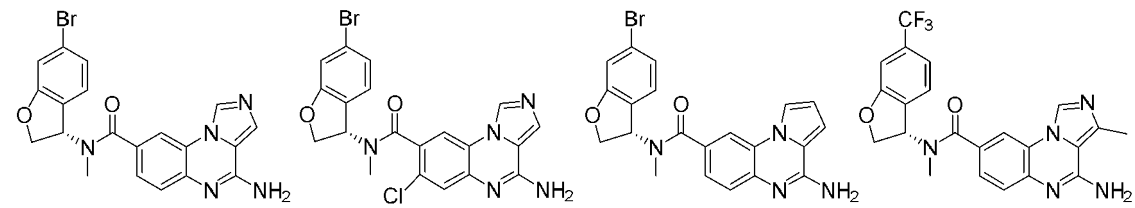

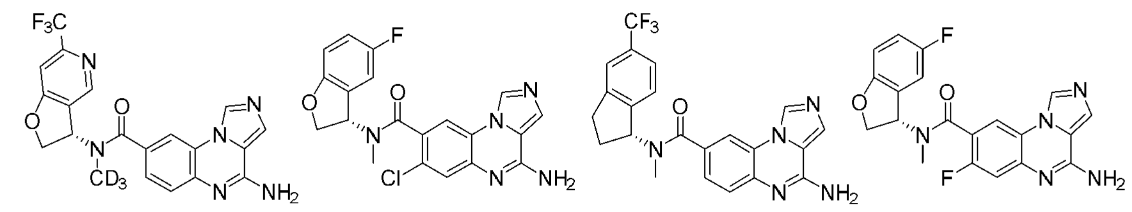

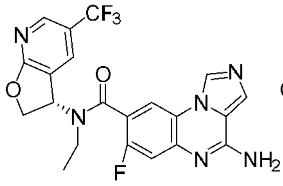
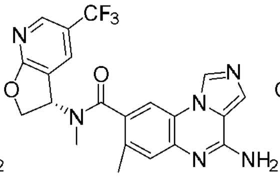
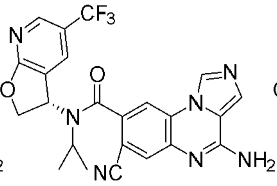
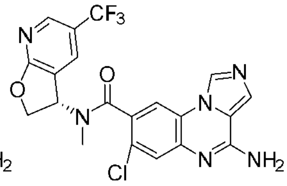

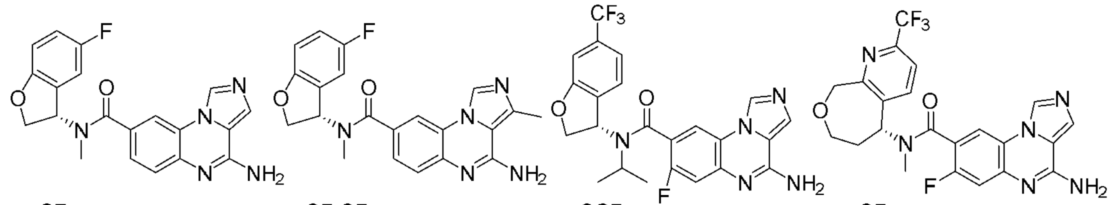

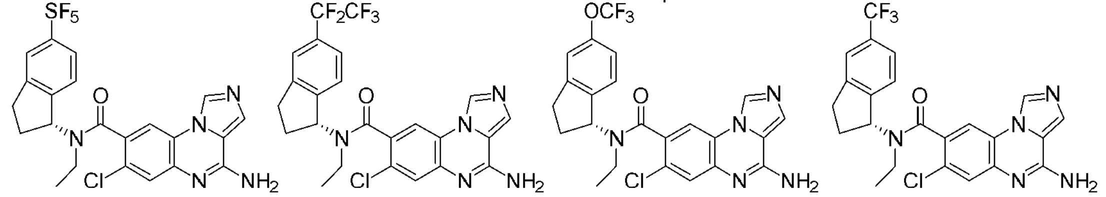

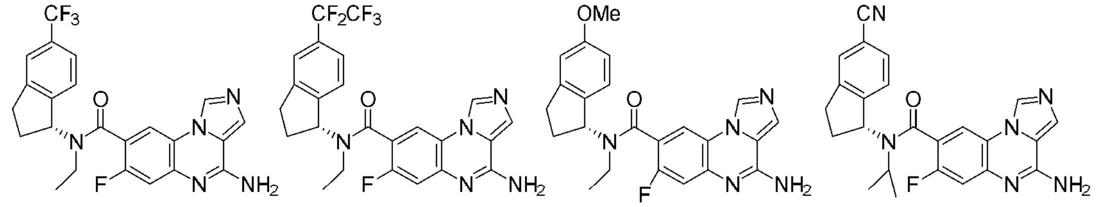

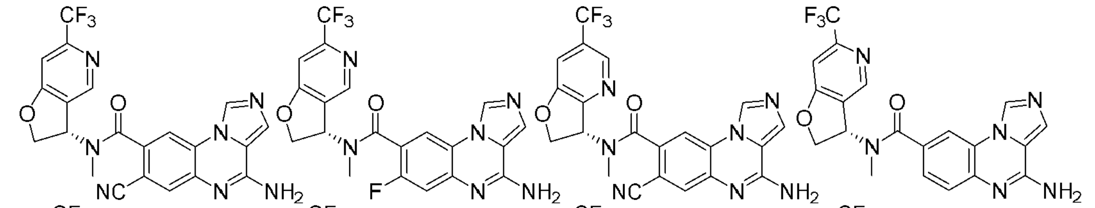

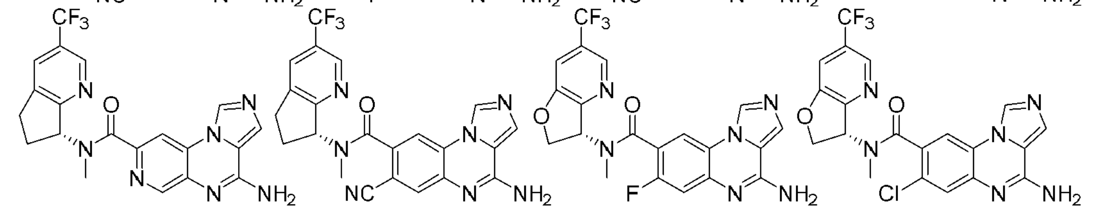

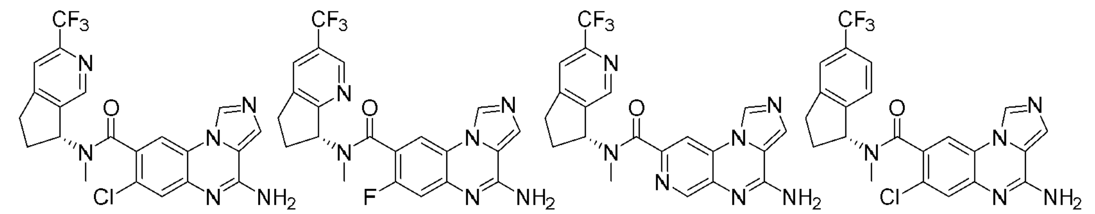

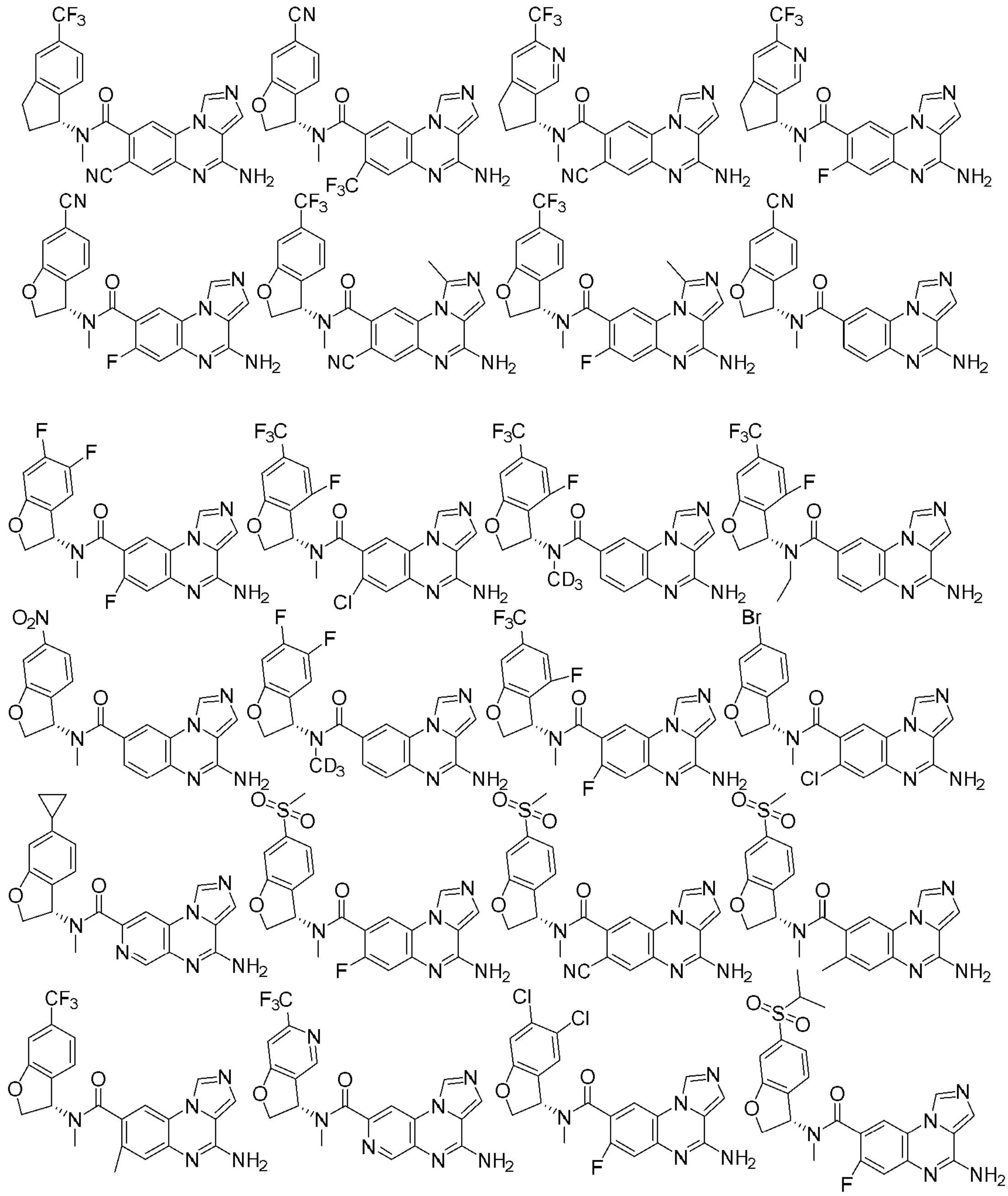

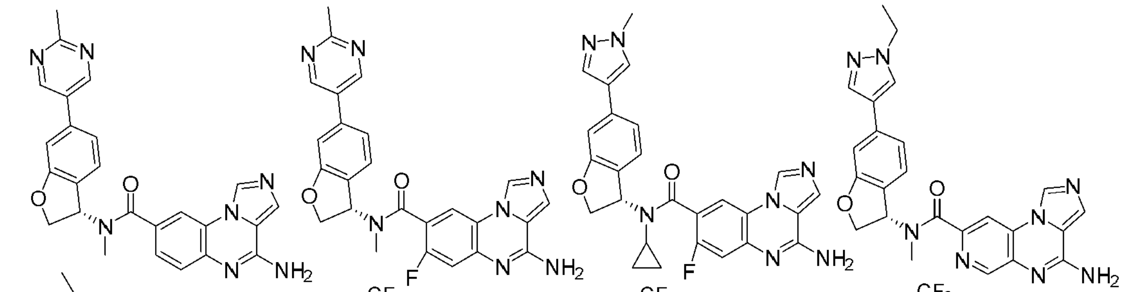

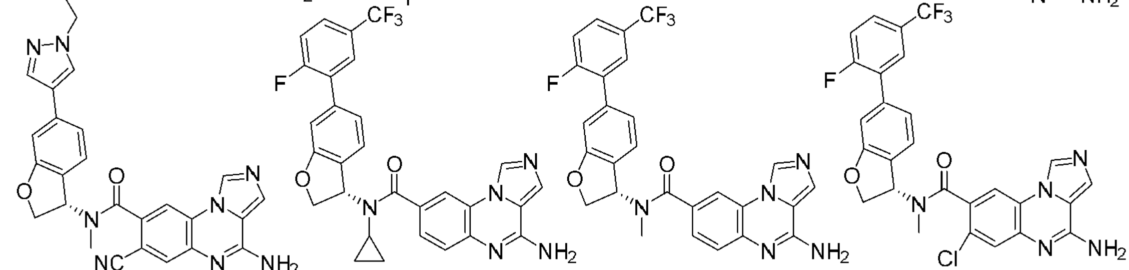

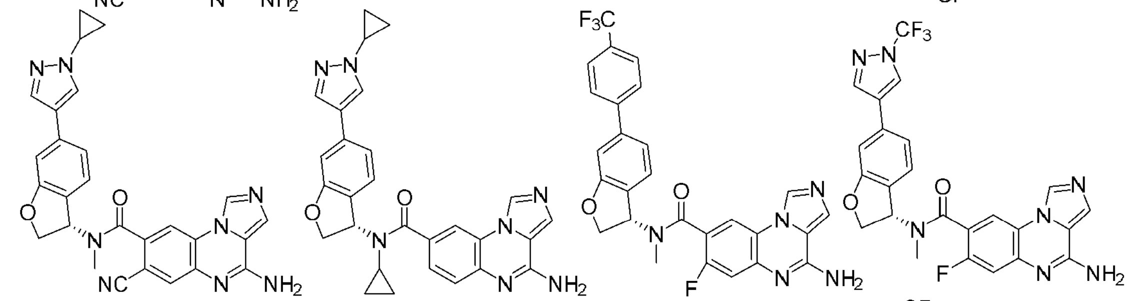

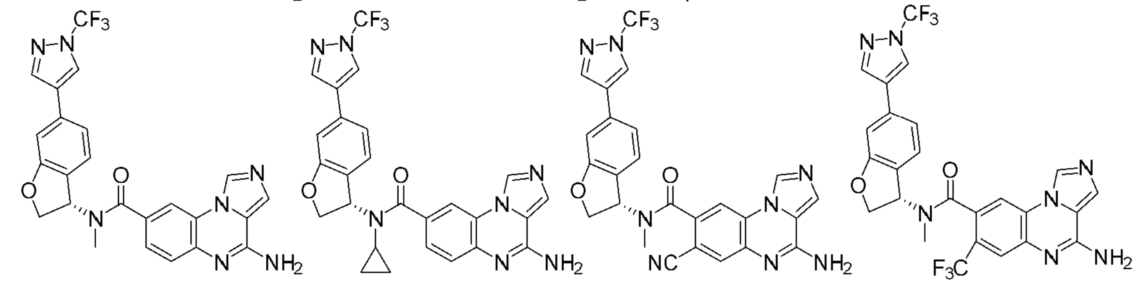

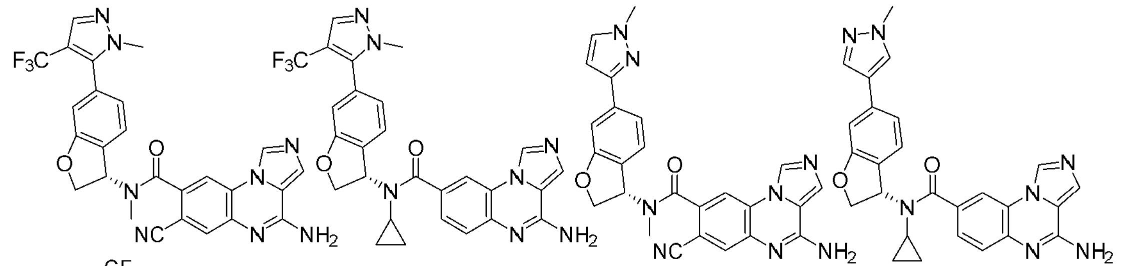

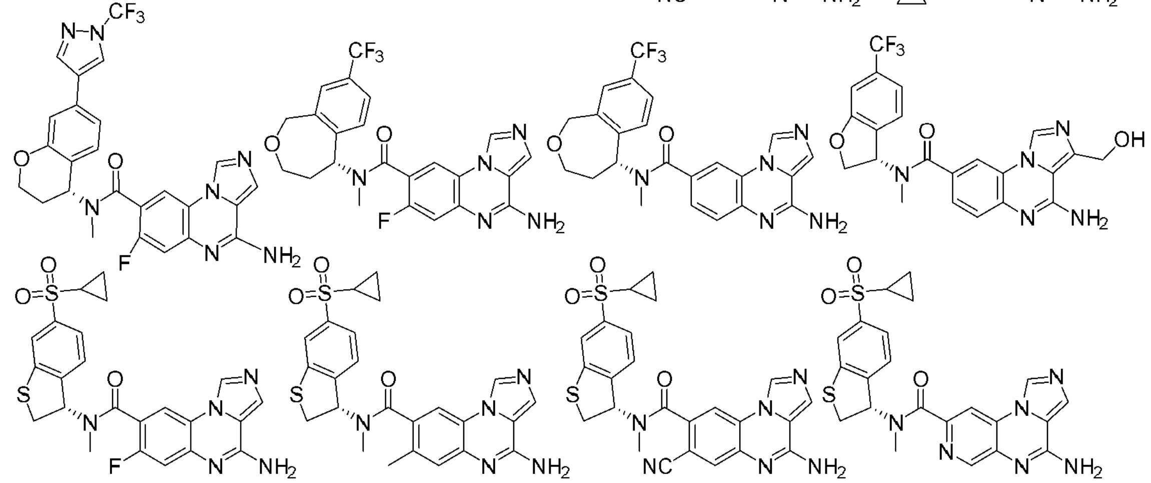

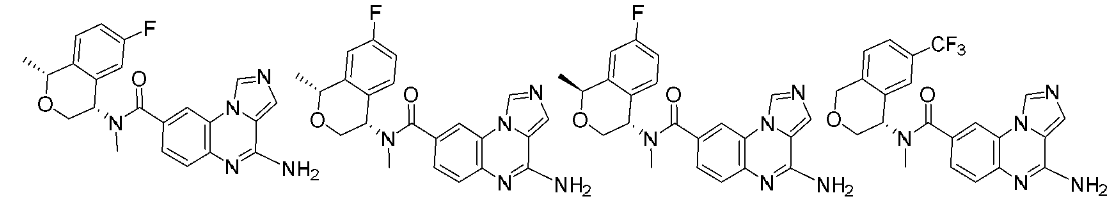

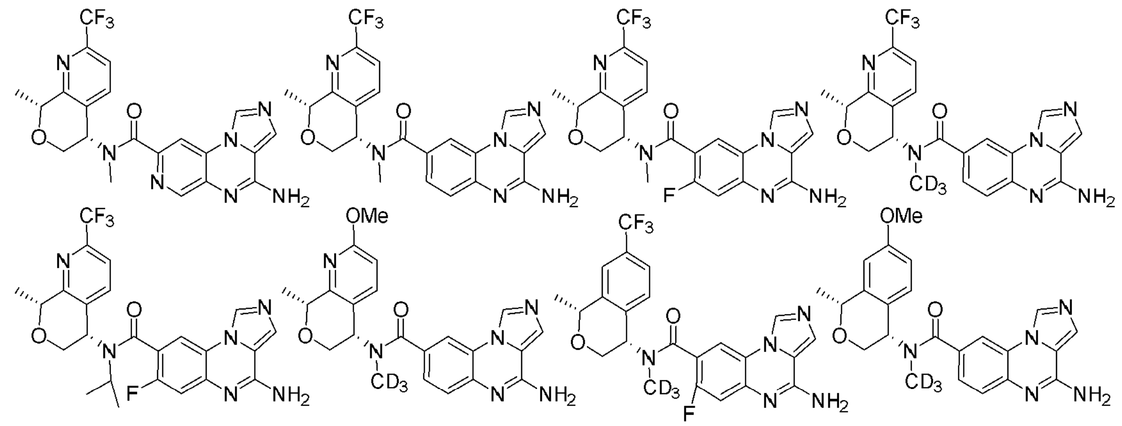

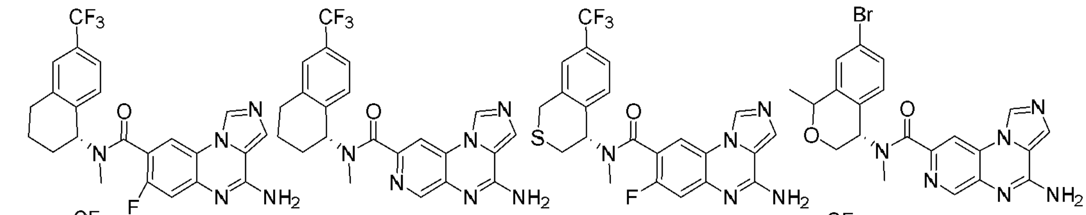

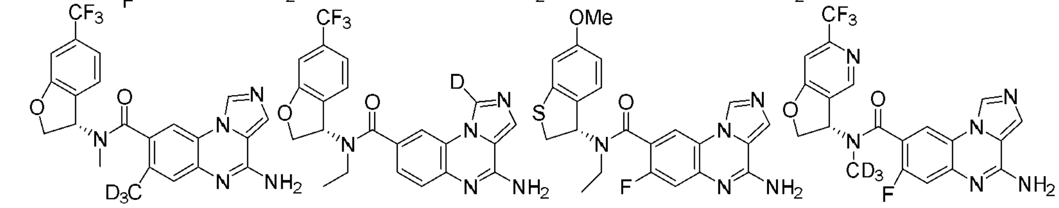

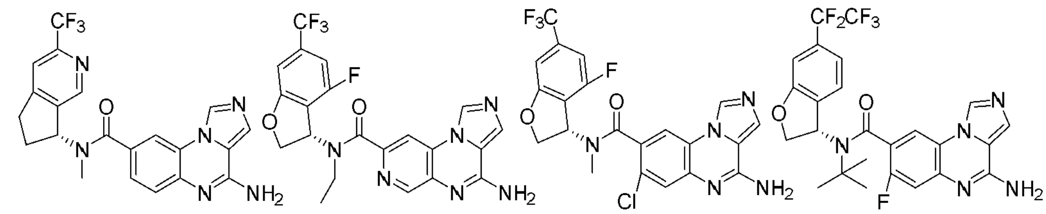

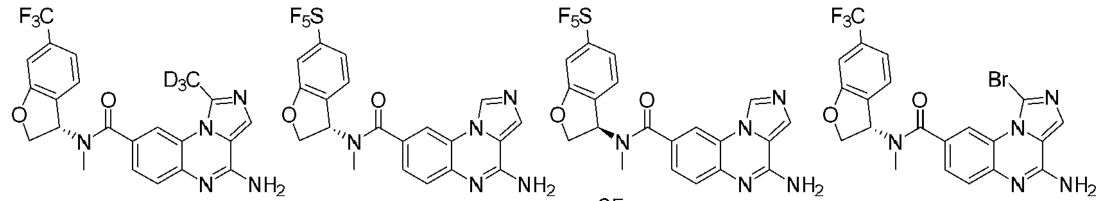

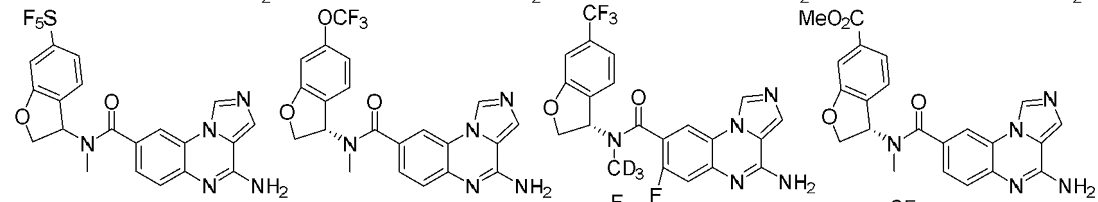

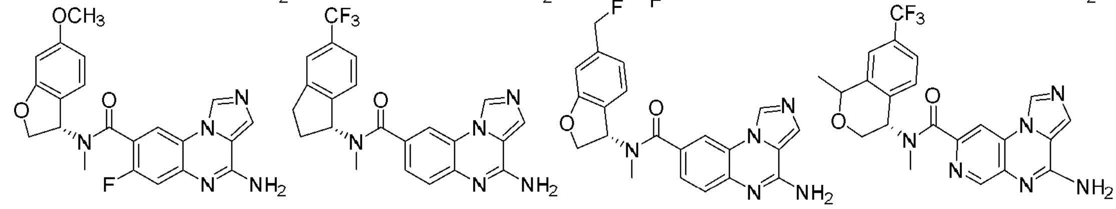

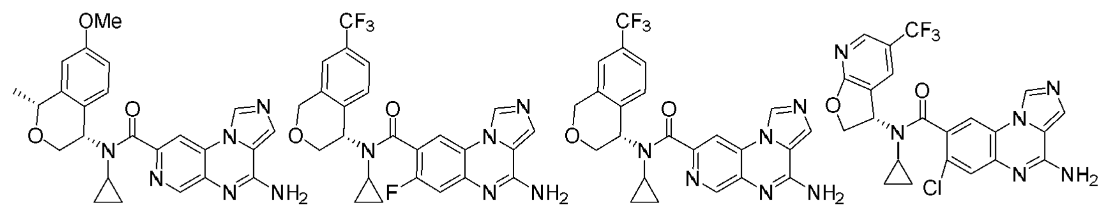

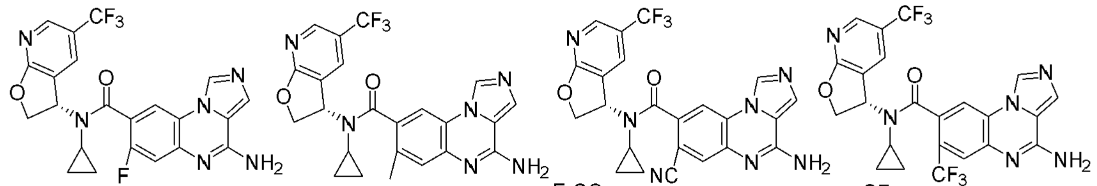

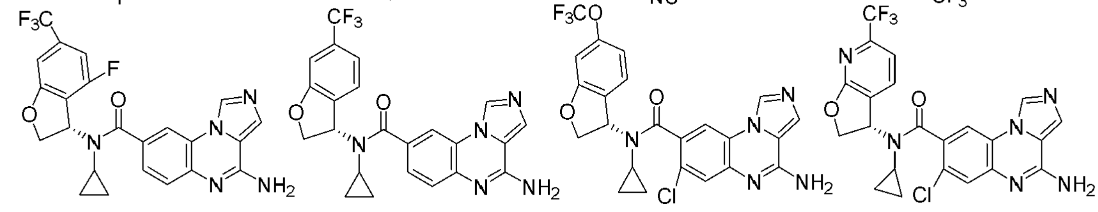

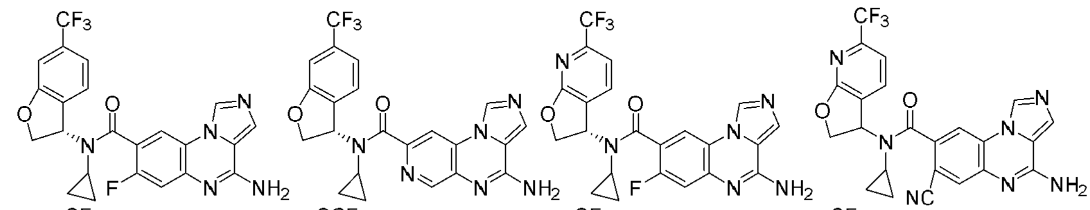

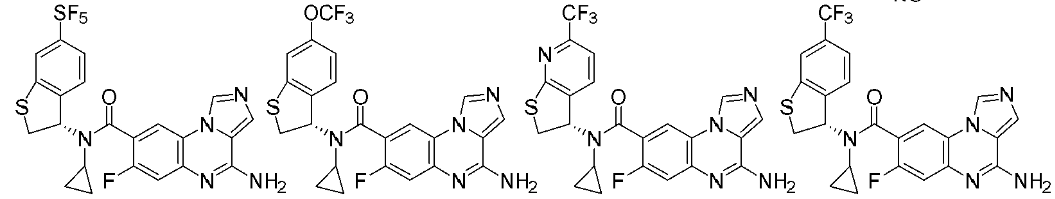

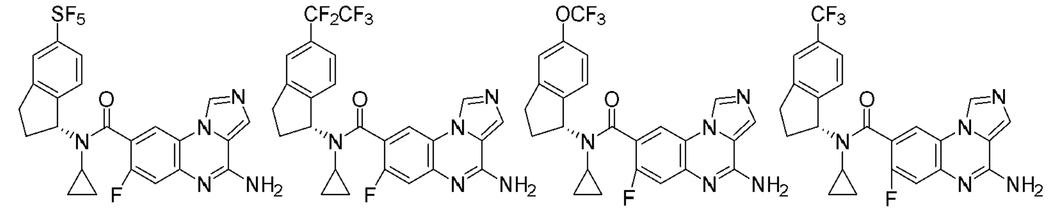

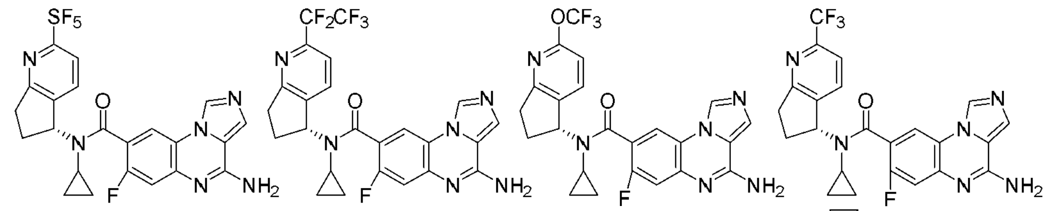

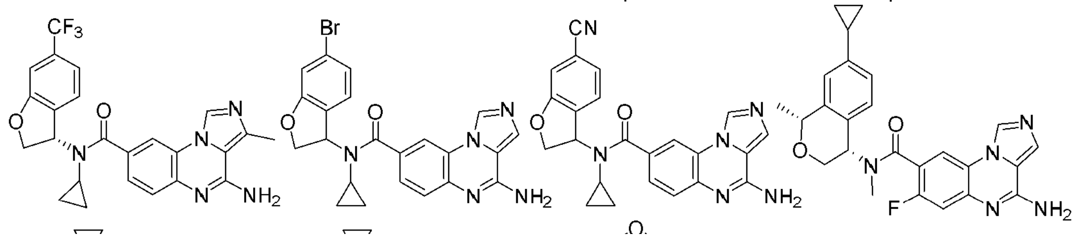

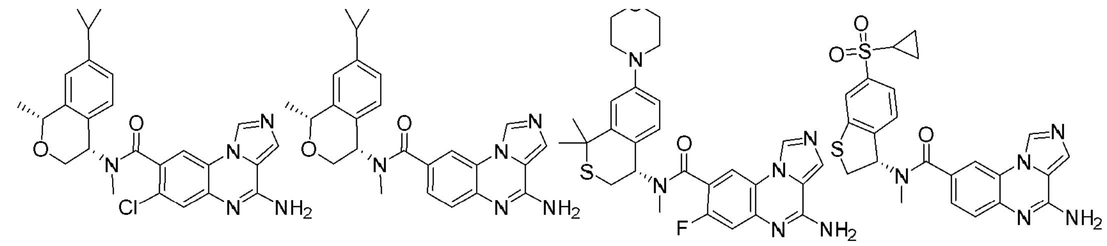

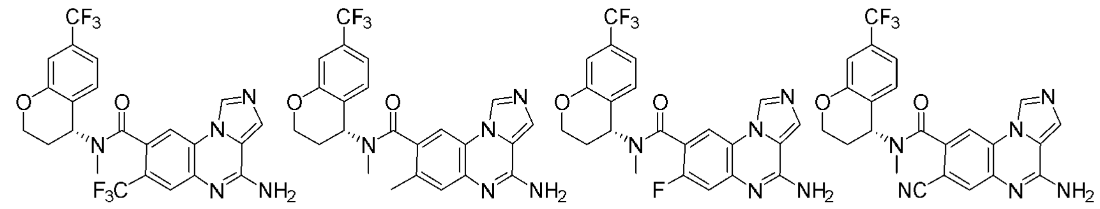

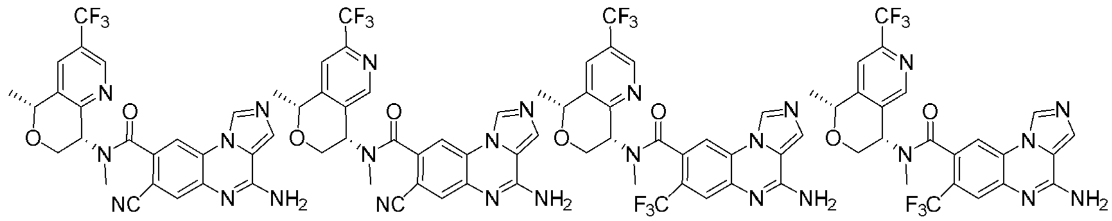

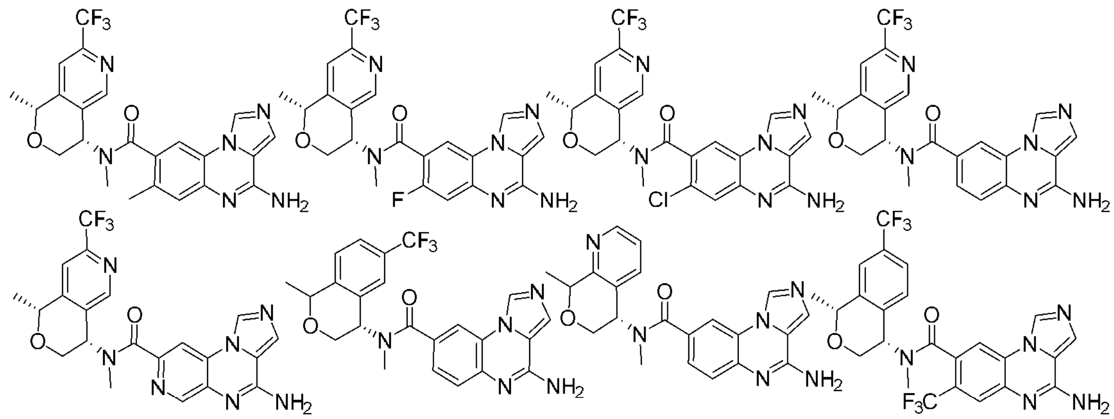

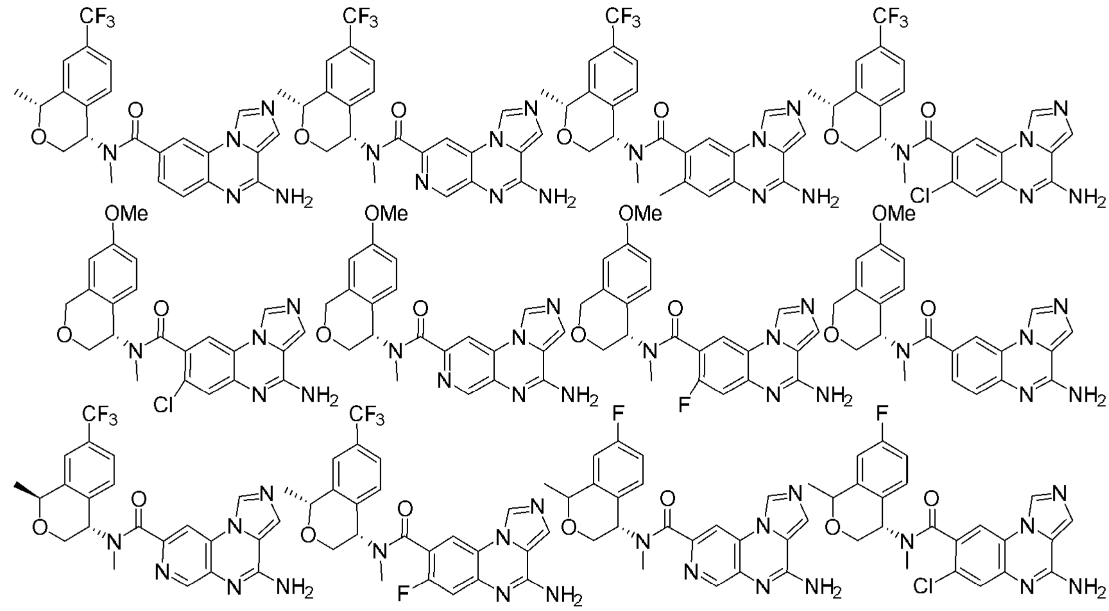

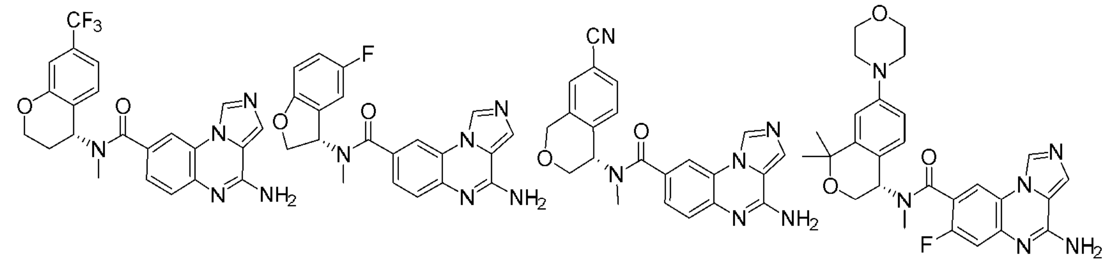

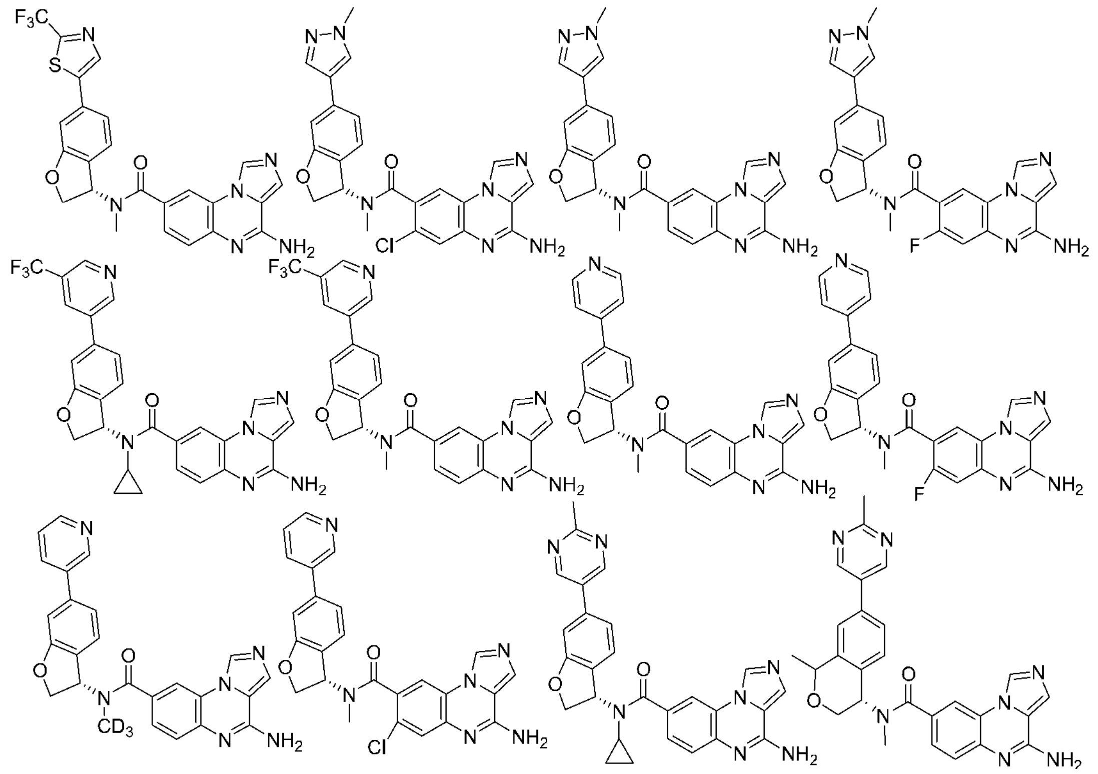

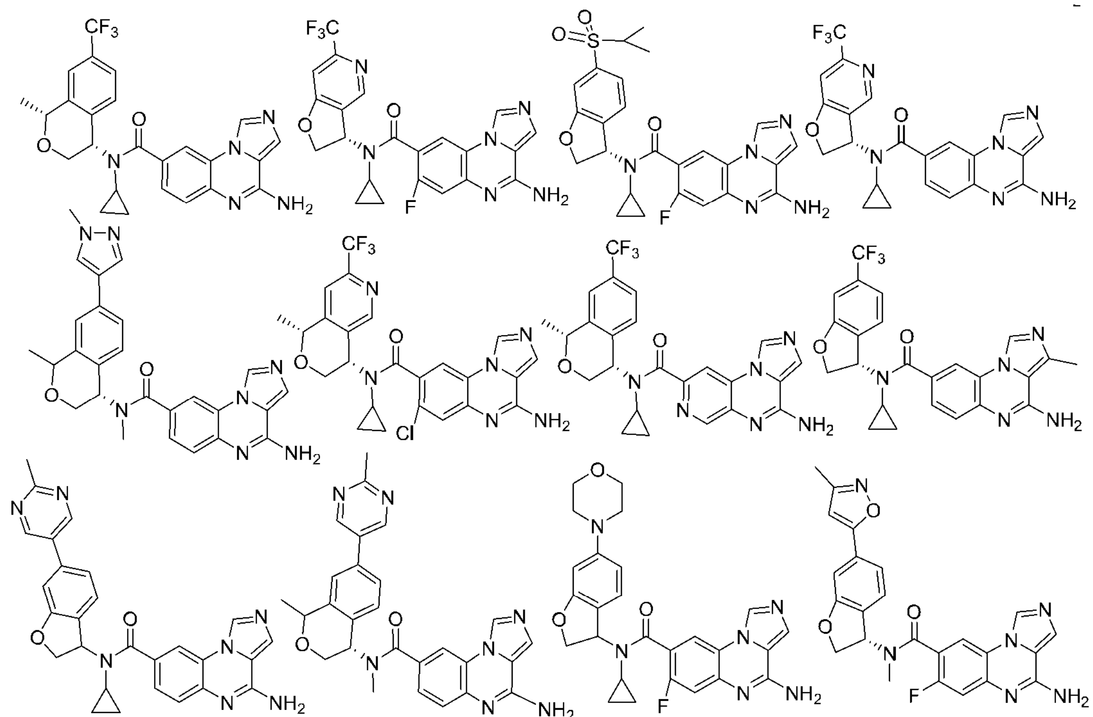

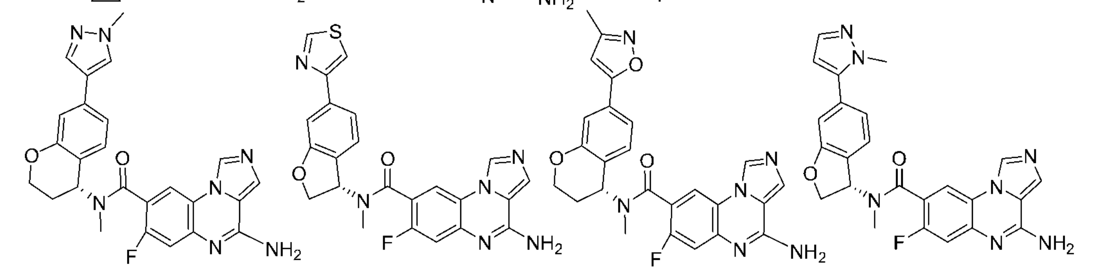

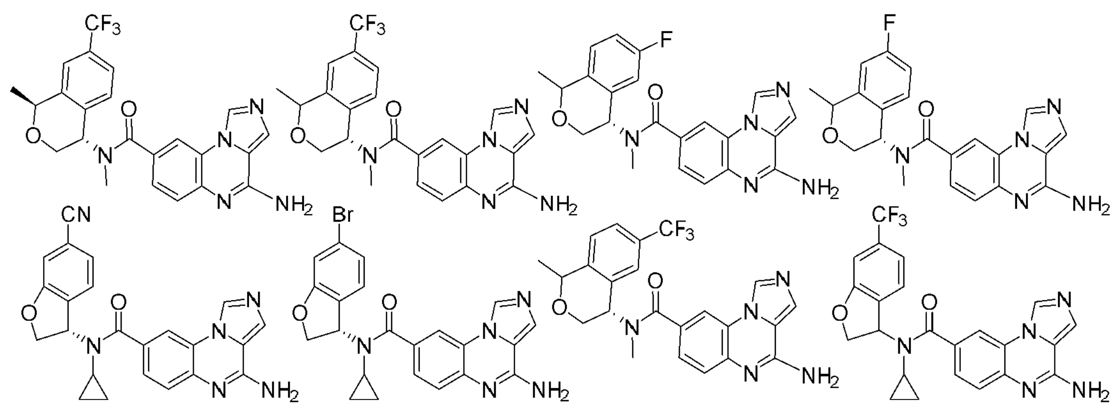

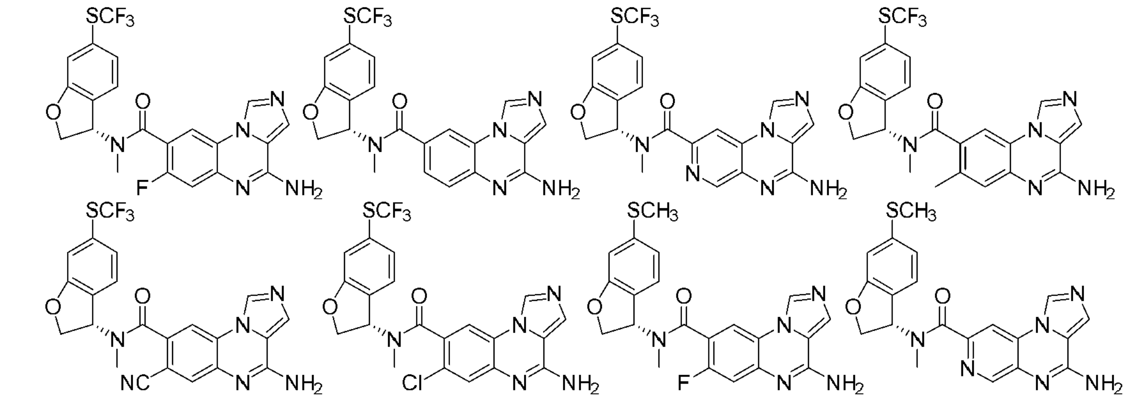

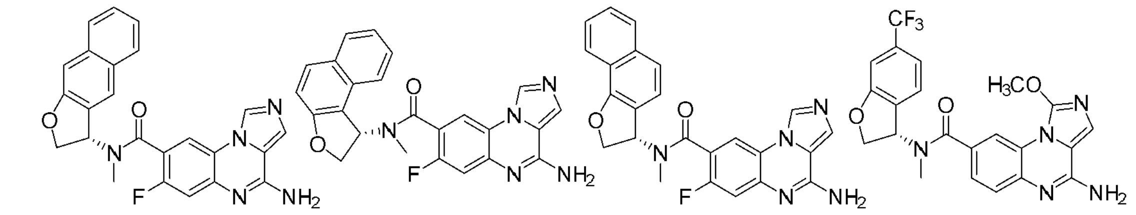

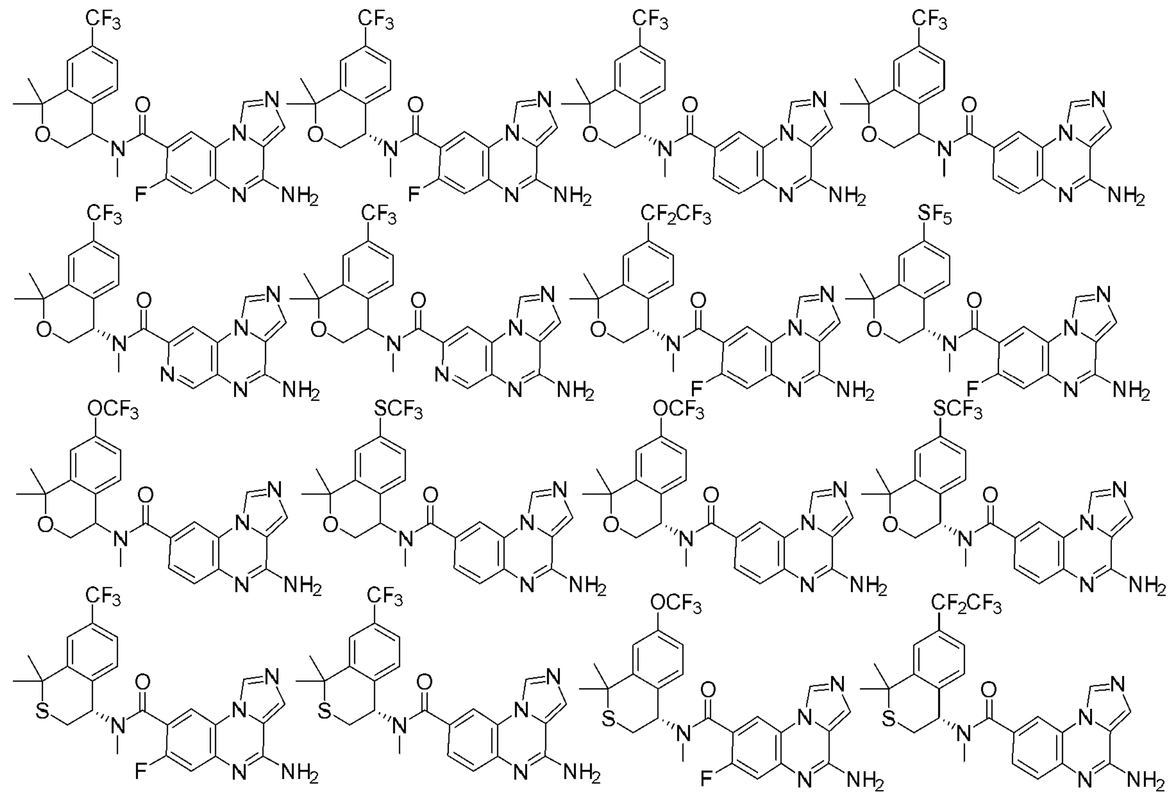

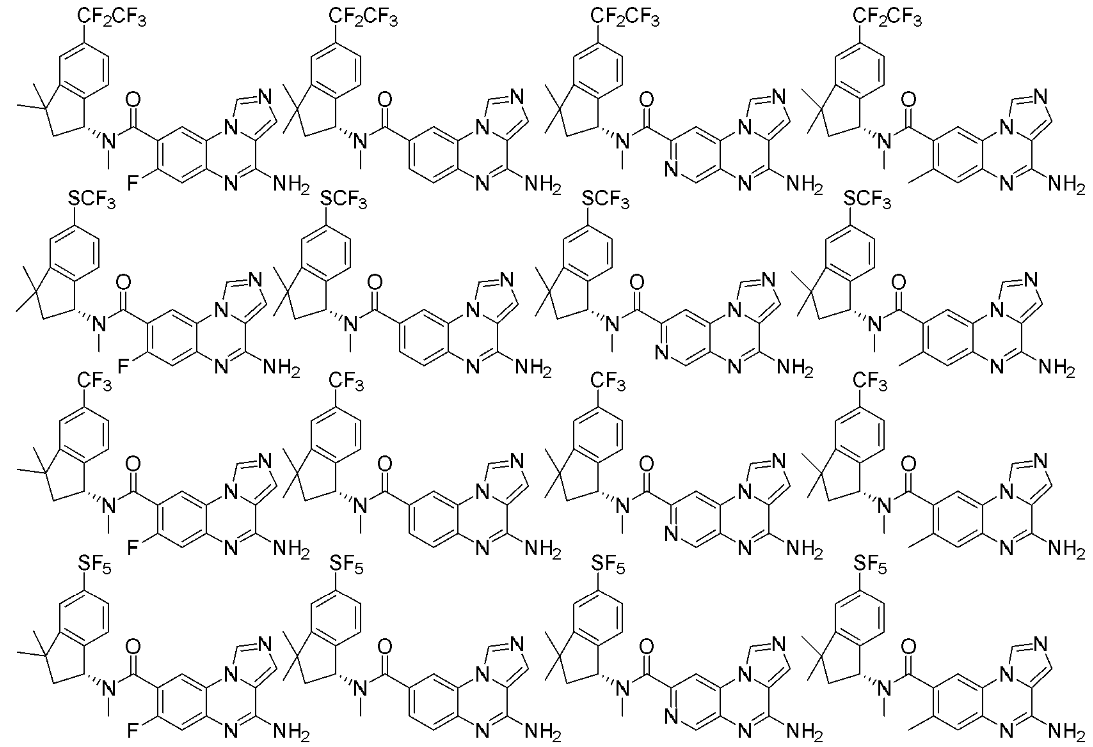

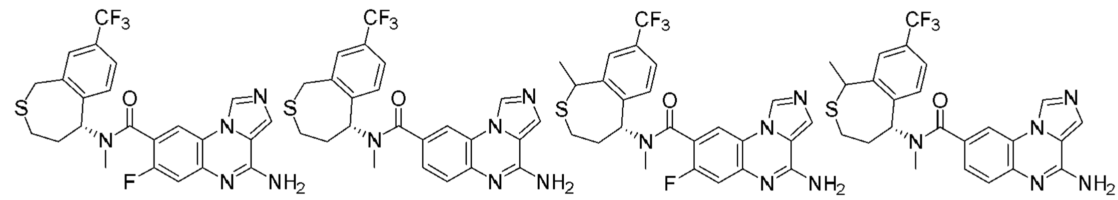

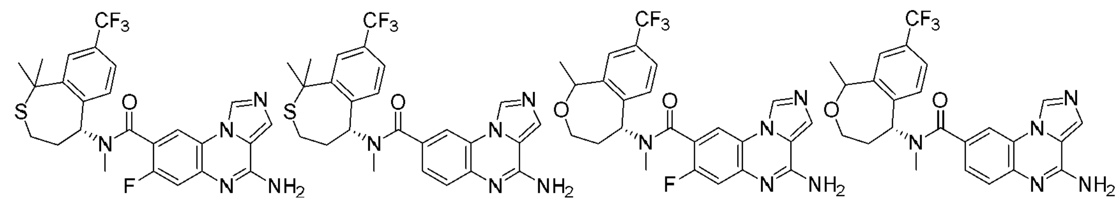

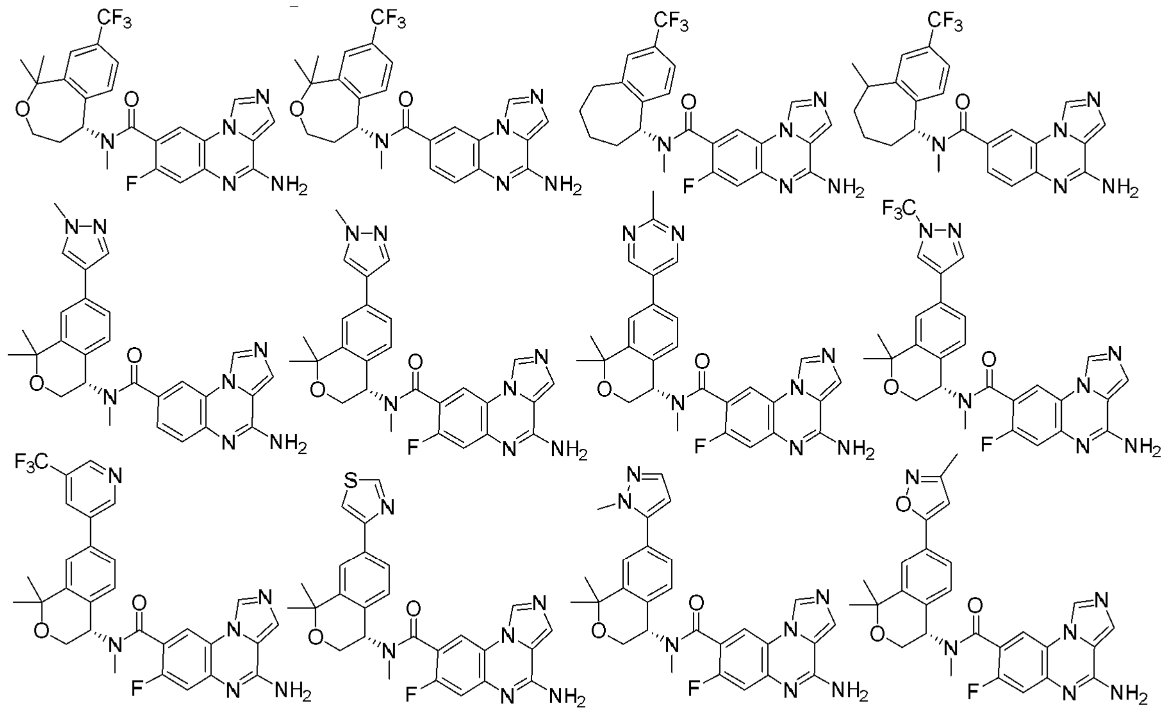

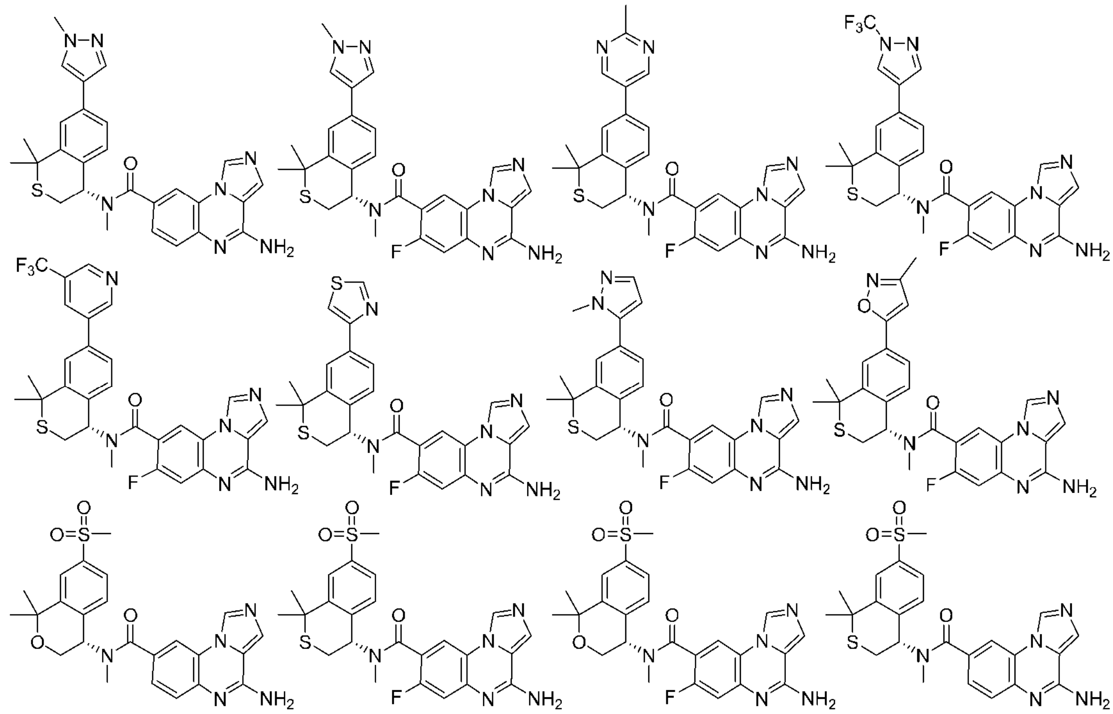

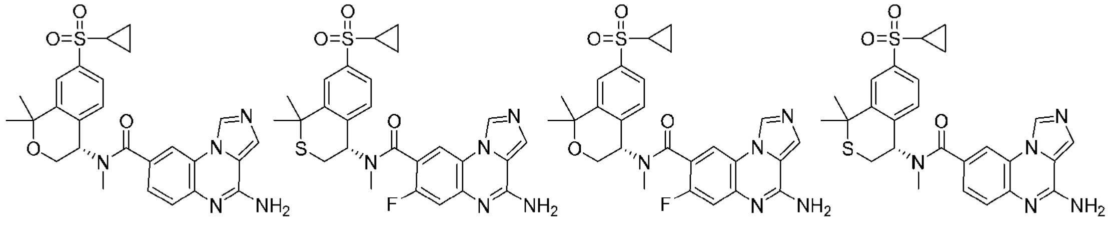

.

In a preferred embodiment of the invention, wherein, the compound is selected from the group consisting of:

(S)-4-amino-N-methyl-N-(6-(trifluoromethyl)-2,3-dihydrobenzofuran-3-yl)imidazo[1,5-a]quinoxaline-8-carboxamide
(S)-4-amino-N-methyl-N-(6-(trifluoromethyl)-2,3-dihydrobenzofuran-3-yl)imidazo[1,5-a]pyrido[3,4-e]pyrazine-8-carboxamide
(S)-4-amino-7-fluoro-N-methyl-N-(6-(trifluoromethyl)-2,3-dihydrobenzofuran-3-yl)imidazo[1,5-a]quinoxaline-8-carboxamide
(S)-4-amino-N-(methyl-d3)-N-(6-(trifluoromethyl)-2,3-dihydrobenzofuran-3-yl)imidazo[1,5-a]quinoxaline-8-carboxamide
(S)-4-amino-N-methyl-N-(6-(pentafluoro-$\lambda^6$-sulfaneyl)-2,3-dihydrobenzofuran-3-yl)imidazo[1,5-a]quinoxaline-8-carboxamide
(S)-4-amino-N-methyl-N-(6-(perfluoroethyl)-2,3-dihydrobenzofuran-3-yl)imidazo[1,5-a]quinoxaline-8-carboxamide
(S)-4-amino-7-fluoro-N-methyl-N-(6-(trifluoromethyl)-2,3-dihydrobenzofuran-3-yl)imidazo[1,5-a]quinoxaline-8-carboxamide

In a preferred embodiment of the invention, wherein, the compound is selected from the group consisting of: (S)-4-amino-N-methyl-N-(6-(trifluoromethyl)-2,3-dihydrobenzofuran-3-yl)imidazo[1,5-a]quinoxaline-8-carboxamide

In a preferred embodiment of the invention, wherein the tumor or cancer comprises a homozygous deletion of the methylthioadenosine phosphorylase (MTAP) gene.

In a preferred embodiment of the invention, wherein the tumor or cancer further comprises a homozygous deletion of the cyclin-dependent kinase inhibitor 2A (CDKN2A) gene.

In a preferred embodiment of the invention, wherein the tumor or cancer is selected from: lung cancer (non-small cell lung cancer (including adenocarcinoma and squamous cell carcinoma) or small cell lung cancer), pancreatic cancer, head and neck cancer, bladder cancer, esophageal cancer, mesothelioma, prostate cancer, breast cancer, brain cancer (e.g., glioblastoma multiforme and astrocytoma), skin cancer, cervical cancer, testicular cancer, colorectal cancer, endometrial cancer, gastric cancer, liver cancer (e.g., hepatoblastoma and hepatocellular adenoma), laryngeal cancer, oral cancer, ovarian cancer, thyroid cancer, cholangiocarcinoma, angiosarcoma, hemangioma, gallbladder cancer, papillary carcinoma, colon cancer, renal cancer, melanoma, multiple myeloma, chronic myeloid leukemia, hematological tumor, lymphoid tumor (e.g., diffuse large B-cell lymphoma), bone cancer, adrenal cancer, thymoma, malignant peripheral nerve sheath tumor, papillary renal cell carcinoma, and clear cell renal cell carcinoma.

In a preferred embodiment of the invention, wherein the tumor or cancer is selected from: brain cancer (e.g., glioblastoma multiforme and astrocytoma), lung cancer, colorectal cancer, bone cancer, gastric cancer, esophageal cancer, cholangiocarcinoma, liver cancer, ovarian cancer, breast cancer, hematological tumor, lymphoid tumor, malignant peripheral nerve sheath tumor, pancreatic cancer, bladder cancer, prostate cancer, and head and neck cancer.

In a preferred embodiment of the invention, wherein the tumor or cancer comprises metastatic lesions in tissues or organs distant from the primary tumor site.

In a preferred embodiment of the invention, wherein the tumor or cancer is selected from: glioma, lung adenocarcinoma, lung squamous cell carcinoma, bone cancer, gastric cancer, esophageal cancer, cholangiocarcinoma, liver cancer, ovarian cancer, malignant peripheral nerve sheath tumor, and lymphoid tumor.

In a preferred embodiment of the invention, wherein the tumor or cancer is a cancer associated with brain metastasis.

Unless explicitly stated otherwise, the compounds in this invention, apart from their specific structures, are expansively interpreted to include pharmaceutically acceptable salts, stereoisomers, isotopic variants (such as deuterated compounds), solvates, hydrates, prodrugs, and metabolites. Namely, the pharmaceutically acceptable salts, stereoisomers, isotopic variants (such as deuterated compounds), solvates, hydrates, prodrugs, and metabolites of the compound are also encompassed within the protective scope of the compound.
Preferably, the pharmaceutical composition of the present invention may further comprise a second active substance, wherein the second active substance is an anticancer agent, and the anticancer agent includes chemotherapeutic drugs, tumor-targeted treatment drugs, tumor treatment antibody drugs, or a combination thereof.

Furthermore, the present invention provides a compound of the invention, its pharmaceutically acceptable salts, esters, prodrugs, stereoisomers, or isotopic derivatives, and methods of treating diseases, preferably tumors, by inhibiting the action of PRMT5.

Definition:

Unless specified otherwise, the term 'alkyl,' either independently or as part of another substituent, denotes a hydrocarbon group that can be straight-chain (unbranched) or branched, cyclic, or a combination thereof. It may be saturated, mono- or polyunsaturated, and can include divalent or multivalent moieties, with a specified carbon atom count (e.g., $C_1$-$C_{10}$ indicating one to ten carbon atoms). Examples of unsaturated alkyl groups include, but are not limited to, ethenyl, 2-propenyl, crotonyl, 2-(buta-1,3-dienyl), 2-isopentenyl, 2,4-pentadienyl, 3-(1,4-pentadienyl), ethynyl, 1-propynyl and 3-propynyl, 3-butynyl, as well as higher homologs and isomers. Alkyl groups limited to hydrocarbons are referred to as 'homoalkyl. The alkyl groups may optionally be substituted with one or more halogen atoms.

The term 'haloalkyl' refers to the alkyl groups as defined above, in which one or more hydrogen atoms are replaced by halogen atoms.

The term 'alkylene' by itself or as part of another substituent refers to a divalent group derived from an alkyl, such as, but not limited to, $-CH_2CH_2CH_2CH_2-$, $-CH_2CH=CHCH_2-$, $-CH_2C\equiv CCH_2-$, $-CH_2CH_2CH(CH_2CH_2CH_3)CH_2-$. Alkyl (or alkylene) groups typically have 1 to 24 carbon atoms, and the present invention preferably encompasses groups with 10 or fewer carbon atoms. 'Lower alkyl' or 'lower alkylene' refers to shorter chain alkyl or alkylene groups, usually with eight or fewer carbon atoms. The alkylene groups may optionally be substituted with one or more halogen atoms.

The term 'alkynyl' denotes a carbon chain containing at least one carbon-carbon triple bond, which may be linear, branched, or a combination thereof. Examples of alkynyl include ethynyl, propynyl, 3-methyl-1-pentynyl, 2-heptynyl, etc. The alkynyl groups may optionally be substituted with one or more halogen atoms.

The term 'cycloalkyl' refers to a monocyclic or bicyclic saturated carbon ring, each having 3 to 10 carbon atoms. 'Fused analogs' of cycloalkyl groups refer to mono-cycle fuse with aryl or heteroaryl, where the connecting point is in the non-aromatic portion. Examples of cycloalkyl and its fused analogs include cyclopropyl, cyclobutyl, cyclopentyl, cyclohexyl, tetrahydronaphthyl, decahydronaphthyl, dihydroindanyl, etc. The cycloalkyl groups may optionally be substituted with one or more halogen atoms. Furthermore, in the context of the present invention, the term 'cycloalkyl' encompasses bridged and spirocyclic systems.

The term 'alkoxy' refers to linear or branched alkoxyl groups indicating the number of carbon atoms. For example, $C_{1-6}$ alkoxy, includes methoxy, ethoxy, propoxy, isopropoxy, etc.

Unless specified otherwise, the term 'heteroalkyl,' either alone or in combination with other terms, refers to stable linear or branched hydrocarbon groups, or cyclic hydrocarbon groups, or their combination, incorporating at least one carbon atom and at least one heteroatom chosen from O, N, P, Si, and S. Optional oxidation of nitrogen, phosphorus, or sulfur atoms, and potential quaternization of nitrogen atoms are permitted. The heteroatoms O, N, P, S, and Si can be positioned at any location within the heteroalkyl or location at which the alkyl connected to the rest part of the molecule. Examples include, but are not limited to, $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_2$, $-S(O)-CH_3$, $-CH_2-CH_2-S(O)_2-CH_3$, $-CH=CH-O-CH_3$, $-Si(CH_3)_3$, $-CH_2-CH=N-OCH_3$, $-CH=CH-N(CH_3)-CH_3$, $-O-CH_3$, $-O-CH_2-CH_3$, and -CN. Up to two or three contiguous heteroatoms are allowed. For instance, $-CH_2-NH-OCH_3$ and $-CH_2-O-Si(CH_3)_3$. Similarly, the term 'heteroalkylidene,' alone or in combination, designates divalent groups derived from heteroalkyl, including but not limited to $-CH_2-CH_2-S-CH_2-CH_2-$ and $-CH_2-S-CH_2-CH_2-NH-CH_2-$. Heteroatoms in heteroalkylidene can be positioned at either end or both ends of the chain (e.g., alkoxy, dialkoxy, amino, diamino, etc.). Additionally, for alkoxy and heteroalkoxy linking groups, the molecular formula is written without indicating the orientation of the linking group (e.g., -C(O)OR'- represents both -C(O)OR'- and -R'OC(O)-). As stated above, the term 'heteroalkyl' encompasses groups connected to the rest of the molecule through heteroatoms, such as -C(O)R', -C(O)NR', -NR'R", -OR', -SR', and/or $-SO_2R'$. When 'heteroalkyl' is mentioned and specific heteroalkyls like -NR'R" are subsequently cited, it should be understood that the term 'heteroalkyl' and -NR'R" are distinct and not mutually exclusive. The inclusion of specific heteroalkyls is for clarification and does not preclude others like -NR'R"."

The term 'cycloalkyloxy' refers to the cyclic alkyl groups as defined above, bound to an oxygen atom, such as cyclopropyloxy.

The term 'haloalkoxy' refers to an alkoxyl group as defined above with one or more hydrogen atoms substituted by halogen.

The term 'aryl' refers to a monocyclic or bicyclic aryl group consisting exclusively of carbon atoms. The 'fused analogs' of aryl are those in which aryl is combined with a monocyclic cycloalkyl or monocyclic heterocyclyl, with the connection point located in the aryl portion. Examples of aryl and its fused analogs include phenyl, naphthyl, indanyl, indenyl, tetrahydronaphthyl, 2,3-dihydrobenzofuranyl, 1,4-benzodioxanyl, etc.

The term 'heteroaryl' refers to a monocyclic or bicyclic aryl group containing at least one heteroatom selected from N, O, and S. The 'fused analogs' of heteroaryl are those in which heteroaryl is combined with a monocyclic cycloalkyl or monocyclic heterocyclyl, with the connection point located in the heteroaryl portion. Examples of heteroaryl include pyrrolyl, isoxazolyl, isothiazolyl, pyrazolyl, pyridinyl, oxazolyl, oxadiazolyl, thiadiazolyl, thiazolyl, imidazolyl, triazolyl, tetrazolyl, furanyl, triazine, thiophenyl, pyrimidinyl, quinazolinyl, pyrazinyl, quinoxalinyl, benzoxazolyl, benzothiazolyl, benzimidazolyl, benzofuranyl, benzothiophenyl, furan[2,3-b]pyridinyl, quinolinyl, indolyl, isoquinolinyl, etc.

"Substituted or unsubstituted": The defined alkyl, aryl, and heteroaryl groups can be unsubstituted or substituted with at least one substituent selected from the group consisting of halogen atoms, alkyl groups with 1 to 6 carbon atoms, alkoxy groups with 1 to 6 carbon atoms, haloalkyl groups with 1 to 6 carbon atoms, halogenated alkoxy groups with 1 to 6 carbon atoms, -CN, alkynyl groups with 2 to 6 carbon atoms, acyl groups with 1 to 6 carbon atoms, cycloalkyl groups with 3 to 7 ring atoms, heteroaryl groups, aryl groups, arylalkoxy groups with 7-10 carbon atoms, aryloxycarbonyl groups, aminocarbonyl groups, vinyl groups with 2 to 5 carbon atoms, alkylthio groups with 1 to 6 carbon atoms, amino sulfonyl groups, sulfonyl amino groups, hydroxy groups, -SF5, hydroxyalkyl groups with 1 to 4 carbon atoms, nitro groups, amino

groups, carboxyl groups, alkoxy carbonyl groups with 2 to 5 carbon atoms, alkoxyalkyl groups with 1 to 4 carbon atoms, alkylsulfonyl groups with 1 to 4 carbon atoms, acylamino groups with 1 to 4 carbon atoms, acyl(amino)amino groups with 1 to 6 carbon atoms, acyl(amino)aminoalkyl groups with 1 to 6 carbon atoms in both acyl and alkyl parts, sulfonylamino groups with 1 to 4 carbon atoms, single alkylamino groups with 1 to 6 carbon atoms or double alkylamino groups with 1 to 6 carbon atoms, single alkylaminoalkyl groups with 1 to 6 carbon atoms or double alkylaminoalkyl groups with 1 to 6 carbon atoms, aminoalkyl groups with 1 to 4 carbon atoms, single alkylamino groups with 1 to 6 carbon atoms or double alkylamino groups with 1 to 6 carbon atoms in both alkyl parts, arylalkyl groups with 7 to 10 carbon atoms, heteroarylalkyl groups with 1 to 4 carbon atoms in alkyl parts, heteroaryloxyalkyl groups with 1 to 4 carbon atoms in alkoxy parts, and alkylsulfonylamino groups with 1 to 4 carbon atoms.

As used herein, the term "heterocycle" or "heterocyclic" or "heterocycloalkyl" or "heterocyclo" refers to a saturated, partially saturated, or unsaturated group (but not aromatic) with a single ring or fused rings (including bridged and spiro systems), having 1 to 10 carbon atoms and 1 to 4 heteroatoms selected from nitrogen, sulfur, or oxygen. In fused systems, one or more rings can be cycloalkyl, aryl, or heteroaryl, as long as the connection points are through non-aromatic rings. In one embodiment, nitrogen and/or sulfur atoms of heterocyclic groups may optionally be oxidized to provide N-oxides, sulfinyl, and sulfonyl portions. Examples of "heterocyclic" and its fused analogs include pyrrolidinyl, piperidinyl, pyrazinyl, imidazolidinyl, 2,3-dihydrofuran(2,3-b)pyridinyl, quinazolinyl, tetrahydroquinoline, tetrahydroisoquinoline, dihydroindole, etc. The term also encompasses non-aromatic, partially unsaturated monocycles, such as 2- or 4-pyridones or N-substituted -(1H,3H)-pyrimidin-2,4-diones (N-substituted uracils).

As used herein, the term "substituted heterocycloalkyl" or "substituted heterocyclo" refers to a heterocycloalkyl group substituted with 1 to 5 (such as 1 to 3) substituents, wherein the substituents are the same as those defined for substituted cycloalkyl.

Unless otherwise specified, the term "halogenated" or "halo" refers to fluorine, chlorine, bromine, or iodine atoms, either by themselves or as part of another substituent. Additionally, the term "halogenated alkyl" refers to both mono-halogenated alkyl and poly-halogenated alkyl. For example, the term "halogenated ($C_1$-$C_6$) alkyl" includes, but is not limited to, trifluoromethyl, 2,2,2-trifluoroethyl, pentafluoroethyl, 4-chlorobutyl, 3-bromopropyl, and so on.

"Prodrug" refers to a substance that, when administered into the body, undergoes a conversion to the parent drug. In certain situations, prodrugs are frequently used because they might be more easily administered than the parent drug. For instance, a prodrug administered orally may be more bioavailable than the parent drug. In drug combinations, prodrugs can also exhibit higher solubility than the parent drug. Examples of prodrugs include, but are not limited to, any compound from compound of formula (I) administered in the form of an ester (prodrug) to facilitate transmembrane transport. This aids in overcoming barriers posed by cell membranes, as the water solubility of the prodrug is detrimental to migration across the membrane. Once inside the beneficial aqueous environment of the cell, the ester is metabolized and hydrolyzed to the active carboxylic acid substance. Another example of a prodrug can be short peptide (polyamino acid) conjugated with an acid moiety, where the peptide undergoes metabolism to release the active portion.

Optical isomers, diastereomers, geometric isomers, and tautomers:
The compound of Formula (I) contains one or more asymmetric centers, and thus, it can exist as a racemate and racemic mixtures, individual enantiomers, diastereomers, and individual diastereomers. The present invention encompasses all these stereoisomeric forms of the compound of Formula (I).

Some compounds described in this disclosure contain olefinic double bonds, which, unless otherwise specified, refers to both E and Z geometric isomers.

Certain compounds of the present invention may comprise one or more ring systems, giving rise to cis- and trans-isomers. The invention is intended to encompass all these cis- and trans- isomers.

Certain compounds described herein may exist with different points of hydrogen attachment, known as tautomerism. An example is keto-enol tautomerism, where a keto form and its enol form are involved. Individual tautomers and their mixtures are included in the compounds of the present invention.

The compounds of the present invention can be separated into diastereoisomeric pairs of enantiomers, for example, by fractional crystallization from suitable solvents such as methanol or ethyl acetate or mixtures thereof. Enantiomeric pairs thus obtained can be separated into individual stereoisomers through conventional methods, including using optically active amines or acids as resolving agents or by separation on a chiral HPLC column.

Alternatively, any diastereoisomer of the compounds of the present invention can be stereoselectively synthesized using optically pure starting materials or reagents of known configuration.

Stable isotope-labeled analogs: One or more protons in the compounds of the present disclosure may be replaced by deuterium atoms, thereby providing deuterated analogs with improved pharmacological activities.

Salt and Dosage Form

The compounds mentioned in this disclosure, as used herein, are understood to include pharmaceutically acceptable salts.

Application

These compounds can be employed for the treatment of PRMTS-related diseases.

The PRMT5-related diseases described in the present disclosure are tumors or cancers, including lung cancer (non-small cell lung cancer or small cell lung cancer), prostate cancer, breast cancer, brain cancer, skin cancer, cervical cancer, testicular cancer, and other tumors. More specifically, the compositions and methods of the present disclosure can be used to treat tumor types including, but not limited to, astrocytoma, breast cancer, cervical cancer, colorectal cancer, endometrial cancer, esophageal cancer, gastric cancer, head and neck cancer, hepatocellular carcinoma, laryngeal cancer, lung cancer, oral cancer, ovarian cancer, prostate cancer, thyroid cancer, and sarcoma. More specifically, these compounds can be used to treat: cardiac tumors, such as sarcoma (angiosarcoma, fibrosarcoma, rhabdomyosarcoma, lipoma), myxoma, rhabdomyoma, fibroma, lipoma, and teratoma; lung tumors, such as bronchogenic carcinoma (squamous cell carcinoma, undifferentiated small cell carcinoma, undifferentiated large cell carcinoma, adenocarcinoma), alveolar (bronchiolar) carcinoma, bronchial adenoma, sarcoma, lymphoma, chondromatous hamartoma, pleural mesothelioma; gastrointestinal tumors, such as esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors), small intestine (adenocarcinoma, lymphoma, carcinoid tumors, Kaposi's sarcoma, leiomyoma, hemangioma, lipoma, neurofibroma, fibroma), large intestine (adenocarcinoma, tubular adenoma, villous adenoma, hamartoma, leiomyoma); genitourinary tract: kidney (adenocarcinoma, Wilms' tumor (nephroblastoma), lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); liver: hepatoma (hepatocellular carcinoma), cholangiocarcinoma, hepatoblastoma, angiosarcoma, hepatocellular adenoma, hemangioma; biliary tract: gallbladder carcinoma, papillary carcinoma, cholangiocarcinoma; bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochrondroma (osteocartilaginous exostoses), benign chondroma, chondroblastoma, chondromyxofibroma, osteoid osteoma, and giant cell tumors; nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma (pinealoma), glioblastoma multiforme, oligodendroglioma, schwannoma, retinoblastoma, congenital tumors), spinal cord (neurofibroma, meningioma, glioma, sarcoma); gynecological: uterus (endometrial cancer), cervix (cervical cancer, pre-cancerous cervical dysplasia), ovary (ovarian cancer (serous cystadenocarcinoma, mucinous cystadenocarcinoma, undifferentiated carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, sarcoma botryoides); and malignant peripheral nerve sheath tumors. The specific lists above include metastatic lesions in other tissues or organs distant from the primary tumor site. Preferably, said cancer includes, but is not limited to: colon cancer, breast cancer, gastric cancer, lung cancer, large intestine cancer, pancreatic cancer, ovarian cancer, prostate cancer, renal cancer, liver cancer, brain cancer, cervical cancer or head and neck cancer, melanoma, multiple myeloma, chronic myeloid leukemia, hematological tumors, and lymphoid tumors, including metastatic lesions in other tissues or organs distant from the primary tumor site.

Preferably, the compounds of the present invention can be used for treating brain tumors (e.g., glioma) or diseases associated with brain metastasis.

As used herein, "pharmaceutically acceptable carrier or excipient" includes diluents, fillers, binders, disintegrants, lubricants, glidants, granulating agents, coating agents, wetting agents, solvents, co-solvents, suspending agents, emulsifiers, sweeteners, flavoring agents, taste-masking agents, coloring agents, anti-caking agents, humectants, chelating agents, plasticizers, viscosity increasing agents, antioxidants, preservatives, stabilizers, surfactants, and buffers; those skilled in the art will appreciate that certain pharmaceutically acceptable excipients may serve more than one function and may serve alternative functions depending on how much of the excipient is present in the formulation and what other ingredients are present in the formulation. For example, when used for oral administration, they can be formulated into oral preparations such as tablets, capsules, granules, and pills, comprising fillers (e.g., sugar derivatives such as lactose, sucrose, glucose, mannitol, and sorbitol; starch derivatives such as corn starch, potato starch, dextrin, and carboxymethyl starch; cellulose derivatives such as crystalline cellulose, hydroxypropyl cellulose, carboxymethyl cellulose, calcium carboxymethyl cellulose, sodium carboxymethyl cellulose; gum arabic; dextran; silicate derivatives such as magnesium aluminum metasilicate; phosphate derivatives such as calcium phosphate; carbonate derivatives such as calcium carbonate; sulfate derivatives such as calcium sulfate, etc.), binders (e.g., gelatin, polyvinylpyrrolidone, and polyethylene glycol), disintegrants (e.g., cellulose derivatives such as sodium carboxymethyl cellulose and polyvinylpyrrolidone), lubricants (e.g., talc, calcium stearate, magnesium stearate, spermaceti, boric acid, sodium benzoate, leucine), stabilizers (methyl parahydroxybenzoate, propyl parahydroxybenzoate, etc.), and flavoring agents (e.g., commonly used sweeteners, acidulants, and fragrances, etc.). When used for parenteral administration, they can be

formulated into injections, including sterile powders for injection and solvents for injection, wherein the carriers or excipients used include sterile water, Ringer's solution, and isotonic sodium chloride solution, and appropriate additives such as antioxidants, buffers, and bacteriostatic agents may also be added depending on the nature of the drug. When used for rectal administration, the drugs can be formulated into suppositories, etc.; and when used for pulmonary administration, the drugs can be formulated into inhalants or sprays, etc. There are many resources available to those skilled in the art that describe pharmaceutically acceptable excipients and can be used to select suitable pharmaceutically acceptable excipients, such as Remington: The Science and Practice of Pharmacy, Chinese Pharmaceutical Yearbook, Pharmaceutics, and other books.

The present disclosure can be administered by any suitable method known in the art, for example, by oral, intravenous, intraperitoneal, intramuscular, topical, transdermal, ocular, nasal, inhalation, subcutaneous, intramuscular, buccal, sublingual, or rectal administration, etc. The compound described above can be administered in any amount on the order of $\mu$g to mg/kg of the subject's body weight, for example, in an amount of 0.1 $\mu$g to 1000 mg/kg body weight/day. For example, in some embodiments, the dose administered to the patient is from about 1 mg/kg to about 75 mg/kg of the subject's body weight. In some embodiments, the dose administered to the patient is from 1 mg/kg to 20 mg/kg of the subject's body weight, for example, 1 mg/kg to 5 mg/kg of the subject's body weight. In some embodiments, the dose administered to the patient is about 0.5 mg/kg of the subject's body weight. In some embodiments, the dose administered to the patient is about 0.75 mg/kg of the subject's body weight. In some embodiments, the dose administered to the patient is about 1 mg/kg of the subject's body weight. In some embodiments, the dose administered to the patient is about 25 mg/kg of the subject's body weight. In some embodiments, the dose administered to the patient is about 5 mg/kg of the subject's body weight. In some embodiments, the dose administered to the patient is about 2 mg/kg of the patient's body weight. In some embodiments, the dose administered to the patient is about 2.5 mg/kg of the subject's body weight. In some embodiments, the dose administered to the patient is about 3 mg/kg of the subject's body weight. In some embodiments, the dose administered to the patient is about 3.5 mg/kg of the subject's body weight. In some embodiments, the dose administered to the patient is about 4 mg/kg of the subject's body weight. In some embodiments, the dose administered to the patient is about 4.5 mg/kg of the subject's body weight. In some embodiments, the dose administered to the patient is about 5 mg/kg of the subject's body weight. In some embodiments, the dose administered to the patient is about 5.5 mg/kg of the subject's body weight. In some embodiments, the dose administered to the patient is about 6 mg/kg of the patient's body weight. In some embodiments, the dose administered to the patient is about 6.5 mg/kg of the subject's body weight. In some embodiments, the dose administered to the patient is about 7 mg/kg of the subject's body weight. In some embodiments, the dose administered to the patient is about 7.5 mg/kg of the subject's body weight. In some embodiments, the dose administered to the patient is about 8 mg/kg of the subject's body weight. In some embodiments, the dose administered to the patient is about 8.5 mg/kg of the patient's body weight.

In some embodiments of the present invention, the compound described above may be administered 4 times daily, 3 times daily, 2 times daily, once daily, once every two days, once weekly, or at other intervals, and the dosing regimen described above is optionally repeated weekly or monthly as appropriate. In the present disclosure, the administered dose of the compound may be adjusted according to factors such as the severity of the disease, age, body weight, gender, route of administration, and course of treatment of the patient or subject.

## DESCRIPTION OF THE DRAWINGS

Figure 1 shows the tumor growth inhibition of Example Compound 107 in a U87MG mouse orthotopic xenograft model.

General Synthetic Route

The compounds of the present invention can be prepared through the following reaction scheme:

Method A:

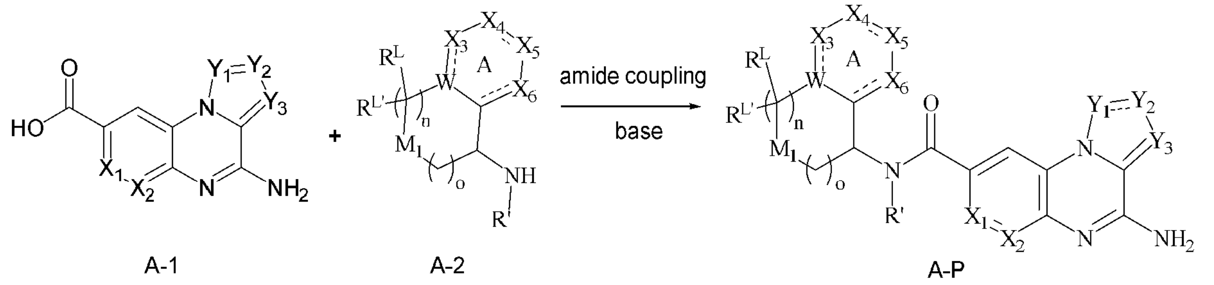


A-1 A-2 A-P

Method A-SFC

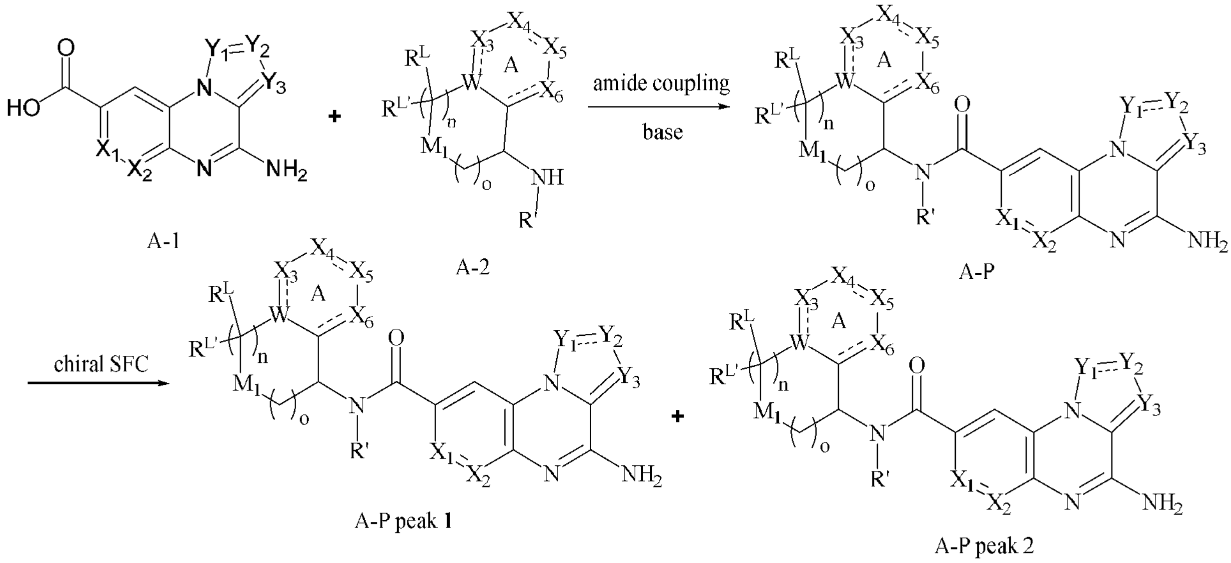


A-1 A-2 A-P

A-P peak 1 A-P peak 2

Wherein, $R^L$, $R^{L'}$, R', ring A, W, n, o, $M_1$, $X_1$, $X_2$, $X_3$, $X_4$, $X_5$, $X_6$, $Y_1$, $Y_2$, $Y_3$ and $\overline{---}$ as defined in claim 1.

Method A: Compound A-P can be obtained through the condensation reaction of carboxylic acid A-1 and amine A-2, using HATU or PyBrOP as the condensing agent and DIPEA or TEA as the base, with DMF or DMAc as the solvent. If the used amine is a racemate, chiral SFC will be employed for resolution, and the stereochemistry of the resulting isomer will be arbitrarily assigned as R or S.

Analytical HPLC:

Instrument: Agilent 1260;

Column specifications: Agilent Poroshell HPH-C18 (3.0×50mm, 2.7μm);

Binary solvent system, mobile phase A: water (0.1% v/v ammonium bicarbonate), mobile phase B: acetonitrile;

Flow rate: 1 milliliter/minute;

Gradient: from 10% B to 90% B;

Duration: 12 minutes;

Detector: DAD detector;

Wavelength: 254/220 nanometers.

Preparative HPLC:

HPLC equipment: Waters 2489;

Column specifications: Ultimate μ XB-C18 (130A, 5um, 30mm× 150mm);

Binary solvent system, mobile phase A: water (0.1% v/v ammonium bicarbonate), mobile phase B: acetonitrile;

Flow rate: 60-100 milliliters/minute;

Gradient: from 10% B to 90% B;

Detector: DAD detector;

Wavelength: 254/220 nanometers;

Mass spectrometer: Agilent G6125B.

The compounds of the present invention can be prepared through chemical synthesis, as illustrated in the following examples. It should be understood that the order of steps in the process can be altered, specific mentioned reagents, solvents, and reaction conditions can be substituted, and, if necessary, reactive sites can be protected and deprotected.

The abbreviations below have the following meanings:
ACN: acetonitrile; EA: ethyl acetate; CDI: N, N'-carbonyldiimidazole; DBU: 1,8-diazabicyclo[5.4.0]undec-7-ene; DIBAL-H: diisobutylaluminum hydride; DIEA: diisopropylethylamine; DMAP: N, N-dimethylaminopyridine; DME: 1,2-dimethoxyethane; DMF: N, N-dimethylformamide; DMA and DMAc: N, N-dimethylacetamide; DMPE: 1,2-bis(dimethylphosphino) ethane; DMSO: dimethyl sulfoxide; DPPB: 1,4-bis(diphenylphosphino)butane; dppe: 1,2-bis(diphenylphosphino)ethane; dppf: 1,1'-bis(diphenylphosphino)ferrocene; dppm: 1,1'-bis(diphenylphosphino)methane; DIAD: diisopropyl azodicarboxylate; EDCI: 1-(3-dimethylaminopropyl)-3-ethylcarbodiimide; HATU: 2-(7-aza-1H-benzotriazol-1-yl)-1,1,3,3-tetramethyluronium hexafluorophosphate; HMPA: hexamethylphosphoramide; IPA: isopropanol; LDA: lithium diisopropylamide; LHMDS: lithium bis(trimethylsilyl)amide; LAH: lithium aluminum hydride; NCS: N-chlorosuccinimide; NaHMDS: sodium bis(trimethylsilyl)amide; PyBOP: benzotriazol-1-yloxytripyrrolidinophosphonium hexafluorophosphate; PyBrOP: tripyrrolidinophosphonium bromide hexafluorophosphate; TDA-I: tri(2-(2-methoxyethoxy)ethyl)amine; DCM: dichloromethane; TEA: triethylamine; TFA: trifluoroacetic acid; THF: tetrahydrofuran; NMM: N-methylmorpholine; NMP: N-methyl-2-pyrrolidone; PPh3: triphenylphosphine; r.t.: room temperature; PMB: para-methoxybenzyl; Tosmic: p-toluenesulfonylmethyl isocyanide; (Boc)2O: di-tert-butyl decarbonate; PE: petroleum ether; o/n: overnight reaction.

The following preparation and implementation examples illustrate the present invention but do not limit it in any way.

A detailed description of selected embodiments will provide a clearer understanding of the features and advantages of the subject matter of the present invention. As one skilled in the art would appreciate, the disclosed and claimed subject matter can be modified in various aspects, and all such modifications are within the scope of the claims. Therefore, the description should be considered as explanatory rather than restrictive in nature. The full scope of the subject matter of the present invention is defined in the claims.

For a better understanding of the present invention, reference may be made to the following examples, which are provided for illustrative purposes and not to limit the scope of the invention.

_

## Intermediates

The intermediate raw materials were purchased from different suppliers:

| Int. No. | structure | name | CAS |
|---|---|---|---|
| 1 | 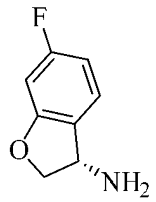 | (*S*)-6-fluoro-2,3-dihydrobenzofuran-3-amine | 1228559-33-8 |
| 2 | 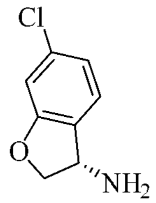 | (*S*)-6-chloro-2,3-dihydrobenzofuran-3-amine | 1228561-83-8 |
| 3 | 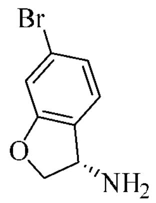 | (*S*)-6-bromo-2,3-dihydrobenzofuran-3-amine | 1228568-69-1 |
| 4 | 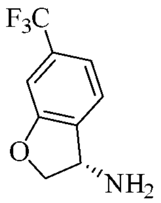 | (S)-6-(trifluoromethyl)-2,3-dihydrobenzofuran-3-amine | 1272724-36-3 |
| 5 | 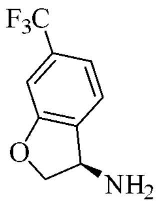 | (R)-6-(trifluoromethyl)-2,3-dihydrobenzofuran-3-amine | 1272732-77-0 |
| 6 | 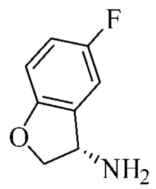 | (S)-5-fluoro-2,3-dihydrobenzofuran-3-amine | 1228571-72-9 |
| 7 | 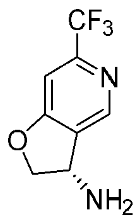 | (S)-6-(trifluoromethyl)-2,3-dihydrofuro[3,2-c]pyridin-3-a mine | |

(continued)

| Int. No. | structure | name | CAS |
|---|---|---|---|
| 8 | 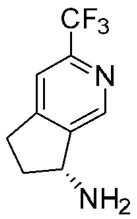 | (R)-3-(trifluoromethyl)-6,7-dihydro-5H-cyclopenta[c] pyridin-7-amine | |
| 9 | 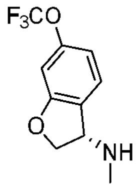 | (S)-N-methyl-6-(trifluoromethoxy)-2,3-dihydrobenzofuran -3-amine | 2814522-50-2 |
| 10 | 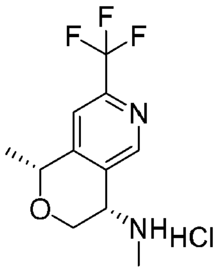 | (1R,4S)-N,1-dimethyl-7-(trifluoromethyl)-3,4-dihydro-1H-pyrano[4,3-c]pyridin-4-amine hydrochloride | |
| 11 | 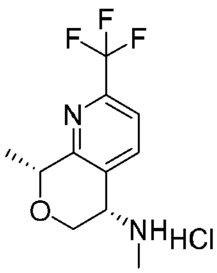 | (5S,8R)-N,8-dimethyl-2-(trifluoromethyl)-5,8-dihydro-6H-pyrano[3,4-b]pyridin-5-amine hydrochloride | |
| 99 | 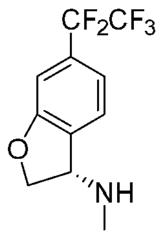 | (S)-N-methyl-6-(perfluoroethyl)-2,3-dihydrobenzofuran-3-amine | |

**Int. 12: (S)-N-methyl-6-(trifluoromethyl)-2,3-dihydrobenzofuran-3-amine**

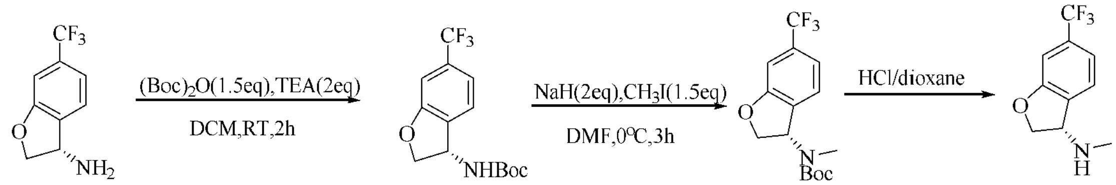

Step1: synthesis of tert-butyl (S)-(6-(trifluoromethyl)-2,3-dihydrobenzofuran-3-yl)carbamate

(S)-6-(trifluoromethyl)-2,3-dihydrobenzofuran-3-amine (200mg, 0.99mmol) was dissolved in 2ml DCM, then di-tert-butyl dicarbonate(325mg, 1.49mmol) and triethylamine(200mg, 1.98mmol) were added. The mixture was stirred at room temperature for 2 hours. The reaction was monitored by LCMS. Residues were purified by rapid column chromatography (20g silica gel column, 20% ethyl acetate/petroleum ether) to yield a yellow liquid: tert-butyl (S)-(6-(trifluoromethyl)-2,3-dihydrobenzofuran-3-yl)carbamate(300mg, yield:99%).
LCMS (ESI) m/z: 304 $[M+H]^+$

Step 2: synthesis of tert-butyl (S)-methyl(6-(trifluoromethyl)-2,3-dihydrobenzofuran-3-yl)carbamate

At 0 °C, (S)-(6-(trifluoromethyl)-2,3-dihydrobenzofuran-3-yl)carbamate (300 mg, 0.99 mmol) was dissolved in DMF (5.00 mL), followed by the addition of sodium hydride (48 mg, 2.00 mmol). The mixture was stirred at 0 °C for 1 hour. Methyl iodide (211 mg, 1.49 mmol) was then added. The mixture was stirred at room temperature for 2 hours. The reaction progress was monitored by LCMS. Residues were purified by rapid column chromatography (20g silica gel column, 15% ethyl acetate/petroleum ether) to yield a yellow liquid of tert-butyl (S)-methyl(6-(trifluoromethyl)-2,3-dihydrobenzofuran-3-yl)carbamate(200.0 mg, 64% yield).
LCMS (ESI) m/z: 318 $[M+H]^+$

Step 3: synthesis of (S)-N-methyl-6-(trifluoromethyl)-2,3-dihydrobenzofuran-3-amine

At room temperature, (S)-methyl(6-(trifluoromethyl)-2,3-dihydrobenzofuran-3-yl)carbamate (200.0 mg, 0.63 mmol) was dissolved in a solution of hydrogen chloride in 1,4-dioxane (5.00 mL). The mixture was stirred at room temperature for 0.5 hours. The reaction progress was monitored by LCMS. The mixture was concentrated to yield a yellow liquid of (S)-N-methyl-6-(trifluoromethyl)-2,3-dihydrobenzofuran-3-amine (130.0 mg, 95% yield).
LCMS (ESI) m/z: 218 $[M+H]^+$

Employing the preparation method and steps of Intermediate 12, with the sole replacement of the corresponding raw material intermediate, the following intermediates were obtained:

| Int. No. | Structure | name | m/z(ESI): $(M+H)^+$ |
|---|---|---|---|
| 13 | 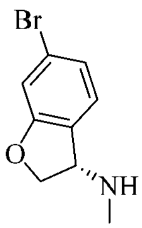 | (S)-6-bromo-N-methyl-2,3-dihydrobenzofuran-3-amine | 228.1 |
| 14 | 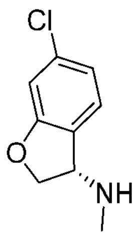 | (S)-6-chloro-N-methyl-2,3-dihydrobenzofuran-3-amine | 184 |
| 15 | 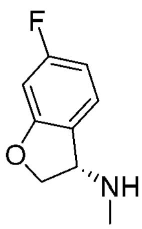 | (S)-6-fluoro-N-methyl-2,3-dihydrobenzofuran-3-amine | 168.2 |

(continued)

| Int. No. | Structure | name | m/z(ESI): (M+H)+ |
|---|---|---|---|
| 16 | 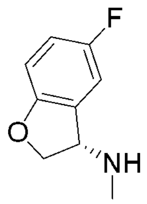 | (S)-5-fluoro-N-methyl-2,3-dihydrobenzofuran-3-amine | 168.2 |
| 17 | 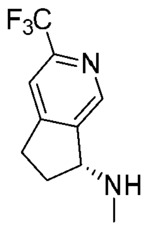 | (R)-N-methyl-3-(trifluoromethyl)-6,7-dihydro-5H-cyclopenta[c]pyridin-7-amine | 217.2 |
| 18 | 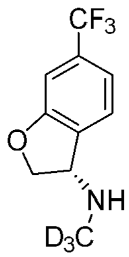 | (S)-N-(methyl-$d_3$)-6-(trifluoromethyl)-2,3-dihydrobenzofuran-3-amine | 221.2 |
| 19 | 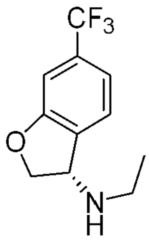 | (S)-N-ethyl-6-(trifluoromethyl)-2,3-dihydrobenzofuran-3-amine | 232.2 |
| 20 | 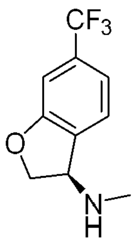 | (R)-N-methyl-6-(trifluoromethyl)-2,3-dihydrobenzofuran-3-amine | 218.2 |
| 21 | 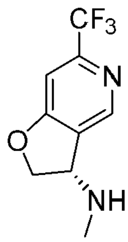 | (S)-N-methyl-6-(trifluoromethyl)-2,3-dihydrofuro[3,2-c]pyridin-3-amine | 219.2 |

Int. 22: (S)-3-(methylamino)-2,3-dihydrobenzofuran-6-carbonitrile

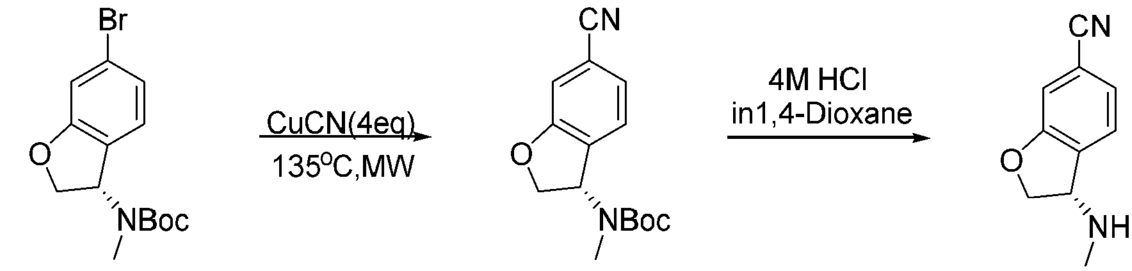


Step 1: synthesis of tert-butyl (S)-(6-cyano-2,3-dihydrobenzofuran-3-yl)(methyl)carbamate

In a microwave reactor, a reaction mixture of tert-butyl (S)-(6-bromo-2,3-dihydrobenzofuran-3-yl)(methyl) carbamate (328 mg, 1 mmol), cuprous cyanide (352 mg, 4 mmol), and N-methylpyrrolidone (4 mL) was heated at 135°C for 4 hours. After completion of the reaction, the system was cooled to room temperature, then poured into water, and extracted three times with ethyl acetate (30 mL×3). The combined organic phases were washed with saturated brine, dried over anhydrous sodium sulfate, filtered, and the filtrate was concentrated under reduced pressure. The residue was purified by silica gel column chromatography (ethyl acetate:petroleum ether = 40:60) to yield tert-butyl (S)-(6-cyano-2,3-dihydrobenzofuran-3-yl)(methyl)carbamate (66 mg, 23.93% yield), a colorless oily substance.
LCMS (ESI): 275 $[M+H]^+$

Step 2: (S)-3-(methylamino)-2,3-dihydrobenzofuran-6-carbonitrile

tert-butyl (S)-(6-cyano-2,3-dihydrobenzofuran-3-yl)(methyl)carbamate (66 mg, 0.24 mmol) was added to a solution of hydrogen chloride in 1,4-dioxane (3 mL, 4 mol/L). The mixture was stirred for 2 hours. After completion of the reaction, the resulting mixture was concentrated under reduced pressure to obtain crude (S)-3-(methylamino)-2,3-dihydrobenzofuran-6-carbonitrile (40 mg), a white solid. The crude product was used directly in the next step without purification.
LCMS (ESI): 175 $[M+H]^+$

Int. 23: synthesis of (S)-6-methoxy-N-methyl-2,3-dihydrobenzofuran-3-amine

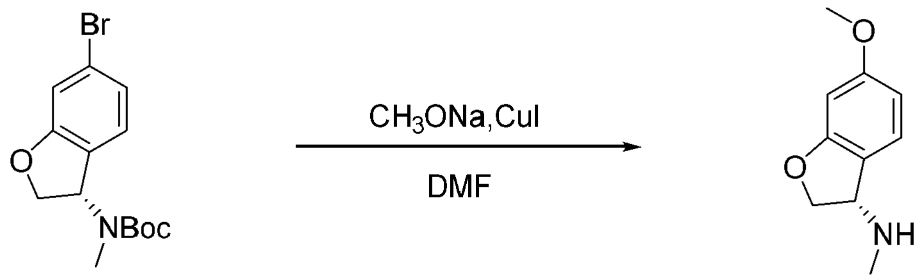


tert-butyl (S)-(6-bromo-2,3-dihydrobenzofuran-3-yl)(methyl)carbamate (328 mg, 1 mmol), CuI (352 mg, 4 mmol), and a solution of sodium methoxide in methanol (180 mg, 40 mmol) in N,N-dimethylformamide (4 mL) were reacted at 120°C for four hours. After completion of the reaction, the system was cooled to room temperature, and the mixture was extracted three times with ethyl acetate (30 mL each time). The combined organic phases were washed with saturated brine and dried over anhydrous sodium sulfate. After concentration under reduced pressure,
(S)-6-methoxy-N-methyl-2,3-dihydrobenzofuran-3-amine(150 mg, 0.8 mmol, 80% yield) was yielded, a colorless oily substance.
LCMS (ESI):180 $[M+H]^+$

Int. 24 (S)-dimethyl(3-(methylamino)-2,3-dihydrobenzofuran-6-yl)phosphine oxide

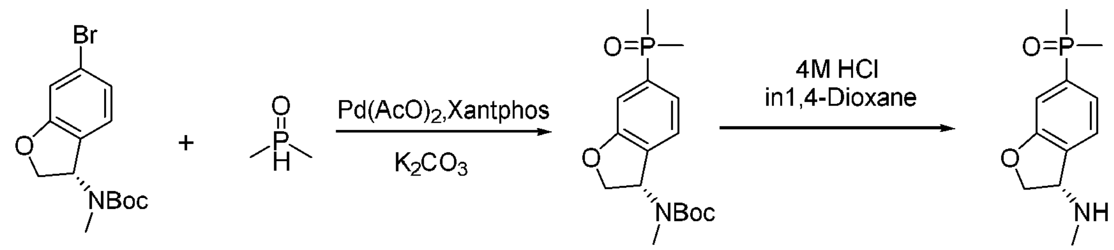


Step 1: tert-butyl (S)-(6-(dimethylphosphoryl)-2,3-dihydrobenzofuran-3-yl)(methyl)carbamate

tert-butyl (S)-(6-bromo-2,3-dihydrobenzofuran-3-yl)(methyl)carbamate (200 mg, 0.61 mmol), dimethyl oxophosphorane (57 mg, 0.73 mmol), potassium carbonate (101 mg, 0.73 mmol), palladium acetate (10 mg, 0.06 mmol), 4,5-bis(diphenylphosphino)-9,9-dimethylxanthene (35 mg, 0.06 mmol), and 5 mL of dimethylformamide were reacted in a microwave reactor at 150°C for 20 minutes. After completion of the reaction, the system was cooled to room temperature, and the mixture was extracted three times with ethyl acetate (30 mL in total, 10 mL each time). The combined organic phases were washed with saturated brine and dried over anhydrous sodium sulfate. After concentration under reduced pressure, tert-butyl (S)-(6-(dimethylphosphoryl)-2,3-dihydrobenzofuran-3-yl)(methyl)carbamate (80 mg, 0.245 mmol, 40.35% yield) was yielded, a white solid.
LCMS (ESI): 326 $[M+H]^+$

Step 2: (S)-dimethyl(3-(methylamino)-2,3-dihydrobenzofuran-6-yl)phosphine oxide

tert-butyl (S)-(6-(dimethylphosphoryl)-2,3-dihydrobenzofuran-3-yl)(methyl)carbamate (80 mg, 0.25 mmol) was added to a solution of 4 M hydrochloric acid in 1,4-dioxane (3 mL) and stirred for 2 hours. After completion of the reaction, the resulting mixture was concentrated under reduced pressure. This yielded (S)-dimethyl(3-(methylamino)-2,3-dihydrobenzofuran-6-yl)phosphine oxide (25 mg, 0.11 mmol, 45.14% yield), a white solid.
LCMS (ESI): 226$[M+H]^+$

Int. 25: (S)-N-methyl-6-(methylsulfonyl)-2,3-dihydrobenzofuran-3-amine

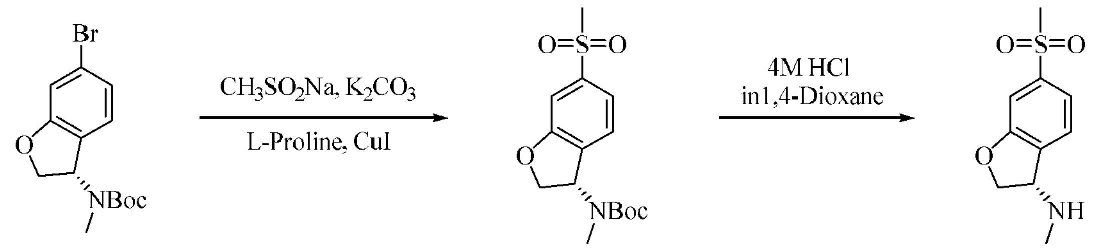


Step 1: synthesis of tert-butyl (S)-methyl(6-(methylsulfonyl)-2,3-dihydrobenzofuran-3-yl)carbamate

tert-butyl (S)-(6-bromo-2,3-dihydrobenzofuran-3-yl)(methyl)carbamate (328 mg, 1 mmol), sodium methanesulfinate (122 mg, 1.2 mmol), L-proline (12 mg, 0.1 mmol), potassium carbonate (166 mg, 1.2 mmol), cuprous iodide (19 mg, 0.1 mmol), and N,N-dimethylformamide (5 mL) were reacted in a microwave reactor at 140°C for 2 hours. After completion of the reaction, the system was cooled to room temperature, and the mixture was extracted three times with ethyl acetate (30 mL in total, 10 mL each time). The combined organic phases were washed with saturated brine and dried over anhydrous sodium sulfate. After concentration under reduced pressure, tert-butyl (S)-methyl(6-(methylsulfonyl)-2,3-dihydrobenzofuran-3-yl)carbamate(60 mg, 0.18 mmol, 18.32% yield) was yielded, a colorless oily substance.
LCMS (ESI): 328 $[M+H]^+$

Step 2: synthesis of (S)-N-methyl-6-(methylsulfonyl)-2,3-dihydrobenzofuran-3-amine

tert-butyl (S)-methyl(6-(methylsulfonyl)-2,3-dihydrobenzofuran-3-yl)carbamate (60 mg, 0.24 mmol) was added to a solution of 4 M hydrochloric acid in 1,4-dioxane (3 mL) and stirred for 2 hours. After completion of the reaction, the

resulting mixture was concentrated under reduced pressure. This yielded synthesis of (S)-N-methyl-6-(methylsulfonyl)-2,3-dihydrobenzofuran-3-amine (30 mg, 0.13 mmol, 72.03% yield), a white solid.

LCMS (ESI): 228[M+H]+

Int. 101 Synthesis of (S)-6-(cyclopropylsulfonyl)-N-methyl-2,3-dihydrobenzofuran-3-amine

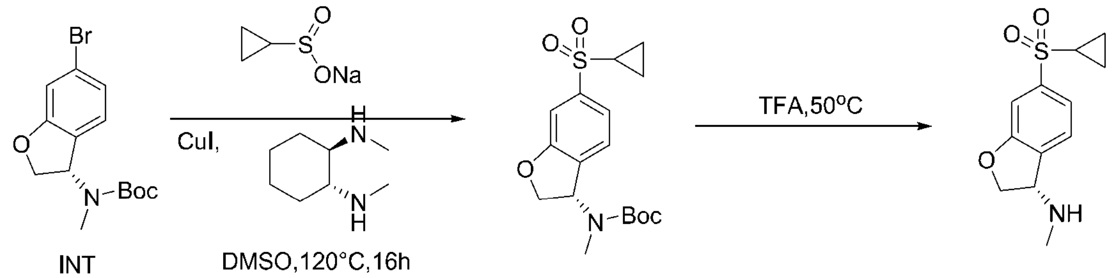


Step 1: Synthesis of tert-butyl (S)-(6-(cyclopropylsulfonyl)-2,3-dihydrobenzofuran-3-yl)(methyl)carbamate

tert-butyl (S)-(6-bromo-2,3-dihydrobenzofuran-3-yl)(methyl)carbamate (300 mg, 0.914 mmol) and sodium cyclopropanesulfinate (118 mg, 0.914 mmol) were dissolved in DMSO (10 mL). Copper(I) iodide (35 mg, 0.182 mmol) and (1R,2R)-N1,N2-dimethylcyclohexane-1,2-diamine (52 mg, 0.365 mmol) were added, and the reaction mixture was stirred at 25°C for 10 hours. After LCMS indicated complete reaction, water (10 mL) was added, followed by extraction with ethyl acetate (20 mL × 2). The combined organic phases were washed with anhydrous sodium sulfate, filtered, and concentrated under reduced pressure. Purification by column chromatography (0-10% ethyl acetate/petroleum ether) yielded Synthesis of tert-butyl (S)-(6-(cyclopropylsulfonyl)-2,3-dihydrobenzofuran-3-yl)(methyl)carbamate (150 mg, 46.4% yield).
LCMS (ESI) m/z: 354.1 [M+H]+

Step 2: Synthesis of (S)-6-(cyclopropylsulfonyl)-N-methyl-2,3-dihydrobenzofuran-3-amine

At room temperature, compound tert-butyl (S)-(6-(cyclopropylsulfonyl)-2,3-dihydrobenzofuran-3-yl)(methyl) carbamate(150 mg, 0.424 mmol) was dissolved in dichloromethane (3 mL), and trifluoroacetic acid (1 mL) was added. The reaction mixture was stirred at room temperature for 16 hours, and the crude product (S)-6-(cyclopropylsulfonyl)-N-methyl-2,3-dihydrobenzofuran-3-amine(150 mg, crude) was obtained by concentration under reduced pressure, without the need for purification, and directly used for the next step.
LCMS (ESI) m/z: 254.1[M+H]+

Int. 26 synthesis of (S)-N-methyl-6-(1-(trifluoromethyl)-1H-pyrazol-4-yl)-2,3-dihydrobenzofuran-3-amine

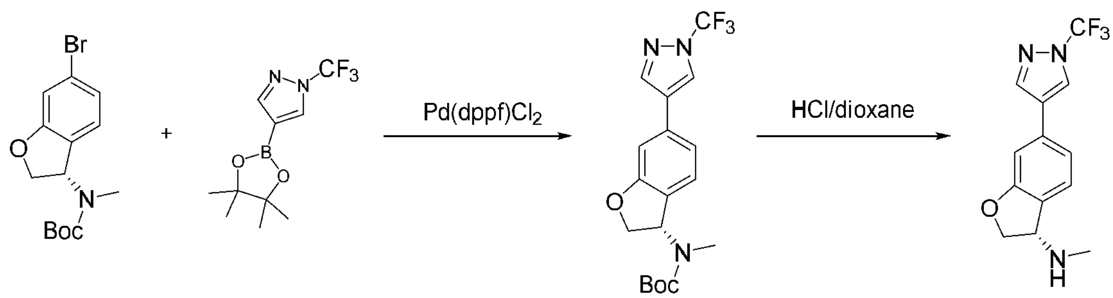


Step 1: Synthesis of tert-butyl (S)-methyl(6-(1-(trifluoromethyl)-1H-pyrazol-4-yl)-2,3-dihydrobenzofuran-3-yl)carbamate

Under $N_2$ atmosphere, tert-butyl (S)-(6-bromo-2,3-dihydrobenzofuran-3-yl)(methyl)carbamate(300 mg, 0.91

mmol) was dissolved in 1,4-dioxane and water (4:1, 10 mL), and 4-(4,4,5,5-tetramethyl-1,3,2-dioxaborolan-2-yl)-1-(trifluoromethyl)-1H-pyrazole (239.0 mg, 1.37 mmol), potassium carbonate (377 mg, 2.73 mmol), and 1,1'-bis(di-tert-butylphosphino)ferrocene palladium dichloride (67.0 mg, 0.09 mmol) were added. The reaction was carried out at 100°C for 16 hours. The mixture was then poured into water (30 mL), extracted with ethyl acetate (20 mL × 3), and the combined organic phase was dried over anhydrous sodium sulfate. After filtration to remove sodium sulfate, the filtrate was rotary evaporated to obtain the crude product. Purification by silica gel column chromatography (petroleum ether: ethyl acetate = 10:1) yielded tert-butyl (S)-methyl(6-(1-(trifluoromethyl)-1H-pyrazol-4-yl)-2,3-dihydrobenzofuran-3-yl) carbamate(240.0 mg, 68.8% yield), a white solid.
LCMS (ESI) m/z: 384.1 $[M+H]^+$

Step 2: Synthesis of (S)-N-methyl-6-(1-(trifluoromethyl)-1H-pyrazol-4-yl)-2,3-dihydrobenzofuran-3-amine

At room temperature, tert-butyl

(S)-methyl(6-(1-(trifluoromethyl)-1H-pyrazol-4-yl)-2,3-dihydrobenzofuran-3-yl)carbamate(240 mg, 0.626 mmol) was dissolved in 4 M hydrochloric acid in ethyl acetate (3 mL). After stirring at room temperature for 1 hour, the reaction mixture was concentrated under reduced pressure to obtain the crude product,
(S)-N-methyl-6-(1-(trifluoromethyl)-1H-pyrazol-4-yl)-2,3-dihydrobenzofuran-3-amine(25.0 mg, crude product).
LCMS (ESI) m/z:283.1 $[M+H]^+$

Using the methods and steps employed for Intermediate 26, with only the replacement of the corresponding raw materials, the following intermediates were synthesized:

| Int. No. | Structure | name | m/z(ESI): $(M+H)^+$ |
|---|---|---|---|
| 27 | 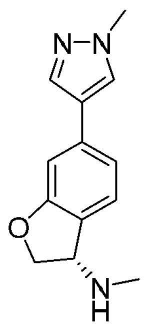 | (S)-N-methyl-6-(1-methyl-1H-pyrazol-4-yl)-2,3-dihydrobenzofuran-3-amine | 230.1 |
| 28 | 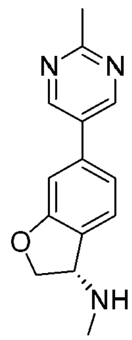 | (S)-N-methyl-6-(2-methylpyrimidin-5-yl)-2,3-dihydrobenzofuran-3-amine | 242.1 |
| 29 | 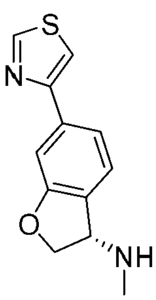 | (S)-N-methyl-6-(thiazol-4-yl)-2,3-dihydrobenzofuran-3-amine | 202.1 |

(continued)

| Int. No. | Structure | name | m/z(ESI): (M+H)+ |
|---|---|---|---|
| 30 | 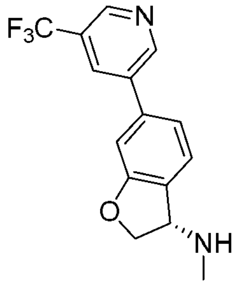 | (S)-N-methyl-6-(5-(trifluoromethyl)pyridin-3-yl)-2,3-dihydrobenzofuran-3-amine | 295.1 |
| 31 | 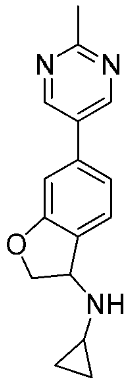 | N-cyclopropyl-6-(2-methylpyrimidin-5-yl)-2,3-dihydrobenzofuran-3-amine | 268.1 |
| 32 | 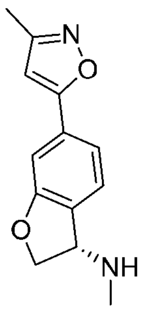 | (S)-N-methyl-6-(3-methylisoxazol-5-yl)-2,3-dihydrobenzofuran-3-amine | 231.1 |
| 33 | 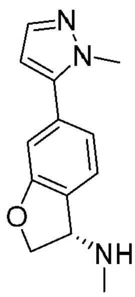 | (S)-N-methyl-6-(1-methyl-1H-pyrazol-5-yl)-2,3-dihydrobenzofuran-3-amine | 230.2 |
| 34 | 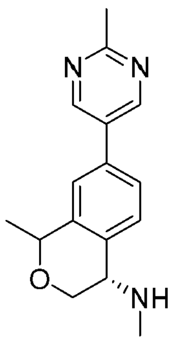 | (4S)-N,1-dimethyl-7-(2-methylpyrimidin-5-yl)isochroman-4-amine | 270.2 |

(continued)

| Int. No. | Structure | name | m/z(ESI): (M+H)$^+$ |
|---|---|---|---|
| 35 | 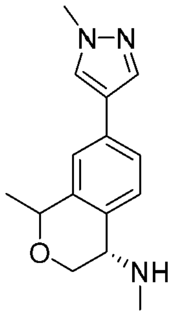 | (4S)-N,1-dimethyl-7-(1-methyl-1H-pyrazol-4-yl)isochroman-4-amine | 258.2 |
| 36 | 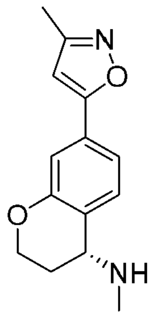 | (R)-N-methyl-7-(3-methylisoxazol-5-yl)chroman-4-amine | 245.2 |
| 37 | 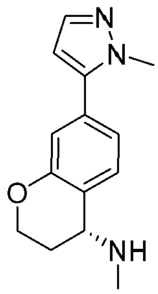 | (R)-N-methyl-7-(1-methyl-1H-pyrazol-5-yl)chroman-4-amine | 244.2 |
| 38 | 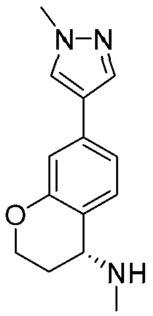 | (R)-N-methyl-7-(1-methyl-1H-pyrazol-4-yl)chroman-4-amine | 244.2 |
| 39 | 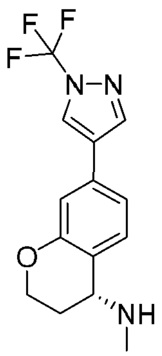 | (R)-N-methyl-7-(1-(trifluoromethyl)-1H-pyrazol-4-yl)chroman-4-amine | 298.2 |

(continued)

| Int. No. | Structure | name | m/z(ESI): $(M+H)^+$ |
|---|---|---|---|
| 40 | 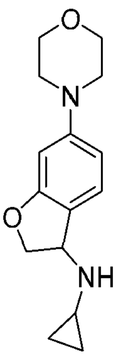 | N-cyclopropyl-6-morpholino-2,3-dihydrobenzofuran-3-amine | 261.2 |
| 103 | 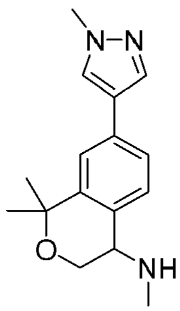 | N,1,1-trimethyl-7-(1-methyl-1H-pyrazol-4-yl)isochroman-4-amine | 272.17 |
| 104 | 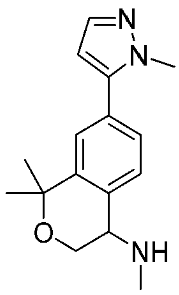 | N,1,1-trimethyl-7-(1-methyl-1H-pyrazol-5-yl)isochroman-4-amine | 272.17 |

Int. 41 synthesis of 6-(trifluoromethyl)benzo[b]thiophen-3(2H)-one

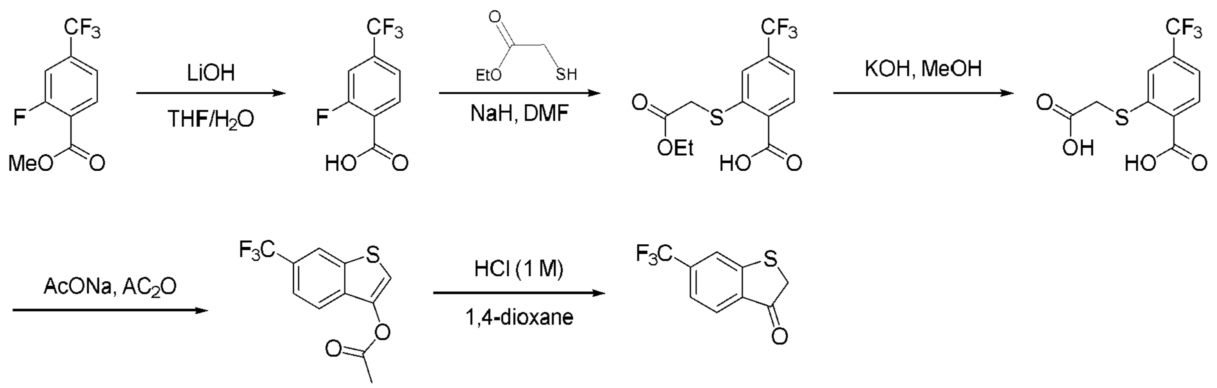


Step 1: Synthesis of 2-fluoro-4-(trifluoromethyl)benzoic acid

At room temperature, lithium hydroxide (9.70 g, 405.15 mmol) is added to a solution of methyl 2-fluoro-4-(trifluoromethyl)benzoate (30.00 g, 135.05 mmol) in tetrahydrofuran:water (300 mL:30 mL). The mixture is stirred at 50 °C for 2 hours. The reaction mixture is poured into water (100 mL), and the pH is adjusted to 4 with formic acid. After extraction with ethyl acetate (100 mL × 3), drying the organic layer over anhydrous sodium sulfate, filtration, and concentration, the crude product is purified by rapid silica gel column chromatography (petroleum ether:ethyl acetate = 30%) to obtain 2-fluoro-4-(trifluoromethyl)benzoic acid (22.00 g, yield: 78%).
LCMS (ESI) m/z: 209.1 $[M+H]^+$

Step 2: Synthesis of 2-((2-ethoxy-2-oxoethyl)thio)-4-(trifluoromethyl)benzoic acid

At 0 °C, sodium hydride (8.46 g, 211.43 mmol) was added to a solution of ethyl 2-mercaptoacetate (12.70 g, 105.72 mmol) in N,N-dimethylformamide (200.00 mL). The mixture was stirred at room temperature for 0.5 hours. Then, 2-fluoro-4-(trifluoromethyl)benzoic acid (22.00 g, 105.71 mmol) is added to the solution, and the mixture is stirred for 1 hour at room temperature. The reaction mixture is slowly poured into water (100 mL), extracted with ethyl acetate (200 mL), dried over anhydrous sodium sulfate, filtered, and concentrated. The crude product is purified by rapid silica gel column chromatography (petroleum ether:ethyl acetate = 10%) to yield 2-((2-ethoxy-2-oxoethyl)thio)-4-(trifluoromethyl)benzoic acid (13.00 g, yield: 40%).
LCMS (ESI) m/z: 309.2 $[M+H]^+$

Step 3: synthesis of 2-((carboxymethyl)thio)-4-(trifluoromethyl)benzoic acid

The mixture of 2-((2-ethoxy-2-oxoethyl)thio)-4-(trifluoromethyl)benzoic acid (13.00 g, 42.17 mmol) and potassium hydroxide (4.72 g, 84.34 mmol) in methanol (130.00 mL) was stirred at room temperature for 2 hours, then concentrated to obtain 2-((carboxymethyl)thio)-4-(trifluoromethyl)benzoic acid(7.00 g, crude).
LCMS (ESI) m/z: 281.2 $[M+H]^+$

Step 4: synthesis of 6-(trifluoromethyl)benzo[b]thiophen-3-yl acetate

At room temperature, sodium acetate trihydrate (10.19 g, 74.94 mmol) was added to a solution of 2-((carboxymethyl)thio)-4-(trifluoromethyl)benzoic acid (7.00 g, 24.98 mmol) in acetic anhydride (70.00 mL). The mixture was stirred at 140°C for 30 minutes. Afterward, the mixture was poured into water, extracted with ethyl acetate (100 mL), dried over anhydrous sodium sulfate, filtered, and concentrated. Purification by quick column chromatography (petroleum ether:ethyl acetate = 50%) yielded 6-(trifluoromethyl)benzo[b]thiophen-3-yl acetate (5.00 g, yield: 77%).
LCMS (ESI) m/z:261.2 $[M+H]^+$

Step 5: synthesis of 6-(trifluoromethyl)benzo[b]thiophen-3(2H)-one

At room temperature, hydrochloric acid solution (10.00 mL, 1 M) was added to a solution of 6-(trifluoromethyl)benzo[b]thiophen-3-yl acetate (1.00 g, 3.84 mmol) in 1,4-dioxane (5.00 mL). The mixture was stirred at 100°C for 2 hours. After cooling, the reaction mixture was poured into water, and the aqueous layer was extracted with ethyl acetate (2 × 200 mL). The combined organic layers were washed with saturated brine (300 mL), dried over anhydrous sodium sulfate, concentrated to obtain the crude product. Purification by quick column chromatography (petroleum ether:ethyl acetate = 70%) yielded 6-(trifluoromethyl)benzo[b]thiophene-3(2H)-one (0.50 g, yield: 59%), a black solid.
LCMS (ESI) m/z:219.2 $[M+H]^+$

Int. 42 Synthesis of 1-methyl-7-(trifluoromethyl)isochroman-4-one

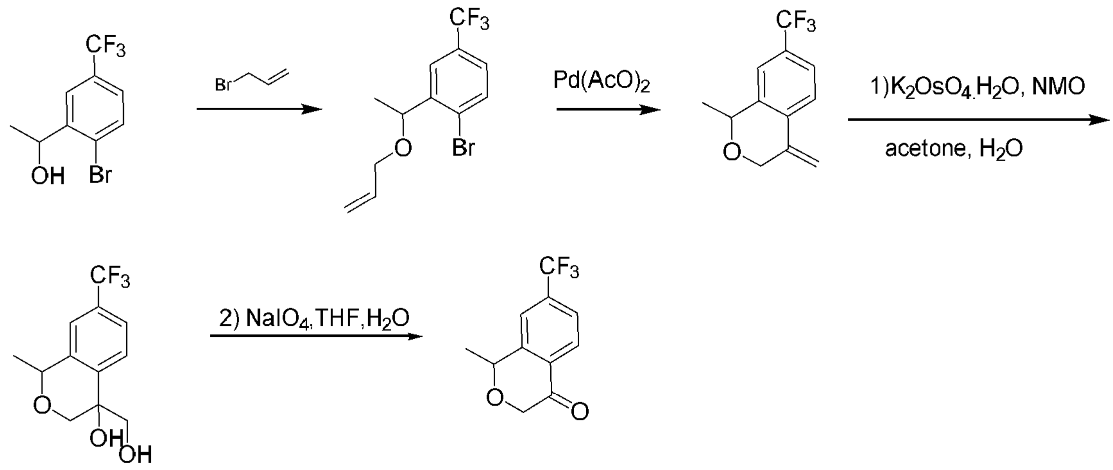


Step 1: Synthesis of 2-(1-(allyloxy)ethyl)-1-bromo-4-(trifluoromethyl)benzene

At room temperature, 1-(2-bromo-5-(trifluoromethyl)phenyl)ethan-1-ol (10 g, 37.16 mmol) was dissolved in tetrahydrofuran (100 mL), followed by the addition of potassium hydroxide (4.2 g, 74.33 mmol), tetrabutylammonium hydrogen sulfate (2.6 g, 7.433 mmol), and 3-bromopropene (5.4 g, 44.60 mmol). The reaction proceeded for 4 hours at 25°C. After completion, the reaction mixture was concentrated under reduced pressure and poured into water (100 mL). The aqueous layer was extracted with ethyl acetate (3 × 100 mL), and the combined organic phases were dried over anhydrous sodium sulfate, filtered, and concentrated under reduced pressure to obtain the crude product (12 g, crude). No further purification was needed, and it was used directly for the next step.
LCMS (ESI) m/z: 309.2 $[M+H]^+$

Step 2: synthesis of 1-methyl-4-methylene-7-(trifluoromethyl)isochromane

2-(1-(allyloxy)ethyl)-1-bromo-4-(trifluoromethyl)benzene (12 g, 38.82 mmol) was dissolved in DMF (100 mL), and cesium carbonate (15.5 g, 46.59 mmol), tri(o-tolyl)phosphine (5.1 g, 19.41 mmol), and palladium acetate (0.89 g, 3.882 mmol) were added. The mixture was stirred at 90°C under a nitrogen atmosphere for 16 hours. The reaction mixture was poured into 400 mL of water, extracted with ethyl acetate (3 × 100 mL), the combined organic phases were dried over anhydrous sodium sulfate, concentrated under reduced pressure to obtain the crude product, and further purified by column chromatography (petroleum ether:ethyl acetate = 10:1) to yield 1-methyl-4-methylene-7-(trifluoromethyl)isochromane (5.5 g, yield: 62.1%).
LCMS (ESI) m/z: 229.1 $[M+H]^+$

Step 3: Synthesis of 4-(hydroxymethyl)-1-methyl-7-(trifluoromethyl)isochroman-4-ol

At room temperature, 1-methyl-4-methylene-7-(trifluoromethyl)isochromane (5.5 g, 24.10 mmol) was dissolved in a mixed solvent of acetone (100 mL) and water (20 mL). N-Methylmorpholine N-oxide (9.2 g, 76.68 mmol) and potassium ruthenate (0.91 g, 2.410 mmol) were added. Under a nitrogen atmosphere, the mixture was stirred at 25°C for 16 hours. After completion of the reaction, sodium sulfite solid (5 g) was added to the reaction mixture and stirred for ten minutes. The solution was concentrated under reduced pressure to remove a certain amount of acetone, poured into water (200 mL), extracted with ethyl acetate (3 × 100 mL), the combined organic phases were dried over anhydrous Na2SO4, concentrated under reduced pressure to obtain the crude product, and further purified to yield 4-(hydroxymethyl)-1-methyl-7-(trifluoromethyl)isochroman-4-ol(6.0 g, yield: 94.9%).
LCMS (ESI) m/z: 263.0 $[M+H]^+$

Step 4: Synthesis of 1-methyl-7-(trifluoromethyl)isochroman-4-one

At room temperature, 4-(hydroxymethyl)-1-methyl-7-(trifluoromethyl)isochroman-4-ol (6.0 g, 22.88 mmol) was dissolved in a mixed solvent of tetrahydrofuran (100 mL) and water (3.5 mL). Sodium periodate (15.0 g, 68.64 mmol) was added under a nitrogen atmosphere, and the mixture was stirred at 25°C for 4 hours. After completion of the reaction, the reaction mixture was diluted with ethyl acetate, filtered, washed, the combined filtrate was dried over anhydrous sodium sulfate, filtered, and the filtrate was concentrated under reduced pressure to obtain the crude product 1-methyl-7-(trifluoromethyl)isochroman-4-one (5 g, crude product).

LCMS (ESI) m/z:230.2 [M+H]+

Using the methods and steps employed for Intermediate42, with only the replacement of the corresponding raw materials, the following intermediates were synthesized:

| Int. No. | Structure | Name | m/z(ESI): (M+H)+ |
|---|---|---|---|
| 43 | 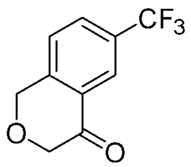 | 6-(trifluoromethyl)isochroman-4-one | 217.2 |
| 44 | 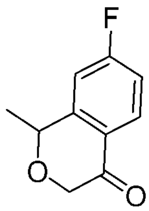 | 7-fluoro-1-methylisochroman-4-one | 180.2 |
| 45 | 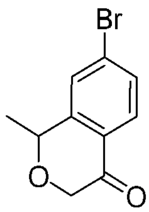 | 7-bromo-1-methylisochroman-4-one | 242.1 |
| 46 | 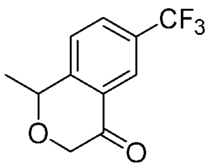 | 1-methyl-6-(trifluoromethyl)isochroman-4-one | 230.2 |
| 47 | 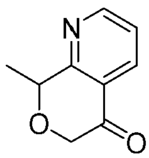 | 8-methyl-6H-pyrano[3,4-b]pyridin-5(8H)-one | 164.2 |
| 48 | 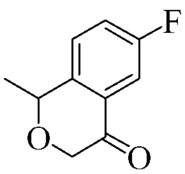 | 6-fluoro-1-methylisochroman-4-one | 181 |
| 49 | 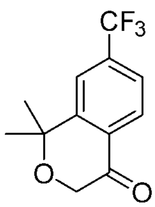 | 1,1-dimethyl-7-(trifluoromethyl)isochroman-4-one | 245.2 |

The following ketones were purchased from different suppliers:

| Int. No. | Structure | Name | CAS |
|---|---|---|---|
| 50 | 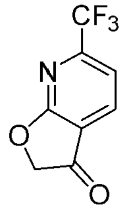 | 6-(trifluoromethyl)furo[2,3-b]pyridin-3(2H)-one | 1196155-86-8 |
| 51 | 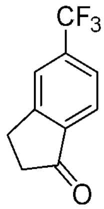 | 5-(trifluoromethyl)-2,3-dihydro-1H-inden-1-one | 150969-56-5 |
| 52 | 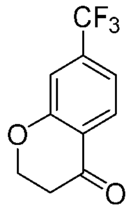 | 7-(trifluoromethyl)chroman-4-one | 111141-02-7 |
| 53 | 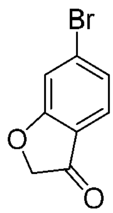 | 6-bromobenzofuran-3(2H)-one | 3260-78-4 |
| 54 | 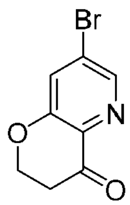 | 7-bromo-2,3-dihydro-4H-pyrano[3,2-b]pyridin-4-one | 1624261-71-7 |
| 102 | 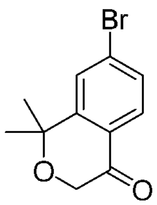 | 7-bromo-1,1-dimethylisochroman-4-one | 2763779-54-8 |

Int. 55 N-methyl-6-(trifluoromethyl)isochroman-4-amine hydrochloride

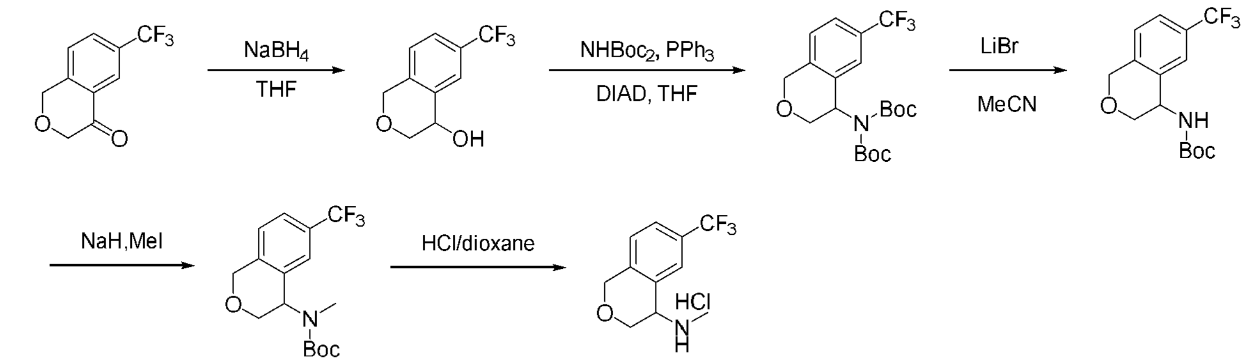


Step 1: Synthesis of 6-(trifluoromethyl)isochroman-4-ol

At room temperature, 6-(trifluoromethyl)isochroman-4-one (1.1 grams, 5.09 mmol) was dissolved in THF (10 mL), followed by the addition of sodium borohydride (251 mg, 6.62 mmol) in an ice bath, and then 2 drops of methanol were slowly added. After complete addition, the reaction was left overnight at room temperature. Upon completion, the reaction was quenched with a solution of 1 M hydrochloric acid (2 mL), extracted with ethyl acetate (10 mL × 2), then the organic phases were combined, dried over sodium sulfate, then filtered, and obtained the crude product, which was further purified by column chromatography to yield 6-(trifluoromethyl)isochroman-4-ol(1.16 grams).

Step 2: synthesis of tert-butyl (tert-butoxycarbonyl)(6-(trifluoromethyl)isochroman-4-yl)carbamate

At room temperature, 6-(trifluoromethyl)isochroman-4-ol (100.0 mg, 0.459 mmol) was dissolved in THF (5.0 mL), then di-tert-butyl dicarbonate (110 mg, 0.505 mmol) and triphenylphosphine (132 mg, 0.505 mmol) were added successively. The reaction mixture was stirred at 0°C for 5 minutes, then DIAD (102 mg, 0.505 mmol) was added, and the reaction was stirred at room temperature overnight. After completion, water (10 mL) was added. And the reaction mixture was extracted with ethyl acetate (10 mL × 3). Then organic phases were combined, dried over anhydrous sodium sulfate, filtered. And the resulting filtrate was concentrated under reduced pressure to obtain the crude product. Further purification by column chromatography (pure petroleum ether) yielded tert-butyl (tert-butoxycarbonyl) (6-(trifluoromethyl) isochroman-4-yl)carbamate (90.0 mg).

Step 3: Synthesis of tert-butyl (6-(trifluoromethyl)isochroman-4-yl)carbamate

Tert-butyl (tert-butoxycarbonyl)(6-(trifluoromethyl)isoxazol-4-yl)methylamine (60.0 mg, 0.14 mmol) was dissolved in acetonitrile (2.0 mL), then lithium bromide (37.6 mg, 0.432 mmol) was added. The reaction mixture was heated at 60°C for 20 hours. After completion, saturated sodium bicarbonate (10 mL) was added, and extracted with ethyl acetate (20 mL). Then the organic phase was dried with sodium sulfate, filtered. The resulting filtrate was concentrated under reduced pressure to obtain (6-(trifluoromethyl)isochroman-4-yl)carbamate (30 mg).

Step 4: Synthesis of tert-butyl methyl(6-(trifluoromethyl)isochroman-4-yl)carbamate

(6-(trifluoromethyl)isochroman-4-yl)carbamate( 360.0 mg, 1.14 mmol) was dissolved in 5.0 mL of DMF and stirred in an ice bath for 2 minutes. Sodium hydride (90.8 mg, 2.27 mmol) was added at this temperature and stirred for 1 hour. Then, methyl iodide (487 mg, 3.41 mmol) was added, and the reaction mixture was stirred overnight at room temperature. After completion, saturated sodium bicarbonate (15 mL) was added, extracted with ethyl acetate (15 mL × 3), dried the organic phase with sodium sulfate, then filtered, and the filtrate was concentrated to obtain the crude product tert-butyl methyl(6-(trifluoromethyl)isochroman-4-yl)carbamate (400.0 mg).

Step 5: Synthesis of N-methyl-6-(trifluoromethyl)isochroman-4-amine hydrochloride

At room temperature, tert-butyl methyl(6-(trifluoromethyl)isochroman-4-yl)carbamate (400 mg, 1.21 mmol) was dissolved in 2 mL of dichloromethane, followed by addition of a solution of 4 M hydrochloric acid in dioxane (2 mL). The reaction was stirred overnight at room temperature. After completion, the reaction mixture was directly evaporated to obtain N-methyl-6-(trifluoromethyl)isochroman-4-amine hydrochloride(390mg).

Using the methods and steps employed for Intermediate 55, with only the replacement of the corresponding raw materials, the following intermediates were synthesized:

| Int. No. | Structure | Name | m/z(ESI): $(M+H)^+$ |
|---|---|---|---|
| 56 | 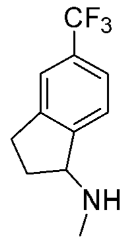 | N-methyl-5-(trifluoromethyl)-2,3-dihydro-1H-inden-1-amine | 216.2 |
| 57 | 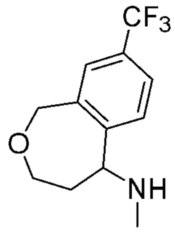 | N-methyl-8-(trifluoromethyl)-1,3,4,5-tetrahydrobenzo[c]oxepin-5-amine | 246.2 |
| 58 | 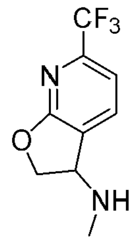 | N-methyl-6-(trifluoromethyl)-2,3-dihydrofuro[2,3-b]pyridin-3-amine | 219.2 |
| 59 | 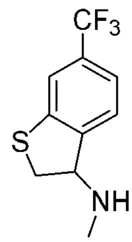 | N-methyl-6-(trifluoromethyl)-2,3-dihydrobenzo[b]thiophen-3-amine | 234.2 |

Int 60 Synthesis of (R)-N-methyl-7-(trifluoromethyl)chroman-4-amine hydrochloride

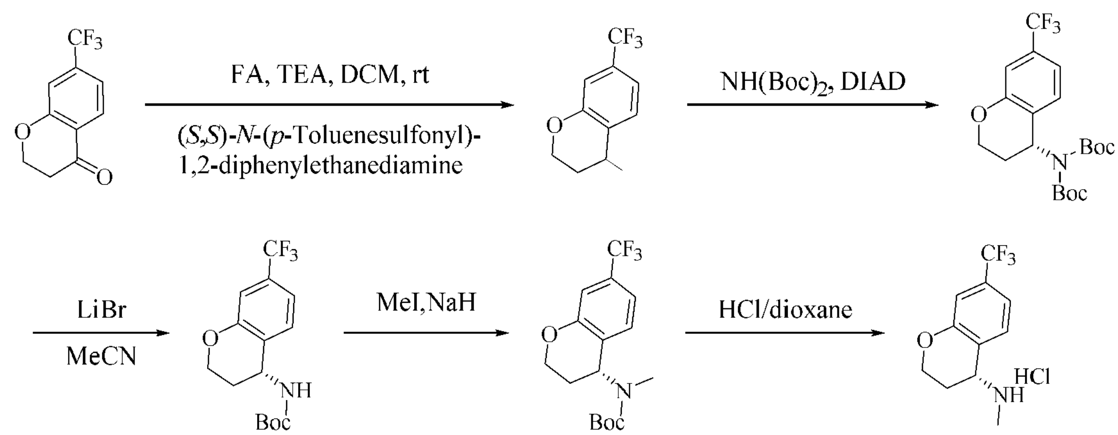

Step 1: Synthesis of (S)-7-(trifluoromethyl)chroman-4-ol

At room temperature, 7-(trifluoromethyl)chroman-4-one (500.0 mg, 2.31 mmol) was dissolved in DCM (10 mL), followed by the addition of (S, S)-N-(p-toluenesulfonyl)-1,2-diphenylethane-1,2-diamine (147.0 mg, 0.23 mmol) cooled by an ice bath. Then, formic acid (372.0 mg, 8.10 mmol) was added dropwise, and finally, triethylamine (701.0 mg, 6.94 mmol) was added dropwise. After completion, the reaction mixture was left overnight at room temperature. Upon completion, saturated sodium bicarbonate solution (10 mL) was added, and the mixture was extracted with ethyl acetate (10 mL × 2). After combining the organic phases, drying over sodium sulfate, and filtration, the crude product was obtained. Purification by column chromatography (petroleum ether:ethyl acetate = 0-40%) yielded (S)-7-(trifluoromethyl)chroman-4-ol(370 mg, yield: 73%).

Step 2: Synthesis of tert-butyl (R)-(tert-butoxycarbonyl)(7-(trifluoromethyl)chroman-4-yl)carbamate

$^1$H NMR (400 MHz, $CDCl_3$) δ 7.25-7.22(m, 1H), 7.12-7.09(m, 1H), 7.07(d, 1H), 5.59-5.54(m, 1H), 4.46-4.41(m, 1H), 4.18-4.10(m, 1H), 2.67-2.60(m, 1H), 2.16-2.10(m, 1H), 1.46(s, 18H).

Under a nitrogen atmosphere, (S)-7-(trifluoromethyl)chroman-4-ol (370.0 mg, 1.70 mmol) was first dissolved in THF (5.0 mL), and then di-tert-butyl iminodicarbonate (405 mg, 1.88 mmol) and triphenylphosphine (489 mg, 1.88 mmol) were added. The reaction mixture was stirred for 5 minutes at 0 °C, and DIAD (377.0 mg, 1.88 mmol) was added dropwise. After stirring at room temperature overnight, the reaction was complete. Water (10 mL) was added, and the mixture was extracted with ethyl acetate (10 mL × 3). After combining the organic phases, drying over anhydrous sodium sulfate, and filtration, the filtrate was concentrated under reduced pressure to obtain the crude product. Further purification by column chromatography (petroleum ether:ethyl acetate = 0-5%) resulted in tert-butyl (R)-(tert-butoxycarbonyl)(7-(trifluoromethyl) chroman-4-yl)carbamate (145.0 mg, yield: 20%).

$^1$H NMR (400 MHz, $CDCl_3$) δ 7.25-7.22(m, 1H), 7.12-7.09(m, 1H), 7.07(d, 1H), 5.59-5.54(m, 1H), 4.46-4.41(m, 1H), 4.18-4.10(m, 1H), 2.67-2.60(m, 1H), 2.16-2.10(m, 1H), 1.46(s, 18H).

Step 3: Synthesis of tert-butyl (R)-(7-(trifluoromethyl)chroman-4-yl)carbamate

tert-butyl (R)-(tert-butoxycarbonyl)(7-(trifluoromethyl)chroman-4-yl)carbamate (150.0 mg, 0.36 mmol) was dissolved in acetonitrile (5.0 mL), and lithium bromide (94.0 mg, 1.08 mmol) was added. The reaction mixture was heated at 60°C for 20 hours. After completion of the reaction, saturated sodium bicarbonate solution (10 mL) was added, and the mixture was extracted with ethyl acetate (20 mL). After drying the organic phase over sodium sulfate and filtration, the filtrate was concentrated to obtain the crude product, tert-butyl (R)-(7-(trifluoromethyl)chroman-4-yl)carbamate(83.0 mg, crude product).

Step 4: Synthesis of tert-butyl (R)-methyl(7-(trifluoromethyl)chroman-4-yl)carbamate

tert-butyl (R)-(7-(trifluoromethyl)chroman-4-yl)carbamate(83.0 mg, 0.26 mmol) was dissolved in 2.0 mL DMF. The mixture was stirred cooled by an ice bath for 2 minutes, and sodium hydride (26.2 mg, 0.66 mmol) was added in one portion. The reaction mixture was stirred at this temperature for 1 hour, followed by the addition of iodomethane (74.4 mg, 0.52 mmol); the reaction continued overnight at room temperature. After completion of the reaction, saturated sodium bicarbonate solution (10 mL) was added, and the mixture was extracted with ethyl acetate (15 mL). The organic phase was dried over sodium sulfate, filtered, and the filtrate was concentrated to obtain the crude product, tert-butyl (R)-methyl(7-(trifluoromethyl)chroman-4-yl)carbamate (100.0 mg, crude product).

LCMS (ESI) m/z:276.1$[M+H]^+$

Step 5: Synthesis of (R)-N-methyl-7-(trifluoromethyl)chroman-4-amine hydrochloride

tert-butyl (R)-methyl(7-(trifluoromethyl)chroman-4-yl)carbamate (100 mg, 0.3 mmol) was dissolved in 2 mL of dichloromethane, and a solution of 4 M hydrogen chloride in dioxane (2 mL) was added. The reaction was stirred at room temperature overnight. After completion of the reaction, the reaction mixture was directly evaporated to dryness, obtaining the crude product, (R)-N-methyl-7-(trifluoromethyl)chroman-4-amine hydrochloride (90 mg, crude product).

Using the methods and steps employed for Intermediate 60, with only the replacement of the corresponding raw materials, the following intermediates were synthesized:

| Int. No. | Structure | Name | m/z(ESI): (M+H)+ |
|---|---|---|---|
| 61 | 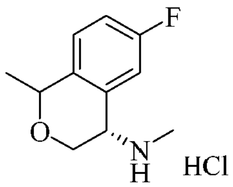 | (4S)-6-fluoro-N,1-dimethylisochroman-4-amine hydrochloride | 196.2 |
| 62 | 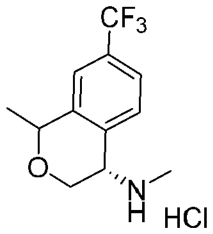 | (4S)-N,1-dimethyl-7-(trifluoromethyl)isochroman-4-amine hydrochloride | 246.2 |
| 63 | 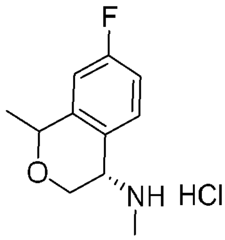 | (4S)-7-fluoro-N,1-dimethylisochroman-4-amine hydrochloride | 196.2 |
| 64 | 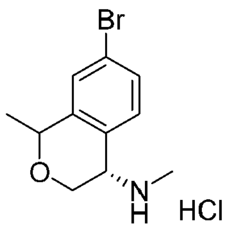 | (4S)-7-bromo-N,1-dimethylisochroman-4-amine hydrochloride | 256.2 |
| 65 | 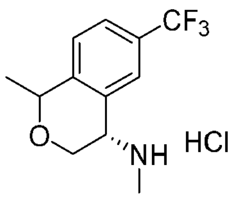 | (4S)-N,1-dimethyl-6-(trifluoromethyl)isochroman-4-amine hydrochloride | 246.2 |
| 66 | 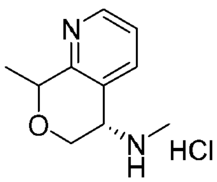 | (5S)-N,8-dimethyl-5,8-dihydro-6H-pyrano[3,4-b]pyridin-5-amine hydrochloride | 179.2 |
| 67 | 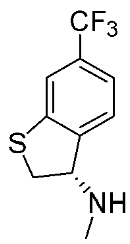 | (S)-N-methyl-6-(trifluoromethyl)-2,3-dihydrobenzo[b]thiophen-3-amine | 234.2 |

(continued)

| Int. No. | Structure | Name | m/z(ESI): (M+H)+ |
|---|---|---|---|
| 68 | 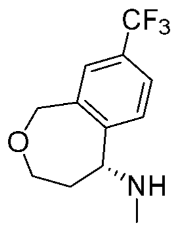 | (R)-N-methyl-8-(trifluoromethyl)-1,3,4,5-tetrahydrobenzo[c] oxepin-5-amine | 246.2 |

**Int. 100 Synthesis of N,1,1-trimethyl-7-(trifluoromethyl)isochroman-4-amine**

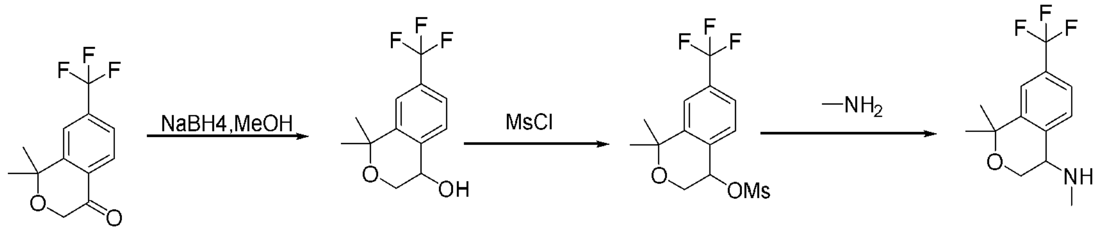


Step 1: Synthesis of 1,1-dimethyl-7-(trifluoromethyl)isochroman-4-ol

1,1-dimethyl-7-(trifluoromethyl)isochroman-4-one (300 mg, 1.229 mmol) was dissolved in MeOH (3 mL) and Sodium borohydride (70 mg, 1.844 mmol) was added. The reaction was stirred at 25°C for 10 hours. Upon completion indicated by LCMS, $NH_4Cl$ solution (10 mL) was added, and the mixture was extracted with ethyl acetate (20 mL × 2). The combined organic phases were dried over anhydrous sodium sulfate, filtered, and the filtrate was concentrated under reduced pressure. Purification by column chromatography (0-10% ethyl acetate/petroleum ether) yielded 1,1-dimethyl-7-(trifluoromethyl)isochroman-4-ol(120 mg, yield: 39.4%).

LCMS (ESI) m/z: 247.1 $[M+H]^+$

Step 2: Synthesis of 1,1-dimethyl-7-(trifluoromethyl)isochroman-4-yl methanesulfonate

1,1-dimethyl-7-(trifluoromethyl)isochroman-4-ol (120 mg, 0.487 mmol) and methanesulfonyl chloride (112 mg, 0.974 mmol) were dissolved in DCM (10 mL), and triethylamine (148 mg, 1.461 mmol) was added. The reaction was stirred at 25°C for 10 hours. Upon completion indicated by LCMS, water (10 mL) was added, and the mixture was extracted with ethyl acetate (20 mL × 2). The combined organic phases were dried over anhydrous sodium sulfate, filtered, and the filtrate was concentrated under reduced pressure, yielding crude 1,1-dimethyl-7-(trifluoromethyl)isochroman-4-yl methanesulfonate (130 mg, yield: 13.3%).

LCMS (ESI) m/z: 326.1 $[M+H]^+$

Step 3: Synthesis of N,1,1-trimethyl-7-(trifluoromethyl)isochroman-4-amine

At room temperature, compound 1,1-dimethyl-7-(trifluoromethyl)isochroman-4-yl methanesulfonate (130 mg, 0.398 mmol) was dissolved in DMF (3 mL), and a solution of methylamine (0.3 mL, 30%) in MeOH and DIEA (155 mg, 1.194 mmol) was added. The reaction was stirred at room temperature for 16 hours. Upon completion indicated by LCMS, water (10 mL) was added, and the mixture was extracted with ethyl acetate (20 mL × 2). The combined organic phases were dried over anhydrous sodium sulfate, filtered, and the filtrate was concentrated under reduced pressure. Purification by column chromatography (50-100% ethyl acetate/petroleum ether) afforded N,1,1-trimethyl-7-(trifluoromethyl)isochroman-4-amine (52 mg, yield: 50.4%).

LCMS (ESI) m/z: 260.1 $[M+H]^+$

Using the methods and steps employed for Intermediate 100, with only the replacement of the corresponding raw materials, the following intermediates were synthesized:

| Int. No. | Structure | Name | m/z(ESI): (M+H)$^+$ |
|---|---|---|---|
| 105 | 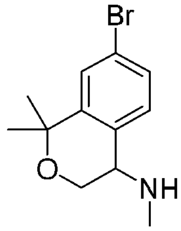 | 7-bromo-N,1,1-trimethylisochroman-4-amine | 270.04 |

Int 69 Synthesis of 6-bromo-N-cyclopropyl-2,3-dihydrobenzofuran-3-amine

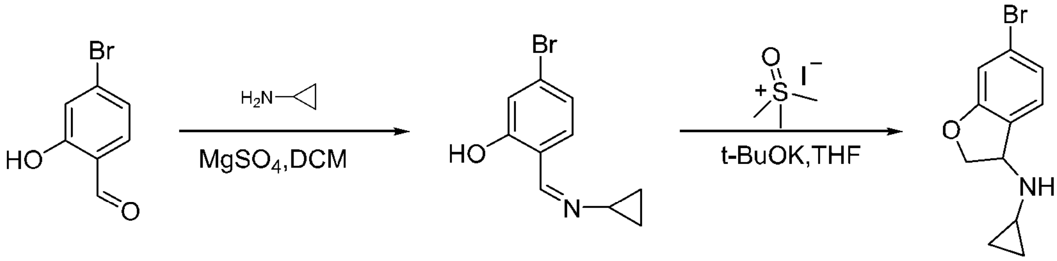


Step 1: Synthesis of 5-bromo-2-((cyclopropylimino)methyl)phenol

At room temperature, 4-bromo-2-hydroxybenzaldehyde (2.0 g, 9.95 mmol) was dissolved in dichloromethane (40 mL), and cyclopropylamine (1.13 g, 19.9 mmol) and anhydrous magnesium sulfate (4.79 g, 39.8 mmol) were added. The mixture was stirred at 25°C for 20 hours. After completion of the reaction, the reaction mixture was concentrated, and the residue was dissolved in ethyl acetate, filtered, and the filter cake was washed with ethyl acetate. The resulting filtrate was concentrated under reduced pressure to obtain the crude product 5-bromo-2-((cyclopropylimino)methyl)phenol, 1.7 g, crude), as a yellow solid, used directly in the next step without purification.

LCMS (ESI) m/z: 241.1 [M+H]$^+$

Step 2: Synthesis of 6-bromo-N-cyclopropyl-2,3-dihydrobenzofuran-3-amine

At room temperature, potassium tert-butoxide (1.99 g, 17.70 mmol) was slowly added to a solution of trimethylsilyl iodide (3.89 g, 17.70 mmol) in THF (5 mL). The mixture was stirred at room temperature for 0.5 hours, and then 5-bromo-2-((cyclopropylimino)methyl)phenol (1.7 g, 7.08 mmol) dissolved in THF was slowly added to the reaction mixture. The resulting suspension was stirred at room temperature for 1 hour and then at 50°C for 3 hours. The reaction mixture was cooled to room temperature, and 1 equivalent of potassium tert-butoxide (0.79 g, 7.08 mmol) was added. The mixture was stirred at room temperature for 12 hours. The reaction mixture was filtered, and the filtrate was diluted with water and extracted with ethyl acetate. After concentrating the organic layer, purification of the residue was performed by column chromatography (petroleum ether/ethyl acetate = 3:1) to obtain an oily substance, 6-bromo-N-cyclopropyl-2,3-dihydrobenzofuran-3-amine (1.0 g, 3.93 mmol), as a yellow oily substance.

LCMS (ESI) m/z: 255.1 [M+H]$^+$

Using the methods and steps employed for Intermediate 69, with only the replacement of the corresponding raw materials, the following intermediates were synthesized:

| Int. No. | Structure | Name | m/z(ESI): (M+H)+ |
|---|---|---|---|
| 70 | 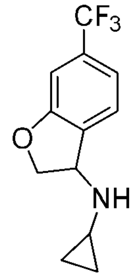 | *N*-cyclopropyl-6-(trifluoromethyl)-2,3-dihydrobenzofuran-3-amine | 244.2 |

Int. 71 Synthesis of 3-(cyclopropylamino)-2,3-dihydrobenzofuran-6-carbonitrile

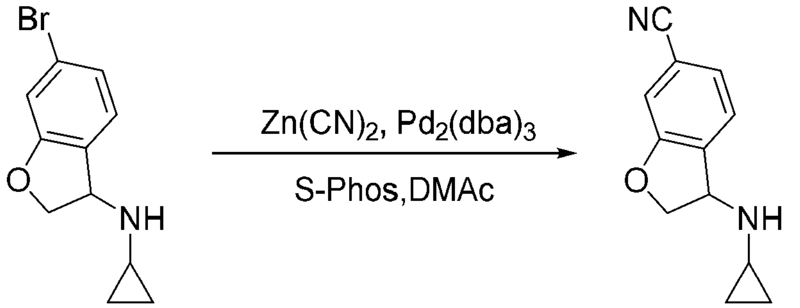


At room temperature, 6-bromo-N-cyclopropyl-2,3-dihydrobenzofuran-3-amine (100 mg, 0.41 mmol) was added in a 50 mL single-neck flask, and $Pd_2(dba)_3$ (18.8 mg, 0.02 mmol), S-Phos (9.8 mg, 0.02 mmol), zinc cyanide (96.5 mg, 0.82 mmol) were added. Then, N,N-dimethylacetamide (1.0 mL) was added, and the reaction mixture was heated to 110 °C under microwave irradiation with stirring for 1 hour. After adding water (10 mL), the mixture was extracted with ethyl acetate (20 mL × 2), and the combined organic phase was dried over anhydrous sodium sulfate, filtered, and the organic phase was concentrated to obtain the crude product. After column chromatography purification using ethyl acetate:petroleum ether (0-50%), the final product 3-(cyclopropylamino)-2,3-dihydrobenzofuran-6-carbonitrile (55 mg, yield: 67%) was obtained.

$^1H$ NMR (400 MHz, $CDCl_3$) δ 7.44-7.42(dd, 1H), 7.2-7.19(dd, 1H), 7.07(d, 1H), 4.65-4.61(m, 1H), 4.58-4.55(m, 1H), 4.47-4.46(m, 1H), 1.25-2.19(m, 1H), 0.52-0.49(m, 2H), 0.43-0.40(m, 2H).

Int. 72 Synthesis of (S)-6-(fluoromethyl)-N-methyl-2,3-dihydrobenzofuran-3-amine

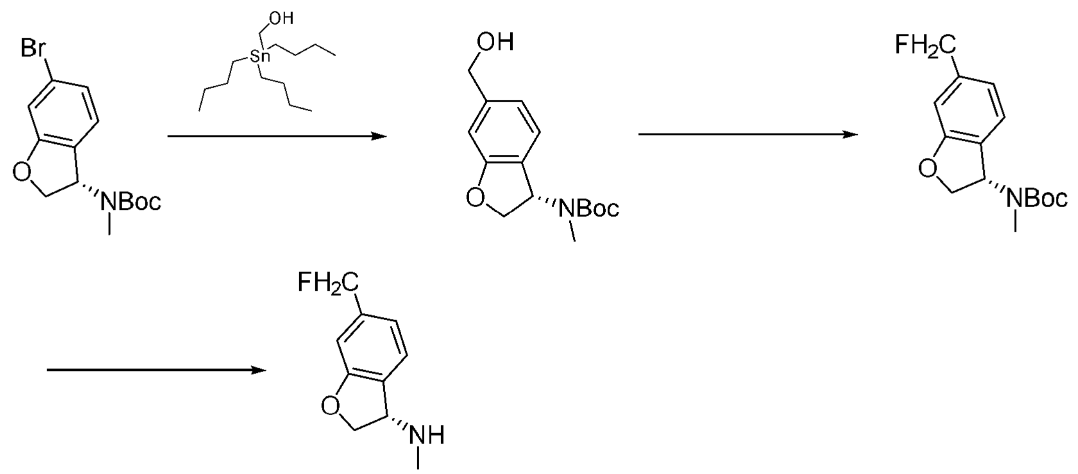

Step 1: Synthesis of tert-butyl (S)-(6-(hydroxymethyl)-2,3-dihydrobenzofuran-3-yl)(methyl)carbamate

The mixture of tert-butyl (S)-(6-bromo-2,3-dihydrobenzofuran-3-yl)(methyl)carbamate (400 mg, 1.22 mmol), (tributylstannyl)methanol (391 mg, 1.22 mmol), and 1,1'-bis(diphenylphosphino)ferrocene palladium(II) dichloride (99 mg, 0.12 mmol) in dioxane (5 mL) was reacted at 100 °C for 12 hours. After completion of the reaction, the reaction mixture was poured into water (10 mL), extracted with ethyl acetate (10 mL × 2), and the combined organic layers were washed with saturated brine (10 mL), dried over anhydrous sodium sulfate, filtered, and the filtrate was concentrated to obtain the crude product. The crude product was purified by column chromatography (ethyl acetate:petroleum ether = 40:60), yielding tert-butyl (S)-(6-(hydroxymethyl)-2,3-dihydrobenzofuran-3-yl)(methyl)carbamate(110 mg, yield: 29%).
LCMS (ESI): 280 $[M+H]^+$

Step 2: Synthesis of tert-butyl (S)-(6-(fluoromethyl)-2,3-dihydrobenzofuran-3-yl)(methyl)carbamate

At -78 °C, [Bis(2-methoxyethyl)amino]sulfur trifluoride (396 mg, 1.79 mmol) was added to the mixture of tert-butyl (S)-(6-(hydroxymethyl)-2,3-dihydrobenzofuran-3-yl)(methyl)carbamate (100 mg, 0.36 mmol) in dichloromethane (1.00 mL). The mixture was stirred for 1 hour at -78 °C. After completion of the reaction, it was quenched with ice water (5 mL), extracted with ethyl acetate (5 mL × 2), and the combined organic layers were washed with saturated brine (5 mL), dried over anhydrous sodium sulfate, filtered, and the filtrate was concentrated to obtain the crude product. The crude product was purified by column chromatography (ethyl acetate:petroleum ether = 20:80), yielding tert-butyl (S)-(6-(fluoromethyl)-2,3-dihydrobenzofuran-3-yl)(methyl)carbamate(80 mg, yield: 70%).
LCMS (ESI): 282 $[M+H]^+$

Step 3: Synthesis of (S)-6-(fluoromethyl)-N-methyl-2,3-dihydrobenzofuran-3-amine

LCMS (ESI): 182 $[M+H]^+$
The mixture of tert-butyl (S)-(6-(fluoromethyl)-2,3-dihydrobenzofuran-3-yl)(methyl)carbamate (80 mg, 0.36 mmol) and hydrochloric acid in dioxane (1 mL) was stirred at room temperature for 2 hours. After completion of the reaction, the reaction mixture was directly evaporated to obtain (S)-6-(fluoromethyl)-N-methyl-2,3-dihydrobenzofuran-3-amine (100 mg, crude product).
LCMS (ESI): 182 $[M+H]^+$

Int. 73 Synthesis of (S)-3-(methylamino)-2,3-dihydrobenzofuran-6-carboxamide

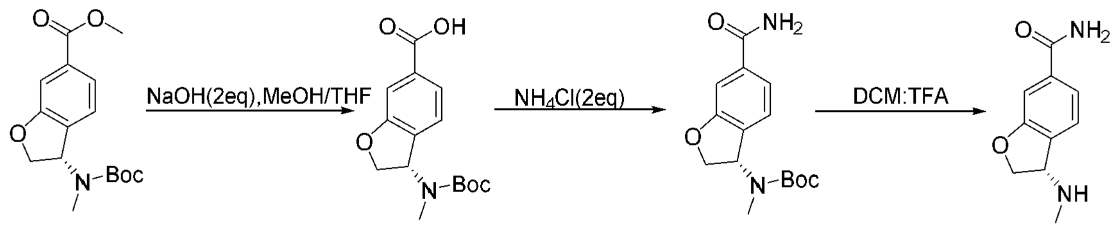


Step 1: Synthesis of (S)-3-((tert-butoxycarbonyl)(methyl)amino)-2,3-dihydrobenzofuran-6-carboxylic acid

At room temperature, the solution of methyl (S)-3-((tert-butoxycarbonyl)(methyl)amino)-2,3-dihydrobenzofuran-6-carboxylate(1 g, 3.25 mmol) in methanol (10.00 mL) and tetrahydrofuran (10.00 mL) was treated with sodium hydroxide (260 mg, 6.5 mmol). The reaction mixture was stirred for 1 hour at room temperature. After completion, the reaction solution was concentrated to yield a white (S)-3-((tert-butoxycarbonyl)(methyl)amino)-2,3-dihydrobenzofuran-6-carboxylic acid (600 mg, crude product).
LCMS (ESI): 294 $[M+H]^+$

Step 2: Synthesis of tert-butyl (S)-(6-carbamoyl-2,3-dihydrobenzofuran-3-yl)(methyl)carbamate

At room temperature, (S)-3-((tert-butoxycarbonyl)(methyl)amino)-2,3-dihydrobenzofuran-6-carboxylic acid (600 mg, 2.04 mmol) was dissolved in N,N-dimethylformamide (5.00 mL), followed by the addition of N,N,N',N'-Tetramethylchloroformamidinium-hexafluorophosphate (TCFH)(1.62 g, 4.38 mmol), N,N-diisopropylethylamine (1.38

g, 10.68 mmol), and ammonium chloride (360 mg, 6.78 mmol). The mixture was allowed to react at room temperature for 30 minutes. After completion, the reaction was diluted with ethyl acetate (30 mL) and water (30 mL). The aqueous layer was extracted with ethyl acetate (2 × 20 mL). The combined organic layers were washed with saturated brine (30 mL), dried over anhydrous sodium sulfate, concentrated, and yielded the crude product. The crude product was purified by flash column chromatography (ethyl acetate/petroleum ether = 17%), providing tert-butyl (S)-(6-carbamoyl-2,3-dihydrobenzofuran-3-yl)(methyl)carbamate(240 mg, 40% yield), a yellow liquid.

LCMS (ESI): 293 $[M+H]^+$

Step 3: Synthesis of (S)-3-(methylamino)-2,3-dihydrobenzofuran-6-carboxamide

At room temperature, tert-butyl (S)-(6-carbamoyl-2,3-dihydrobenzofuran-3-yl)(methyl)carbamate (240 mg, 0.82 mmol) was dissolved in dichloromethane (2.00 mL) and trifluoroacetic acid (0.40 mL). The mixture was stirred at room temperature for half an hour. After completion, the reaction solution was concentrated to yield a black solid (S)-3-(methylamino)-2,3-dihydrobenzofuran-6-carboxamide (100 mg, 66.66% yield).

In. 74 Synthesis of (S)-$N^3$-methyl-2,3-dihydrobenzofuran-3,6-diamine

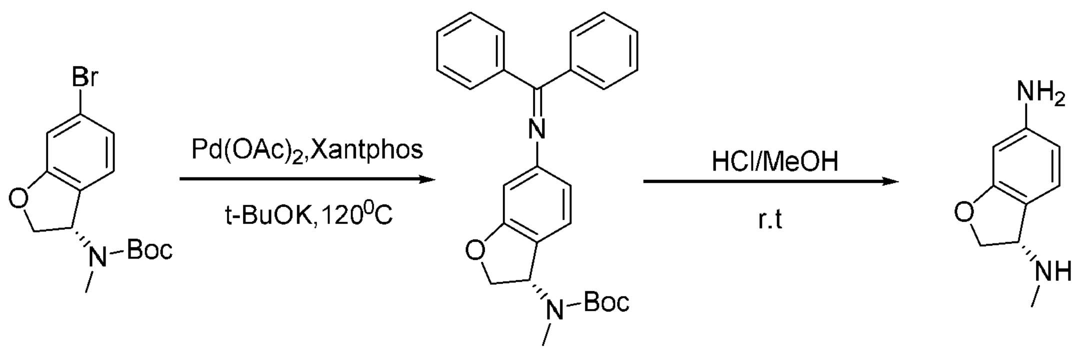


Step 1: Synthesis of tert-butyl (S)-(6-((diphenylmethylene)amino)-2,3-dihydrobenzofuran-3-yl)(methyl)carbamate

At room temperature, tert-butyl (S)-(6-bromo-2,3-dihydrobenzofuran-3-yl)(methyl)carbamate (120 mg, 0.35 mmol) was dissolved in 1,4-dioxane (5.00 mL). To the solution, Benzophenone imine (190 mg, 1.06 mmol), potassium tert-butoxide (80 mg, 0.71 mmol), 4,5-Bis(diphenylphosphino)-9,9-diMethylxanthene, Xantphos (40 mg, 0.07 mmol), and palladium acetate (8 mg, 0.03 mmol) were added. The reaction was carried out at 100°C for 2 hours. After completion, the reaction mixture was poured into water (2 mL), extracted with ethyl acetate (2 mL × 2), and the combined organic layers were washed with saturated saline solution (2 mL), dried over anhydrous sodium sulfate, filtered, and the filtrate was concentrated. The crude product was purified by flash column chromatography (petroleum ether:ethyl acetate = 10%) to yield the white solid tert-butyl (S)-(6-((diphenylmethylene)amino)-2,3-dihydrobenzofuran-3-yl)(methyl)carbamate(90 mg, 59% yield).

LCMS (ESI): 429 $[M+H]^+$

Step 2: Synthesis of (S)-$N^3$-methyl-2,3-dihydrobenzofuran-3,6-diamine

At room temperature, tert-butyl (S)-(6-((diphenylmethylene)amino)-2,3-dihydrobenzofuran-3-yl)(methyl)carbamate (90 mg, 0.21 mmol) was dissolved in hydrochloric acid-methanol solution (5.00 mL). The mixture was stirred at room temperature for 0.5 hours. After completion, the reaction solution was poured into a dichloromethane solution to precipitate a white solid, yielding (S)-$N^3$-methyl-2,3-dihydrobenzofuran-3,6-diamine (60 mg, 90% yield).

LCMS (ESI): 165 $[M+H]^+$

Int. 75 Synthesis of (S)-1,1,1-trifluoro-N-(3-(methylamino)-2,3-dihydrobenzofuran-6-yl)methanesulfonamide

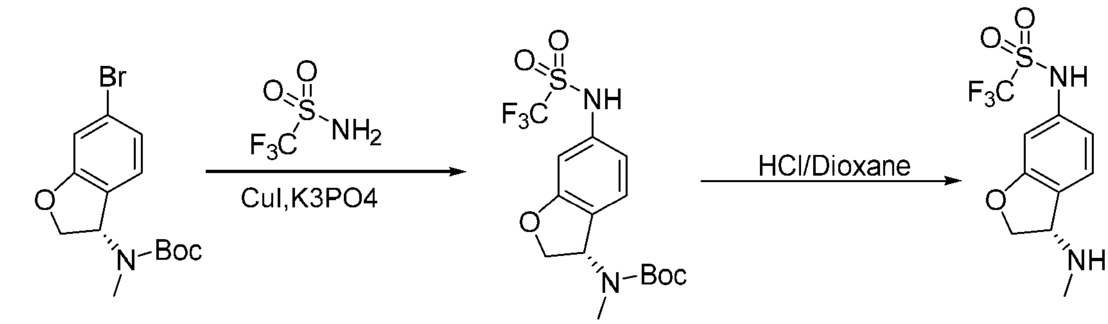


Step 1: Synthesis of tert-butyl (S)-methyl(6-((trifluoromethyl)sulfonamido)-2,3-dihydrobenzofuran-3-yl)carbamate

tert-butyl (S)-(6-bromo-2,3-dihydrobenzofuran-3-yl)(methyl)carbamate (200 mg, 0.61 mmol), trifluoromethanesulfonyl amide (136 mg, 0.91 mmol), cuprous iodide (1.16 g, 6.09 mmol), and potassium phosphate (259 mg, 1.22 mmol) were successively dissolved in N,N-dimethylformamide (9 mL) solution. The mixture was stirred at 90°C for 2 hours. After completion, the system was cooled to room temperature, extracted three times with ethyl acetate (10 mL each). The organic phases were combined, washed with saturated brine (10 mL, 3 times), dried over anhydrous sodium sulfate, and filtered. The filtrate was concentrated under vacuum to obtain tert-butyl (S)-methyl(6-((trifluoromethyl)sulfonamido)-2,3-dihydrobenzofuran-3-yl)carbamate (800 mg, 51.85% yield) as a yellow liquid.

LCMS (ESI): 397.2 $[M+H]^+$

Step 2: Synthesis of (S)-1,1,1-trifluoro-N-(3-(methylamino)-2,3-dihydrobenzofuran-6-yl)methanesulfonamide

tert-butyl (S)-methyl(6-((trifluoromethyl)sulfonamido)-2,3-dihydrobenzofuran-3-yl)carbamate (80 mg, 3.246 mmol) was dissolved in a solution of hydrogen chloride in 1,4-dioxane (4.0 M, 5 mL). The mixture was stirred at room temperature for 2 hours. After completion of the reaction, the system was cooled to room temperature, and the reaction mixture was concentrated under vacuum to obtain (S)-1,1,1-trifluoro-N-(3-(methylamino)-2,3-dihydrobenzofuran-6-yl) methanesulfonamide(60 mg, 1.18 mmol, yield: 82.46%).

Int. 76 Synthesis of 4-aminoimidazo[1,5-a]quinoxaline-8-carboxylic acid

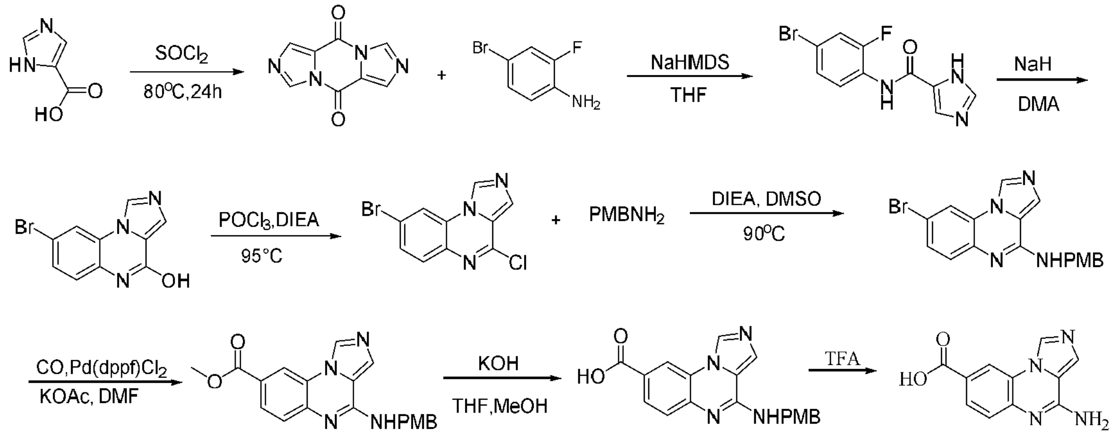


Step 1: Synthesis of 5H,10H-diimidazo[1,5-a:1',5'-d]pyrazine-5,10-dione

Imidazo-5-carboxylic acid (100 g, 892.14 mmol) was added to sulfurous dichloride (500 mL) and stirred at 80°C for 12 hours. The mixture was concentrated, and the concentrated solution was washed with toluene (500 mL × 2), filtered to obtain a solid product. After removing the residual solvent under vacuum, 5H,10H-diimidazo[1,5-a:1',5'-d]pyrazine-5,10-dione (70 g, 0.37 mol, yield: 42%) was obtained as a yellow solid.

LCMS (ESI) m/z: 189$[M+H]^+$

Step 2: Synthesis of N-(4-bromo-2-fluorophenyl)-1H-imidazole-5-carboxamide

At 0°C, a solution of 4-bromo-2-fluorophenylamine (60.60 g, 318.91 mmol) in tetrahydrofuran (600 mL) was slowly added dropwise to a mixture of Sodium bis(trimethylsilyl)amide (318.91 mL, 637.82 mmol, 2 mol/L) over 1 hour. Then, 5H,10H-diimidazo[1,5-a:1',5'-d]pyrazine-5,10-dione (60 g, 318.91 mmol) was added, and the mixture was stirred at room temperature for 12 hours. The reaction mixture was then poured into water, filtered, and the crude product was purified by silica gel column chromatography (petroleum ether: ethyl acetate = 40%) to obtain N-(4-bromo-2-fluorophenyl)-1H-imidazole-5-carboxamide(60 g, 0.21 mol, yield: 66%), as a white solid.
LCMS (ESI) m/z: 284.2[M+H]$^+$

Step 3: Synthesis of 8-bromoimidazo[1,5-a]quinoxalin-4-ol

N-(4-bromo-2-fluorophenyl)-1H-imidazole-5-carboxamide (60 g, 211.20 mmol) and sodium hydride (16.88 g, 422.40 mmol, 60% dispersion) were stirred in dimethylacetamide (600 mL) at 140°C for 12 hours. The mixture was then poured into water, filtered to obtain 8-bromoimidazo[1,5-a]quinoxalin-4-ol (50 g, yield: 90%), as a yellow solid.
LCMS (ESI) m/z: 264.2[M+H]$^+$

Step 4: Synthesis of 8-bromo-4-chloroimidazo[1,5-a]quinoxaline

To the mixture of 8-bromoimidazo[1,5-a]quinoxalin-4-ol (50 g, 189.34 mmol) and N,N-diisopropylethylamine (48.85 g, 378.67 mmol), phosphorous oxychloride (500 mL) was added. The mixture was stirred at 90°C for 2 hours, then concentrated. The residue was dissolved in acetonitrile, slowly added to ice water, resulting in the precipitation of a solid. Filtration yielded 8-bromo-4-chloroimidazo[1,5-a]quinoxaline (50 g, yield: 93%).
LCMS (ESI) m/z: 282.2[M+H]$^+$.

Step 5: Synthesis of 8-bromo-N-(4-methoxybenzyl)imidazo[1,5-a]quinoxalin-4-amine

To the mixture of 8-bromo-4-chloroimidazo[1,5-a]quinoxaline (50 g, 176.98 mmol) and 4-methoxybenzylamine (29.13 g, 212.38 mmol) in dimethyl sulfoxide (500 mL), N,N-diisopropylethylamine (45.66 g, 353.96 mmol) was added. The mixture was stirred at 80°C for 2 hours, then poured into water. Filtration yielded a yellow oily substance, 8-bromo-N-(4-methoxybenzyl)imidazo[1,5-a]quinoxalin-4-amine (50 g, yield: 74%).
LCMS (ESI) m/z: 383.2[M+H]$^+$

Step 6: synthesis of Methyl 4-((4-methoxybenzyl)amino)imidazo[1,5-a]quinoxaline-8-carboxylate

8-bromo-N-(4-methoxybenzyl)imidazo[1,5-a]quinoxalin-4-amine(50 g, 130.47 mmol), [1,1'-bis(diphenylphosphino)ferrocene]dichloropalladium (10.83 g, 13.05 mmol), and potassium acetate (25.57 g, 260.93 mmol) were successively added to a solution of dimethylformamide (50 mL) and methanol (250 mL). The system was reacted at 100°C under a carbon monoxide atmosphere (4 MPa) for 12 hours, then poured into water and filtered. Purification by silica gel chromatography (petroleum ether: ethyl acetate = 80%) yielded Methyl 4-((4-methoxybenzyl)amino)imidazo[1,5-a]quinoxaline-8-carboxylate(30 g, yield: 63%), as a yellow solid.
LCMS (ESI) m/z: 363.2 [M+H]$^+$

Step 7 Synthesis of 4-((4-methoxybenzyl)amino)imidazo[1,5-a]quinoxaline-8-carboxylic acid

Methyl 4-((4-methoxybenzyl)amino)imidazo[1,5-a]quinoxaline-8-carboxylate (30 g, 82.87 mmol) was added to a mixture of methanol, water, and tetrahydrofuran (1:1:1, 150 mL), along with potassium hydroxide (92.40 g, 165.0 mmol). The reaction proceeded at 60°C for 12 hours, and after vacuum concentration to remove the organic solvent, the residue was poured into water. Then the pH was adjusted to 7-8 with HCl (2 M), followed by extraction with ethyl acetate (100 mL × 3) and vacuum concentration, yielded 4-((4-methoxybenzyl)amino)imidazo[1,5-a]quinoxaline-8-carboxylic acid(25 g, yield: 86%).
LCMS (ESI) m/z: 349.2 [M+H]$^+$

Step 8: Synthesis of 4-aminoimidazo[1,5-a]quinoxaline-8-carboxylic acid

At room temperature, 4-((4-methoxybenzyl)amino)imidazo[1,5-a]quinoxaline-8-carboxylic acid (100.0 mg, 0.28 mmol) was dissolved in trifluoroacetic acid (2 mL). The mixture was stirred at 100 °C for 2 hours, and the reaction was monitored by LCMS. The reaction mixture was concentrated to yield 4-aminoimidazo[1,5-a]quinoxaline-8-carboxylic acid

(60.0 mg, yield: 94%), as a white solid.

LCMS (ESI) m/z: 229.2 [M+H]$^+$

Using the methods and steps employed for Intermediate 76, with only the replacement of the corresponding raw materials, the following intermediates were synthesized:

| Int. No. | Structure | Name | m/z(ESI): (M+H)$^+$ |
|---|---|---|---|
| 77 | 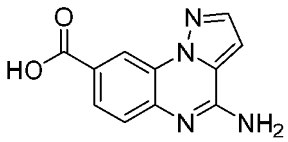 | 4-aminopyrazolo[1,5-a]quinoxaline-8-carboxylic acid | 229.2 |
| 78 | 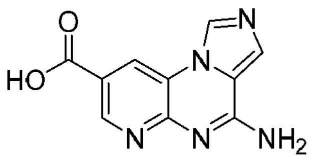 | 4-aminoimidazo[1,5-a]pyrido[2,3-e]pyrazine-8-carboxylic acid | 230.2 |
| 79 | 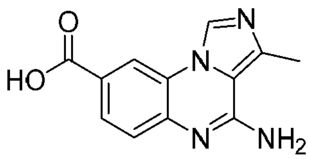 | 4-amino-3-methylimidazo[1,5-a]quinoxaline-8-carboxylic acid | 243.2 |
| 80 | 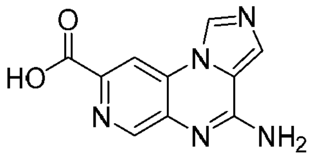 | 4-aminoimidazo[1,5-a]pyrido[3,4-e]pyrazine-8-carboxylic acid | 230.2 |
| 81 | 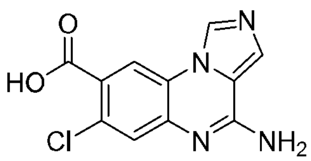 | 4-amino-7-chloroimidazo[1,5-a]quinoxaline-8-carboxylic acid | 263.2 |
| 82 | 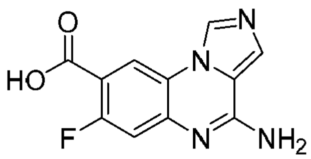 | 4-amino-7-fluoroimidazo[1,5-a]quinoxaline-8-carboxylic acid | 247.2 |
| 83 | 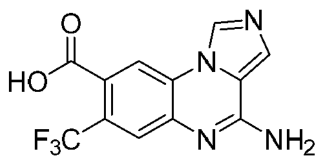 | 4-amino-7-(trifluoromethyl)imidazo[1,5-a]quinoxaline-8-carboxylic acid | 297.2 |

Int. 84 Synthesis of 4-amino-1-methylimidazo[1,5-a]quinoxaline-8-carboxylic acid

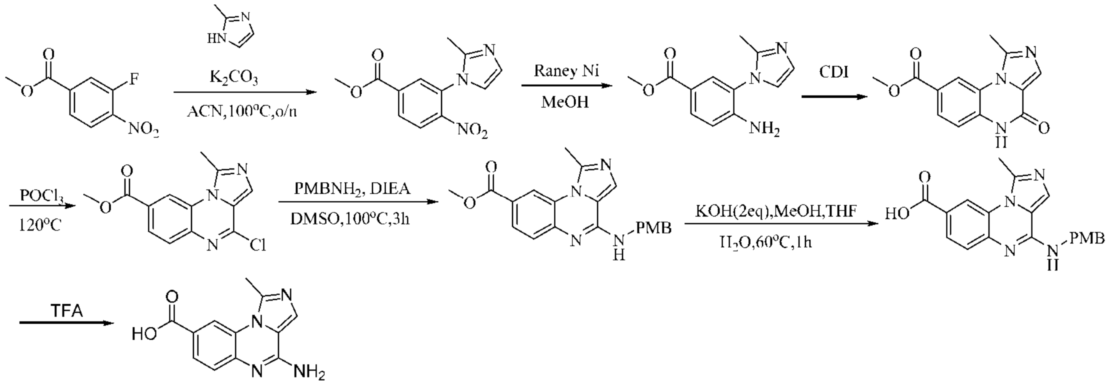


Step 1: Synthesis of methyl 3-(2-methyl-1H-imidazol-1-yl)-4-nitrobenzoate

Methyl 3-fluoro-4-nitrobenzoate (20.0 g, 100 mmol) was dissolved in acetonitrile (100 mL), followed by the addition of 2-methyl-1H-imidazole (8.2 g, 100 mmol) and potassium carbonate (27.6 g, 200 mmol). The mixture was stirred at 100 °C for 12 hours, and the reaction progress was monitored by liquid chromatography-mass spectrometry (LCMS). The reaction mixture was poured into 300 mL water, and then extracted with ethyl acetate (200 mL × 3). The combined organic phase was dried over anhydrous sodium sulfate, and the organic phase was concentrated under vacuum and the residues undegone further purification by silica gel column chromatography (petroleum ether: ethyl acetate = 10%) to yield the yellow solid Synthesis of methyl 3-(2-methyl-1H-imidazol-1-yl)-4-nitrobenzoate (25.0 g, yield: 95%).
LCMS (ESI) m/z: 262 $[M+H]^+$

Step 2: Synthesis of methyl 4-amino-3-(2-methyl-1H-imidazol-1-yl)benzoate

At room temperature, 3-(2-methyl-1H-imidazol-1-yl)-4-nitrobenzoate (25 g, 95.8 mmol) was dissolved in methanol (300 mL), and then Raney Nickel (2 g) was added. The mixture was stirred for 12 hours under a hydrogen atmosphere at room temperature. The reaction was monitored by LCMS. The residue was filtered, and the filtrate was concentrated and purified by silica gel column chromatography (petroleum ether: ethyl acetate = 20%) to yield the yellow solid methyl 4-amino-3-(2-methyl-1H-imidazol-1-yl)benzoate(20.5 g, yield: 93%).
LCMS (ESI) m/z: 232 $[M+H]^+$

Step 3: Synthesis of methyl 1-methyl-4-oxo-4,5-dihydroimidazo[1,5-a]quinoxaline-8-carboxylate

At room temperature, methyl 4-amino-3-(2-methyl-1H-imidazol-1-yl)benzoate (2.0 g, 8.7 mmol) was dissolved in o-dichlorobenzene (40 mL), and then carbonyldiimidazole (2.8 g, 17.4 mmol) was added. The mixture was stirred for 12 hours at 180 °C. The reaction was monitored by liquid chromatography-mass spectrometry (LCMS). The mixture was filtered to obtain a filter cake, and the filter cake was slurried with ethyl acetate (5 mL) to yield the black solid methyl 1-methyl-4-oxo-4,5-dihydroimidazo[1,5-a]quinoxaline-8-carboxylate(920.0 mg, yield: 41%).
LCMS (ESI) m/z: 258 $[M+H]^+$

Step 4: Synthesis of methyl 4-chloro-1-methylimidazo[1,5-a]quinoxaline-8-carboxylate

At room temperature, methyl 1-methyl-4-oxo-4,5-dihydroimidazo[1,5-a]quinoxaline-8-carboxylate (100.0 mg, 0.39 mmol) was dissolved in Phosphorus oxychloride (5 mL), and the mixture was stirred for 4 hours at 120 °C. The reaction was monitored by liquid chromatography-mass spectrometry (LCMS). The reaction mixture was concentrated under reduced pressure, diluted with a small amount of acetonitrile, poured into water (5 mL), filtered, and the filter cake was washed with water. The resulting solid, methyl 4-chloro-1-methylimidazo[1,5-a]quinoxaline-8-carboxylate(60.0 mg, yield: 56%), was obtained as a black solid.
LCMS (ESI) m/z: 276 $[M+H]^+$

Step 5: Synthesis of methyl 4-((4-methoxybenzyl)amino)-1-methylimidazo[1,5-a]quinoxaline-8-carboxylate

At room temperature, methyl 4-chloro-1-methylimidazo[1,5-a]quinoxaline-8-carboxylate (60 mg, 0.22 mmol) was dissolved in dimethyl sulfoxide (2 mL), followed by the addition of (4-methoxyphenyl)methanamine (60 mg, 0.44 mmol) and N,N-diisopropylethylamine (67 mg, 0.52 mmol). The mixture was stirred for 1 hour at 100 °C. The reaction was

monitored by liquid chromatography-mass spectrometry (LCMS). The reaction mixture was poured into water (10 mL), extracted with ethyl acetate (10 mL × 3). Then the combined organic phase was dried over anhydrous sodium sulfate, and the organic phase was concentrated and purified by silica gel chromatography (petroleum ether: ethyl acetate = 40%) to obtain the black solid of methyl 4-((4-methoxybenzyl)amino)-1-methylimidazo[1,5-a]quinoxaline-8-carboxylate(60 mg, yield: 73%).

LCMS (ESI) m/z: 377 $[M+H]^+$

Step 6: Synthesis of 4-((4-methoxybenzyl)amino)-1-methylimidazo[1,5-a]quinoxaline-8-carboxylic acid

At room temperature, methyl 4-((4-methoxybenzyl)amino)-1-methylimidazo[1,5-a]quinoxaline-8-carboxylate (60 mg, 0.16 mmol) was dissolved in methanol (1 mL), tetrahydrofuran (1 mL), and water (1 mL), then potassium hydroxide (26 mg, 0.44 mmol) was added. The mixture was stirred for 1 hour at room temperature. The reaction progress was monitored by liquid chromatography-mass spectrometry (LCMS). The reaction mixture was poured into water (10 mL), then carefully adjusted to acidic pH with formic acid, extracted with ethyl acetate (10 mL × 3), the combined organic phase was dried over anhydrous sodium sulfate, and the organic phase was concentrated to obtain the white solid of 4-((4-methoxybenzyl)amino)-1-methylimidazo[1,5-a]quinoxaline-8-carboxylic acid(50 mg, yield: 86%).

LCMS (ESI) m/z: 363 $[M+H]^+$

Step 7: Synthesis of 4-amino-1-methylimidazo[1,5-a]quinoxaline-8-carboxylic acid

The solution of 4-((4-methoxybenzyl)amino)-1-methylimidazo[1,5-a]quinoxaline-8-carboxylic acid (400 mg, 1.10 mmol) in trifluoroacetic acid (5.00 mL) was stirred at 90°C for 2 hours. The reaction solution was then vacuum-concentrated to obtain 4-amino-1-methylimidazo[1,5-a]quinoxaline-8-carboxylic acid (250 mg, yield: 94%).

LCMS (ESI) m/z:243 $[M+H]^+$

Int. 85 Synthesis of 4-amino-7-cyanoimidazo[1,5-a]quinoxaline-8-carboxylic acid

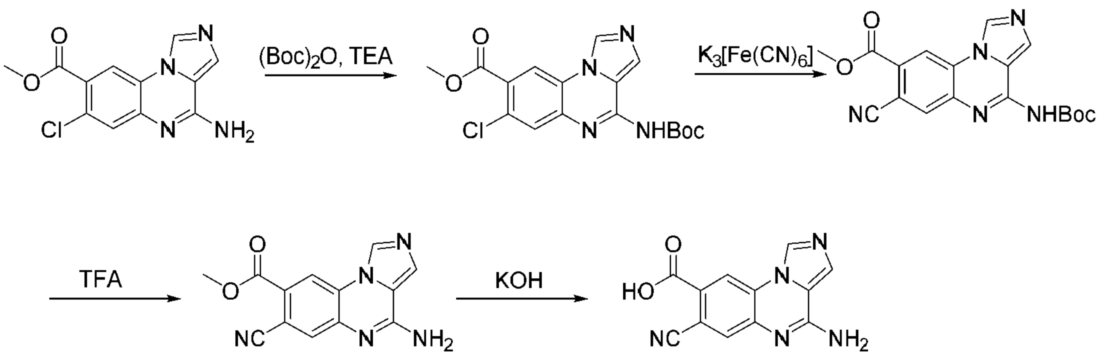


Step 1: Synthesis of methyl 4-((tert-butoxycarbonyl)amino)-7-chloroimidazo[1,5-a]quinoxaline-8-carboxylate

The solution of methyl 4-amino-7-chloroimidazo[1,5-a]quinoxaline-8-carboxylate (1.20 g, 4.34 mmol), di-tert-butyl dicarbonate (1.89 g, 8.67 mmol), and triethylamine (1.30 g, 13.01 mmol) in dichloromethane (10 mL) was stirred at room temperature for 2 hours. After completion of the reaction, the mixture was poured into water (5 mL), extracted with ethyl acetate (5 mL), dried over anhydrous sodium sulfate, and filtered. The solution was then concentrated and purified by column chromatography (petroleum ether: ethyl acetate = 70:30) to yield 4-((tert-butoxycarbonyl)amino)-7-chloroimidazo[1,5-a]quinoxaline-8-carboxylate(1.00 g, 61% yield), a white solid.

LCMS (ESI): 376 $[M+H]^+$

Step 2: Synthesis of methyl 4-((tert-butoxycarbonyl)amino)-7-cyanoimidazo[1,5-a]quinoxaline-8-carboxylate

Under a nitrogen atmosphere, a mixture of 4-((tert-butoxycarbonyl)amino)-7-chloroimidazo[1,5-a]quinoxaline-8-carboxylate(0.20 g, 0.53 mmol), potassium ferricyanide (0.05 g, 0.16 mmol), 2-Di- tert -butylphosphino-2',4',6'-triisopropylbiphenyl (0.09 g, 0.21 mmol), 2'-(Amino)[1,1'-biphenyl]-2-yl][bis(1,1-dimethylethyl)[2',4',6'-tris(1-methylethyl) [1,1'-biphenyl]-2-yl]phosphine](methanes ulfonato)palladium (0.17 g, 0.21 mmol), and potassium acetate (0.01 g, 0.07

mmol) in 1,4-dioxane/water (4.00 mL) was stirred at 100 °C for 2 hours under reflux. The mixture was then concentrated and purified by column chromatography (petroleum ether: ethyl acetate = 70:30) to yield methyl 4-((tert-butoxycarbonyl) amino)-7-cyanoimidazo[1,5-a]quinoxaline-8-carboxylate (0.10 g, 51%) as a white solid.

LCMS (ESI): 368[M+H]$^+$

Step 3: Synthesis of methyl 4-amino-7-cyanoimidazo[1,5-a]quinoxaline-8-carboxylate

Trifluoroacetic acid (0.10 mL) was added to a solution of 4-((tert-butoxycarbonyl)amino)-7-cyanoimidazo[1,5-a] quinoxaline-8-carboxylate(0.20 g, 0.54 mmol) in dichloromethane (0.50 mL). The reaction mixture was stirred at room temperature for 2 hours, then concentrated to yield methyl 4-amino-7-cyanoimidazo[1,5-a]quinoxaline-8-carboxylate (0.05 g, crude product)

LCMS (ESI): 268[M+H]$^+$

Step 4: Synthesis of 4-amino-7-cyanoimidazo[1,5-a]quinoxaline-8-carboxylic acid

A mixture of methyl 4-amino-7-cyanoimidazo[1,5-a]quinoxaline-8-carboxylate (0.20 g, 0.75 mmol), potassium hydroxide (0.07 g, 1.50 mmol) and the mixture of tetrahydrofuran/methanol/water (1 mL/1 mL/1 mL), was stirred at room temperature for 2 hours. After completion of the reaction, the system was concentrated to obtain the crude product, 4-amino-7-cyanoimidazo[1,5-a]quinoxaline-8-carboxylic acid(210 mg, crude product). The crude product is used directly in the next step without purification.

LCMS (ESI): 254[M+H]$^+$

Int. 86 Synthesis of 4-amino-7-methylimidazo[1,5-a]quinoxaline-8-carboxylic acid

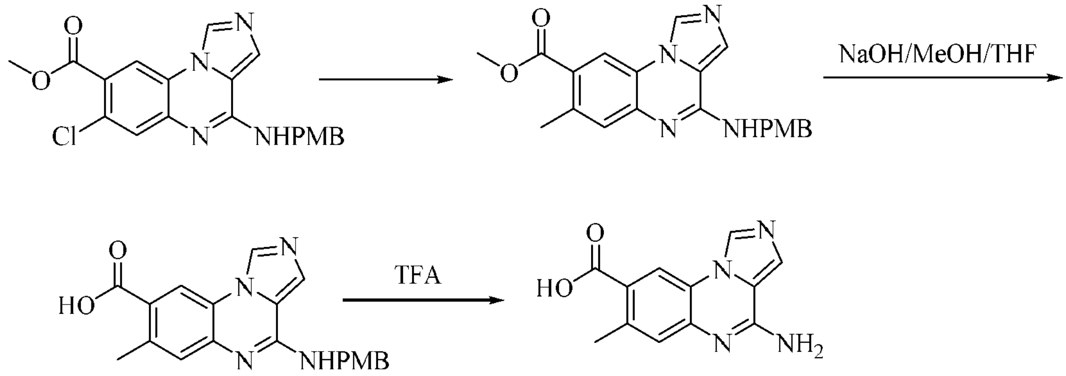


Step 1: Synthesis of methyl 4-((4-methoxybenzyl)amino)-7-methylimidazo[1,5-a]quinoxaline-8-carboxylate

A mixture of methyl 7-chloro-4-((4-methoxybenzyl)amino)imidazo[1,5-a]quinoxaline-8-carboxylate (200 mg, 0.5 mmol), potassium carbonate (300 mg, 2 mmol), 1,1'-Bis (di-t-butylphosphino)ferrocene palladium dichloride (0.08 g, 0.1 mmol), and Trimethylboroxine (0.01 g, 0.10 mmol) in 1,2-dimethoxyethane (2.00 mL) was stirred at 100 °C for 12 hours, then poured into water, extracted with ethyl acetate (5 mL), dried over anhydrous sodium sulfate, filtered, and purified by column chromatography (petroleum ether:ethyl acetate = 30%) to yield methyl 4-((4-methoxybenzyl)amino)-7-methylimidazo[1,5-a]quinoxaline-8-carboxylate(100mg, 53%).

LCMS (ESI):268 [M+H] $^+$

Step 2: Synthesis of 4-((4-methoxybenzyl)amino)-7-methylimidazo[1,5-a]quinoxaline-8-carboxylic acid

Methyl 4-((4-methoxybenzyl)amino)-7-methylimidazo[1,5-a]quinoxaline-8-carboxylate (500 mg, 1.86 mmol) was dissolved in a solvent mixture of methanol: tetrahydrofuran: saturated aqueous potassium hydroxide solution (1 mL: 1 mL: 1 mL). The reaction mixture proceeded at 60 °C for 12 hours, then evaporate to dryness. The pH was adjusted to 3 with formic acid, filtered to obtain 4-((4-methoxybenzyl)amino)-7-methylimidazo[1,5-a]quinoxaline-8-carboxylic acid(52 mg, 12% yield).

LCMS (ESI):363 [M+H] $^+$

Step 3: Synthesis of 4-amino-7-methylimidazo[1,5-a]quinoxaline-8-carboxylic acid

4-((4-methoxybenzyl)amino)-7-methylimidazo[1,5-a]quinoxaline-8-carboxylic acid (500 mg, 1.86 mmol) was dissolved in trifluoroacetic acid (5 mL). The reaction mixture was stirred at 100 °C for 12 hours. After completion, the reaction system was evaporated to dryness to obtain 4-amino-7-methylimidazo[1,5-a]quinoxaline-8-carboxylic acid (520 mg, crude product).

LCMS (ESI):363 [M+H] $^+$

By employing the method used in the third step for Intermediate 86, the following intermediate acids were prepared:

| Int. No. | Structure | Name | m/z(ESI): $(M+H)^+$ |
|---|---|---|---|
| 87 | 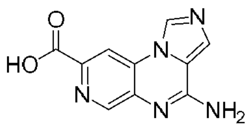 | 4-aminoimidazo[1,5-a]pyrido[3,4-e]pyrazine-8-carboxylic acid | 230.1 |
| 88 | 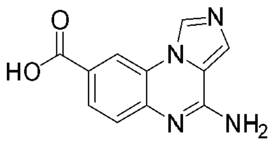 | 4-aminoimidazo[1,5-a]quinoxaline-8-carboxylic acid | 229.1 |
| 89 | 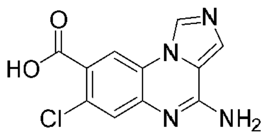 | 4-amino-7-chloroimidazo[1,5-a]quinoxaline-8-carboxylic acid | 263.1 |
| 90 | 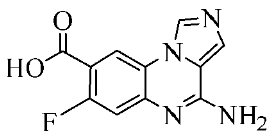 | 4-amino-7-fluoroimidazo[1,5-a]quinoxaline-8-carboxylic acid | 247.1 |
| 91 | 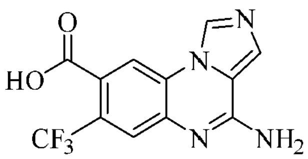 | 4-amino-7-(trifluoromethyl)imidazo[1,5-a]quinoxaline-8-carboxylic acid | 297.1 |
| 92 | 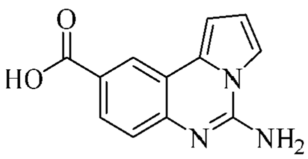 | 5-aminopyrrolo[1,2-c]quinazoline-9-carboxylic acid | 228.1 |

Int. 93 4-amino-[1,2,4]triazolo[4,3-a]quinoxaline-8-carboxylic acid

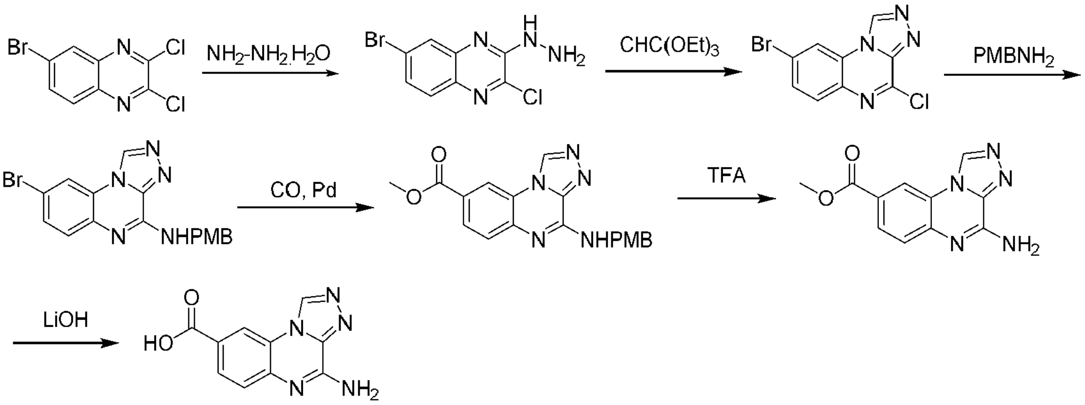


Step 1: Synthesis of 6-bromo-2-chloro-3-hydrazineylquinoxaline

At room temperature, 6-bromo-2,3-dichloroquinoxaline (278.0 mg, 1.0 mmol) was firstly added into a 25 mL single-neck flask. Then hydrazine hydrate (156 mg, 2.5 mmol, 80% wt) was added. After completion, the reaction proceeded overnight at room temperature. After the reaction was complete, the reaction was filtered to obtain a filter cake and the filter cake was washed with water (10 mL × 2), washed with ethyl acetate (5 mL × 2) to obtain 6-bromo-2-chloro-3-hydrazineylquinoxaline(150 mg).
LCMS (ESI) m/z:273.0/275.0 $[M+H]^+$

Step 2: Synthesis of 8-bromo-4-chloro-[1,2,4]triazolo[4,3-a]quinoxaline

At room temperature, 6-bromo-2-chloro-3-hydrazineylquinoxaline (116.0 mg, 0.5 mmol) was added into a 25 mL single-neck flask. Then triethyl orthoformate (4.0 mL) was added. Then the reaction was heated to 100 °C, and proceeded for 1 hour at this temperature. When reaction was complete indicated by LCMS, the reaction was cooled to room temperature, then filtered to get a filter cake. The filter cake was washed with methanol (3.0 mL × 3), then dried to obtain 8-bromo-4-chloro-[1,2,4]triazolo[4,3-a]quinoxaline (110 mg).
$^1$H NMR (400 MHz, $CDCl_3$) δ 10.21(s, 1H), 8.84(d, 1H), 7.98(d, 1H), 7.90(dd, 1H).

Step 3: Synthesis of 8-bromo-N-(4-methoxybenzyl)-[1,2,4]triazolo[4,3-a]quinoxalin-4-amine

8-bromo-4-chloro-[1,2,4]triazolo[4,3-a]quinoxaline (1.70 g, 6.00 mmol) was dissolved in DMSO (15.0 mL), then benzylamine (1.23 g, 9.00 mmol) and DIEA (2.32 g, 18.00 mmol) were added successively. The reaction system proceeded at 90 °C for 4 hours. After completion, water (60 mL) was added, then extracted with ethyl acetate (40 mL × 3), dried with sodium sulfate, filtered, and concentrated to obtain the crude product, 8-bromo-N-(4-methoxybenzyl)-[1,2,4]triazolo[4,3-a]quinoxalin-4-amine (2.20 g).
LCMS (ESI) m/z:384.1/386.1 $[M+H]^+$

Step 4: Methyl 4-((4-methoxybenzyl)amino)-[1,2,4]triazolo[4,3-a]quinoxaline-8-carboxylate

Potassium acetate (1.7 g, 17.2 mmol) and Pd(dppf)Cl2 (420 mg, 0.57 mmol) were successively added to a solution of 8-bromo-N-(4-methoxybenzyl)-[1,2,4]triazolo[4,3-a]quinoxalin-4-amine (2.2 g, 5.74 mmol) in MeOH (30 mL) and DMF (30 mL). Under a CO atmosphere, the mixture was stirred at 100 °C for 12 hours. After removing methanol, 100 mL of water was added, extracted with ethyl acetate (50 mL × 3). Then the organic phase was combined, dried over anhydrous $Na_2SO_4$, and concentrated to obtain the crude product. The crude product was slurried in ethyl acetate (20 mL) to obtain Methyl 4-((4-methoxybenzyl)amino)-[1,2,4]triazolo[4,3-a]quinoxaline-8-carboxylate (1.1 g, yield: 53%).
LCMS (ESI) m/z: 364.2$[M+H]^+$

Step 5: Synthesis of methyl 4-amino-[1,2,4]triazolo[4,3-a]quinoxaline-8-carboxylate

Methyl 4-((4-methoxybenzyl)amino)-[1,2,4]triazolo[4,3-a]quinoxaline-8-carboxylate (300.0 mg, 0.78 mmol) was added into a 25 mL single-neck flask followed by addition of TFA (5.0 mL). The reaction system was heated to 80 °C, and stirred for 16 hours at this temperature. After the reaction was complete, the reaction mixture was evaporated to obtain the crude product, methyl 4-amino-[1,2,4]triazolo[4,3-a]quinoxaline-8-carboxylate(190 mg).
LCMS (ESI) m/z: 244.3 $[M+H]^+$

Step 6: Synthesis of 4-amino-[1,2,4]triazolo[4,3-a]quinoxaline-8-carboxylic acid

methyl 4-amino-[1,2,4]triazolo[4,3-a]quinoxaline-8-carboxylate was dissolved in THF(5ml) and methanol(5.0ml). The pH was adjusted to 7 using a 3M potassium hydroxide solution followed by addition of lithium hydroxide (65.0 mg, 1.56 mmol). The reaction proceeded at 50 °C for 16 hours. After the reaction was complete, methanol and THF were evaporated from the system, and the pH was adjust to 6.5 with 1M hydrochloric acid. The mixture was filtered to get a filter cake and the filter cake was washed with water (5.0 mL × 3), dried to obtain the crude product, 4-amino-[1,2,4]triazolo [4,3-a]quinoxaline-8-carboxylic acid(120 mg).
LCMS (ESI) m/z: 230.1 [M+H]$^+$

Int. 94 Synthesis of 5-aminoimidazo[1,5-c]quinazoline-9-carboxylic acid

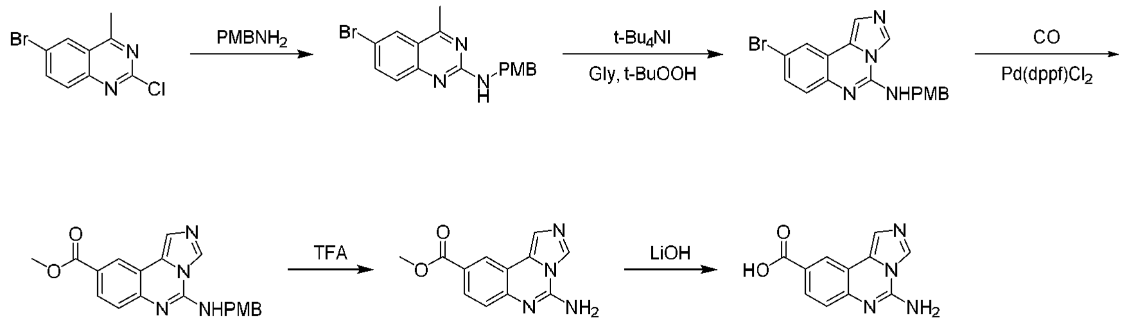


Step 1: Synthesis of 6-bromo-N-(4-methoxybenzyl)-4-methylquinazolin-2-amine

At room temperature, 6-bromo-2-chloro-4-methylquinazoline (0.5 g, 1.942 mmol) was dissolved in DMSO (15 mL) followed by addition of DIEA (768 mg, 5.825 mmol) and 4-methoxybenzylamine (600 mg, 3.883 mmol). The reaction mixture proceeded at room temperature for 16 hours. Then water (50 mL) was added, and extracted with ethyl acetate (20 mL × 3). The organic phase was combined and dried over anhydrous sodium sulfate, filtered. The filtrate was concentrated under reduced pressure and the residue was further purified by column chromatography (0-10% methanol/dichloromethane) to obtain 6-bromo-N-(4-methoxybenzyl)-4-methylquinazolin-2-amine (500 mg, yield: 72%).

LCMS (ESI) m/z: 359.2[M+H]$^+$

Step 2: Synthesis of 9-bromo-N-(4-methoxybenzyl)imidazo[1,5-c]quinazolin-5-amine

At room temperature, 6-bromo-N-(4-methoxybenzyl)-4-methylquinazolin-2-amine (0.5 g, 1.396 mmol) was dissolved in DMSO (20 mL). Glycine (211 mg, 2.792 mmol), tert-butanol hydrogen peroxide (719 mg, 5.583 mmol), tetrabutylammonium iodide (104 mg, 0.2792 mmol), and acetic acid (251 mg, 4.187 mmol) were added successively. The reaction mixture was stirred at 90 °C under nitrogen protection for 16 hours. After the reaction was complete, water (60 mL) was added, followed by extraction with ethyl acetate (30 mL × 3). The organic phases were combined, dried over anhydrous sodium sulfate, filtered, and the filtrate was concentrated under reduced pressure. The product was purified by column chromatography (0-10% methanol/dichloromethane) to yield 9-bromo-N-(4-methoxybenzyl)imidazo[1,5-c]quinazolin-5-amine(300mg, yield: 56%).

LCMS (ESI) m/z: 384.2 [M+H]$^+$

Step 3 Synthesis of methyl 5-((4-methoxybenzyl)amino)imidazo[1,5-c]quinazoline-9-carboxylate

At room temperature, 9-bromo-N-(4-methoxybenzyl)imidazo[1,5-c]quinazolin-5-amine (300 mg, 0.785 mmol) was dissolved in a solution of MeOH (10 mL) and DMF (10 mL). Potassium acetate (230 mg, 2.36 mmol) and Pd(dppf)Cl2 (58 mg, 0.0785 mmol) were added successively. The mixture was stirred at 100 °C for 12 hours under a CO atmosphere. After removing of methanol, water (60 mL) was added, and the solution was extracted with ethyl acetate (30 mL × 3). The organic phases were combined, dried over anhydrous sodium sulfate, filtered, and the filtrate was concentrated under reduced pressure. The product was purified by column chromatography (0-10% methanol/dichloromethane) to yield methyl 5-((4-methoxybenzyl)amino)imidazo[1,5-c]quinazoline-9-carboxylate (200 mg, yield: 56%).

LCMS (ESI) m/z: 363.2 [M+H]$^+$

Step 4: Synthesis of Methyl 5-aminoimidazo[1,5-c]quinazoline-9-carboxylate

At room temperature, methyl 5-((4-methoxybenzyl)amino)imidazo[1,5-c]quinazoline-9-carboxylate (200 mg, 0.5519 mmol) was placed into a 20 mL round-bottom flask, and trifluoroacetic acid (2 mL) was added. The mixture was stirred at 78 °C for 3 hours. After removing trifluoroacetic acid, water (5 mL) was added, and the solution was filtered. The filter cake was washed with water, yielding Methyl 5-aminoimidazo[1,5-c]quinazoline-9-carboxylate(200 mg, crude product).

LCMS (ESI) m/z:243.2 $[M+H]^+$

Step 5: Synthesis of 5-aminoimidazo[1,5-c]quinazoline-9-carboxylic acid

At room temperature, Methyl 5-aminoimidazo[1,5-c]quinazoline-9-carboxylate (200 mg, 0.8257 mmol) was dissolved in a mixed solvent of methanol/tetrahydrofuran/water (9 mL, 4:4:1). Lithium hydroxide (40 mg, 1.651 mmol) was added to the mixture, and the reaction was stirred at 78 °C for 3 hours. After removing trifluoroacetic acid, water (5 mL) was added, and the solution was filtered. The filter cake was washed with water to obtain 5-aminoimidazo[1,5-c]quinazoline-9-carboxylic acid(200 mg, crude product).
LCMS (ESI) m/z:243.2 $[M+H]^+$

Int. 95 Synthesis of 4-aminotetrazolo[1,5-a]quinoxaline-8-carboxylic acid

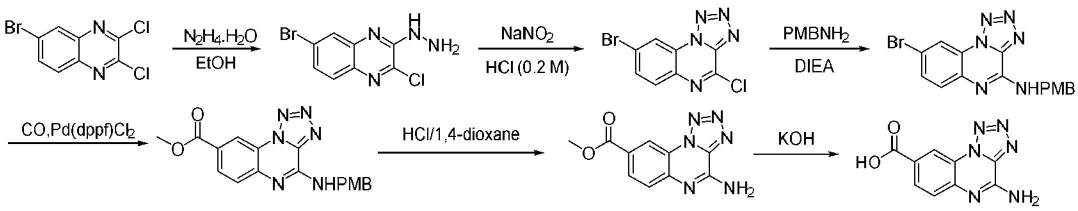


Step 1: Synthesis of 6-bromo-2-chloro-3-hydrazineylquinoxaline

A mixture of 6-bromo-2,3-dichloroquinoxaline (6.00 g, 21.73 mmol) and hydrazine hydrate (1.15 g, 35.98 mmol) in ethanol (50.00 mL) was stirred for 1 hour at 0 °C and then for 2 hours at room temperature. After filtration, washing with ethanol (100 mL) yielded 6-bromo-2-chloro-3-hydrazineylquinoxaline (5.00 g, 18.31 mmol).

LCMS (ESI): 273 $[M+H]^+$

Step 2: Synthesis of 8-bromo-4-chlorotetrazolo[1,5-a]quinoxaline

A mixture of 6-bromo-2-chloro-3-hydrazineylquinoxaline (5.00 g, 18.31 mmol) and sodium nitrite (2.52 g, 36.56 mmol) in hydrochloric acid solution (0.2 M, 50.00 mL) was stirred for 2 hours at room temperature. The mixture was extracted with ethyl acetate (100 mL × 3), and the organic phase was concentrated under vacuum. Further purification by silica gel column chromatography (petroleum ether: ethyl acetate = 70%) yielded 8-bromo-4-chlorotetrazolo[1,5-a] quinoxaline (4.00 g, 18.28 mmol).

LCMS (ESI): 284$[M+H]^+$

Step 3: Synthesis of 8-bromo-N-(4-methoxybenzyl)tetrazolo[1,5-a]quinoxalin-4-amine

A mixture of 8-bromo-4-chlorotetrazolo[1,5-a]quinoxaline (4.00 g, 14.13 mmol) with para-methoxybenzylamine (3.87 g, 28.26 mmol) and N,N-diisopropylethylamine (7.30 g, 56.52 mmol) in dichloromethane (50 mL) was stirred for 2 hours at room temperature. After extraction with ethyl acetate (100 mL × 3), the organic phase was concentrated under vacuum. Further purification by silica gel column chromatography (petroleum ether:ethyl acetate = 30%) yielded 8-bromo-N-(4-methoxybenzyl)tetrazolo[1,5-a]quinoxalin-4-amine (3.00 g, 7.81 mmol).

LCMS (ESI): 385$[M+H]^+$

Step 4: Synthesis of methyl 4-((4-methoxybenzyl)amino)tetrazolo[1,5-a]quinoxaline-8-carboxylate

8-bromo-N-(4-methoxybenzyl)tetrazolo[1,5-a]quinoxalin-4-amine (3.00 g, 7.81 mmol), potassium acetate (1.53

g, 15.61 mmol), and 1,1'-Bis(diphenylphosphino)ferrocene-palladium(II)dichloride dichloromethane complex (0.63 g, 0.77 mmol) were successively dissolved in a mixed solvent of N,N-dimethylformamide and methanol (1:1, 30 mL). The reaction proceeded overnight in a high-pressure vessel under carbon monoxide (4 mPa), at 100 °C. After filtration, extraction with water (100 mL) and ethyl acetate (100 mL × 3), and vacuum concentration of the organic phase, methyl 4-((4-methoxybenzyl)amino)tetrazolo[1,5-a]quinoxaline-8-carboxylate(3.00 g, 8.24 mmol) was obtained.

LCMS (ESI): 365[M+H] +

Step 5: Synthesis of methyl 4-aminotetrazolo[1,5-a]quinoxaline-8-carboxylate

Methyl 4-((4-methoxybenzyl)amino)tetrazolo[1,5-a]quinoxaline-8-carboxylate (3.00 g, 8.24 mmol) was dissolved in hydrochloric acid/dioxane (4M, 50 mL) solution. The reaction was stirred at room temperature for 2 hours. After extraction with water (100 mL) and ethyl acetate (100 mL × 3), the organic phase was concentrated under vacuum. Further purification by silica gel column chromatography (petroleum ether: ethyl acetate = 50%) yielded methyl 4-aminotetrazolo[1,5-a]quinoxaline-8-carboxylate (2.00 g, 8.16 mmol).

LCMS (ESI): 245[M+H] +

Step 6: Synthesis of 4-aminotetrazolo[1,5-a]quinoxaline-8-carboxylic acid

methyl 4-aminotetrazolo[1,5-a]quinoxaline-8-carboxylate (2.00 g, 8.16 mmol) and potassium hydroxide (0.91 g, 16.32 mmol) were successively dissolved in a mixed solvent: tetrahydrofuran (10 mL), water (10 mL), and methanol (10 mL). The reaction was stirred at 60 °C for 2 hours. After extraction with water (50 mL) and ethyl acetate (50 mL × 3), the organic phase was concentrated under vacuum to yield 4-aminotetrazolo[1,5-a]quinoxaline-8-carboxylic acid (1.00 g, 4.34 mmol).

LCMS (ESI): 231[M+H] +

Int. 96 Synthesis of 6-aminoimidazo[1,5-a]pyrido[3,2-e]pyrazine-2-carboxylic acid

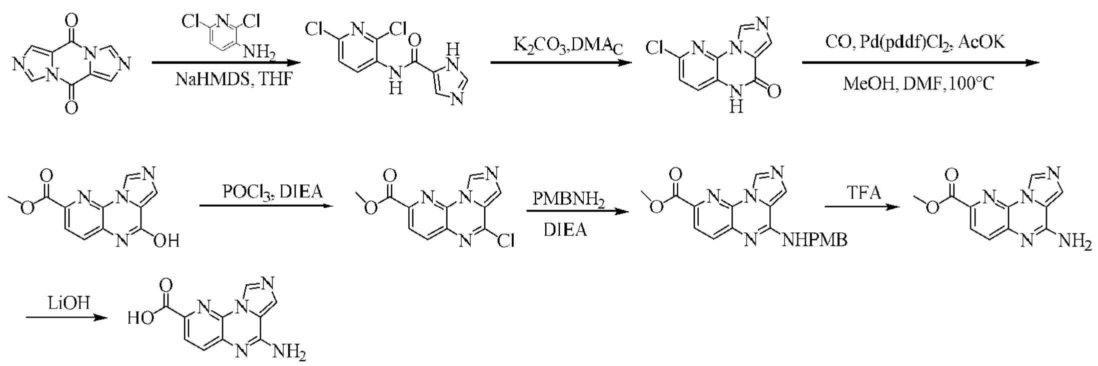


Step 1: Synthesis of N-(2,6-dichloropyridin-3-yl)-1H-imidazole-5-carboxamide

2,6-dichloropyridin-3-amine (163 mg, 1.0 mmol) was dissolved in tetrahydrofuran (5.0 mL). After the reaction system was cooled by an ice bath for 5 minutes, NaHMDS (0.125 mL, 2.0 M, 0.25 mmol) was slowly added to the reaction mixture and stirred for 1 hour. Then, 5H,10H-diimidazo[1,5-a:1',5'-d]pyrazine-5,10-dione (188.0 mg, 1.0 mmol) was added, and the reaction mixture was stirred overnight at room temperature. The reaction progress was monitored by LCMS. After completion, the reaction mixture was poured into water, adjusted to pH = 7, resulting in the precipitation of solid. The solid was collected by filtration, dried, yielding crude N-(2,6-dichloropyridin-3-yl)-1H-imidazole-5-carboxamide(175 mg, crude product).

LCMS (ESI) m/z:257.0[M+H]+

Step 2: Synthesis of 2-chloroimidazo[1,5-a]pyrido[3,2-e]pyrazin-6(5H)-one

N-(2,6-dichloropyridin-3-yl)-1H-imidazole-5-carboxamide (256.0 mg, 1.0 mmol) was dissolved in N,N-dimethylacetamide (2.0 mL). Potassium carbonate (276 mg, 2.0 mmol) was added, and the reaction was heated to 140°C, stirred for 2 hours. After completion indicated by LCMS, the reaction mixture was cooled to room temperature, poured into water,

stirred for 30 minutes, leading to the precipitation of solid. The solid was collected by filtration, yielding crude 2-chloroimidazo[1,5-a]pyrido[3,2-e]pyrazin-6(5H)-one (153.0 mg, crude product).

LCMS (ESI) m/z:221.1[M+H]$^+$

Step 3: Synthesis of methyl 6-hydroxyimidazo[1,5-a]pyrido[3,2-e]pyrazine-2-carboxylate

2-chloroimidazo[1,5-a]pyridine[3,2-e]pyrimidin-6(5H)-one (2.0 g, 9.09 mmol) was dissolved in a solution of MeOH (30.00 mL) and DMF (310.00 mL), followed by the addition of potassium acetate (1.78 g, 18.18 mmol) and Pd(dppf) Cl2 (660 mg, 0.91 mmol). Under a CO atmosphere, the mixture was stirred at 100°C for 12 hours. After removing of methanol, the reaction mixture was added to 100 mL of water, resulting in the precipitation of solid. The solid was collected by filtration, yielding crude product. The crude product was slurried with ethyl acetate (50 mL × 2) to obtain the product, methyl 6-hydroxyimidazo[1,5-a]pyrido[3,2-e]pyrazine-2-carboxylate (1.5 g, yield: 68%), as a brown solid.

LCMS(ESI) m/z: 245.2 [M+H]$^+$

Step 4: Synthesis of Methyl 6-chloroimidazo[1,5-a]pyrido[3,2-e]pyrazine-2-carboxylate

Methyl 6-hydroxyimidazo[1,5-a]pyrido[3,2-e]pyrazine-2-carboxylate (200.0 mg, 0.82 mmol) was added to phosphorus oxychloride (2.0 mL), and N,N-diisopropylethylamine (528 mg, 4.09 mmol) was added dropwise. The reaction was heated to 90°C and maintained for 2.5 hours. After LCMS indicated completion, the mixture was evaporated to remove phosphorus oxychloride. The resulting solution was poured into ice-water, stirred for 15 minutes, filtered, and the filter cake was collected, dried, yielding the crude product of Methyl 6-chloroimidazo[1,5-a]pyrido[3,2-e]pyrazine-2-carboxylate(200.0mg, crude product).

LCMS (ESI) m/z:263.0[M+H]$^+$

Step 5: Synthesis of methyl 6-((4-methoxybenzyl)amino)imidazo[1,5-a]pyrido[3,2-e]pyrazine-2-carboxylate

Methyl 6-chloroimidazo[1,5-a]pyrido[3,2-e]pyrazine-2-carboxylate (200.0 mg, 0.76 mmol) was dissolved in DMSO (3.0 mL), and para-methoxybenzylamine (115.0 mg, 0.92 mmol) and N,N-diisopropylethylamine (295 mg, 2.29 mmol) were added successively. The reaction was heated to 90°C and stirred for 16 hours. After completion, the reaction mixture was cooled to room temperature, poured into ice-water, stirred, and then extracted with ethyl acetate (20 mL). The organic phase was dried over anhydrous sodium sulfate, filtered, and the filtrate was concentrated. The crude product was purified by silica gel column chromatography (dichloromethane:methanol = 0-5%), yielding methyl 6-((4-methoxybenzyl) amino)imidazo[1,5-a]pyrido[3,2-e]pyrazine-2-carboxylate(250mg, 91% yield).

LCMS(ESI) m/z:364.2[M+H]$^+$

Step 6: Synthesis of Methyl 6-aminoimidazo[1,5-a]pyrido[3,2-e]pyrazine-2-carboxylate

Methyl 6-((4-methoxybenzyl)amino)imidazo[1,5-a]pyrido[3,2-e]pyrazine-2-carboxylate (250 mg, 0.69 mmol) was dissolved in trifluoroacetic acid (TFA) (5.0 mL), and the reaction was heated to 80 °C for 16 hours. After complete reaction, the mixture was cooled, and TFA was removed by evaporation, resulting in the crude product Methyl 6-aminoimidazo[1,5-a]pyrido[3,2-e]pyrazine-2-carboxylate (160 mg, crude).

LCMS (ESI) m/z:244.1[M+H]$^+$

Step 7: Synthesis of 6-aminoimidazo[1,5-a]pyrido[3,2-e]pyrazine-2-carboxylic acid

Methyl 6-aminoimidazo[1,5-a]pyrido[3,2-e]pyrazine-2-carboxylate (83.0 mg, yield: 55%) was dissolved in a mixture of THF/MeOH (1.2 mL/1.2 mL), and lithium hydroxide solution (0.66 mL, 2.0 M, 1.32 mmol) was added. The reaction was heated to 50°C and stirred for 16 hours. After complete reaction, the solvent was evaporated, and the pH of the reaction mixture was adjusted to around 7 using 1 N HCl aqueous solution. After stirring for 15 minutes, the mixture was filtered, and the solid product was collected to obtain 6-aminoimidazo[1,5-a]pyrido[3,2-e]pyrazine-2-carboxylic acid(83mg, yield: 55%)

LCMS (ESI) m/z:230.1[M+H]$^+$

Int. 97 Synthesis of 4-amino-6-methylimidazo[1,5-a]quinoxaline-8-carboxylic acid

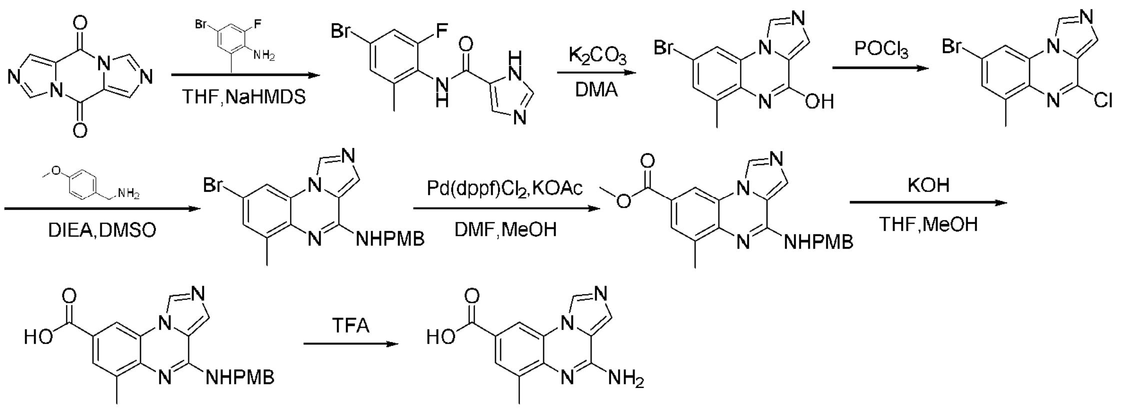


Step 1: Synthesis of N-(4-bromo-2-fluoro-6-methylphenyl)-1H-imidazole-5-carboxamide

At room temperature, 4-bromo-2-fluoro-6-methylaniline (15 g, 73.89 mmol) was added to a 500 mL round-bottom flask, followed by the addition of tetrahydrofuran (150 mL). At 0 °C, di(trimethylsilyl)amine (74 mL) was added, and after stirring the resulting mixture at 0 °C for half an hour, 5H,10H-diimidazo[1,5-a:1',5'-d]pyrazine-5,10-dione (6.95 g, 36.95 mmol) was added. The mixture proceeded for an additional hour at room temperature. Then the reaction mixture was poured into ice water, filtered to obtain the red solid residue as the product, N-(4-bromo-2-fluoro-6-methylphenyl)-1H-imidazole-5-carboxamide (10 g, yield: 90.9%).

LCMS (ESI)m/z: 298 $[M+H]^+$

Step 2: Synthesis of 8-bromo-6-methylimidazo[1,5-a]quinoxalin-4-ol

At room temperature, N-(4-bromo-2-fluoro-6-methylphenyl)-1H-imidazole-5-carboxamide (10 g, 33.67 mmol) was added to a 250 mL round-bottom flask. Then, N,N-dimethylacetamide (100 mL) was poured into the flask, then potassium carbonate (13.94 g, 101.01 mmol) was added. The resulting mixture was stirred at 140 °C for 2 hours. After the reaction was complete, the reaction mixture was poured into water to adjust the pH to 3-4, filtered, and the black solid residue was collected as the product, obtaining 8-bromo-6-methylimidazo[1,5-a]quinoxalin-4-ol (8 g, yield: 85.74%).

LCMS (ESI)m/z: 278 $[M+H]^+$

Step 3: Synthesis of 8-bromo-4-chloro-6-methylimidazo[1,5-a]quinoxaline

At room temperature, 8-bromo-6-methylimidazo[1,5-a]quinoxalin-4-ol (8 g, 28.88 mmol) was added to a 250 mL round-bottom flask. Then, phosphorus oxychloride (100 mL) was added into the flask, and the resulting mixture was stirred at 120 °C for 12 hours. The reaction solution was concentrated under reduced pressure, poured into ice water, filtered, and the black solid residue was collected as the product, obtaining 8-bromo-4-chloro-6-methylimidazo[1,5-a]quinoxaline(7.5 g, yield: 88.23%).

LCMS (ESI)m/z: 296 $[M+H]^+$

Step 4: Synthesis of 8-bromo-N-(4-methoxybenzyl)-6-methylimidazo[1,5-a]quinoxalin-4-amine

At room temperature, 8-bromo-4-chloro-6-methylimidazo[1,5-a]quinoxaline (7.5 g, 25.42 mmol) was added to a 250 mL round-bottom flask. Then, dimethyl sulfoxide (75 mL) was poured into the flask, and (4-methoxyphenyl) methanamine (4.18 g, 30.51 mmol) and N,N-diisopropylethylamine (6.56 g, 50.85 mmol) were added. The resulting mixture was stirred at 90 °C for 2 hours. The reaction solution was concentrated under reduced pressure, poured into ice water, filtered, and the red solid residue was collected as the product, obtaining 8-bromo-N-(4-methoxybenzyl)-6-methylimidazo[1,5-a]quinoxalin-4-amine (6 g, yield: 60%).

LCMS (ESI)m/z: 397$[M+H]^+$

Step 5: Synthesis of methyl 4-((4-methoxybenzyl)amino)-6-methylimidazo[1,5-a]quinoxaline-8-carboxylate

At room temperature, 8-bromo-N-(4-methoxybenzyl)-6-methylimidazo[1,5-a]quinoxalin-4-amine (6 g, 15.15 mmol) was added to a 250 mL high-pressure reaction vessel. Then, N,N-dimethylformamide (30 mL) and methanol (60 mL) were poured into the reaction vessel, and potassium acetate (2.97 g, 30.3 mmol) and 1,1'-Bis(diphenylphosphino) ferrocene]dichloropalladium(II) (1.24 g, 1.52 mmol) were added. Carbon monoxide (4 MPa) was introduced, and the

resulting mixture was stirred at 100 °C for 12 hours. The reaction solution was concentrated under reduced pressure, poured into water, filtered, and the red solid residue was collected as the product, obtaining methyl 4-((4-methoxybenzyl)amino)-6-methylimidazo[1,5-a]quinoxaline-8-carboxylate(4g, yield: 70.54%).

LCMS (ESI)m/z: 377$[M+H]^+$

Step 6: Synthesis of 4-((4-methoxybenzyl)amino)-6-methylimidazo[1,5-a]quinoxaline-8-carboxylic acid

At room temperature, methyl 4-((4-methoxybenzyl)amino)-6-methylimidazo[1,5-a]quinoxaline-8-carboxylate (4 g, 10.61 mmol) was added to a 250 mL round-bottom flask. Then, tetrahydrofuran (40 mL) and methanol (40 mL) were poured into the flask, and potassium hydroxide (1.19 g, 21.22 mmol) was added. The resulting mixture was stirred at 60 °C for 1 hour. The reaction solution was concentrated under reduced pressure, poured into water, and the pH was adjusted to around 3 using formic acid, resulting in solid precipitation. The mixture was filtered, and the red solid residue was collected as the product, obtaining 4-((4-methoxybenzyl)amino)-6-methylimidazo[1,5-a]quinoxaline-8-carboxylic acid(2.9 g, yield: 76.31%).

LCMS (ESI)m/z: 363$[M+H]^+$

Step 7: Synthesis of 4-amino-6-methylimidazo[1,5-a]quinoxaline-8-carboxylic acid

At room temperature, 4-((4-methoxybenzyl)amino)-6-methylimidazo[1,5-a]quinoxaline-8-carboxylic acid (2.9 g, 7.99 mmol) was added to a 50 mL round-bottom flask. Then, trifluoroacetic acid (30 mL) was poured into the flask, and the resulting mixture was stirred at 100 °C for 3 hours. The reaction solution was concentrated under reduced pressure, yielding a black solid, 4-amino-6-methylimidazo[1,5-a]quinoxaline-8-carboxylic acid(1.5 g, yield: 51.28%).

LCMS (ESI)m/z:243$[M+H]^+$

Int. 98 Synthesis of 4-amino-1-cyclopropylimidazo[1,5-a]quinoxaline-8-carboxylic acid

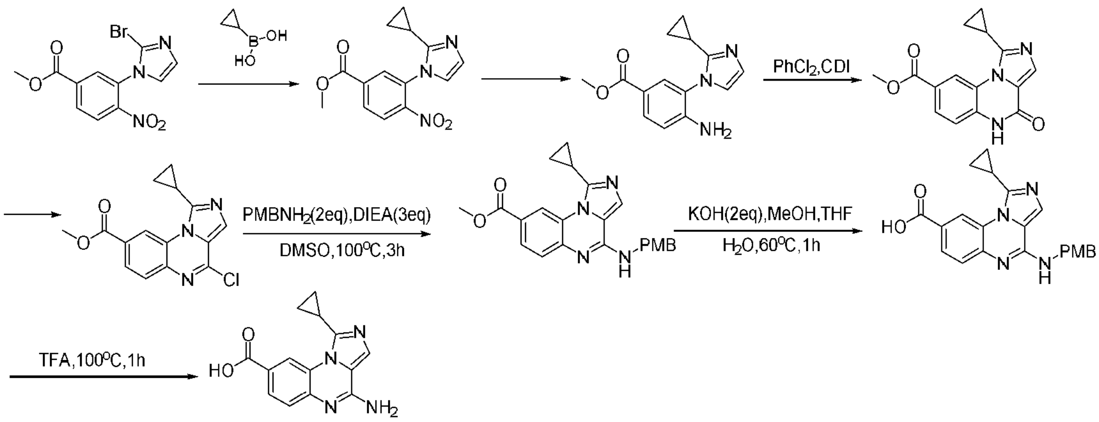


Step 1: Synthesis of methyl 3-(2-cyclopropyl-1H-imidazol-1-yl)-4-nitrobenzoate

A mixture of 3 methyl 3-(2-bromo-1H-imidazol-1-yl)-4-nitrobenzoate (1.00 g, 3.07 mmol), cyclopropylboronic acid (0.26 g, 3.07 mmol), Pd(dppf)Cl2 (0.25 g, 0.31 mmol), and potassium carbonate (0.85 g, 6.13 mmol) in dioxane (10 mL) was stirred at 100 ° C for 2 hours. After completion of the reaction, the reaction mixture was poured into water (10 mL), extracted with ethyl acetate (10 mL × 2), and the combined organic layers were washed with saturated brine (10 mL), dried over anhydrous sodium sulfate, filtered, and concentrated to obtain the crude product. The crude product was purified by column chromatography (ethyl acetate: petroleum ether = 10:90), yielding methyl 3-(2-cyclopropyl-1H-imidazol-1-yl)-4-nitrobenzoate(0.50 g, yield: 57%).

LCMS (ESI): 288 $[M+H]^+$

Step 2: Synthesis of methyl 4-amino-3-(2-cyclopropyl-1H-imidazol-1-yl)benzoate

A mixture of methyl 3-(2-cyclopropyl-1H-imidazol-1-yl)-4-nitrobenzoate (1.00 g, 3.48 mmol), boronic acid (0.94 g, 10.44 mmol), and 4,4'-bipyridine (0.27 g, 1.74 mmol) in N,N-dimethylformamide (10.00 mL) was stirred at room

temperature for 2 hours. The reaction mixture was then concentrated, poured into water (10 mL), extracted with ethyl acetate (10 mL × 2), and the combined organic layers were washed with saturated brine (10 mL), dried over anhydrous sodium sulfate, filtered, and concentrated to obtain the crude product. The crude product was purified by column chromatography (ethyl acetate: petroleum ether = 53%), yielding methyl 4-amino-3-(2-cyclopropyl-1H-imidazol-1-yl) benzoate (0.60 g, yield: 67%).

LCMS (ESI): 257 $[M+H]^+$

Step 3: Synthesis of methyl 1-cyclopropyl-4-oxo-4,5-dihydroimidazo[1,5-a]quinoxaline-8-carboxylate

A mixture of methyl 4-amino-3-(2-cyclopropyl-1H-imidazol-1-yl)benzoate (500 mg, 1.94 mmol) and carbonyl imidazole (377 mg, 2.33 mmol) in chlorobenzene (5 mL) was stirred at 140°C for 12 hours. After concentration, the reaction mixture was poured into water (10 mL), extracted with ethyl acetate (10 mL × 2), and the combined organic layers were washed with saturated brine (10 mL), dried over anhydrous sodium sulfate, filtered, and concentrated to obtain the crude product. The crude product was purified by column chromatography (ethyl acetate: petroleum ether = 20%), yielding methyl 1-cyclopropyl-4-oxo-4,5-dihydroimidazo[1,5-a]quinoxaline-8-carboxylate(500mg, yield: 91%).

LCMS (ESI): 283 $[M+H]^+$

Step 4: Synthesis of methyl 4-chloro-1-cyclopropylimidazo[1,5-a]quinoxaline-8-carboxylate

A solution of methyl 1-cyclopropyl-4-oxo-4,5-dihydroimidazo[1,5-a]quinoxaline-8-carboxylate (500.00 mg, 1.77 mmol) in phosphorus oxychloride (5.00 mL) was stirred at 120 °C for 12 hours. After concentration, methyl 4-chloro-1-cyclopropylimidazo[1,5-a]quinoxaline-8-carboxylate (300 mg, crude) was obtained.

LCMS (ESI): 301 $[M+H]^+$

Step 5: Synthesis of methyl 1-cyclopropyl-4-((4-methoxybenzyl)amino)imidazo[1,5-a]quinoxaline-8-carboxylate

A solution of the mixture of methyl 4-chloro-l-cyclopropylimidazo[1,5-a]quinoxaline-8-carboxylate(300 mg, 0.99 mmol), p-methoxybenzylamine (417 mg, 2.98 mmol), and diisopropylamine (384 mg, 2.98 mmol) in dimethyl sulfoxide (3 mL, 0.00 mmol) was stirred at 120 °C for 2 hours. After concentration, the reaction mixture was poured into water (10 mL), and extracted with ethyl acetate (10 mL × 2). The combined organic layers were washed with saturated brine (10 mL), dried over anhydrous sodium sulfate, filtered, and concentrated to obtain the crude product. The crude product was purified by column chromatography (ethyl acetate:petroleum ether = 42%) to yield methyl 1-cyclopropyl-4-((4-methoxybenzyl)amino)imidazo[1,5-a]quinoxaline-8-carboxylate(300mg, yield: 75%).

LCMS (ESI): 402 $[M+H]^+$

Step 6: Synthesis of 1-cyclopropyl-4-((4-methoxybenzyl)amino)imidazo[1,5-a]quinoxaline-8-carboxylic acid

Lithium hydroxide (89.45 mg, 2.24 mmol) was added to a solution of methyl 1-cyclopropyl-4-((4-methoxybenzyl) amino)imidazo[1,5-a]quinoxaline-8-carboxylate (300 mg, 0.75 mmol) in methanol/tetrahydrofuran (5.00 mL/5.00 mL). The reaction mixture was stirred at 60°C for 2 hours, then concentrated to obtain 1-cyclopropyl-4-((4-methoxybenzyl) amino)imidazo[1,5-a]quinoxaline-8-carboxylic acid(160 mg, yield: 55%).

LCMS (ESI): 388 $[M+H]^+$

Step 7: Synthesis of 4-amino-1-cyclopropylimidazo[1,5-a]quinoxaline-8-carboxylic acid

A mixture of 1-cyclopropyl-4-((4-methoxybenzyl)amino)imidazo[1,5-a]quinoxaline-8-carboxylic acid (200 mg, 0.51 mmol) in trifluoroacetic acid (2.00 mL) was heated at 100°C for 2 hours, then concentrated to obtain 4-amino-1-cyclopropylimidazo[1,5-a]quinoxaline-8-carboxylic acid (100 mg, crude).

LCMS (ESI):268 $[M+H]^+$

**Example 1**

4-amino-N-(2,3-dihydrobenzofuran-3 -yl)-N-methylimidazo[1,5-a]quinoxaline-8-carboxamide

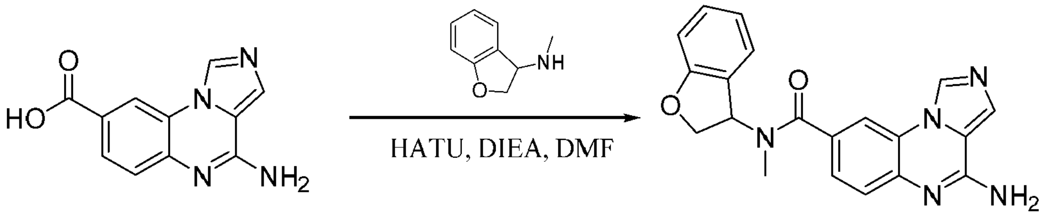


4-aminoimidazo[1,5-a]quinoxaline-8-carboxylic acid(50mg, 0.21mmol) was dissolved in DMF(2.5ml) followed by addition of 2-(7-Azabenzotriazol-1-yl)-N,N,N',N'-tetramethyluronium hexafluorophosphate(125mg, 0.32mmol), N,N-diisopropylethylamine(85mg, 0.65mmol) and N-methyl-2,3-dihydrobenzofuran-3-amine(33mg, 0.21mmol) successively. The reaction mixture was stirred at room temperature for 0.5h. After completion of the reaction, the reaction solution was concentrated and further purified by HPLC(Column: Xbridge BEH Shield RP18 5m, 30mm× 150mm; mobile phase A: water(10mmol/L ammonium bicarbonate), mobile phase B: acetonitrile, flow rate: 60ml/min; Gradient: From 20% B to 36% B within 8 minutes; Wavelength: 254 nanometers/220 nanometers.) to obtain 4-amino-N-(2,3-dihydrobenzofuran-3-yl)-N-methylimidazo[1,5-a]quinoxaline-8-carboxamide

$^1$H NMR (400 MHz, DMSO-$d_6$) δ 9.19 (s, 1H), 8.33 (s, 1H), 7.92 (s, 1H), 7.48-7.41 (m, 5H), 7.30-7.26 (m, 1H), 7.00-6.99 (m, 1H),6.91-6.89 (M, 1H), 6.35-5.76 (m, 1H), 4.79-4.56 (m, 2H), 2.68 (s, 3H). LCMS (ESI): 360.30 [M+H]$^+$

By employing the synthetic steps described in Example 1, with only the replacement of corresponding starting materials, embodiments listed in the table below can be prepared:

| Ex. | Structure | Name | $^1$HNMR&$^{19}$F NMR& LCMS |
|---|---|---|---|
| 2 | 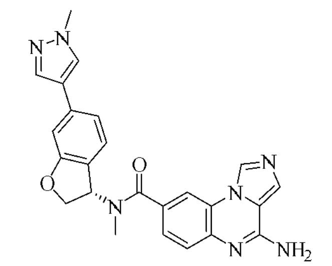 | (S)-4-amino-N-methy 1-N-(6-(1-methyl-1H-pyrazol-4-yl)-2,3-dihy drobenzofuran-3-yl)i midazo[1,5-a]quinoxa line-8-carboxamide | $^1$HNMR (400 MHz, DMSO-$d_6$): δppm 9.11(s, 1H),8.25(s, 1H),8.07(s, 1H),7.84-7.79 (m, 2H), 7.40-7.36(m, 4H), 7.31-7.29(m, 1H), 7.12-7.10(m, 1H), 7.02(s, 1H), 6.21-4.61(m,1H), 4.60-4.54(m, 1H), 3.66(s, 1H),2.52(s, 1H). LCMS(ESI)m/z:440.35[M+H]$^-$ |
| 3 | 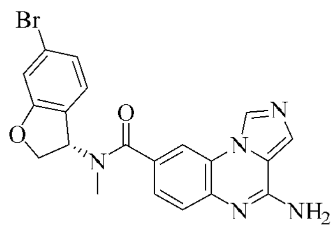 | (S)-4-amino-N-(6-bromo-2,3-dihydrobenzofuran-3-yl)-N-methyli midazo[1,5-a]quinoxaline-8-carboxamide | $^1$H NMR (400 MHz, DMSO-$d_6$) δ 9.17 (s, 1H), 8.31 (s, 1H), 7.91 (s, 1H), 7.50-7.45 (m, 4H), 7.38-7.37 (m, 1H), 7.16 (s, 2H), 6.33-5.59 (m, 1H), 4.80-4.55 (m, 2H), 2.71 (s, 3H). LCMS (ESI) m/z: 438.00 [M+H]$^+$ |
| 4 | 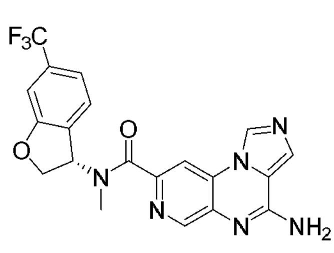 | (S)-4-amino-N-methy 1-N-(6-(trifluoromethy 1)-2,3-dihydrobenzofuran-3-yl)imidazo[1,5-a] pyrido[3,4-e]pyrazi ne-8-carboxamide | $^1$H NMR (400 MHz, DMSO-$d_6$) δ 9.31 (d, *J* = 7.8 Hz, 1H), 8.67 (d, *J* = 19.6 Hz, 1H), 8.53 (d, *J* = 19.7 Hz, 1H), 7.99 (d, *J* = 3.8 Hz, 1H), 7.89 - 7.65 (m, 2H), 7.57 (d, *J* = 7.8 Hz, 1H), 7.45 - 7.19 (m, 2H), 6.51 - 5.98 (m, 1H), 4.95 - 4.41 (m, 2H), 2.72 (d, *J= 25.9* Hz, 3H). LCMS (ESI) m/z: 429.1 [M+H]$^-$ |
| 5 | 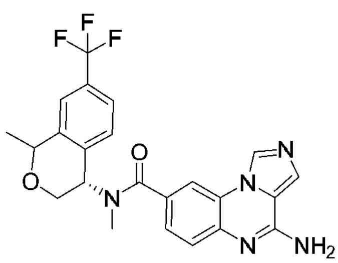 | 4-amino-N-methyl-N-((4S)-1-methyl-7-(trifluoromethyl)isochroman-4-yl)imidazo[1,5-a ]quinoxaline-8-carbox amide | $^1$H NMR (400 MHz, DMSO-$d_6$) δ 9.19 (d, *J* = 18.9 Hz, 1H), 8.37 (d, *J*= 12.5 Hz, 1H), 7.93 (s, 1H), 7.73 - 7.41 (m, 7H), 5.78 (d, *J* = 37.1 Hz, 1H), 4.77 (d, *J* = 40.0 Hz, 2H), 4.42 - 3.87 (m, 2H), 2.79 (s, 3H), 1.56 (t, *J* = 7.8 Hz, 3H). $^{19}$F NMR (376 MHz, DMSO-$d_6$) δ-60.92. LCMS (ESI) m/z: 456 [M+H]$^+$ |

(continued)

| Ex. | Structure | Name | $^1$HNMR&$^{19}$F NMR& LCMS |
|---|---|---|---|
| 6 | 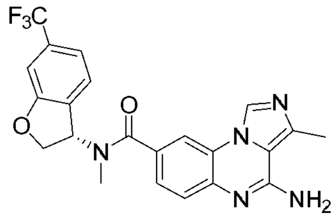 | (S)-4-amino-N,3-dimethyl-N-(6-(trifluoromethyl)-2,3-dihydrobenzofuran-3-yl)imidazo[1 ,5-a]quinoxaline-8-car boxamide | $^1$H NMR (400 MHz, DMSO-$d_6$) δ 9.05 (s, 1H), 8.28 - 8.23 (m, 1H), 7.64 (d, *J* = 7.8 Hz, 1H), 7.47 - 7.38 (m, 2H), 7.33 (d, *J* = 7.9 Hz, 1H), 7.26 (s, 1H), 6.90 (s, 2H), 6.38 (s, 1H), 4.81 (s, 1H), 4.71 (dd, *J* = 10.3, 4.4 Hz, 1H), 2.67 (s, 3H), 2.63 (s, 3H).<br>$^{19}$F NMR (376 MHz, DMSO-$d_6$) δ -60.80.<br>LCMS (ESI) m/z: 442 [M+H]$^+$ |
| 7 | 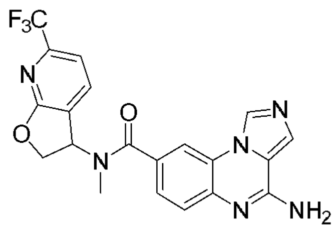 | 4-amino-N-methyl-N-(6-(trifluoromethyl)-2, 3-dihydrofuro[2,3-b]pyridin-3-yl)imidazo[1, 5-a]quinoxaline-8-car boxamide | $^1$H NMR (400 MHz, DMSO-$d_6$) δ 9.17 (s, 1H), 8.33 (s, 1H), 8.13 *(d, J*= 7.4 Hz, 1H), 7.92(s, 1H), 7.55 -7.42 (m, 5H), 6.49 - 6.03 (m, 1H), 5.01 - 4.84 (m, 1H), 4.79 - 4.69 (m, 1H), 2.78 (s, 3H).<br>LCMS (ESI) m/z: 429.20 [M+H]$^+$ |
| 8 | 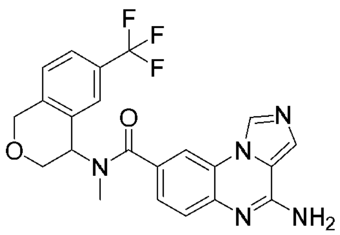 | 4-amino-N-methyl-N-(6-(trifluoromethyl)isochroman-4-yl)imidaz o[1,5-a]quinoxaline-8 -carboxamide | $^1$H NMR (400 MHz, DMSO-ds) δ 9.20(s, 1H), 8.37(s, 1H), 7.91(s, 1H), 7.68-7.66(m, 2H), 7.51-7.44(m, 5H), 5.77(s, 1H), 4.95-4.91(m, 1H), 4.76-4.72(m, 1H), 4.22-4.11(m, 2H), 2.77(s, 3H). |
| 9 | 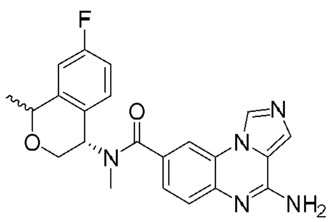 | 4-amino-N-((4S)-7-fluoro-1-methylisochroman-4-yl)-N-methylimidazo[1,5-a]quinoxaline-8-carboxamide | $^1$H NMR (400 MHz, DMSO-$d_6$) δ 9.18(d, 1H), 8.35(d, 1H), 7.91(s, 1H), 7.47-7.42(m, 5H), 7.18-7.13(m, 2H), 5.76-5.63(m, 1H), 4.91-4.61(m, 2H), 4.34-3.90(m, 2H), 2.75-2.69(m, 3H), 1.53-1.33(m, 3H).<br>LCMS (ESI) m/z:406.3[M+H]$^+$ |
| 10 | 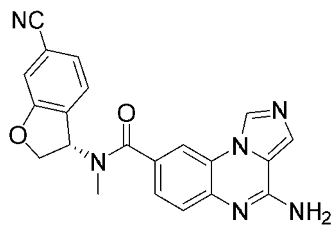 | (S)-4-amino-N-(6-cyano-2,3-dihydrobenzofuran-3-yl)-N-methylimidazo[1,5-a]quinoxaline-8-carboxamide | $^1$H NMR (400 MHz, DMSO-$d_6$) δ 9.17 (s, 1H), 8.34-8.32 (m, 1H), 7.91 (s, 1H), 7.63-7.61 (m, 1H), 7.47-7.42 (m, 6H), 6.52-5.81 (m, 1H), 4.81-4.68 (m, 2H), 2.71 (s, 3H).<br>LCMS (ESI): 385.05 [M+H]$^+$ |
| 11 | 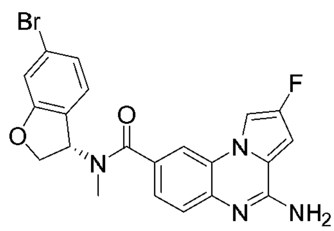 | (S)-4-amino-N-(6-bromo-2,3-dihydrobenzofuran-3-yl)-2-fluoro-N-methylpyrrolo[1,2-a]quinoxaline-8-carbox amide | $^1$H NMR (400 MHz, DMSO-$d_6$) δ 8.40 (s, 1H), 8.10 (d, *J* = 1.6 Hz, 1H), 7.45-7.44 (m, 1H), 7.42 - 7.33 (m, 2H), 7.16 (s, 4H), 6.95 (d, *J* = 1.8 Hz, 1H), 4.80-4.61(m,3H), 2.65 (s, 3H).<br>LCMS (ESI) m/z: **455.20**[M+H]$^-$ |

(continued)

| Ex. | Structure | Name | $^1$HNMR&$^{19}$F NMR& LCMS |
|---|---|---|---|
| 12 | 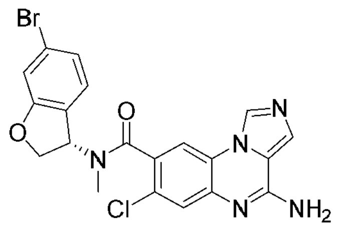 | (S)-4-amino-N-(6-bromo-2,3-dihydrobenzofuran-3-yl)-7-chloro-N-methylimidazo[1,5-a] quinoxaline-8-carbox amide | $^1$H NMR (400 MHz, DMSO-$d_6$) δ 9.20-9.06 (m, 1H), 8.54-8.68 (m, 1H), 7.93-7.92 (m, 1H), 7.64 - 7.58(m, 2H), 7.54-7.46 (m, 1H), 7.43 - 7.25 (m, 1H), 7.20 (s, 1H), 7.16-7.12 (m, 1H), 6.88-6.44 (m, 1H), 5.42 - 5.30 (m, 1H), 4.85-4.62 (m, 1H), 2.05-1.95 (s, 3H). LCMS (ESI) m/z: 472.15 [M+H]$^+$. |
| 14 | 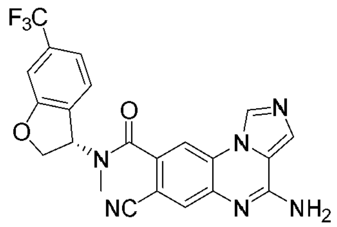 | (S)-4-amino-7-cyano-N-methyl-N-(6-(triflu oromethyl)-2,3-dihydr obenzofuran-3-yl)imi dazo[1,5-a]quinoxalin e-8-carboxa-mide | $^1$H NMR (400 MHz, DMSO-$d_6$) δ 9.21 (s, 1H), 8.55-8.44 (m, 1H), 7.97-7.90(m, 2H), 7.76 (s, 2H), 7.60-7.59 (m, 1H), 7.38-7.36 (m, 1H), 7.30-7.26 (m, 1H), 6.53-5.65 (m, 1H), 4.94-4.68 (m, 2H), 2.73-2.62 (m, 3H). LCMS (ESI): 452.95[M+H]$^+$ |
| 15 | 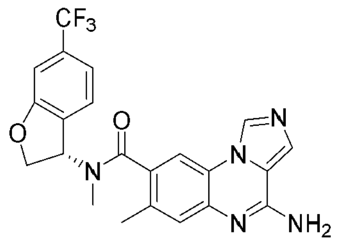 | (S)-4-amino-N,7-dime thyl-N-(6-(trifluorome thyl)-2,3-dihydrobenz ofuran-3-yl)imidazo[1 ,5-a] quinoxaline-8-car boxa-mide | $^1$H NMR (400 MHz, DMSO-$d_6$) δ 9.05 (s, 1H), 8.21(s,1H),7.90 (s, 1H), 7.62 (s, 1H), 7.35-7.32 (m, 2H), 7.22 (s, 1H), 7.16-7.15 (m, 2H), 6.53-5.71(m,1H), 4.89-4.70 (m, 2H), 2.71-2.69 (s, 3H), 2.33 (s, 3H). LCMS (ESI): 442.2[M+H]$^-$ |
| 16 | 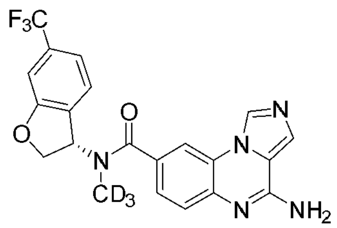 | (S)-4-amino-N-(meth yl-d3)-N-(6-(trifluoro methyl)-2,3-dihydrob enzofuran-3-yl)imidaz o[1,5-a]quinoxaline-8 -carboxa-mide | $^1$H NMR (400 MHz, DMSO-$d_6$) δ 9.19 (s, 1H), 8.34 (t, J = 1.1 Hz, 1H), 7.91 (s, 1H), 7.65-7.63 (d, J = 7.8 Hz, 1H), 7.48 - 7.44 (m, 4H), 7.34-7.32 (m, 1H), 7.26 (s, 1H), 6.39-5.78 (m, 1H), 4.84-4.69 (m, 2H). LCMS (ESI): 430.95 [M+H] $^+$ |
| 17 | 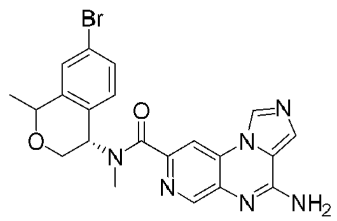 | 4-amino-N-((4S)-7-br omo-1-methylisochro man-4-yl)-N-methyli midazo[1,5-a]pyrido[ 3,4-e]pyr-azine-8-carb oxamide | $^1$H NMR (400 MHz, $CDCl_3$) δ 9.31-9.28(m, 1H), 8.67-8.64(m, 1H), 8.56-8.49(m, 1H), 7.98(s, 1H), 7.72-7.66(m, 2H), 7.56-7.45(m, 2H), 7.41-7.26(m, 1H), 5.63-5.62(m, 0.5H), 5.10-5.09(m, 0.5H), 4.75-4.64(m, 1H), 4.24-4.17(m, 1H), 4.06-4.02(m, 1H), 3.92-3.88(m, 1H), 2.85-2.75(d, 3H), 1.54-1.52(m, 3H). LCMS ( ESI ) m/z: 467.1/469.1 [M+H]$^+$ |
| 18 | 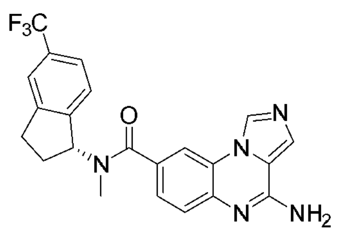 | (R)-4-amino-N-methy 1-N-(5-(trifluoromethy 1)-2,3-dihydro-1H-ind en-1-yl)imidazo[1,5-a ]qui-noxaline-8-carbox amide | $^1$HNMR (400 MHz, DMSO-$d_6$) δ 9.19 (d, *J* = 22.0 Hz, 1H), 8.36 (s, 1H), 7.91 (s, 1H), 7.62 (d, *J* = 8.0 Hz, 2H), 7.58-7.29 (m, 5H), 5.83 (d, *J* = 320.9 Hz, 1H), 3.05 (d, *J* = 33.9 Hz, 2H), 2.71 (s, 3H), 2.28 (d, *J* = 69.4 Hz, 2H). $^{19}$FNMR (376 MHz, DMSO-$d_6$) δ -60.51 (d, *J*= 23.8 Hz). LCMS (ESI) m/z: 426.1 [M+H]$^-$ |

(continued)

| Ex. | Structure | Name | $^1$HNMR&$^{19}$F NMR& LCMS |
|---|---|---|---|
| 19 | 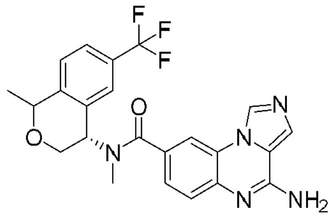 | 4-amino-N-methyl-N-((4S)-1-methyl-6-(trifluoromethyl)isochroman-4-yl)imidazo[1,5-a ]quinoxaline-8-carbox amide | $^1$H NMR (400 MHz, DMSO-$d_6$) δ 9.14 (d, *J* = 53.8 Hz, 1H), 8.85 - 8.31 (m, 1H), 7.92 (d, *J* = 3.4 Hz, 1H), 7.68 (t, *J*= 10.1 Hz, 2H), 7.62 - 7.39 (m, 5H), 5.92 - 5.18 (m, 1H), 5.09 - 4.73 (m, 1H), 4.46 - 3.84 (m, 2H), 2.78 (d, *J* = 21.2 Hz, 3H), 1.77 - 1.35 (m, 3H).<br>$^{19}$F NMR (376 MHz, DMSO-$d_6$) δ -60.15 - -61.37 (m).<br>LCMS (ESI) m/z: 456 [M+H]$^+$ |
| 20 | 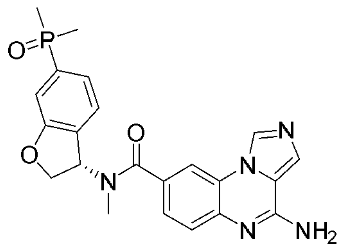 | (S)-4-amino-N-(6-(dimethylphosphoryl)-2, 3-dihydrobenzofuran-3-yl)-N-methylimidaz o[1,5-a]quinoxaline-8 -carboxamide | $^1$H NMR (400 MHz, DMSO-$d_6$) δ 9.19 (s, 1H), 8.33 (s, 1H), 7.92 (s, 1H), 7.57-7.55 (m, 1H), 7.47-7.46 (m, 4H), 7.41-7.39 (m, 1H), 7.33-7.26 (m, 1H), 6.55-5.71 (m, 1H), 4.79-4.65 (m, 2H), 2.71 (s, 3H), 1.71-1.62(m, 6H).<br>LCMS (ESI): 436.20 [M+H]$^+$ |
| 21 | 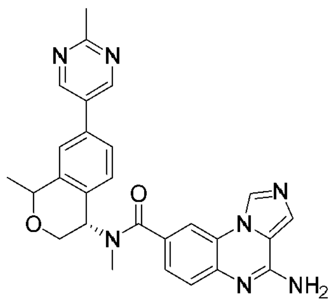 | 4-amino-N-methyl-N-((4S)-1-methyl-7-(2-methylpyrimidin-5-yl) isochroman-4-yl)imid azo [1,5-a]quinoxaline -8-carboxamide | $^1$HNMR (400 MHz, DMSO-$d_6$) δ9.22-9.20(m, 1H), 9.06-9.04(m, 2H), 8.42-8.36(m, 1H), 7.92(s, 1H), 7.74-7.67(m, 2H), 7.55-7.39(m, 5H), 5.70-4.90(m, 1H), 4.71-4.69(m, 1H), 4.36-4.24(m, 1H), 4.10-3.94(m, 1H), 2.82-2.73(m, 3H), 2.67-2.66(m, 3H), 1.62-1.40(m, 3H).<br>LCMS (ESI) m/z: 480.4[M+H]$^-$ |
| 22 | 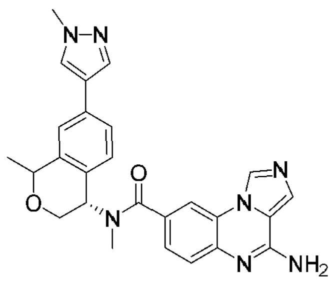 | 4-amino-N-methyl-N-((4S)-1-methyl-7-(1-methyl-1H-pyrazol-4-yl) isochroman-4-yl)i midazo [1,5-a]quinoxa line-8-carboxamide | $^1$H NMR (400 MHz, DMSO-$d_6$) δ 9.22-9.19(m, 1H), 8.40-8.34(m, 1H), 8.19-8.17(m, 1H), 7.92-7.89(m, 2H), 7.48-7.34(m, 5H), 7.30-7.23(m, 2H), 5.62-4.78(m, 1H), 4.74-4.64(m, 1H), 4.32-4.27(m, 1H), 4.06-4.00(m, 1H), 3.86(s, 3H), 2.79-2.71(m, 3H), 1.51-1.38(m, 3H).<br>LCMS (ESI) m/z: 468.3[M+H]$^-$ |
| 23 | 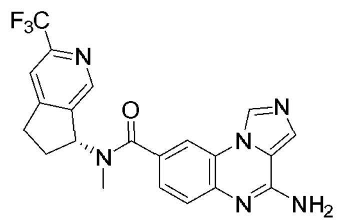 | (R)-4-amino-N-methy 1-N-(3-(trifluoromethy 1)-6,7-dihydro-5H-cyc lopenta[c]pyridin-7-yl )imidazo[1,5-a]quinox aline-8-carboxamide | $^1$H NMR (400 MHz, DMSO-$d_6$) δ 9.12 (s, 1H), 8.32 (d, *J* = 6.6 Hz, 1H), 7.93 (d, *J* = 3.2 Hz, 1H), 7.58 (d, *J* = 26.7 Hz, 3H), 7.41 - 7.19 (m, 3H), 6.51 - 5.61 (m, 1H), 4.96 - 4.60 (m, 2H), 2.65 (d, *J* = 31.4 Hz, 3H).<br>$^{19}$F NMR (376 MHz, DMSO-$d_6$) δ -65.97.<br>LCMS (ESI) m/z: 427.5 [M+H]$^-$ |

(continued)

| Ex. | Structure | Name | $^1$HNMR&$^{19}$F NMR& LCMS |
|---|---|---|---|
| 24 | 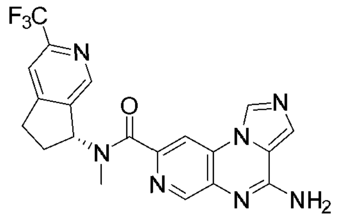 | (R)-4-amino-N-methy 1-N-(3-(trifluoromethy 1)-6,7-dihydro-5H-cyc lopenta[c]pyridin-7-yl )imidazo[1,5-a]pyrido [3,4-e] pyrazine-8-carb oxamide | $^1$H NMR (400 MHz, DMSO-$d_6$) δ 9.30 (d, *J* = 7.2 Hz, 1H), 8.81 - 8.49 (m, 3H), 7.99 (d, *J* = 2.6 Hz, 1H), 7.89 (d, *J* = 23.8 Hz, 1H), 7.73 (s, 2H), 6.07 (dt, *J* = 194.2, 8.2 Hz, 1H), 3.19 - 3.04 (m, 1H), 2.90 (t, *J* = 8.7 Hz, 1H), 2.79 (d, *J* = 18.9 Hz, 3H), 2.46 - 2.35 (m, 1H), 2.22 (dq, *J* = 13.3, 8.6 Hz, 1H).<br>$^{19}$F NMR (376 MHz, DMSO-$d_6$) δ -65.90.<br>LCMS (ESI) m/z: 428.4 $[M+H]^-$ |
| 25 | 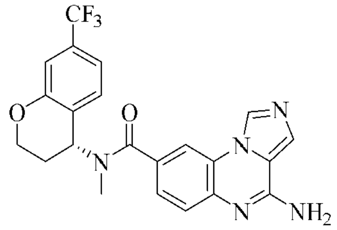 | (R)-4-amino-N-methy 1-N-(7-(trifluoromethy 1) chroman-4-yl)imida zo [1,5-a]quinoxaline-8-car-boxamide | $^1$H NMR (400 MHz, DMSO-$d_6$) δ 9.24-9.13(m, 1H), 8.37(s, 1H), 7.92(s, 1H), 7.53-7.39(m, 5H), 7.29-7.24(m, 1H), 7.15-7.10(m, 1H), 5.99-5.16(m, 1H), 4.50-4.10(m, 2H), 2.73(d, *J* = 4.4 Hz, 3H), 2.36-2.15(m, 2H).<br>LCMS (ESI) m/z: 442.2 $[M+H]^-$ |
| 26 | 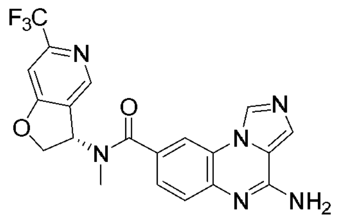 | (S)-4-amino-N-methy 1-N-(6-(trifluoromethy 1)-2,3-dihydrofuro[3,2 -c] pyridin-3-yl)imidaz o[1,5-a] quinoxaline-8 -carboxa-mide | $^1$H NMR (400 MHz, DMSO-$d_6$) δ 9.19 (s, 1H), 8.79 - 8.45 (m, 2H), 8.34 (d, *J* = 1.7 Hz, 1H), 7.93 (s, 1H), 7.65 - 7.36 (m, 4H), 6.37 (s, 1H), 5.05 -4.77 (m, 2H), 2.64 (d, *J* = 82.3 Hz, 3H).<br>$^{19}$F NMR (376 MHz, DMSO-$d_6$) δ -66.36.<br>LCMS (ESI) m/z: 429.4 $[M+H]^-$ |
| 27 | 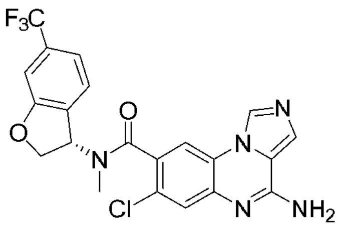 | (S)-4-amino-7-chloro-N-methyl-N-(6-(triflu oro-methyl)-2,3-dihydr oben-zofuran-3-yl)imi dazo[1,5-a]quinoxalin e-8-carboxa-mide | $^1$H NMR (400 MHz, DMSO-$d_6$) δ 9.20-9.06 (m, 1H), 8.57-7.92 (m, 2H), 7.68-7.47 (m, 4H),7.37 - 7.23 (m, 2H), 6.54-5.50(m, 1H), 4.91-4.61 (m, 2H), 2.71-2.66(m, 3H).<br>LCMS (ESI) m/z: 462 .10$[M+H]^+$ |
| 28 | 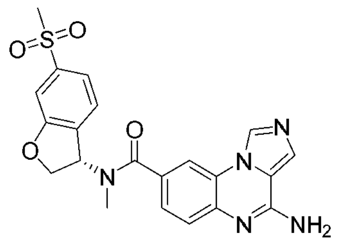 | (S)-4-amino-N-methy 1-N-(6-(methylsulfony 1)-2,3-dihydrobenzofu ran-3-yl)imidazo[1,5-a]qui-noxaline-8-carbo xamide | $^1$H NMR (400 MHz, DMSO-$d_6$) δ 9.18 (s, 1H), 8.34 (s, 1H), 7.92 (s, 1H), 7.69 (d, *J* = 7.8 Hz, 1H), 7.54 (d, *J* = 7.9 Hz, 1H), 7.47 *(d, J*= 10.5 Hz, 4H), 7.41 (s, 1H), 6.40 (s, 1H), 4.84 (s, 1H), 4.74 (dd, *J* = 10.2, 4.5 Hz, 1H), 3.24 (s, 3H), 2.69 (s, 3H).<br>LCMS (ESI): 438.25 $[M+H]^+$ |
| 29 | 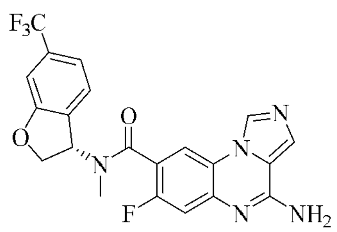 | (S)-4-amino-7-fluoro-N-methyl-N-(6-(triflu oro-methyl)-2,3-dihydr oben-zofuran-3-yl)imi dazo[1,5-a]quinoxalin e-8-carboxa-mide | $^1$H NMR (400 MHz, DMSO-$d_6$) δ 9.12 (s, 1H), 8.32 (d, *J* = 6.6 Hz, 1H), 7.93 (d, *J* = 3.2 Hz, 1H), 7.58 (d, *J* = 26.7 Hz, 3H), 7.41-7.19 (m, 3H), 6.51-5.61 (m, 1H), 4.96-4.60 (m, 2H), 2.65 (d, *J* = 31.4 Hz, 3H).<br>$^{19}$F NMR (376 MHz, DMSO-$d_6$) δ -60.83 (d, *J* = 10.8 Hz).<br>LCMS (ESI) m/z: 579.2 $[M+H]^-$ |

(continued)

| Ex. | Structure | Name | $^1$HNMR& $^{19}$F NMR& LCMS |
|---|---|---|---|
| 30 | 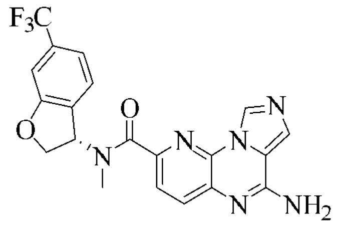 | (S)-6-amino-N-methy 1-N-(6-(trifluoromethy 1)-2,3-dihydrobenzofu ran-3-yl)imidazo[1,5-a] pyrido[3,2-e]pyrazi ne-2-carboxamide | $^1$H NMR (400 MHz, DMSO-$d_6$) δ 8.97 (d, *J* = 24.2 Hz, 1H), 8.04-7.85 (m, 2H), 7.83-7.58 (m, 4H), 7.31 (dd, *J*= 36.5, 11.5 Hz, 2H), 6.48-6.08 (m, 1H), 4.78 (dt, *J* = 30.4, 9.0 Hz, 2H), 2.86 -2.69 (s, 3H).<br>LCMS (ESI) m/z:429.20[M+H]$^-$ |
| 31 | 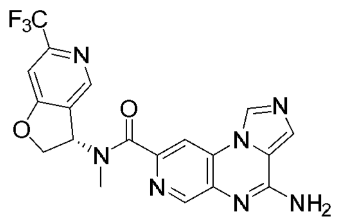 | (S)-4-amino-N-methy 1-N-(6-(trifluoromethy 1)-2,3-dihydrofuro[3,2 -c] pyridin-3-yl)imidaz o[1,5-a] pyrido[3,4-e]p yrazine-8-carboxamid e | $^1$H NMR (400 MHz, DMSO-$d_6$) δ 9.69-9.21 (m, 1H), 8.86-8.41 (m, 3H), 8.12-7.97 (m, 1H), 7.76 (d, *J* = 12.4 Hz, 2H), 7.58-7.32 (m, 1H), 6.47-6.09 (m, 1H), 4.89-4.84 (m, 1H), 3.17 (d, *J* = 5.2 Hz, 1H), 2.80 (d, *J* = 55.3 Hz, 3H).<br>$^{19}$F NMR (376 MHz, DMSO-$d_6$) δ -66.33, -66.34.<br>LCMS (ESI) m/z: 430.4 [M+H]$^-$ |
| 32 | 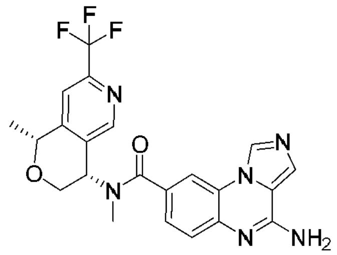 | 4-amino-N-methyl-N-((1R,4S)-1-methyl-7-(tri-fluoromethyl)-3,4-d ihy-dro-1H-pyrano[4,3 -c]pyridin-4-yl)imidazo[1,5-a]qui-noxaline-8 -carboxamide | $^1$H NMR (400 MHz, DMSO-$d_6$) δ 9.20 (s, 1H), 8.80 (s, 1H), 8.37 (s, 1H), 8.03-7.76 (m, 2H), 7.67-7.27 (m, 4H), 5.76 (s, 1H), 4.82 (s, 1H), 4.55-3.99 (m, 2H), 2.82 (s, 3H), 1.59 (d, *J* = 6.6 Hz, 3H).<br>LCMS (ESI): 457.40 [M+H]$^+$ |
| 33 | 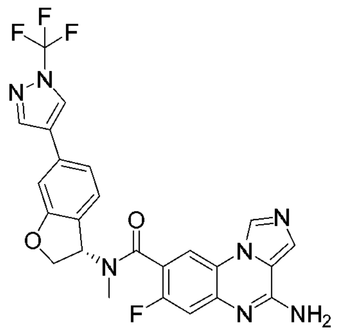 | (S)-4-amino-7-fluoro-N-methyl-N-(6-(1-(tri fluoromethyl)-1H-pyr azol-4-yl)-2,3-dihydro benzofuran-3-yl)imid azo[1,5-a]qui-noxaline -8-carboxamide | $^1$H NMR (400 MHz, DMSO-$d_6$) δ 9.14 (s, 1H), 9.01 (d, *J* = 7.1 Hz, 1H), 8.49 (d, *J* = 8.4 Hz, 1H), 8.33 (d, *J* = 6.6 Hz, 1H), 7.93 (d, *J* = 3.6 Hz, 1H), 7.61 (s, 2H), 7.44 - 7.19 (m, 4H), 6.48 - 5.53 (m, 1H), 4.88 - 4.52 (m, 2H), 2.76 - 2.57 (m, 3H).<br>LCMS (ESI) m/z: 512.2 [M+H]$^-$ |
| 34 | 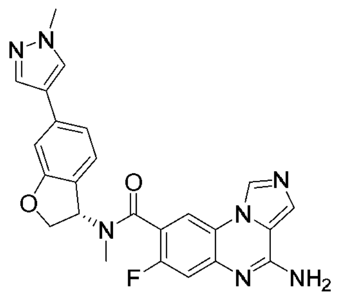 | (S)-4-amino-7-fluoro-N-methyl-N-(6-(1-me thyl-1H-pyrazol-4-yl) -2,3-dihydrobenzofura n-3-yl) imidazo[1,5-a] quinoxa-line-8-carbox amide | $^1$H NMR (400 MHz, DMSO-$d_6$) δ 9.14 (s, 1H), 8.33 (d, J = 6.6 Hz, 1H), 8.15 (d, J = 7.2 Hz, 1H), 7.98 - 7.84 (m, 2H), 7.61 (s, 2H), 7.35 - 7.05 (m, 4H), 6.44 - 5.47 (m, 1H), 4.86 - 4.48 (m, 2H), 3.85 (d, J = 5.3 Hz, 3H), 2.75 -2.55 (m, 3H).<br>LCMS (ESI) m/z: 457.2 [M+H]$^-$ |

(continued)

| Ex. | Structure | Name | $^1$HNMR&$^{19}$F NMR& LCMS |
|---|---|---|---|
| 35 | 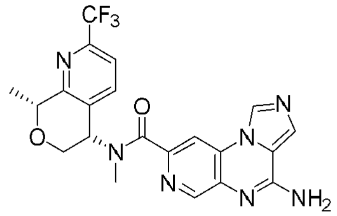 | 4-amino-N-methyl-N-((5S,8R)-8-methyl-2-(trifluoromethyl)-5,8-d ihydro-6H-pyrano[3,4 -b]pyridin-5-yl)imida zo[1,5-a] pyrido[3,4-e] pyrazine-8-carboxami de | $^1$H NMR (400 MHz, DMSO-$d_6$) δ 9.29 (s, 1H), 8.66 (d, $J$ = 6.0 Hz, 1H), 8.55 (d, $J$ = 30.0 Hz, 1H), 8.15 (dd, $J$ = 95.7, 8.1 Hz, 1H), 7.99 (s, 1H), 7.98 - 7.86 (m, 1H), 7.73 (d, $J$ = 9.9 Hz, 2H), 5.63 (d, $J$ = 169.5 Hz, 1H), 4.80 (dq, $J$ = 40.6, 6.6 Hz, 1H), 4.26 (d, $J$ = 12.3 Hz, 1H), 4.10 (ddd, $J$ = 60.3, 12.6, 4.5Hz, 1H), 2.86 (d, $J$= 10.3 Hz, 3H), 1.58 (d, $J$ = 6.6 Hz, 3H).<br>LCMS (ESI) m/z:458.5[M+H]$^+$ |
| 36 | 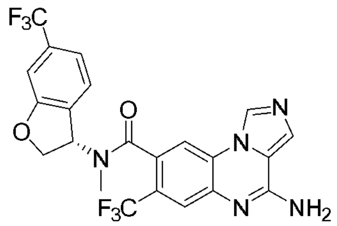 | (S)-4-amino-N-methy 1-7-(trifluoromethyl)-N-(6-(trifluoromethyl) -2,3-dihydrobenzofura n-3-yl)imidazo[1,5-a] quinoxaline-8-carbox amide | $^1$H NMR (400 MHz, DMSO-$d_6$) δ 9.23 (d, $J$ = 73.2 Hz, 1H), 8.46 (d, $J$ = 113.8 Hz, 1H), 8.07 - 7.80(m, 1H), 7.71 (d, $J$ = 9.0 Hz, 1H), 7.53 (s, 3H), 7.34 (dd, $J$ = 16.4, 7.8 Hz, 1H), 7.21 (d, $J$ = 25.6 Hz, 1H), 6.71 - 5.28 (m, 1H), 5.09 - 4.29 (m, 2H), 2.94 (d, $J$ = 178.2 Hz, 3H).<br>LCMS (ESI) m/z:496.4[M+H]$^+$ |
| 37 | 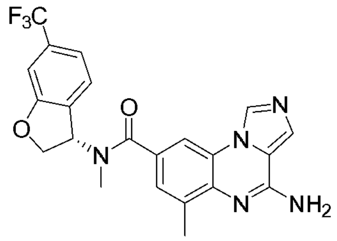 | (S)-4-amino-N,6-dime thyl-N-(6-(trifluorome thyl)-2,3-dihydrobenz ofuran-3-yl)imidazo[1 ,5-a] quinoxaline-8-car boxamide | $^1$H NMR (400 MHz, DMSO-$d_6$) δ 9.15 (s, 1H), 8.17 (d, $J$ = 2.2 Hz, 1H), 7.91 (s, 1H), 7.65-7.63 (d, $J$ = 7.8 Hz, 1H), 7.43 (s, 2H), 7.39 (s, 1H), 7.35-7.33 (d, $J$ = 7.8 Hz, 1H), 7.26 (s, 1H), 6.53-5.75 (m, 1H), 4.82 -4.69(m, 2H), 2.67 (s, 3H), 2.53 (s, 3H).<br>LCMS (ESI)m/z:442.10[M+H]$^-$ |
| 38 | 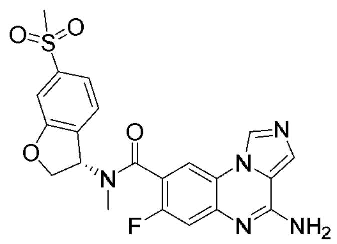 | (S)-4-amino-7-fluoro-N-methyl-N-(6-(meth ylsulfonyl)-2,3-dihydr obenzofuran-3-yl)imi dazo[1,5-a]quinoxalin e-8-carboxamide | $^1$H NMR (400 MHz, DMSO-$d_6$) δ 9.12 (s, 1H), 8.32-8.31 (m, 1H), 7.93-7.92 (m, 1H), 7.65-7.61 (m, 2H), 7.59-7.57 (m, 1H), 7.47-7.39 (m, 1H), 7.28-7.23 (m, 1H), 6.55-6.47 (m, 1H), 4.94 - 4.85 (m, 1H), 4.69-4.62 (m, 2H), 3.24-3.21(m, 3H), 2.70-2.53 (m, 3H).<br>LCMS (ESI): 456.10 [M+H]$^+$ |
| 39 | 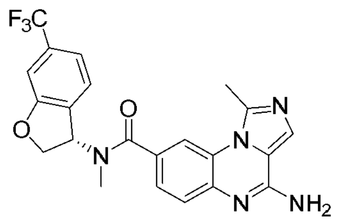 | (S)-4-amino-N,1-dime thyl-N-(6-(trifluorome thyl)-2,3-dihydrobenz ofuran-3-yl)imidazo[1 ,5-a] quinoxaline-8-carboxamide | $^1$H NMR (400 MHz, DMSO-$d_6$) δ 8.15-8.10 (m, 1H), 7.83 (s, 1H), 7.65-7.61 (m, 1H), 7.55 - 7.45 (m, 2H), 7.38-7.32 (m, 2H), 7.33 (d, J = 8.0 Hz, 1H), 7.25-7.21 (m, 1H), 6.29-5.95 (m, 1H), 4.86 - 4.71 (m, 2H), 2.99 (s, 3H), 2.68 (s, 3H).<br>LCMS (ESI) m/z: 442.20 [M+H]$^+$ |
| 40 | 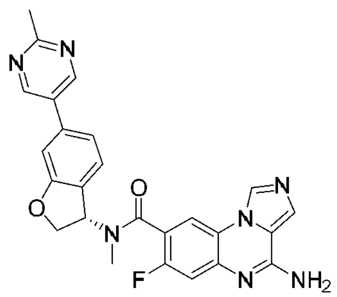 | (S)-4-amino-7-fluoro-N-methyl-N-(6-(2-me thylpyrimidin-5-yl)-2, 3-dihydrobenzofuran-3-yl)imidazo[1,5-a]qu inoxaline-8-carboxam ide | $^1$H NMR (400 MHz, DMSO-$d_6$) δ 9.15 (d, $J$ = 5.3 Hz, 1H), 9.02 (d, $J$ = 9.9 Hz, 2H), 8.34 (d, $J$ = 6.6 Hz, 1H), 7.94 (d, $J$ = 3.5 Hz, 1H), 7.63 (s, 2H), 7.52 - 7.13 (m, 4H), 6.56 - 5.56 (m, 1H), 4.93 - 4.52 (m, 2H), 2.83 - 2.57 (m, 6H).<br>LCMS (ESI) m/z: 470.1 [M+H]$^-$ |

(continued)

| Ex. | Structure | Name | $^{1}$HNMR&$^{19}$F NMR& LCMS |
|---|---|---|---|
| 41 | 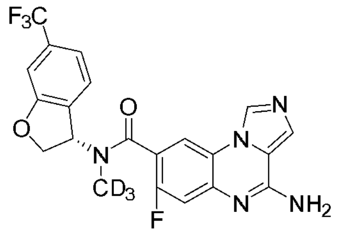 | (S)-4-amino-7-fluoro-N-(methyl-d3)-N-(6-(t ri-fluoromethyl)-2,3-di hy-drobenzofuran-3-yl )imi-dazo[1,5-a]quinox aline-8-carboxamide | $^{1}$H NMR (400 MHz, DMSO-$d_6$) δ 9.14 (s, 1H), 8.34 *(d, J*= 6.5 Hz, 1H), 7.96 (d, *J* = 2.3 Hz, 1H), 7.71 (s, 2H), 7.56 (d, *J* = 7.8 Hz, 1H), 7.42 - 7.15 (m, 3H), 6.53 - 5.58 (m, 1H), 4.88 (t, *J= 9.8* Hz, 1H), 4.68 (dd, *J*= 10.3, 4.3 Hz, 1H)<br>LCMS (ESI) m/z: 449.4 [M+H]$^-$ |
| 42 | 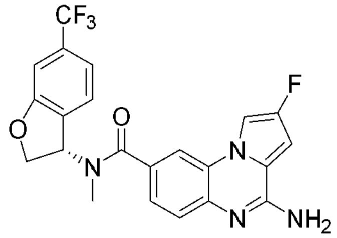 | (S)-4-amino-2-fluoro-N-methyl-N-(6-(triflu oro-methyl)-2,3-dihydr oben-zofuran-3-yl)pyrr olo[1,2-a] quinoxaline-8-carboxa-mide | $^{1}$H NMR (400 MHz, DMSO-$d_6$) δ 8.41 (s, 1H), 8.13 (d, J = 1.6 Hz, 1H), 7.64-7.63 (d, J = 7.8 Hz, 1H), 7.49 - 7.38 (m, 2H), 7.34-7.32 (m, 1H), 7.25-7.24 (m, 1H), 7.18 (s, 2H), 6.97-6.95 (m, 1H), 6.46-5.62 (m, 1H), 4.82-4.54(m, 2H), 2.67 (s, 3H).<br>LCMS (ESI):444.80 [M+H]$^+$ |
| 43 | 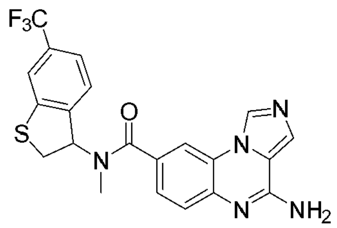 | 4-amino-N-methyl-N-(6-(trifluoromethyl)-2, 3-dihydrobenzo[b]thi ophen-3-yl)imidazo[1, 5-a] quinoxaline-8-car boxa-mide | $^{1}$H NMR (400 MHz, DMSO-$d_6$) δ 9.18 (s, 1H), 8.36 (t, *J* = 1.2 Hz, 1H), 7.92 (s, 1H), 7.79 - 7.66 (m, 1H), 7.61 - 7.37 (m, 6H), 6.13 (d, *J* = 299.0 Hz, 1H), 3.69 (d, *J* = 49.5 Hz, 2H), 2.82 (s, 3H).<br>LCMS (ESI): 442.2 [M+H] $^+$ |
| 44 | 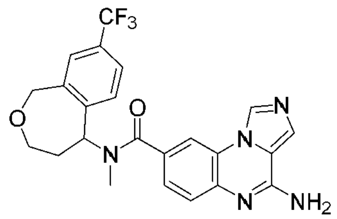 | 4-amino-N-methyl-N-(8-(trifluoromethyl)-1, 3,4,5-tetrahydrobenzo [c] oxepin-5-yl)imidaz o[1,5-a] quinoxaline-8 -carboxa-mide | $^{1}$H NMR (400 MHz, DMSO-$d_6$) δ 9.24 (s, 1H), 8.30 (d, *J* = 69.6 Hz, 1H), 7.94 (s, 1H), 7.70 (s, 2H), 7.62 - 7.41 (m, 4H), 7.29 (d, *J* = 10.2 Hz, 1H), 5.97 (d, *J* = 10.7 Hz, 1H), 4.83 (q, *J* = 14.1 Hz, 2H), 4.11 *(d, J*= 57.7 Hz, 2H), 3.02 (s, 3H), 2.23 (dd, *J* = 48.7, 11.9 Hz, 2H).<br>LCMS (ESI): 455.2 [M+H] $^+$ |
| 45 | 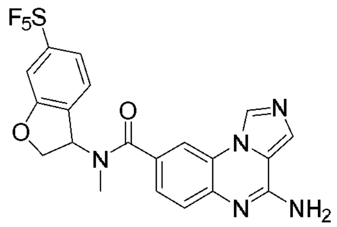 | 4-amino-N-methyl-N-(6-(pentafluoro-λ$^6$-sul faneyl)-2,3-dihydrobe nzofuran-3-yl)imidaz o[1,5-a] quinoxaline-8 -carboxa-mide | $^{1}$H NMR (400 MHz, DMSO-$d_6$) δ 9.17 (s, 1H), 8.33 (s, 1H), 7.98 (s, 1H), 7.65 - 7.63 (m, 1H), 7.52 - 7.42 (m, 6H), 6.51 - 5.57 (m, 1H), 4.84 - 4.69 (m, 2H), 2.70 (s, 3H).<br>LCMS (ESI): 486.1 [M+H] $^+$ |
| 46 | 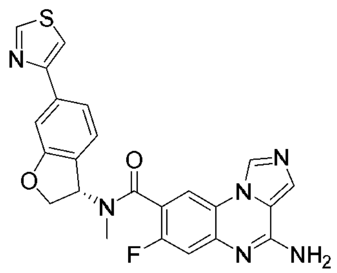 | (S)-4-amino-7-fluoro-N-methyl-N-(6-(thiaz ol-4-yl)-2,3-dihydrobe nzofuran-3-yl)imidaz o[1,5-a]qui-noxaline-8 -carboxamide | $^{1}$H NMR (400 MHz, DMSO-$d_6$) δ 9.41 - 8.97 (m, 2H), 8.63 - 8.03 (m, 2H), 7.93 (d, *J*= 4.4 Hz, 1H),7.77 - 7.47 (m, 3H), 7.42 - 7.06 (m, 3H), 6.71 - 5.34 (m, 1H), 5.00 - 4.49 (m, 2H), 2.66 (d, *J* = 41.7 Hz, 3H).<br>LCMS (ESI) m/z:461.20[M+H]$^-$ |

(continued)

| Ex. | Structure | Name | $^{1}$HNMR&$^{19}$F NMR& LCMS |
|---|---|---|---|
| 47 | 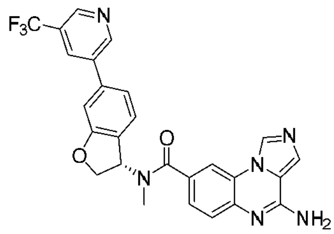 | (S)-4-amino-N-methy 1-N-(6-(5-(trifluorome thyl) pyridin-3-yl)-2,3-dihydrobenzofuran-3-yl)imidazo [1,5-a]quin oxaline-8-car-boxamid e | $^{1}$H NMR (400 MHz, $CDCl_3$) δ 9.20 (d, *J* = 9.1 Hz, 2H), 8.97 (d, *J* = 1.9 Hz, 1H), 8.47 (s, 1H),8.35 (s, 1H), 7.92 (s, 1H), 7.57 (d, *J* = 7.8 Hz, 1H), 7.46 (dd, *J* = 19.2, 6.8 Hz, 6H), 6.73 - 5.58 (m, 1H),4.75 (d, *J* = 41.0 Hz, 2H), 2.70 (s, 3H).<br>LCMS (ESI) m/z:505.30[M+H]$^-$ |
| 48 | 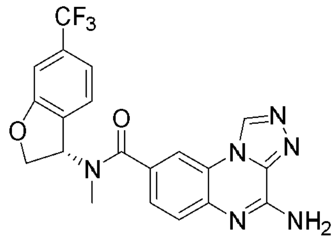 | (S)-4-amino-N-methy 1-N-(6-(trifluoromethy 1)-2,3-dihydrobenzofu ran-3-yl)-[1,2,4]triazo lo [4,3-a]quinoxaline-8 -car-boxamide | $^{1}$H NMR (400 MHz, DMSO-$d_6$) δ 10.04 (s, 1H), 8.37 (t, *J* = 1.2 Hz, 1H), 7.88 (s, 2H), 7.68 - 7.56 (m, 3H), 7.34 (d, *J* = 7.8 Hz, 1H), 7.27 (s, 1H), 6.09 (d, *J* = 257.8 Hz, 1H), 5.20 - 4.40 (m, 2H), 2.69 (s, 3H).<br>LCMS (ESI) m/z: 429.2 [M+H]$^-$ |
| 49 | 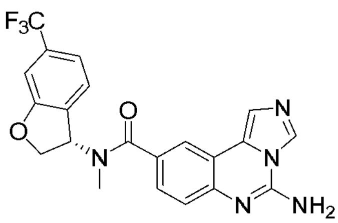 | (S)-5-amino-N-methy 1-N-(6-(trifluoromethy 1)-2,3-dihydrobenzofu ran-3-yl)imidazo[1,5-c]qui-nazoline-9-carbo xamide | $^{1}$H NMR (400 MHz, DMSO-$d_6$) δ 8.66 (s, 1H), 8.17 (d, *J* = 2.0 Hz, 1H), 7.99 (s, 1H), 7.81 (s, 2H), 7.66 *(d, J* = 7.8 Hz, 1H), 7.53(dd, *J* = 8.3, 2.0 Hz, 1H), 7.44 (d, *J* = 8.4 Hz, 1H), 7.34 (d, *J= 7.8* Hz, 1H), 7.26 (s, 1H), 6.39 (s, 1H), 4.90 - 4.60 (m, 2H), 2.68 (s, 3H)<br>LCMS (ESI) *m/z:* 428.2 [M+H] $^-$ |
| 50 | 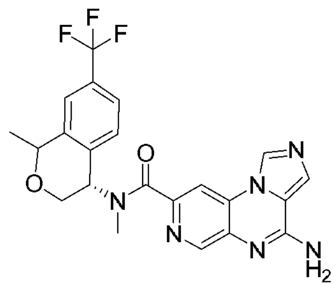 | 4-amino-N-methyl-N-((4S)-1-methyl-7-(trifl uoromethyl)isochrom an-4-yl)imidazo[1,5-a ]pyrido[3,4-e]pyrazin e-8-car-boxamide | $^{1}$H NMR (400 MHz, DMSO-$d_6$) δ 9.34-9.25 (m, 1H), 8.70-8.62 (m, 1H), 8.62-8.47 (m, 1H), 7.99 (d, *J* = 2.0 Hz, 1H), 7.78-7.45 (m, 5H), 5.92-5.18 (m, 1H), 4.88 (ddq, *J* = 74.5, 37.9, 6.4 Hz, 1H), 4.34-4.19 (m, 1H), 4.13-3.88 (m, 1H), 2.83-2.68 (m, 3H), 1.63-1.41 (m, 3H).<br>$^{19}$F NMR (376 MHz, DMSO) δ -60.91.<br>LCMS: LCMS (ESI) m/z: 457 [M+H]$^+$ |
| 51 | 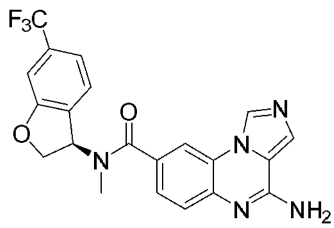 | (R)-4-amino-N-methy 1-N-(6-(trifluoromethy 1)-2,3-dihydrobenzofu ran-3-yl)imidazo[1,5-a]qui-noxaline-8-carbo xamide | $^{1}$H NMR (400 MHz, DMSO-$d_6$) 9.19 (s, 1H), 8.35 (s, 1H), 7.93 (s, 1H), 7.65 (d, *J* = 7.8 Hz, 1H), 7.48 (d, *J* = 8.6 Hz, 4H), 7.40 -7.22 (m, 2H), 6.08 (d, *J* = 254.7 Hz, 1H), 5.00 -4.65 (m, 2H), 2.69 (s, 3H).<br>LCMS (ESI) m/z: 428.1 [M+H]$^-$ |
| 52 | 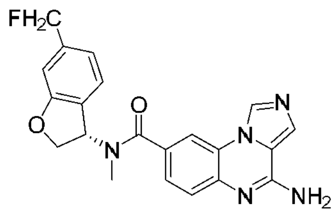 | (S)-4-amino-N-(6-(flu oro-methyl)-2,3-dihydr oben-zofuran-3-yl)-N-methylimi-dazo[1,5-a] quinoxaline-8-carbox amide | $^{1}$H NMR (400 MHz, DMSO-$d_6$) δ 9.08 (s, 1H), 8.26 (s, 1H), 7.94 (s, 1H), 7.58-7.55 (m, 1H), 7.54-7.52 (m, 1H), 7.49-7.30(m,1H) ,7.05-7.01 (m, 1H), 6.93-6.88(m,1H),6.48-5.75(m,1H),5.4 4-5.38(m,1H),5.28(s,1H),4.69-4.63(m ,1-H),3.59-3.54 (m, 1H),2.80-2.69(m,3H)<br>LCMS (ESI): 392.10 [M+H] $^+$ |

(continued)

| Ex. | Structure | Name | $^1$HNMR&$^{19}$F NMR& LCMS |
|---|---|---|---|
| 53 | 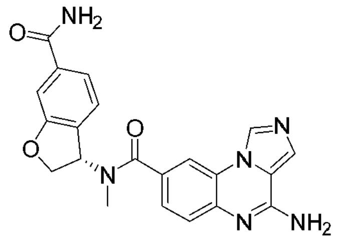 | (S)4-amino-N-(6-car ba-moyl-2,3-dihydrobe nzo-furan-3-yl)-N-met hylimidazo[1,5-a]quin oxaline-8-carboxamid e | $^1$H NMR (400 MHz, DMSO-$d_6$) δ 9.18 (s,1H), 8.33 (s, 1H), 7.95 (s, 1H), 7.91 (s, 1H), 7.50-7.47 (m,4H), 7.43 (s, 2H), 7.38-7.35(m, 2H), 6.42-5.71 (m,1H), 4.89-4.64 (m, 2H),2.66-2.65(m,3H). LCMS (ESI): 403.10[M+H]$^+$ |
| 54 | 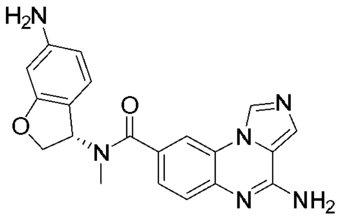 | (S)-4-amino-N-(6-ami no-2,3-dihydrobenzof uran-3-yl)-N-methyli midazo[1,5-a]quinoxa line-8-carboxamide | $^1$H NMR (400 MHz, DMSO-$d_6$) δ 9.18 (s,1H), 8.28 (s, 1H), 7.91 (s, 1H), 7.47 - 7.42 (m, 4H), 7.00 (d, $J$ = 8.0 Hz, 1H), 6.17 (d, $J$ = 8.0 Hz, 1H), 6.05 (s, 1H), 5.40- 5.23 (d, 2H), 4.61-4.48 (s, 2H), 2.77 (s, 1H), 2.61 (s, 3H).<br>LCMS (ESI): 375.10 [M+H]$^+$ |
| 55 | 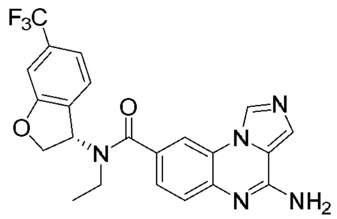 | (S)-4-amino-N-ethyl-N-(6-(trifluoromethyl) -2,3-dihydrobenzofura n-3-yl) imidazo[1,5-a] quinoxa-line-8-carbox amide | $^1$H NMR (400 MHz, DMSO-$d_6$) δ 9.17 (s, 1H), 8.28 (d, J = 1.7 Hz, 1H), 7.91 (d, J = 0.7 Hz, 1H), 7.66 (d, J = 7.8 Hz, 1H), 7.47-7.42 (m, 4H), 7.33 - 7.29(m, 1H), 7.23 (d, J = 1.5 Hz, 1H), 5.85 (s, 1H), 4.79-4.68 (m, 2H), 3.23-3.20 (m, 2H), 0.93 (s, 3H).<br>LCMS (ESI) m/z: 442.15 [M+H]$^+$ |
| 56 | 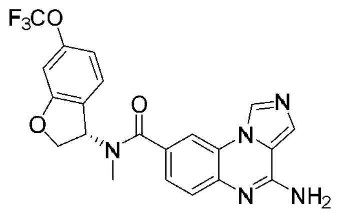 | (S)-4-amino-N-methy 1-N-(6-(trifluorometho xy)-2,3-dihydrobenzo furan-3-yl)imidazo[1, 5-a]qui-noxaline-8-car boxamide | $^1$H NMR (400 MHz, DMSO-$d_6$) δ 9.12 (s, 1H), 8.31 (d, J = 6.6 Hz, 1H), 7.93 (d, J = 3.1 Hz, 1H), 7.86 - 7.03 (m, 5H), 6.94 (s, 2H), 6.42 (dd, J = 9.1, 4.0 Hz, 1H), 4.92 - 4.55 (m, 2H), 3.06 -2.56 (m, 3H).<br>LCMS (ESI): 443.75 [M+H]$^+$ |
| 57 | 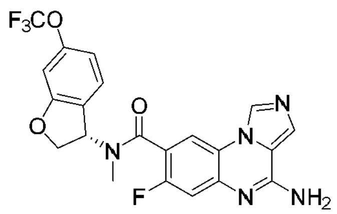 | (S)-4-amino-7-fluoro-N-methyl-N-(6-(triflu oro-methoxy)-2,3-dihy dro-benzofuran-3-yl)i midazo [1,5-a]quinoxa line-8-car-boxamide | $^1$H NMR (400 MHz, DMSO-$d_6$) δ 9.21 (s, 1H), 8.34 (s, 1H), 8.0-7.82(m,1H),7.66 - 7.39 (m, 1H), 7.36-7.30 ( m, 1H ) ,6.96 (s, 2H), 6.52(m,1H),4.71 (d, J = 4.2 Hz, 2H), 2.67 (s, 3H).<br>LCMS (ESI): 461.75 [M+H]$^+$ |
| 107 | 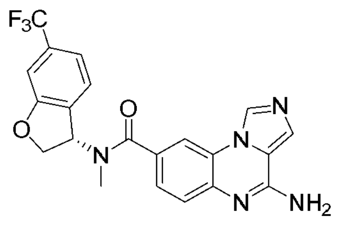 | (S)-4-amino-N-methy 1-N-(6-(trifluoromethy 1)-2,3-dihydrobenzofu ran-3-yl)imidazo[1,5-a]qui-noxaline-8-carboxamide | $^1$H NMR (400 MHz, DMSO-$d_6$) δ 9.18 (s, 1H), 8.33 (t, $J$= 1.1 Hz, 1H), 7.91 (d, $J$ = 0.8 Hz, 1H), 7.64 (d, $J$ = 7.8 Hz, 1H), 7.52 - 7.41 (m, 4H), 7.33 (d, $J$ = 7.8 Hz, 1H), 7.25 (s, 1H), 6.37 (s, 1H), 5.86 (s, 1H), 4.81 (s, 1H), 4.72 (dd, $J$ = 10.3, 4.4 Hz, 1H), 2.68 (s, 3H).<br>LCMS (ESI) m/z: 428.2 [M+H]$^-$ |

(continued)

| Ex. | Structure | Name | $^1$HNMR&$^{19}$F NMR& LCMS |
|---|---|---|---|
| 108 | 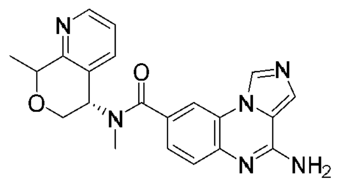 | 4-amino-N-methyl-N-((5S)-8-methyl-5,8-di hydro-6H-pyrano[3,4-b] pyridin-5-yl)imidaz o[1,5-a] quinoxaline-8 -carboxa-mide | $^1$H NMR (400 MHz, DMSO-$d_6$) δ 9.26-9.12 (m, 1H), 8.52 (d, *J* = 18.6 Hz, 1H), 8.43-8.32 (m, 1H), 7.92 (s, 1H), 7.79 (dd, *J* = 23.1, 7.8 Hz, 1H), 7.61 - 7.32 (m, 5H), 5.79 (d, *J* = 49.5 Hz, 1H), 5.09-4.53 (m, 2H), 4.43-3.89 (m, 2H), 2.77(d, *J* = 31.5 Hz, 3H), 1.67-1.32 (m, 3H). LCMS (ESI) m/z: 389.4 $[M+H]^-$ |
| 124 | 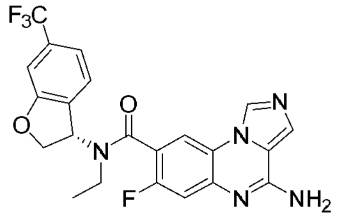 | (S)-4-amino-N-ethyl-7-fluoro-N-(6-(trifluor omethyl)-2,3-dihydro benzofuran-3-yl)imid azo[1,5-a]quinoxaline -8-carboxa-mide | $^1$H NMR (400 MHz, DMSO-$d_6$) δ9.10-9.04 (m, 1H) , 8.42-8.21 (m, 1H), 7.86 (s, 1H), 7.53-7.49 (m, 3H), 7.26-7.24 (m, 1H), 7.21 - 7.15 (m, 2H), 5.94-5.53 (s, 1H), 4.87-4.82 (t, J = 9.7 Hz, 1H), 4.60-4.57 (m, 3H), 0.98-0.79 (m,3H). LCMS (ESI) m/z: 460.20 $[M+H]^+$ |
| 110 | 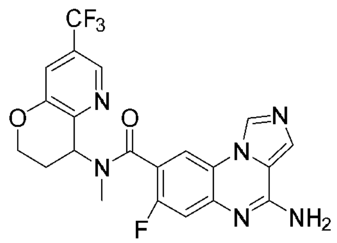 | 4-amino-7-fluoro-N-methyl-N-(7-(trifluoro methyl)-3,4-dihydro-2 H-pyrano[3,2-b]pyridi n-4-yl) imidazo[1,5-a] quinoxa-line-8-carbox amide | $^1$H NMR (400 MHz, DMSO-$d_6$) δ 9.14 (d, *J* = 52.0 Hz, 1H), 8.69 - 8.49 (m, 1H), 8.31 (dd, *J* = 21.8,6.5 Hz, 1H), 7.91 (d, *J* = 11.2 Hz, 1H), 7.63 (dd, *J* = 23.9, 2.0 Hz, 1H), 7.54 (d, *J* = 16.2 Hz, 2H), 7.22(dd, *J* = 11.0, 7.9 Hz, 1H), 6.03 - 5.01 (m, 1H), 4.64 - 4.17 (m, 2H), 2.71 *(d, J*= 2.0 Hz, 3H), 2.46 -2.20 (m, 2H). LCMS(ESI) m/z: 461.20$[M+H]^-$ |
| 125 | 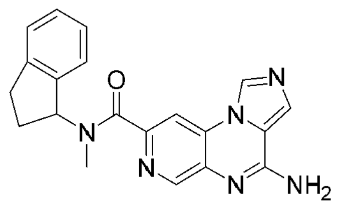 | 4-amino-N-(2,3-dihyd ro-1H-inden-1-yl)-N-methylimidazo[1,5-a] pyrido[3,4-e]pyrazine -8-car-boxamide | $^1$H NMR (400 MHz, DMSO-$d_6$) δ 9.43 - 9.23 (m, 1H), 8.79 - 8.61 (m, 1H), 8.51 (d, *J* = 3.7 Hz, 1H), 8.03 - 7.94 (m, 1H), 7.78 - 7.59 (m, 2H), 7.37 - 7.21 (m, 4H), 6.30 - 5.52 (m, 1H), 3.06 - 2.84 (m, 2H), 2.72 - 2.55 (m, 3H), 2.46 - 2.27 (m, 1H), 2.16 - 2.01 (m, 1H). LCMS(ESI) m/z: 359.15$[M+H]^-$ |
| 117 | 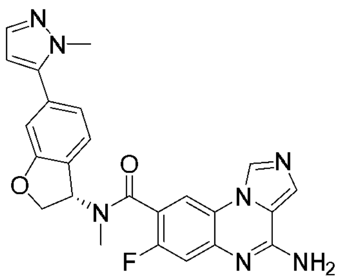 | (S)-4-amino-7-fluoro-N-methyl-N-(6-(1-me thyl-1H-pyrazol-5-yl) -2,3-dihydrobenzofura n-3-yl) imidazo[1,5-a] quinoxa-line-8-carbox amide | $^1$H NMR (400 MHz, DMSO-$d_6$) δ 9.16 (s, 1H), 8.36 (d, *J* = 6.6 Hz, 1H), 7.95 (d, *J* = 3.6 Hz, 1H), 7.64 (s, 2H), 7.46 (dt, *J* = 5.7, 2.9 Hz, 2H), 7.36 - 6.98 (m, 3H), 6.53 - 5.46 (m, 2H), 4.92 - 4.52 (m, 2H), 3.86 (d, *J* = 14.2 Hz, 3H), 2.69 (d, *J* = 41.8 Hz, 3H). LCMS(ESI) m/z: 458.1$[M+H]^-$ |
| 119 | 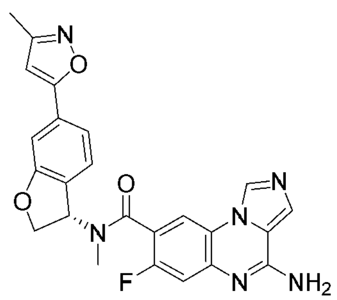 | (S)-4-amino-7-fluoro-N-methyl-N-(6-(3-me thylisoxazol-5-yl)-2,3 -dihydrobenzofuran-3 -yl)imidazo [1,5-a]qui noxaline-8-car-boxami de | $^1$H NMR (400 MHz, DMSO-$d_6$) δ 9.14 (s, 1H), 8.34 (d, *J* = 6.6 Hz, 1H), 7.94 (d, *J* = 3.6 Hz, 1H), 7.62 (s, 2H), 7.48 (d, *J* = 13.2 Hz, 2H), 7.41 -7.20 (m, 2H), 6.93 (d, *J* = 6.6 Hz, 1H), 6.52 -5.60 (m, 1H), 4.93 -4.58 (m, 2H), 2.67 (d, *J* = 37.4 Hz, 3H), 2.29 (d, *J* = 4.6 Hz, 3H). LCMS(ESI) m/z: 459.1$[M+H]^-$ |

(continued)

| Ex. | Structure | Name | $^1$HNMR&$^{19}$F NMR& LCMS |
|---|---|---|---|
| 116 | 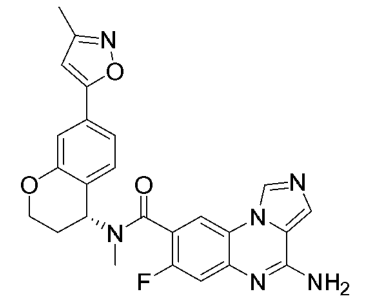 | (R)-4-amino-7-fluoro-N-methyl-N-(7-(3-me thylisoxazol-5-yl)chro man-4-yl)imidazo[1,5 -a]quinoxaline-8-carb oxamide | $^1$H NMR (400 MHz, DMSO-$d_6$) δ 9.27 - 9.07 (m, 1H), 8.42 (d, *J* = 6.6 Hz, 1H), 7.94 (d, *J* = 10.1 Hz, 1H), 7.62 (d, *J* = 14.7 Hz, 2H), 7.43 (dd, *J* = 8.1, 1.8 Hz, 1H), 7.33 - 7.11 (m, 3H), 7.03 - 6.82 (m, 1H), 6.04 (dd, *J* = 10.6, 6.3 Hz, 1H), 4.52 - 4.03 (m, 2H), 2.71 (d, *J* = 38.4 Hz, 3H), 2.42 - 2.06 (m, 5H).<br>LCMS(ESI) m/z: 473.1 $[M+H]^-$ |
| 130 | 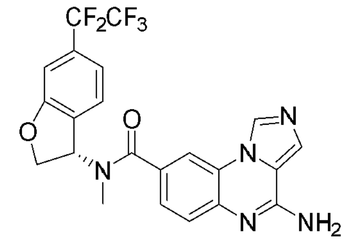 | (S)-4-amino-N-methy 1-N-(6-(perfluoroethyl )-2,3-dihydrobenzofur an-3-yl) imidazo[1,5-a ]quinoxaline-8-carbox amide | $^1$H NMR (400 MHz, $CDCl_3$) δ 9.19 (s, 1H), 8.34 (d, *J* = 1.2 Hz, 1H), 7.92 (s, 1H), 7.68 (d, *J* = 7.8 Hz, 1H), 7.52 - 7.40 (m, 4H), 7.29 (d, *J* = 7.9 Hz, 1H), 7.21 (s, 1H), 6.54 - 5.59 (m, 1H), 5.09 - 4.48 (m, 2H), 2.69 (s, 3H).<br>LCMS(ESI) m/z: 478.3$[M+H]^-$ |
| 129 | 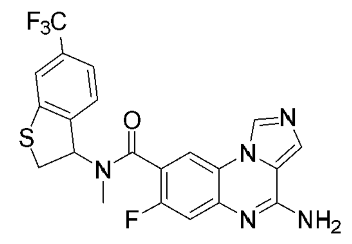 | 4-amino-7-fluoro-N-methyl-N-(6-(trifluoro methyl)-2,3-dihydrob enzo [b]thiophen-3-yl) imidazo [1,5-a]quinox aline-8-carboxamide | $^1$H NMR (400 MHz, DMSO-$d_6$) δ 9.12 (d, *J* = 17.1 Hz, 1H), 8.51 - 8.23 (m, 1H), 7.93 (d, *J* = 3.1 Hz, 1H), 7.83 - 7.67 (m, 1H), 7.61 (d, *J* = 7.1 Hz, 2H), 7.52 (t, *J* = 9.4 Hz, 1H), 7.40 (d, *J* = 8.0 Hz, 1H), 7.26 (dd, *J* = 11.0, 1.7 Hz, 1H), 6.62 - 5.58 (m, 1H), 3.93 - 3.38 (m, 2H), 2.79 (d, *J* = 41.5 Hz, 3H).<br>LCMS(ESI) m/z: 462.2 $[M+H]^-$ |

Example 58:

(S)-4-amino-N-methyl-N-(6-(trifluoromethyl)-2,3-dihydrobenzofuran-3-yl)tetrazolo[1,5-a]quinoxaline-8-carboxamide

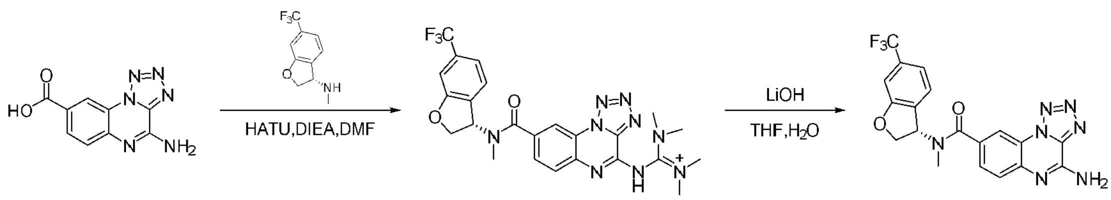


Step 1: Synthesis of (S)-N-((dimethylamino)((8-(methyl(6-(trifluoromethyl)-2,3-dihydrobenzofuran-3-yl)carbamoyl)tetrazolo[1,5-a]quinoxalin-4-yl)amino)methylene)-N-methylmethanaminium

4-aminotetrazolo[1,5-a]quinoxaline-8-carboxylic acid(131mg, 0.56mmol) was dissolved in DMF(4.5mL), followed by addition of 2-(7-Azabenzotriazol-1-yl)-N,N,N',N'-tetramethyluronium hexafluorophosphate(325mg, 0.85mmol), N,N-diisopropylethylamine(220mg, 1.71mmol) and (S)-N-methyl-6-(trifluoromethyl)-2,3-dihydrobenzofuran-3-amine(144mg, 0.660mmol). The reaction proceeded at room temperature for 0.5h. The reaction mixture was diluted with ethyl acetate(30mL) and water(30mL), the water layer was extracted with ethyl acetate(2×20mL). The organic phase was combined and washed with saturated brine(30mL), dried over anhydrous sodium sulfate, filtered, the filtrate was concentrated to obtain (S)-4-amino-N-methyl-N-(6-(trifluoromethyl)-2,3-dihydrobenzofuran-3-yl)tetrazolo[1,5-a]quinoxaline-8-carboxamide(100 mg, 0.19mmol, yield:33.33%), as white solid.

LCMS (ESI): $528[M+H]^+$

Step 2: Synthesis of (S)-4-amino-N-methyl-N-(6-(trifluoromethyl)-2,3-dihydrobenzofuran-3-yl)tetrazolo[1,5-a]quinoxaline-8-carboxamide

(S)-4-amino-N-methyl-N-(6-(trifluoromethyl)-2,3-dihydrobenzofuran-3-yl)tetrazolo[1,5-a]quinoxaline-8-carboxamid e(100mg, 0.19mnol) was dissolved in THF(4mL), followed by addition of water(4mL) and LiOH(27mg, 1.12mmol). After completion of the reaction, the reaction mixture was concentrated and purified by HPLC(column: YMC Triart $C_{18}$ ExRs 5 m,30mm × 150mm; mobile phase A: water(10mmol/L ammonium bicarbonate), mobile phase B: acetonitrile, flow rate: 60ml/min; Gradient: From 25% B to 50% B within 10 minutes; Wavelength: 254 nanometers/220 nanometers; Rention time: 8.43/9.23 minutes. ) to obtain (S)-4-amino-N-methyl-N-(6-(trifluoromethyl)-2,3-dihydrobenaofuran-3-yl)tetrazolo[1,5-a]quinoxaline-8-carboxamide(3.58 mg, 0.01mmol, yield:4.41%), as white solid.

$^1$H NMR (400 MHz, DMSO-$d_6$) δ 11.10 (s, 1H), 8.01-7.95 (d, 2H), 7.74-7.71 (d, 2H), 7.41-7.39 (d, 1H), 7.33(s,1H), 6.50-5.74(m,1H), 4.91-4.73(m,2H), 3.94(s,3H).
LCMS (ESI) *m/z:* 430.10[M+H] $^+$

Example 59 Synthesis of 4-amino-N-cyclopropyl-7-fluoro-N-(7-(5-(trifluoromethyl)pyridin-3-yl)-3,4-dihydro-2H-pyrano [3,2-b]pyridin-4-yl)imidazo [1,5-a]quinoxaline-8-carboxamide

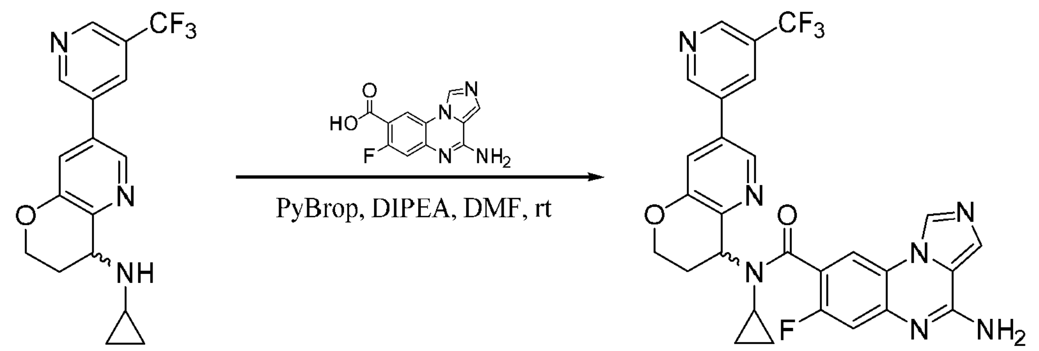


N-cyclopropyl-7-(5-(trifluoromethyl)pyridin-3-yl)-3,4-dihydro-2H-pyrano[3,2-b]pyridin-4-amine(45mg, 0.13mmol) was dissolved in DMF(3mL), followed by addition of 4-amino-7-fluoroimidazo[1,5-a]quinoxaline-8-carboxylic acid(33mg, 0.13mmol), PyBrop(79mg, 0.17mmol) and DIPEA(50.3mg, 0.39mmol). The reaction was stirred at room temperature overnight. Then water(10mL) was added and extracted with ethyl acetate(20mL). The combined organic phase was washed with water(2×10mL), washed with saturated brine(10mL), dried over anhydrous sodium sulfate, filtered. The filtrate was concentrated to obtain crude product. The crude product was purified by column chromatograpy(MeOH/DCM=0-7%) to obtain
4-amino-N-cyclopropyl-7-fluoro-N-(7-(5-(trifluoromethyl)pyridin-3-yl)-3,4-dihydro-2H-pyrano[3,2-b]pyridin-4-yl)imidazo [1,5-a]quinoxaline-8-carboxamide(18mg, yield: 25%).

$^1$H NMR (400 MHz, DMSO-$d_6$) δ 9.31 (s, 1H), 9.18 (s, 1H), 9.02 (s, 1H), 8.63 (d, *J* = 19.1 Hz, 2H),8.24 (s, 1H), 7.93 (s, 1H), 7.77 (s, 1H), 7.56 (s, 2H), 7.22 (d, *J* = 11.4 Hz, 1H), 5.39 (s, 1H), 4.46 (d, *J*= 53.9 Hz, 2H), 3.05 (s, 1H), 2.35 (s, 2H), 0.70 (s, 4H).
LCMS (ESI) m/z:564.30[M+H]$^+$

By using the synthetic procedures described in Example 59, and replacing the corresponding starting materials, the embodiments listed in the table below can be prepared:

| Ex. | Structure | name | $^1$HNMR&$^{19}$F NMR& LCMS |
|---|---|---|---|
| 60 | 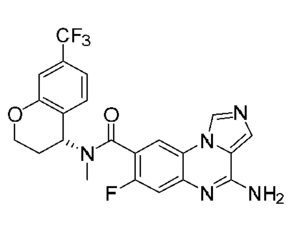 | (R)-4-amino-7-fluoro-N-methyl-N-(7-(triflu oromethyl)chroman-4 -yl) imidazo[1,5-a]qui noxa-line-8-carboxami de | $^1$H NMR (400 MHz, DMSO-$d_6$) δ 9.14 (d, *J* = 28.3 Hz, 1H), 8.40 (d, *J* = 6.6 Hz, 1H), 8.02 - 7.84 (m,1H), 7.59 (d, *J* = 14.5 Hz, 2H), 7.45 - 7.22 (m, 2H), 7.14 (d, *J* = 27.6 Hz, 1H), 5.56 (ddd, *J* = 387.9,10.8, 6.3 Hz, 1H), 4.69 - 3.91 (m, 2H), 2.94 - 2.59 (m, 3H), 2.36 - 2.09 (m, 2H). LCMS (ESI) m/z: 460.20 [M+H]$^-$ |

(continued)

| Ex. | Structure | name | $^{1}$HNMR&$^{19}$F NMR& LCMS |
|---|---|---|---|
| 61 | 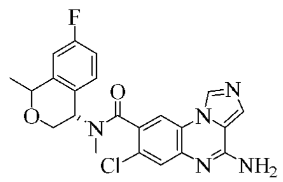 | 4-amino-7-chloro-N-( (4S)-7-fluoro-1-methy lisochroman-4-yl)-N-methylimidazo[1,5-a] quinoxaline-8-carbox amide | $^{1}$HNMR (400MHz, DMSO-$d_6$) δ9.26-9.13(m,1H), 8.41-8.34(m, 1H), 7.93-7.91(m, 1H), 7.59-7.58(m, 2H), 7.56-7.47(m, 1H), 7.47-7.37(m, 1H), 7.23-7.08(m, 2H), 5.68-4.93(m,1H), 4.85-4.61(m, 1H), 4.27-3.88(m, 2H), 2.78-2.51(m, 3H), 1.52-1.48(m, 3H). LCMS(ESI) m/z:440.2 $[M+H]^-$ |
| 62 | 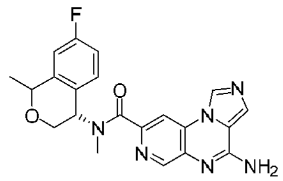 | 4-amino-N-((4S)-7-fl uoro-1-methylisochro man-4-yl)-N-methyli midazo[1,5-a]pyrido[ 3,4-e] pyrazine-8-carb oxamide | $^{1}$HNMR (400MHz, DMSO-$d_6$) δ 9.31-9.27(m, 1H), 8.67-8.65(m, 1H), 8.55-8.48(m, 1H), 7.98(d, 1H), 7.72-7.70(m, 2H), 7.49-7.39(m, 1H), 7.21-7.10(m, 2H), 5.64-5.08(m, 1H), 4.86-4.62(m, 1H), 4.25-3.87(m, 2H), 2.78-2.69(m, 3H), 1.54-1.37(m, 3H). LCMS(ESI) m/z: 407.2$[M+H]^-$ |
| 63 | 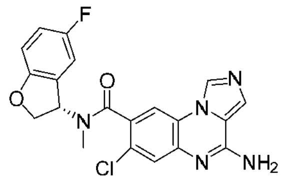 | (S)-4-amino-7-chloro-N-(5-fluoro-2,3-dihyd robenzofuran-3-yl)-N-methylimidazo[1,5-a] quinoxaline-8-carbox amide | $^{1}$H NMR (400 MHz, DMSO-$d_6$) δ 9.03 (s, 1H), 8.45-8.21 (m, 1H), 8.22 (s, 1H), 7.91 (s, 1H), 7.46 (s, 1H), 7.31 (s, 2H), 7.15-7.06 (m, 2H), 6.90- 6.86 (m,lH), 6.42-5.39 (m, 1H), 4.78 -4.54 (m, 2H), 2.72-2.48 (m,3H). LCMS (ESI) m/z:412.1$[M+H]^+$ |
| 64 | 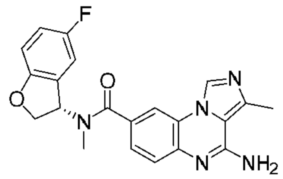 | (S)-4-amino-N-(5-flu oro-2,3-dihydrobenzo furan-3-yl)-N,3-dimet hylimidazo[1,5-a]quin oxa-line-8-carboxamid e | $^{1}$H NMR (400 MHz, DMSO-$d_6$) δ 9.05(s, 1H), 8.25(s, 1H), 744-7.40(m, 2H), 7.30-7.28(m, 1H), 7.11-7.09(m, 1H), 6.70-6.88(m, 3H), 4.72-4.62(m, 2H), 4.62-4.60(m, 1H), 2.67(s, 3H), 2.63(s, 3H). LCMS (ESI) m/z:**392.2**$[M+H]^+$ |
| 65 | 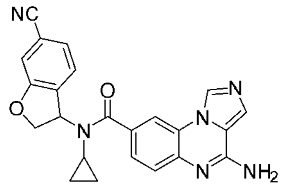 | 4-amino-N-(6-cyano-2,3-dihydrobenzofura n-3-yl)-N-cyclopropyl imidazo[1,5-a]quinox aline-8-carboxamide | $^{1}$H NMR (400 MHz, DMSO-$d_6$) δ 9.17(s, 1H), 8.38(s, 1H), 7.92(s, 1H), 7.73(d, 1H), 7.62(m, 1H), 7.47-7.41(m, 3H), 7.37(s, 1H), 5.95-5.91(m, 1H), 4.89-4.84(m, 1H), 4.70-4.67(m, 1H), 3.0(m, 1H), 0.46-0.09(m, 4H). LCMS (ESI) m/z: 411.30 $[M+H]^-$. |
| 66 | 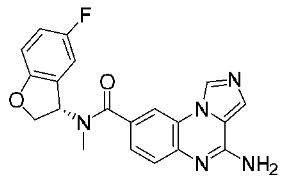 | (S)-4-amino-N-(5-flu oro-2,3-dihydrobenzo furan-3-yl)-N-methyli midazo[1,5-a]quinoxa line-8-carboxamide | $^{1}$H NMR (400 MHz, DMSO-$d_6$) δ 9.18(s, 1H), 8.32(s, 1H), 7.91(s, 1H), 7.46(d, 4H), 7.31-7.28(m, 1H), 7.13-7.08(td, 1H), 6.91-6.88(m, 1H), 4.81-4.65(m, 2H), 4.63-4.60(m, 1H), 2.67(s, 3H). LCMS (ESI) m/z:378.1$[M+H]^+$ |
| 67 | 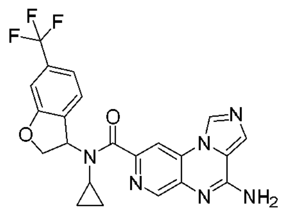 | 4-amino-N-cycloprop yl-N-(6-(trifluorometh yl)-2,3-dihydrobenzof uran-3-yl)imidazo[1,5 -a] pyrido[3,4-e]pyrazi ne-8-carboxamide | $^{1}$H NMR (400 MHz, DMSO-$d_6$) δ 9.27 (s, 1H), 8.65 (d, *J* = 1.9 Hz, 1H), 8.44 (s, 1H), 7.98 (d, *J* = 1.9 Hz, 1H), 7.72 (s, 2H), 7.63 (d, *J* = 7.8 Hz, 1H), 7.30 (d, *J* = 7.8 Hz, 1H), 7.21 (s, 1H), 5.97 (s, 1H), 4.87 (d, *J* = 10.3 Hz, 1H), 4.78 - 4.68 (m, 1H), 2.95 (s, 1H), 0.37 (s, 2H), 0.22 (s, 1H), 0.11 (s, 1H). LCMS (ESI) m/z: 455 $[M+H]^+$ |

(continued)

| Ex. | Structure | name | $^1$HNMR&$^{19}$F NMR& LCMS |
|---|---|---|---|
| 68 | 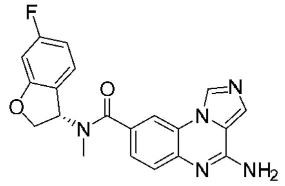 | (S)-4-amino-N-(6-fluoro-2,3-dihydrobenzofuran-3-yl)-N-methylimidazo[1,5-a]quinoxaline-8-carboxamide | $^1$H NMR (400 MHz, DMSO-$d_6$) δ 9.20 (s, 1H), 8.33 (d, *J* = 1.2 Hz, 1H), 7.95 (s, 1H), 7.58 (s, 2H), 7.48 (s, 2H), 7.42 (dd, *J* = 7.8, 5.8 Hz, 1H), 6.80 (t, *J*= 9.1 Hz, 2H), 6.29 (s, 1H), 4.90 -4.57 (m, 2H), 2.65 (s, 3H).<br>LCMS (ESI) m/z: 378 $[M+H]^+$ |
| 69 | 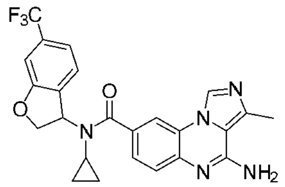 | 4-amino-N-cyclopropyl-3-methyl-N-(6-(trifluoromethyl)-2,3-dihydrobenzofuran-3-yl)imidazo[1,5-a]quinoxaline-8-carboxamide | $^1$H NMR (400 MHz, DMSO-$d_6$) δ 9.05 (s, 1H), 8.32 (d, *J*= 1.8 Hz, 1H), 7.73 (d, *J* = 7.8 Hz, 1H), 7.60 - 7.52 (m, 1H), 7.38 (d, *J* = 8.4 Hz, 1H), 7.30 (d, *J* = 7.8 Hz, 1H), 7.20 (s, 1H), 6.91 (s, 2H), 5.94 (dd, *J* = 9.7, 4.5 Hz, 1H), 4.87 (t, *J* = 9.8 Hz, 1H), 4.70 (dd, *J* = 10.3, 4.6 Hz, 1H), 3.01 - 2.94 (m, 1H), 2.63 (s, 3H), 0.49 - 0.38 (m, 1H), 0.34 (h, *J* = 5.0 Hz, 1H), 0.20 (dq, *J* = 12.7, 6.4 Hz, 1H), 0.10 (dt, *J* = 10.6, 5.2 Hz, 1H).<br>LCMS (ESI) m/z: 468 $[M+H]^+$ |
| 70 | 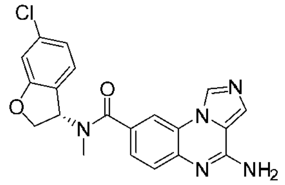 | (S)-4-amino-N-(6-chloro-2,3-dihydrobenzofuran-3-yl)-N-methylimidazo[1,5-a]quinoxaline-8-carboxamide | $^1$H NMR (400 MHz, DMSO-$d_6$) δ 9.19 (s, 1H), 8.32 (d, *J* = 1.4 Hz, 1H), 7.94 (s, 1H), 7.54 (h, *J* = 4.9, 4.4 Hz, 1H), 7.48 (s, 2H), 7.43 (d, *J* = 8.4 Hz, 1H), 7.09 - 6.97 (m, 2H), 4.86 - 4.59 (m, 2H), 2.66 (s, 3H).<br>LCMS (ESI) m/z: 395 $[M+H]^+$ |
| 72 | 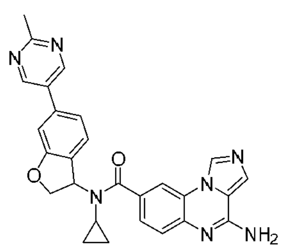 | 4-amino-N-cycloprop yl-N-(6-(2-methylpyri midin-5-yl)-2,3-dihyd robenzofuran-3-yl)imi dazo [1,5-a]quinoxalin e-8-carboxamide | $^1$H NMR (400 MHz, DMSO-$d_6$) δ 9.23 (s, 1H), 9.04 (s, 2H), .42 (d, *J* = 1.8 Hz, 1H), 7.98 (s, 1H), 7.81~7.55 (m, 4H), .46 (d, *J* = 8.4 Hz, 1H), 7.41~7.22 (m, 2H), 5.96 (dd, *J* = .2, 4.2 Hz, 1H), 4.83 *(t, J* = 9.7 Hz, 1H), 4.68 (m, 1H), 2.95 (s, 1H), 2.67 (s, 3H), 0.38 (ddt, *J* = 20.6, 5.9, 6.1 Hz, 2H), 0.26~0.08 (m, 2H).<br>LCMS (ESI) m/z: 478.2 $[M+H]^-$ |
| 73 | 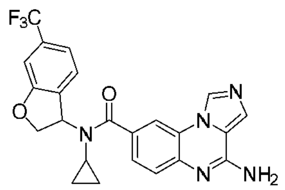 | 4-amino-N-cycloprop yl-N-(6-(trifluorometh yl)-2,3-dihydrobenzof uran-3-yl)imidazo[1,5 -a] quinoxaline-8-carb oxa-mide | $^1$H NMR (400 MHz, DMSO-$d_6$) δ 9.18 (s, 1H), 8.39 (d, *J* = 1.9 Hz, 1H), 7.92 (s, 1H), 7.74 (d, *J* = 7.8 Hz, 1H), 7.61 (dd, *J* = 8.4, 1.9 Hz, 1H), 7.51~7.38 (m, 3H), 7.31 (d, *J* = 7.8 Hz, 1H), 7.21 (s, 1H), 5.95 (dd, *J* = 9.8, 4.5 Hz, 1H), 4.87 (t, *J* = 9.8 Hz, 1H), 4.71 (dd, *J* = 10.1, 4.6 Hz, 1H), 2.98 (dt, *J* = 11.3, 5.1 Hz, 1H), 0.38 (ddt, *J*= 36.4, 10.8, 5.7 Hz, 2H), 0.26~0.04 (m, 2H).<br>LCMS (ESI) m/z: 454.3 $[M+H]^-$ |
| 74 | 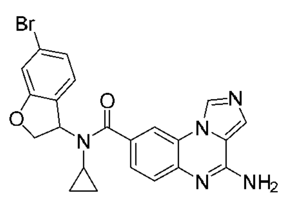 | 4-amino-N-(6-bro-mo-2,3-dihydrobenzo-fura n-3-yl)-N-cyclopropyl imidazo[1,5-a]quinox aline-8-carboxamide | $^1$H NMR (400 MHz, DMSO-$d_6$) δ 9.18 (s, 1H), 8.37 (d, *J* = 1.9 Hz, 1H), 7.92 (s, 1H), 7.59 (dd, *J* = 8.4, 1.8 Hz, 1H), 7.55 -7.37 (m, 4H), 7.24 - 7.03 (m, 2H), 5.86 (dd, *J* = 9.3, 4.2 Hz, 1H), 4.81 (t, *J* = 9.7 Hz, 1H), 4.65 (dd, *J* = 10.2, 4.2 Hz, 1H), 2.92 (d, *J* = 17.5 Hz, 1H), 0.50 - 0.27 (m, 2H), 0.27 - 0.04 (m, 2H).<br>LCMS (ESI) m/z: 465.3 $[M+H]^-$ |

(continued)

| Ex. | Structure | name | $^1$HNMR&$^{19}$F NMR& LCMS |
|---|---|---|---|
| 126 | 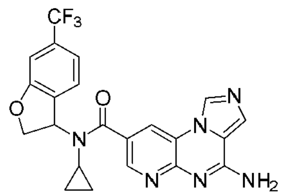 | 4-amino-N-cycloprop yl-N-(6-(trifluorometh yl)-2,3-dihydrobenzof uran-3-yl)imidazo[1,5 -a] pyrido[2,3-e]pyrazi ne-8-carboxamide | $^1$H NMR (400 MHz, DMSO-$d_6$) δ 9.29 (s, 1H) , 8.84(d, 1H), 8.75(d, 1H), 8.5-8.5(m, 2H), 8.10(s, 1H), 7.74(d, 1H), 7.31(d, 1H), 7.22(s, 1H), 5.99-5.95(m, 1H), 4.91-4.86(m, 1H), 4.73-4.70(m, 1H), 3.63-3.59(m, 1H),0.51-0.13(m, 4H). LCMS(ESI) m/z:455.3[M+H]$^+$ |
| 121 | 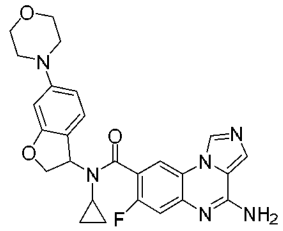 | 4-amino-N-cycloprop yl-7-fluoro-N-(6-mor pholino-2,3-dihydrobe nzofuran-3-yl)imidaz o [1,5-a]quinoxaline-8 -carboxamide | $^1$H NMR (400 MHz, DMSO-$d_6$) δ 9.11 (s, 1H), 8.30 (d, J = 6.6 Hz, 1H), 7.92 (s, 1H), 7.58 (s, 2H), 7.21 (t, J = 9.3 Hz, 2H), 6.60 -6.38 (m, 2H), 5.97 (s, 1H), 4.71 (s, 1H), 4.54 (dd, J = 10.5, 3.5 Hz, 1H), 3.72 (t, J = 4.7 Hz, 4H), 3.10 (t, J = 4.8 Hz, 4H), 2.62 -2.54 (m, 1H), 0.22 (d, J = 76.8 Hz, 4H). LCMS (ESI) m/z: 489.1 [M+H]$^-$ |

Example 75 Synthesis of (S)-4-amino-N-(methyl-d3)-N-(6-(trifluoromethyl)-2,3-dihydrobenzofuran-3-yl)imidazo[1,5-a] pyrido[3,4-e]pyrazine-8-carb oxamide

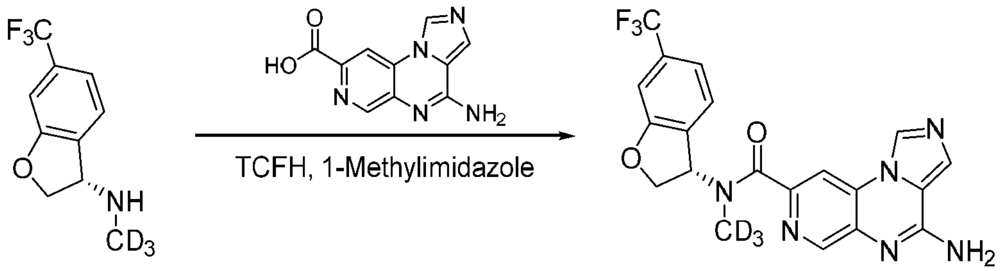


(S)-N-(methyl-d3)-6-(trifluoromethyl)-2,3-dihydrobenzofuran-3-amine(0.1g, 0.45mmol), 4-aminoimidazo[1,5-a] pyrido[3,4-e]pyrazine-8-carboxylic acid(0.1g, 0.45mmol), N,N,N',N'-Tetramethylchloroformamidinium hexafluorophosphate(0.19g, 0.68mmol) and 1-Methylimidazole(0.11g, 1.36mmol) were added in DMF(1mL) and the reaction mixture was stirred for 1 hour. After the reaction was complete, the reaction system was cooled to room temperature. Then the reaction mixture was extracted with ethyl acetate(10mL×3). The combined organic phase was washed with saturated brine(10mL×3), dried over anhydrous sodium sulfate, concentrated to dryness. The residue was further purified by HPLC(column: YMC Triart $C_{18}$ ExRs 5m, 30mm × 150 mm; mobile phase A: water(10mmol/L ammonium bicarbonate), mobile phase B: acetonitrile, flow rate: 60ml/min; Gradient: From 25% B to 55% B within 8 minutes; Wavelength: 254 nanometers/220 nanometers. Retention time(minimum value): 7.22minutes.) to obtain (S)-4-amino-N-(methyl-d3)-N-(6-(trifluoromethyl)-2,3-dihydrobenzofuran-3-yl)imidazo[1,5-a]pyrido[3,4-e]pyrazine-8-carb oxamide(11.94mg, yield:6.09%).

$^1$H NMR (400 MHz, DMSO-$d_6$) δ 9.31-9.30 (m, 1H), 8.70-8.65 (m, 1H),8.56-8.51 (m, 1H), 7.99 (d, *J* = 4.0 Hz, 1H), 7.79 - 7.56 (m, 3H),7.36- 7.34 (m, 1H), 7.27-7.25 (m, 1H), 6.44-6.02 (m, 1H), 4.89-4.71 (m, 2H).

LCMS (ESI): 431.85 [M+H]$^+$

By using the synthetic procedures described in Example 75, and replacing the corresponding starting materials, the embodiments listed in the table below can be prepared:

| Ex. | Structure | name | $^1$HNMR&$^{19}$F NMR& LCMS |
|---|---|---|---|
| 76 | 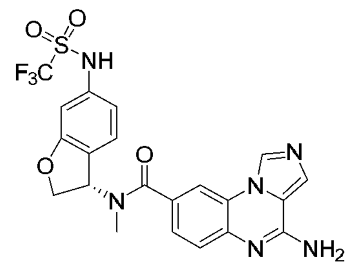 | (S)-4-amino-N-methyl-N-(6-((trifluoromethyl)sulfonamido)-2,3-dihydrobenzofuran-3-yl)imidazo[1,5-a]quinoxaline-8-carboxamide | $^1$H NMR (400 MHz, DMSO-$d_6$) δ 9.19 (s, 1H), 8.30 (s, 1H), 7.91 (s, 1H), 7.80 - 7.16 (m, 4H), 7.08 (s, 1H), 6.52-6.46 (m, 3H), 5.73-5.41(m,1H),4.62-4.49 (m, 2H), 2.98 - 2.50 (m, 3H).<br>LCMS (ESI): 507.1 [M+H] + |
| 118 | 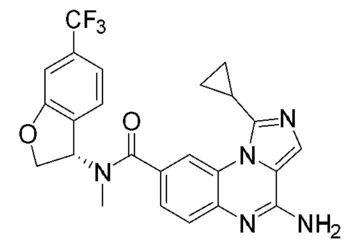 | (S)-4-amino-1-cyclopropyl-N-methyl-N-(6-(trifluoromethyl)-2,3-dihydrobenzofuran-3-yl)imidazo[1,5-a]quinoxaline-8-carboxamide | $^1$H NMR (400 MHz, DMSO-$d_6$) δ 8.66 (s, 1H), 7.79 (s, 1H), 7.62-7.60 (m, 1H), 7.54 - 7.45 (m, 2H), 7.32-7.31 (m, 3H), 7.25 (s, 1H), 6.34-6.11 (m, 1H), 4.84 - 4.70 (m, 2H), 2.67 (d, J = 3.0 Hz, 3H), 2.58 - 2.51 (m, 1H), 1.30 - 0.95 (m, 4H).<br>LCMS (ESI): 467.75 [M+H] + |

Example 77: Synthesis of 4-amino-N-((4S)-6-fluoro-1-methylisochroman-4-yl)-N-methylimidazo[1,5-a]quinoxaline-8-carboxamide

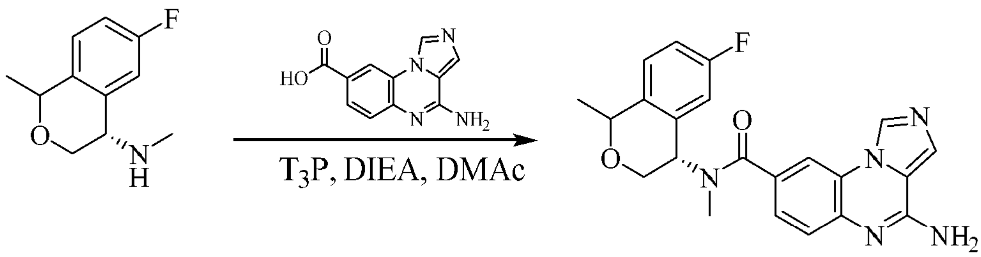


(4S)-6-fluoro-N,1-dimethylisochroman-4-amine hydrochloride(100mg, 0.5122mmol) was dissolved in DMA(4mL), followed by addition of 4-aminoimidazo[1,5-a]quinoxaline-8-carboxylic acid(140mg, 0.6146mmol), T3P(489mg, 0.7683mmol) and N,N-diisopropylethylamine(338mg, 3.561mmol). After the reaction mixture was stirred for 2 hours, the reaction solution was poured into water(40mL), extracted with ethyl acetate(20mL×3). The combined organic phase was dried over anhydrous sodium sulfate, filtered. The filtrate was concentrated to obtain crude product and was further purified by silica column chromatography(DCM:MeOH=10:1) to obtain 4-amino-N-((4S)-6-fluoro-1-methylisochroman-4-yl)-N-methylimidazo[1,5-a]quinoxaline-8-carboxamide(70mg, yield: 33.7%).

$^1$H NMR (400 MHz, DMSO-$d_6$) δ 9.27 - 9.11 (m, 1H), 8.38 (d, *J* = 17.2 Hz, 1H), 7.92 (s, 1H), 7.60 - 6.98 (m, 7H), 5.62 (s, 1H), 4.94 - 4.67 (m, 1H), 4.38 - 3.79 (m, 2H), 2.87 - 2.66 (m, 3H), 1.49 (dd, *J* = 13.0, 6.2 Hz, 3H).

$^{19}$F NMR (376 MHz, DMSO-$d_6$) δ -115.37, -115.39.

LCMS (ESI) m/z: 406 [M+H]$^+$

Example 78 (S)-4-amino-N-methyl-N-(6-(trifluoromethyl)-2,3-dihydrobenzofuran-3-yl)imidazo[1,5-a]quinoxaline-8-carboxamide-1-d

Example 111 (S)-4-amino-1-bromo-N-methyl-N-(6-(trifluoromethyl)-2,3-dihydrobenzofuran-3-yl)imidazo[1,5-a]quinoxaline-8-carboxam ide

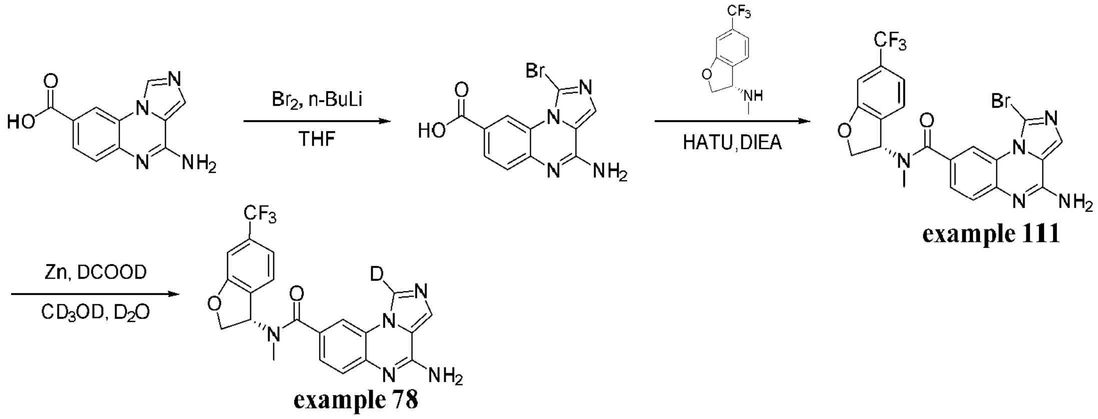


Step 1: Synthesis of 4-amino-1-bromoimidazo[1,5-a]quinoxaline-8-carboxylic acid

4-aminoimidazo[1,5-a]quinoxaline-8-carboxylic acid(500mg, 2.19mmol) was dissolved THF(15mL). Then the reaction system was cooled to -40°C, and n-butyllithium(Sml. 15.34mmol) was added dropwise keeping -40°C. The mixture reacted for 10 minutes at 40°C, then reacted for 10 minutes at room temperature. After the reaction was complete, the reaction mixture was concentrated under vacuum to dryness. After DCM was added, the mixture was filtered then further purified by silica column chromatography (Formic acid: acetonitrile=1:1) to obtain 4-amino-1-bromoimidazo[1,5-a] quinoxaline-8-carboxylic acid(30mg, yield: 4.46%), as a white solid.
LCMS (ESI) m/z: 307 [M+H] $^+$

Step 2: Synthesis of Example 111 (S)-4-amino-1-bromo-N-methyl-N-(6-(trifluoromethyl)-2,3-dihydrobenzofuran-3-yl) imidazo[1,5-a]quinoxaline-8-carboxam ide

4-amino-1-bromoimidazo[1,5-a]quinoxaline-8-carboxylic acid(10mg, 0.03mmol), 2-(7-Azabenzotriazol-1-yl)-N,N,N',N'-tetramethyluronium hexafluorophosphate(19mg, 0.05mmol), N,N-diisopropylethylamine(8mg, 0.05mmol) and (S)-N-methyl-6-(trifluoromethyl)-2,3-dihydrobenzofuran-3-amine (7mg, 0.03mmol) were successively dissolved in DMF(1mL). After completion, the reaction mixture was stirred at room temperature for 2 hours. After completion, the reaction mixture was extracted with ethyl acetate(10mL×3). The combined organic phase was washed with saturated brine(10mL×3), dried over anhydrous sodium sulfate and filtered. The filtrate was concentrated under vacuum to obtain Example 111 (S)-4-amino-1-bromo-N-methyl-N-(6-(trifluoromethyl)-2,3-dihydrobenzofuran-3-yl)imidazo[1,5-a]quinoxaline-8-carboxam ide(30mg, 0.03mmol, yield: 80%), as yellow liquid.
$^1$H NMR (400 MHz, DMSO-$d_6$) δ 9.09 (s, 1H), 7.98 (s, 1H), 7.65 - 7.55 (m, 2H), 7.52 (d, *J* = 7.9 Hz, 3H), 7.32 (d, *J* = 7.7 Hz, 1H), 7.25 (s, 1H), 6.42(s,1H),4.79 - 4.72 (m, 2H), 2.75 - 2.62 (m, 3H).
LCMS (ESI) *m/z*:506 [M+H] $^+$

Step 3 Synthesis of Example 78 (S)-4-amino-N-methyl-N-(6-(trifluoromethyl)-2,3-dihydrobenzofuran-3-yl)imidazo[1,5-a]quinoxaline-8-carboxamide-1-d

(S)-4-amino-1-bromo-N-methyl-N-(6-(trifluoromethyl)-2,3-dihydrobenzofuran-3-yl)imidazo[1,5-a]quinoxaline-8-carb oxamide(20mg, 0.16mmol), Zinc powder(26mg, 1.58mmol), and deuterated formic acid(19mg, 1.58mmol) were dissolved in the solution of deuterated methanol(lmL) and $D_2O$(1mL). The reaction mixture was stirred at room temperature for 1 hour. After the reaction was complete, the mixture was extracted with ethyl acetate (10mL×3). The combined organic phase was washed with saturated brine (10mL×3), dried over anhydrouse sodium sulfate, concentrated under reduced pressure. The residue was purified by HPLC(column: Kinetex 5 m EVO $C_{18}$, 30mm × 150 mm; mobile phase A: water(10mmol/L ammonium bicarbonate), mobile phase B: acetonitrile, flow rate: 60ml/min; Gradient: From 20% B to 47% B within 10 minutes; Wavelength: 254 nanometers/220 nanometers. Retention time: 9.35minutes.) to obtain Example 78 (S)-4-amino-N-methyl-N-(6-(trifluoromethyl)-2,3-dihydrobenzofuran-3-yl)imidazo[1,5-a]quinoxaline-8-carboxamide-1-d(3. 34mg, 0.16mmol, yield: 8.45%), as white solid.
$^1$H NMR (400 MHz, DMSO-$d_6$) δ 8.34 (s, 1H),7.92 (d, *J* = 7.3 Hz, 1H), 7.64-7.62 (d, *J* = 7.6 Hz, 1H), 7.49 (s, 4H), 7.33-7.32 (m, 1H), 7.26 (s, 1H),6.48 (m,1H) ,4.73-4.72(m,2H), 2.68 (s, 3H).
LCMS (ESI) *m/z:* 428 [M+H] $^+$
By using the synthetic procedures described in Example 78, and replacing the corresponding starting materials, the embodiments listed in the table below can be prepared:

| Ex. | Structure | name | $^1$HNMR&$^{19}$F NMR& LCMS |
|---|---|---|---|
| 79 | 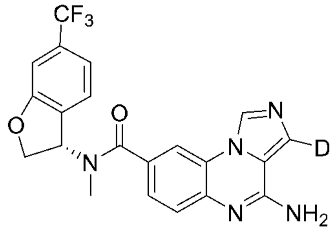 | (S)-4-amino-N-methy 1-N-(6-(trifluoromethy l)-2,3-dihydrobenzofu ran-3-yl)imidazo[1,5-a] quinoxaline-8-carbo xa-mide-3-d | $^1$H NMR (400 MHz, DMSO-$d_6$) δ 9.18 (s, 1H), 8.33 (t, J = 1.1 Hz, 1H), 7.64 (d, J = 7.8 Hz, 1H), 7.52 - 7.37 (m, 4H), 7.33 (d, J = 7.9 Hz, 1H), 7.26 (s, 1H), 6.52(s,1H),4.72 (dd, J = 10.1, 4.5 Hz, 2H), 2.67 (d, J = 2.8 Hz, 3H).<br>LCMS (ESI) *m/z:* 428 [M+H]$^-$ |
| 127 | 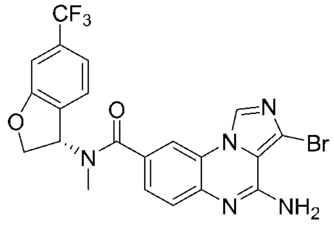 | (S)-4-amino-3-bromo-N-methyl-N-(6-(triflu oromethyl)-2,3-dihydr obenzofuran-3-yl)imidazo[1,5-a]quinoxalin e-8-carboxamide | $^1$H NMR (400 MHz, DMSO-$d_6$) δ 9.25 (s, 1H), 8.33 (s, 1H), 7.63 (d, J = 7.8 Hz, 1H), 7.56 - 7.47 (m, 2H), 7.34-7.32 (m, 1H), 7.26 (s, 1H), 7.05 (s, 2H), 6.52-5.65 (m, 1H), 4.89 - 4.65 (m, 2H), 2.67 (s, 3H).<br>LCMS (ESI): 506 [M+H] $^-$ |

Example 109 Synthesis of ethyl 4-amino-8-(methyl(6-(trifluoromethyl)-2,3-dihydrobenzofuran-3-yl)carbamoyl)imidazo [1,5-a]quinoxaline-3-carboxylate

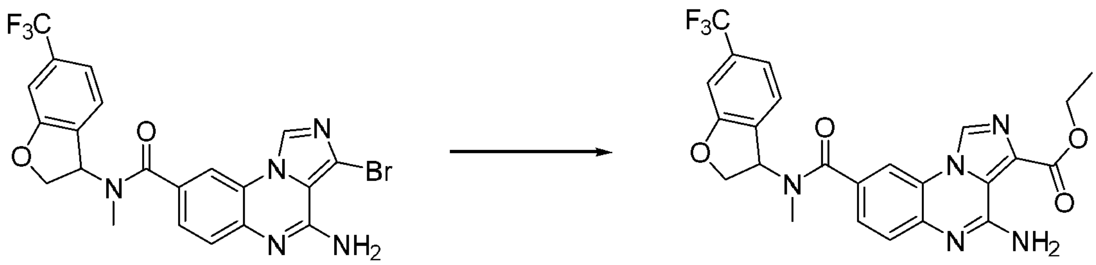

4-amino-3-bromo-N-methyl-N-(6-(trifluoromethyl)-2,3-dihydrobenzofuran-3-yl)imidazo[1,5-a]quinoxaline-8-carbox amide(30mg, 0.006mmol) , potassium acetate(17mg, 0.18mmol) and 1,1'-Bis(diphenylphosphino)ferrocene-palladium(II)dichloride dichloromethane complex(4mg, 0.01mmol) were added to the mixed solvent of DMF(5mL) and EtOH(SmL). The reaction proceeded overnight in a high-pressure vessel under carbon monoxide (4 mPa), at 110 °C. After the reaction was complete, the reaction was diluted with ethyl acetate(10mL) and water(5mL). The water layer was extrated with ethyl acetate(2×10mL). The combined organic phase was washed with saturated brine(10mL), dried over anhydrous sodium sulfate and concentrated to obtain crude product. The crude product was purified by HPLC(Column: Sunfire $C_{18}$ 5 m, 30 mm × 150 mm; Mobile Phase A: Water(0.1% FA), Mobile Phase B: ACN; Flow rate: 60ml/min mL/min; Gradient: 37% B to 64% B in 10 min; Wave Length: 254nm/220nm nm; RT1(min): 5.97) to obtain Ethyl 4-amino-8-(methyl(6-(trifluoromethyl)-2,3-dihydrobenzofuran-3-yl)carbamoyl)imidazo[1,5-a]quinoxaline-3-carboxylate(4. 22mg, 14%).

$^1$H NMR (400 MHz, DMSO-$d_6$) δ 9.36 (s, 1H), 9.30-8.61 (m, 1H), 8.46(s,1H), 8.10-7.92(m, 1H), 7.70-7.64 (m, 1H), 7.61 - 7.50 (m, 2H), 7.34-7.30 (m, 1H), 7.26 (s, 1H),6.38-5.76(m, 1H), 4.84-4.70 (m, 2H), 4.32-4.37 (m, 2H), 2.68-2.66 (m, 3H), 1.37 (t, J = 7.1 Hz, 3H).

LCMS (ESI): 500.10[M+H]$^+$

Example 80 Synthesis of (S)-4-amino-7-fluoro-N-(6-methoxy-2,3-dihydrobenzofuran-3-yl)-N-methylimidazo[1,5-a]quinoxaline-8-carboxamide

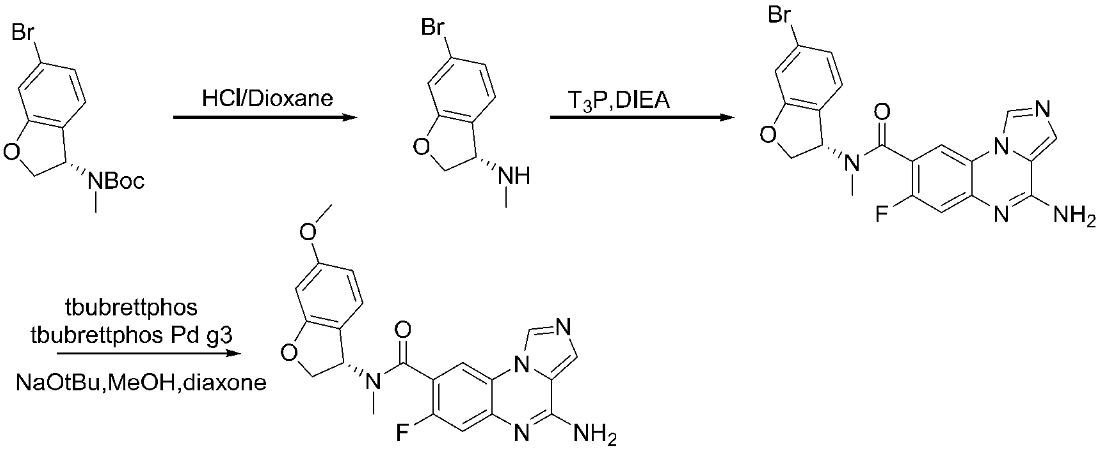


Step 1: Synthesis of (S)-6-bromo-N-methyl-2,3-dihydrobenzofuran-3-amine

tert-butyl (S)-(6-bromo-2,3-dihydrobenzofuran-3-yl)(methyl)carbamate(200mg, 0.6mmol) was dissolved in the solution of hydrochloride acid in 1.4-dioxane and was stirred at room temperature. After the reaction was complete, the reaction solution was concentrated to obtain crude product (S)-6-bromo-N-methyl-2,3-dihydrobenzofuran-3-amine(85mg, yield 61%)

LCMS (ESI) m/z: 228 $[M+H]^+$

Step 2: Synthesis of (S)-4-amino-N-(6-bromo-2,3-dihydrobenzofuran-3-yl)-7-fluoro-N-methylimidazo[1,5-a]quinoxaline-8-carboxamide

4-amino-7-fluoroimidazo[1,5-a]quinoxaline-8-carboxylic acid was dissolved in DMA(2mL), followed by addition of Propylphosphonic anhydride(172mg, 0.74mmol), N,N-diisopropylethylamine(144mg, 1.12mmol) and (S)-6-bromo-N-methyl-2,3-dihydrobenzofuran-3-amine(85mg, 0.37mmol) successively. The reaction system was then stirred overnight at room temperature. After the reaction was complete, the solution was extracted with ethyl acetate(10mL×3). The combined organic phase was washed with saturated brine, dried over anhydrous sodium sulfate, concentrated then purified by silical column chromatography(DCM: MeOH= 10%) to obtain (S)-4-amino-N-(6-bromo-2,3-dihydrobenzofuran-3-yl)-7-fluoro-N-methylimidazo[1,5-a]quinoxaline-8-carboxamide(35mg, 20%).

LCMS (ESI) m/z:456 $[M+H]^+$

Step 3: Synthesis of (S)-4-amino-7-fluoro-N-(6-methoxy-2,3-dihydrobenzofuran-3-yl)-N-methylimidazo[1,5-a]quinoxaline-8-carboxamide

(S)-4-amino-N-(6-bromo-2,3-dihydrobenzofuran-3-yl)-7-fluoro-N-methylimidazo[1,5-a]quinoxaline-8-carboxamide( 35mg, 0.08mmol) was dissolved in dioxane(1mL), followed by addition of [(2-Di-tert-butylphosphino-3,6-dimethoxy-2',4',6'-triisopropyl-1,1'-biphenyl)-2-(2'-amino-1,1'-biphenyl)]palladium(II) methanesulfonate(65mg, 0.07mmol), MeOH(86mg, 2.68mmol) under $N_2$ atmosphere. After completion, the reaction system was stirred for 10 hours at room temperature. After the reaction was complete, the solution was extracted with ethyl acetate(10mL×3). The combined organic phase was washed with saturated brine, dried over anhydrous sodium sulfate and concentrated. The residue was further purified by preparative HPLC Column: XBridge BEH Shield RP18 5 m, 30 mm × 150 mm; Mobile Phase A: Water(10mmol/L $NH_4HCO_3$), Mobile Phase B: ACN; Flow rate: 60ml/min mL/min; Gradient: 28% B to 50 % B in 8 min; Wave Length: 254nm/220nm nm; RT1(min):7.88) to obtain (S)-4-amino-7-fluoro-N-(6-methoxy-2,3-dihydrobenzofuran-3-yl)-N-methylimidazo[1,5-a]quinoxaline-8-carboxamide(14.2 5mg, yield: 44.6%).

$^1H$ NMR (400 MHz, DMSO-$d_6$) δ 9.12 (s, 1H),8.46-8.29 (m, 1H), 7.92 (d, *J* = 3.6 Hz, 1H), 7.59 (s, 2H), 7.28 - 7.20 (m, 2H), 6.58 - 6.31 (m, 2H),, 5.42- 4.79 (m, 1H), 4.74 - 4.53 (m, 2H), 3.74 (d, *J* = 10.0 Hz, 3H), 2.70 -2.58(m, 3H).

LCMS (ESI) m/z: 408.10 $[M+H]^+$

Example 122: Synthesis of methyl (S)-3-(4-amino-N-methylimidazo[1,5-a]quinoxaline-8-carboxamido)-2,3-dihydrobenzofuran-6-carboxylate

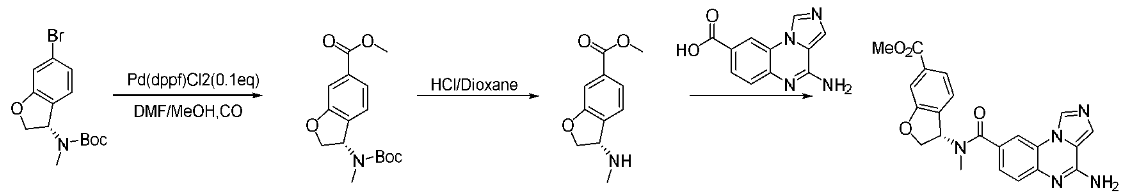


Step 1: Synthesis of methyl (S)-3-((tert-butoxycarbonyl)(methyl)amino)-2,3-dihydrobenzofuran-6-carboxylate

At room temperature, tert-butyl (S)-(6-bromo-2,3-dihydrobenzofuran-3-yl)(methyl)carbamate(100mg, 0.31mmol) was dissolved in the solution of MeOH(SmL) and DMF(10mL). Then [1,1'-Bis(diphenylphosphino)ferrocene]dichloropalladium(II)(56mg, 0.07mmol) and potassium acetate(68mg, 0.69mmol) were added successively. The reaction system proceeded overnight at 100°C under CO atmosphere(4MPa). After the reaction was complete, the solution was concentrated, poured into water and filtered. The filtered cake was slurried in petroleum ether to obtain methyl (S)-3-((tert-butoxycarbonyl)(methyl)amino)-2,3-dihydrobenzofuran-6-carboxylate(70mg, yield: 77.78%), as red solid.

LCMS (ESI): 308 $[M+H]^+$

Step 2: Synthesis of methyl (S)-3-(methylamino)-2,3-dihydrobenzofuran-6-carboxylate

(S)-3-((tert-butoxycarbonyl)(methyl)amino)-2,3-dihydrobenzofuran-6-carboxylate(70mg, 0.23mmol) was dissolved in the solution of HCl in 1,4-dioxane(2mL, 4M). The reaction mixture proceeded for 0.5h at room temperature. After the reaction was complete, the solution was concentrated to obtain methyl (S)-3-(methylamino)-2,3-dihydrobenzofuran-6-carboxylate(46mg, yield 97%), as black solid.

LCMS (ESI): 208 $[M+H]^+$

Step 3 Synthesis of methyl (S)-3-(4-amino-N-methylimidazo[1,5-a]quinoxaline-8-carboxamido)-2,3-dihydrobenzofuran-6-carboxylate

4-aminoimidazo[1,5-a]quinoxaline-8-carboxylic acid was dissolved in DMF(4mL), followed by addition of 2-(7-Azabenzotriazol-1-yl)-N,N,N',N'-tetramethyluronium hexafluorophosphate(128mg, 0.34mmol), N,N-diisopropylethylamine(87mg, 0.67mmol) and (S)-3-(methylamino)-2,3-dihydrobenzofuran-6-carboxylate (46mg, 0.22mmol) successively. The reaction mixture was stirred for 3 hours at room temperature. After the reaction was complete, the mixture was diluted with ethyl acetate(10mL) and water(5mL). Then the water phase was seperated and extracted with ethyl acetate(2×10mL). The combined organic phase was washed with saturated brine(10mL), dried over anhydrous sodium sulfate, filtered and the filtrate was concentrated to obtain crude product. The crude product was further purified by HPLC(column: Sunfire C18 5 m, 30mm×150mm; mobile phase A: water(0.1% formic acid), mobile phase B: acetonitrile; flow rate: 60ml/min; gradient from 5% B to 30% B within 8 mins; wavelength: 254nm/220nm) to obtain methyl (S)-3-(4-amino-N-methylimidazo[1,5-a]quinoxaline-8-carboxamido)-2,3-dihydrobenzofuran-6-carboxylate(21mg, yield: 1%), as white solid.

$^1$H NMR (400 MHz, DMSO-$d_6$) δ9.21 (s, 1H) , 8.35 (s, 1H), 8.13(s,1H), 8.01-7.92 (s, 1H), 7.62-7.60(m,1H), 7.56-7.54 (m,1H), 7.50-7.49 (m, 2H), 7.40-7.36 (m, 1H), 6.39-5.66 (s, 1H), 4.87-4.68 (m, 2H), 3.85-3.84 (m, 3H),2.67-2.64(m,3H), 0.98-0.79 (m,3H)

LCMS (ESI): 418.20 $[M+H]^+$

Example 112: Synthesis of (S)-4-amino-N-(6-(difluoromethyl)-2,3-dihydrobenzofuran-3-yl)-N-methylimidazo[1,5-a]quinoxaline-8-carboxamide

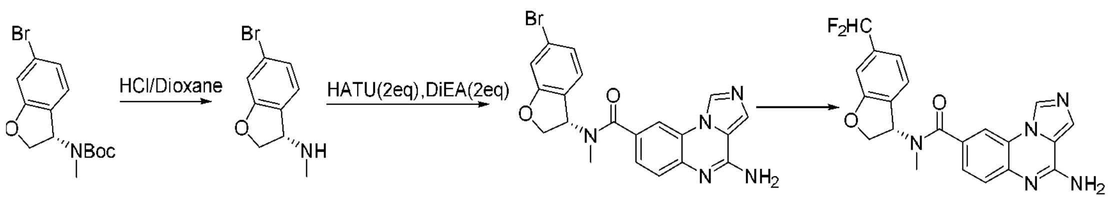

Step 1 Synthesis of (S)-6-bromo-N-methyl-2,3-dihydrobenzofuran-3-amine

tert-butyl (S)-(6-bromo-2,3-dihydrobenzofuran-3-yl)(methyl)carbamate was dissolved in the solution of HCl in 1,4-dioxane(4.0M, 10mL). Then the reaction mixture was stirred at room temperature for 2 hours. After the reaction was complete, the solution was concentrated under vacuum to obtain (S)-6-bromo-N-methyl-2,3-dihydrobenzofuran-3-amine (80mg, 0.30mmol, yield: 95.46%), as yellow liquid.

LCMS (ESI): 228 $[M+H]^+$

Step 2: Synthesis of (S)-4-amino-N-(6-bromo-2,3-dihydrobenzofuran-3-yl)-N-methylimidazo[1,5-a]quinoxaline-8-carboxamide

4-aminoimidazo[1,5-a]quinoxaline-8-carboxylic acid(80mg, 0.35mmol), 2-(7-Azabenzotriazol-1-yl)-N,N,N',N'-tetramethyluronium hexafluorophosphate(200mg, 0.53mmol) and N,N-diisopropylethylamine(136mg, 1.05mmol) were dissolved in DMA(3mL) followed by addition of (S)-6-bromo-N-methyl-2,3-dihydrobenzofuran-3-amine(80mg, 0.35mmol). The reaction mixture was stirred for 2 hours at room temperature. After the reaction was complete, the solution was extracted with ethyl acetate(10mL×3). The combined organic phase was washed with saturated brine(10mL×3), dried over anhydrous sodium sulfate and concentrated to obtain crude product. The crude product was further purified by silica column chromatography(petroleum ether: ethyl acetate=80:20) to obtain (S)-4-amino-N-(6-bromo-2,3-dihydrobenzofuran-3-yl)-N-methylimidazo[1,5-a]quinoxaline-8-carboxamide(70mg, 0.35mmol, yield: 45.54%).

LCMS (ESI): 438 $[M+H]^+$

Step 3: Synthesis of (S)-4-amino-N-(6-(difluoromethyl)-2,3-dihydrobenzofuran-3-yl)-N-methylimidazo[1,5-a]quinoxaline-8-carboxamide

(S)-4-amino-N-(6-bromo-2,3-dihydrobenzofuran-3-yl)-N-methylimidazo[1,5-a]quinoxaline-8-carboxamide(30mg, 0.07mmol) was dissolved in THF(2mL), followed by successively addition of $[Ir(dF(CF_3)ppy)_2(dpy)]PF_6$(8mg, 0.007mmol), nickel(II) bromide ethylene glycol dimethyl ether complex(1mg, 0.003mmol), $PPh_3(CF_2H)_2$(17mg, 0.14mmol), diphenyl phenanthroline(2mg, 0.007mmol). The reaction proceeded overnight at room temperature under wavelength of 465nm. After the reaction was complete, the solution was extracted with ethyl acetate(10mL×3). The combined organic phase was washed with saturated brine(10mL×3), dried over anhydrous sodium sulfate, concentrated to obtain crude product. The crude product was further purified by HPLC(column: Kinetex 5 m EVO C18, 30mm × 150 mm; mobile phase A: water(10mmol/L ammonium bicarbonate), mobile phase B: Acetonitrile; flow rate: 60ml/minutes; Gradient from 20% B to 47% B with 10 minutes; wavelength: 254/220nm; reteintion time(minimum value): 9.35) to obtain (S)-4-amino-N-(6-(difluoromethyl)-2,3-dihydrobenzofuran-3-yl)-N-methylimidazo[1,5-a]quinoxaline-8-carboxamide(0.84 mg, 0.07mmol, yield:2.96%).

$^1$H NMR (400 MHz, Methanol-$d_4$) δ 9.11 (s, 1H), 8.29 (s, 1H), 7.98 (s, 1H), 7.57-7.54 (m, 3H), 7.17-7.16 (s, 1H), 7.03 (s, 1H), 4.73-4.62 (s, 2H),4.60-4.52(m, 1H), 2.78 (s, 3H).

LCMS (ESI): 409 $[M+H]^+$

Example 115: Synthesis of (R)-4-amino-7-fluoro-N-methyl-N-(7-(1-methyl-1H-pyrazol-5-yl)chroman-4-yl)imidazo[1,5-a]quinoxaline-8-carboxamide

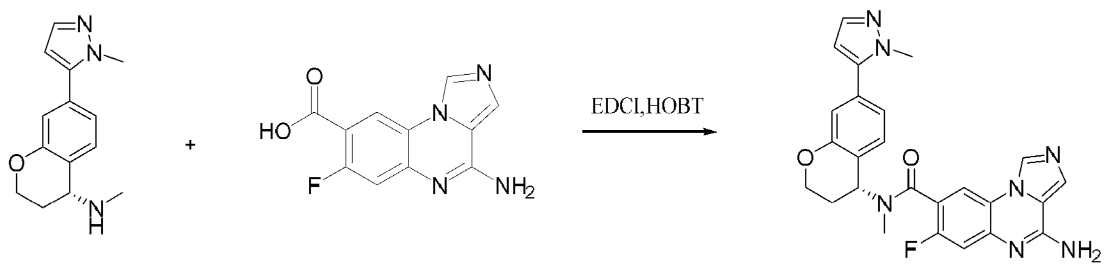


At room temperature, 4-amino-7-fluoroimidazo[1,5-a]quinoxaline-8-carboxylic acid(200mg, 0.813mmol) was dissolved in DMAc(3mL), followed by addition of 1-(3-Dimethylaminopropyl)-3-ethylcarbodiimide(187mg, 0.976mmol) and 1-Hydroxybenzotriazole(133mg, 0.976mmol) then DIEA(421mg, 3.264mmol). The reaction system proceeded at

room temperature for 0.5h. Then (R)-N-methyl-7-(1-methyl-1H-pyrazol-5-yl)chroman-4-amine(1g, 4.184mmol) was added. The reaction mixture preceeded at room temperature for 16 hours. After completion of the reaction indicated by LCMS, water(30mL) was added, and extracted with ethyl acetate(10mL×3). The combined organic phase was washed with saturated brine(30mL), dried over anhydrous sodium sulfate, filtered. And the filtrate was concentrated to obtain crude product. And the crude product was further purified by silica column chromatography(0-5% MeOH/DCM) to obtain (R)-4-amino-7-fluoro-N-methyl-N-(7-(1-methyl-1H-pyrazol-5-yl)chroman-4-yl)imidazo[1,5-a]quinoxaline-8-carboxamide( 45mg, yield: 11.75%)

$^1$H NMR (400 MHz, DMSO-$d_6$) δ 9.17 (d, *J* = 22.9 Hz, 1H), 8.42 (d, *J*= 6.6 Hz, 1H), 7.94 (d, *J* = 8.6 Hz, 1H), 7.61 (d, *J* = 12.8 Hz, 2H), 7.46 (dd, *J* = 6.2, 1.9 Hz, 1H), 7.27 (td, *J* = 11.3, 7.6 Hz, 2H), 7.14 (dd, *J* = 7.9, 1.8 Hz, 1H), 6.98 (dd, *J* = 27.5, 1.8 Hz, 1H), 6.41 (dd, *J*= 9.7, 1.9 Hz, 1H), 6.10 -5.04 (m, 1H), 4.49 -4.05 (m, 2H), 3.86 (d, *J* = 15.6 Hz, 3H), 2.82 -2.62 (m, 3H), 2.32 (dd, *J* = 17.9, 7.3 Hz, 1H), 2.13 (dd, *J* = 7.1, 3.6 Hz, 1H).

LCMS (ESI) m/z: 472.1 $[M+H]^+$

By using the synthetic procedures described in Example 115, and replacing the corresponding starting materials, the embodiments listed in the table below can be prepared:

| Ex. | Structure | name | $^1$HNMR&$^{19}$F NMR& LCMS |
|---|---|---|---|
| 13 | 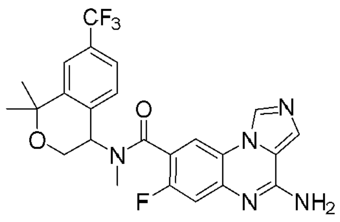 | 4-amino-N-(1,1-dimethyl-7-(trifluoromethyl ) isochroman-4-yl)-7-fluoro-N-methylimidaz o [1,5-a]quinoxaline-8 -carboxamide | $^1$H NMR (400 MHz, DMSO-$d_6$) δ 9.13 (s, 1H), 8.81 (s, 1H), 8.34 (d, *J* = 6.6 Hz, 1H), 8.08 (d, *J* = 8.0 Hz, 1H), 7.96 (d, *J*= 16.4 Hz, 2H), 7.60 (s, 2H), 7.24 (d, *J* = 11.2 Hz, 1H), 4.90 (s, 2H), 2.81 (s, 1H), 0.53 (d, *J* = 12.7 Hz, 4H). LCMS (ESI) m/z: 488.2 $[M+H]^-$ |
| 113 | 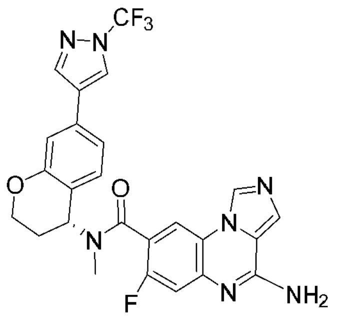 | (R)-4-amino-7-fluoro-N-methyl-N-(7-(1-(trifluoromethyl)-1H-pyrazol-4-yl)chroman-4-ylimidazo[1,5-a]quinoxaline-8-carboxamid e | $^1$H NMR (400 MHz, DMSO-$d_6$) δ 9.16 (d, *J* = 22.8 Hz, 1H), 8.98 (d, *J* = 3.8 Hz, 1H), 8.44 (dd, *J* = 20.3, 7.1 Hz, 2H), 7.93 (d, *J* = 9.8 Hz, 1H), 7.58 (t, *J* = 13.0 Hz, 2H), 7.39 - 7.13 (m, 4H), 5.52 (ddd, *J* = 405.6, 10.5, 6.5 Hz, 1H), 4.49 - 3.96 (m, 2H), 2.81 - 2.58 (m, 3H), 2.28 (qd, *J* = 12.2, 10.9, 6.8 Hz, 1H), 2.19 - 2.02 (m, 1H). LCMS (ESI) m/z: 472.1 $[M+H]^-$ |
| 114 | 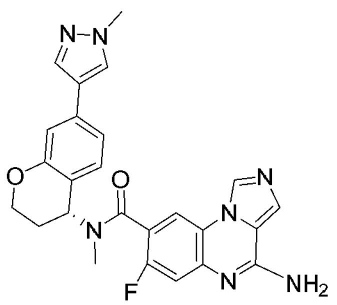 | (R)-4-amino-7-fluoro-N-methyl-N-(7-(1-methyl-1H-pyrazol-4-yl) chroman-4-yl)imidazo [1,5-a]quinoxaline-8-c arboxamide | $^1$H NMR (400 MHz, DMSO-$d_6$) δ 9.19 - 9.13 (m, 1H), 8.43 - 8.19 (m, 1H), 8.12 (d, *J* = 5.2 Hz, 1H), 7.93 (d, *J* = 9.2 Hz, 1H), 7.84 (d, *J* = 7.5 Hz, 1H), 7.60 - 7.53 (m, 2H), 7.29 - 6.96 (m, 4H), 5.48 (ddd, *J* = 409.0, 10.4, 6.4 Hz, 1H), 4.43 - 3.98 (m, 2H), 3.85 (d, *J* = 6.1 Hz, 3H), 2.82 - 2.59 (m, 3H), 2.36 -2.19 (m, 1H), 2.08 (t, *J* = 5.9 Hz, 1H). LCMS (ESI) m/z: 472.1 $[M+H]^-$ |
| 120 | 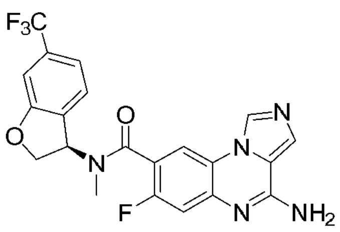 | (R)-4-amino-7-fluoro-N-methyl-N-(6-(triflu oromethyl)-2,3-dihydr obenzofuran-3-yl)imi dazo[1,5-a]quinoxalin e-8-carboxamide | $^1$H NMR (400 MHz, DMSO-$d_6$) δ 9.13 (s, 1H), 8.44- 8.24 (m, 1H), 7.93 (d, *J* = 3.1 Hz, 1H), 7.58 (d, *J*= 28.1 Hz, 3H), 7.43 -7.15 (m, 3H), 6.53 -5.60 (m, 1H), 4.96 -4.58 (m, 2H), 2.65 (d, *J* = 31.4 Hz, 3H). LCMS (ESI) m/z: 446.1 $[M+H]^-$ |

(continued)

| Ex. | Structure | name | $^1$HNMR&$^{19}$F NMR& LCMS |
|---|---|---|---|
| 133 | 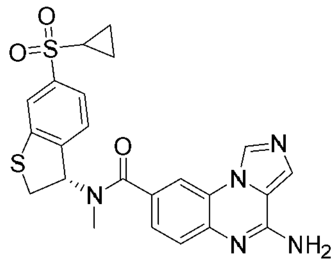 | (S)-4-amino-N-(6-(cyclopropylsulfonyl)-2,3-dihydrobenzo[b]thiophen-3-yl)-N-methylimidazo[1,5-a]quinoxaline-8-carboxamide | $^1$H NMR (400 MHz, DMSO-$d_6$) δ 9.19 (s, 1H), 8.34 (s, 1H), 7.92 (s, 1H), 7.69 (d, J = 7.8 Hz, 1H), 7.60 - 7.29 (m, 6H), 6.55 - 5.83 (m, 1H), 4.97 - 4.58 (m, 2H), 2.98 - 2.84 (m, 1H), 2.70 (s, 3H), 1.19 - 0.94 (m, 4H). LCMS (ESI) m/z: 464.2 $[M+H]^-$ |
| 136 | 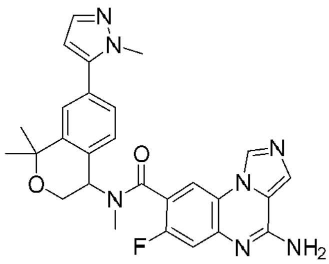 | 4-amino-N-(1,1-dimethyl-7-(1-methyl-1H-pyrazol-5-yl)isochroman-4-yl)-7-fluoro-N-methylimidazo[1,5-a]quinoxaline-8-carboxamide | LCMS (ESI) m/z: 500.2 $[M+H]^-$ |
| 137 | 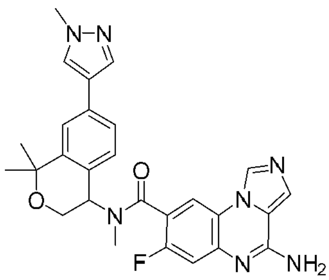 | 4-amino-N-(1,1-dimethyl-7-(1-methyl-1H-pyrazol-4-yl)isochroman-4-yl)-7-fluoro-N-methylimidazo[1,5-a]quinoxaline-8-carboxamide | LCMS (ESI) m/z: 500.2 $[M+H]^-$ |

Example 128 Synthesis of (S)-4-amino-3-(hydroxymethyl)-N-methyl-N-(6-(trifluoromethyl)-2,3-dihydrobenzofuran-3-yl) imidazo[1,5-a]quinoxaline-8 -carboxamide

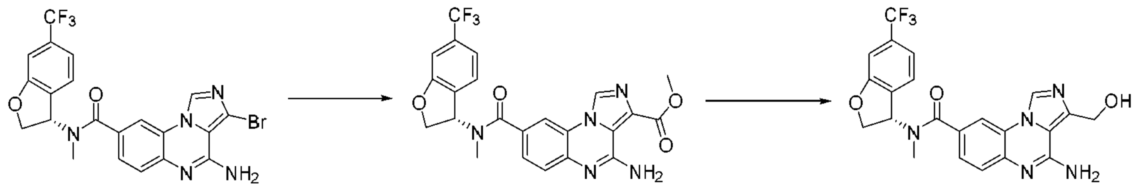

Step 1: Synthesis of methyl (S)-4-amino-8-(methyl(6-(trifluoromethyl)-2,3-dihydrobenzofuran-3-yl)carbamoyl)imidazo [1,5-a]quinoxaline-3-carboxylat e

(S)-4-amino-3-bromo-N-methyl-N-(6-(trifluoromethyl)-2,3-dihydrobenzofuran-3-yl)imidazo[1,5-a]quinoxaline-8-carb oxamide(60mg, 0.12mmol) was dissolved in the solution of DMF(2mL) and MeOH(2mL), followed by addition of [1,1'-Bis(diphenylphosphino)ferrocene]dichloropalladium(II)(3mL, 0.04mmol) and potassium acetate(23mg, 0.24mmol). The reaction proceeded for 16 hours at 100°C under CO atmosphere(4MPa) in high pressure vessel. After the reaction was complete, the solution was extracted with ethyl acetate(10mL × 3). The combined organic phase was washed with saturated brine(10mL×3), dried over anhydrous sodium sulfate and concentrated under vacuum and further purified by silica column chromatography(basic water: acetonitrile=1:1) to obtain methyl (S)-4-amino-8-(methyl(6-(trifluoromethyl)-2,3-dihydrobenzofuran-3-yl)carbamoyl)imidazo[1,5-a]quinoxaline-3-carboxylat e(40mg, yield: 69.53%), as brown solid.

LCMS (ESI): 485.42 $[M+H]^+$

Step 2: Synthesis of (S)-4-amino-3-(hydroxymethyl)-N-methyl-N-(6-(trifluoromethyl)-2,3-dihydrobenzofuran-3-yl)imidazo[1,5-a]quinoxaline-8 -carboxamide methyl

(S)-4-amino-8-(methyl(6-(trifluoromethyl)-2,3-dihydrobenzofuran-3-yl)carbamoyl)imidazo[1,5-a]quinoxaline-3-carboxylat e(30mg, 0.06mmol) was dissolved in methanol(2mL), followed by Sodium borohydride(Smg, 0.12mmol). The reaction mixture was stirred for 2 hours at room temperature. After completion of the reaction, the solution was extracted with ethyl acetate(10mL×3). The combined organic phase was washed with saturated brine(10mL×3), dried over anhydrous sodium sulfate and concentrated under vacuum to obtain crude product. The crude product was purified by HPLC(column: Kinetex 5 m EVO C18, 30mm × 150 mm; mobile phase A: water(10mmol/L ammonium bicarbonate), mobile phase B: Acetonitrile; flow rate: 60ml/min; Gradient from 20% B to 47% B within 10 minutes; wavelength: 254/220nm; retention time(minimum value): 9.35 ) to obtain (S)-4-amino-3-(hydroxymethyl)-N-methyl-N-(6-(trifluoromethyl)-2,3-dihydrobenzofuran-3-yl)imidazo[1,5-a]quinoxaline-8 -carboxamide(3.17mg, yield: 11.13%), as white solid.

$^1$H NMR (400 MHz, DMSO-$d_6$) δ 9.11 (s, 1H), 8.31 (s, 1H), 7.64 (d, *J* = 7.8 Hz, 1H), 7.59 (s, 2H), 7.47 (d, *J* = 5.5 Hz, 2H), 7.34 (d, *J* = 7.8 Hz, 1H), 7.27 - 7.23 (m, 1H), 6.36 (t, *J* = 5.2 Hz, 1H), 4.85 (d, *J*= 5.1 Hz, 3H), 4.73 (d, *J* = 4.6 Hz, 1H), 3.31 (s, 1H),2.67(S,3H).

LCMS (ESI): 457.41 [M+H] $^+$

Example 132: Synthesis of (S)-4-amino-N8-methyl-N8-(6-(trifluoromethyl)-2,3-dihydrobenzofuran-3-yl)imidazo[1,5-a] quinoxaline-3,8-dicarboxamide

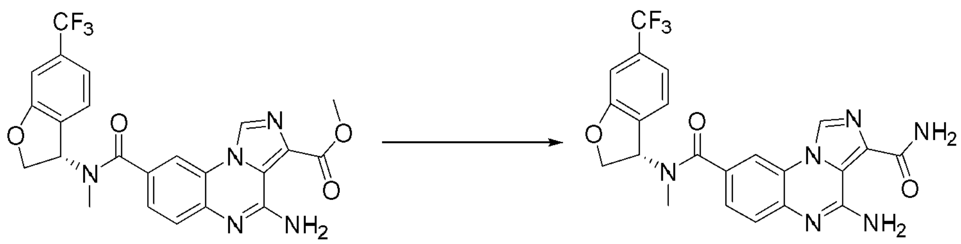

methyl (S)-4-amino-8-(methyl(6-(trifluoromethyl)-2,3-dihydrobenzofuran-3-yl)carbamoyl)imidazo[1,5-a]quinoxaline-3-carboxylat e(30mg, 0.06mmol) was dissolved in MeOH(0.5mL)/THF(0.50mL)/ammonia solution(0.5mL) and proceeded at 60°C for 2 hours. After the reaction was complete, the reaction solution was poured into water(5mL), extracted with ethyl acetate and concentrated to obtain crude product. The crude product was further purified by high pressure preparative HPLC to obtain (S)-4-amino-N$^8$-methyl-N$^8$-(6-(trifluoromethyl)-2,3-dihydrobenzofuran-3-yl)imidazo [1,5-a]quinoxaline-3,8-dicarboxamide( 1.06mg, 4%), as white solid.

$^1$H NMR (400 MHz, DMSO-$d_6$) δ 10.01 (s, 1H), 9.32 (s, 1H), 8.42 (s, 1H), 8.21 (s, 1H), 7.92 (s, 1H), 7.78 (s, 1H), 7.64 (d, *J* = 7.9 Hz, 1H), 7.54 (d, *J* = 8.2 Hz, 1H), 7.48 (d, *J* = 11.1 Hz, 1H), 7.34 (d, *J* = 7.8 Hz, 1H), 7.27 (s, 1H), 6.43 (s, 1H), 4.73 (s, 2H), 2.76 - 2.63 (m, 3H).

LCMS (ESI): 470.80 [M+H] $^+$

Example 131 Synthesis of (S)-4-amino-3-cyano-N-methyl-N-(6-(trifluoromethyl)-2,3-dihydrobenzofuran-3-yl)imidazo [1,5-a]quinoxaline-8-carboxami de

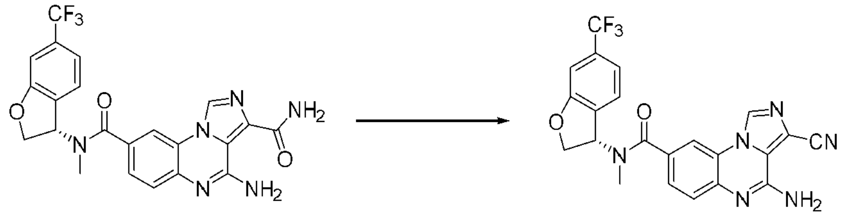

To a stirred solution of (S)-4-amino-N$^8$-methyl-N$^8$-(6-(trifluoromethyl)-2,3-dihydrobenzofuran-3-yl)imidazo[1,5-a]quinoxaline-3,8-dicarboxamide in DCM(2mL), pyridine(10.09mg, 0.13mmol), Trifluoroacetic anhydride(26.79mg,

0.26mmol) was added. The reaction proceeded at room temperature for 12 hours. After completion of the reaction, the solution was poured into water(5mL), extracted with ethyl acetate. The combined organic phase was concentrated to obtain crude product and the crude product was further purified by high pressure preparative HPLC(Column: C18; mobile phase A: water, mobile phase B: Acetonitrile; Gradient from 10% to 50% within 10 minutes.) to obtain (S)-4-amino-3-cyano-N-methyl-N-(6-(trifluoromethyl)-2,3-dihydrobenzofuran-3-yl)imidazo[1,5-a]quinoxaline-8-carboxami de(1.61mg, yield: 5%), as white solid.

$^1H$ NMR (400 MHz, DMSO-$d_6$) δ 9.46 (s, 1H), 8.48 (s, 1H), 7.64 (d, *J*= 8.7 Hz, 3H), 7.33 (s, 1H), 7.26 (s, 1H), 7.12 (s, 2H),6.40(s,1H), 4.72 (d, *J* = 10.5 Hz, 1H),4.71(s,1H), 2.67 (d, *J* = 2.8 Hz, 3H).

LCMS (ESI): 452.80 [M+H]$^+$

Example 81&82:

Example 81 (R)-4-amino-N-(6-bromo-2,3-dihydrobenzofuran-3-yl)-N-cyclopropylimidazo[1,5-a]quinoxaline-8-carboxamide& Example 82 (S)-4-amino-N-(6-bromo-2,3-dihydrobenzofuran-3-yl)-N-cyclopropylimidazo[1,5-a]quinoxaline-8-carboxamide

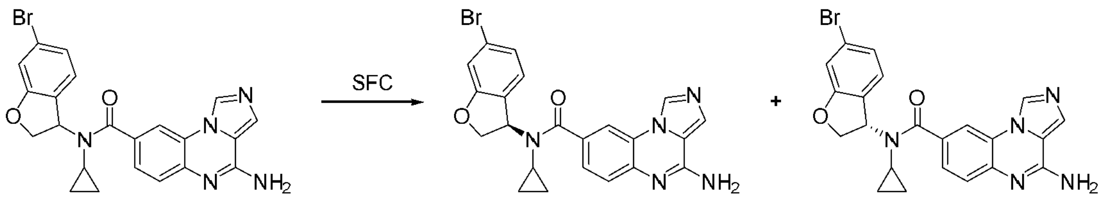


Compound 4-amino-N-(6-bromo-2,3-dihydrobenzofuran-3-yl)-N-cyclopropylimidazo[1,5-a]quinoxaline-8-carboxamide(90mg)was purified by SFC(Column: DAICEL CHIRALCEL OJ(250mm ×30mm, 10um; condition: $CO_2$-i-PrOH(0.1%$NH_3$•$H_2O$), 50%/50%, flow rate : 80ml/min) to obtain (R)-4-amino-N-(6-bromo-2,3-dihydrobenzofuran-3-yl)-N-cyclopropylimidazo[1,5-a]quinoxaline-8-carboxamide(34.61mg) & (S)-4-amino-N-(6-bromo-2,3-dihydrobenzofuran-3-yl)-N-cyclopropylimidazo[1,5-a]quinoxaline-8-carboxamide(28.15mg)

Example 81 (R)-4-amino-N-(6-bromo-2,3-dihydrobenzofuran-3-yl)-N-cyclopropylimidazo[1,5-a]quinoxaline-8-carboxamide

LCMS (ESI) m/z: 465.3 [M+H]$^+$

$^1H$ NMR (400 MHz, DMSO-$d_6$) δ 9.18 (s, 1H), 8.37 (d, *J* = 1.8 Hz, 1H), 7.91 (s, 1H), 7.59 (dd, *J* = 8.4, 1.8 Hz, 1H), 7.55 - 7.39 (m, 4H), 7.18 - 7.09 (m, 2H), 5.86 (dd, *J* = 9.3, 4.1 Hz, 1H), 4.81 (t, *J* = 9.7 Hz, 1H), 4.65 (dd, *J* = 10.2, 4.2Hz, 1H), 2.90 (p, *J* = 3.2 Hz, 1H), 0.45 - 0.26 (m, 2H), 0.24 - 0.03 (m, 2H).

Example 82 (S)-4-amino-N-(6-bromo-2,3-dihydrobenzofuran-3-yl)-N-cyclopropylimidazo[1,5-a]quinoxaline-8-carboxamide

LCMS (ESI) m/z: 465.3 [M+H]$^+$

$^1H$ NMR (400 MHz, DMSO-$d_6$) δ 9.24 (d, *J* = 3.6 Hz, 1H), 8.40 (d, *J* = 3.5 Hz, 1H), 8.03 (s, 1H), 7.85 (s, 2H), 7.62 (dd, *J*= 8.4, 3.3 Hz, 1H), 7.47 (dd, *J* = 8.2, 3.3 Hz, 2H), 7.13 (dd, *J* = 8.7, 3.3 Hz, 2H), 5.86(dt, *J* = 8.6, 4.0 Hz, 1H), 4.80 (td,*J* = 9.7, 3.6 Hz, 1H), 4.65 (dt, *J* = 9.2, 4.1 Hz, 1H), 2.90 (d, *J* = 6.7 Hz, 1H), 0.44 - 0.26 (m, 2H), 0.13 (ddd, *J* = 45.3, 10.6, 4.8 Hz, 2H).

By performing the procedures outlined in Examples 81 and 82, along with the respective chiral SFC separation conditions, the embodiments listed in the table below can be prepared:

| Ex. | Structure | name | SFC condition |
|---|---|---|---|
| 83 | 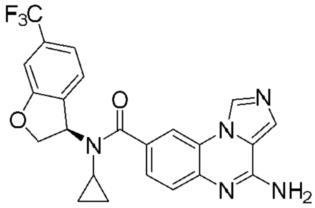 | (R)-4-amino-N-cyclo propyl-N-(6-(trifluoro methyl)-2,3-dihydrob enzofuran-3-yl)imidaz o[1,5-a]quinoxaline-8 -carboxamide | Peak1: SFC(column: DAICEL CHIRALCEL OJ (250×30 mm, 10 μm), condition: A for $CO_2$ and B for MeOH (0.1%$NH_3H_2O$), 40%/40%, flow rate: 80ml/min) |
| 84 | 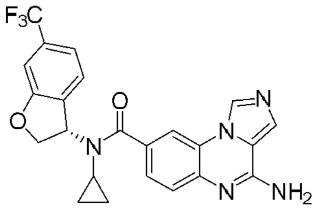 | (S)-4-amino-N-cyclop ropyl-N-(6-(trifluoro methyl)-2,3-dihydrob enzofuran-3-yl)imidaz o[1,5-a]quinoxaline-8 -carboxamide | Peak2: SFC(column: DAICEL CHIRALCEL OJ (250×30 mm, 10 μm), condition: A for $CO_2$ and B for MeOH (0.1%$NH_3H_2O$), 40%/40%, flow rate: 80ml/min) |
| 85 | 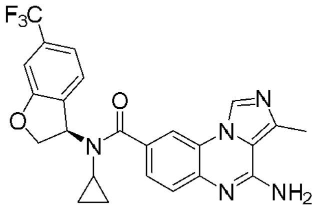 | (R)-4-amino-N-cyclo propyl-3-methyl-N-(6 -(trifluoromethyl)-2,3-dihydrobenzofuran-3-yl)imidazo[1,5-a]quin oxaline-8-carboxamid e | Peak1:SFC(column:Phenomenex-Cellul ose-2, (250mm ×30mm,10um), conditions: A for $CO_2$ and B for EtOH (0.1%$NH_3H_2O$), 45%/45%, flow rate: 80 ml/min) |
| 86 | 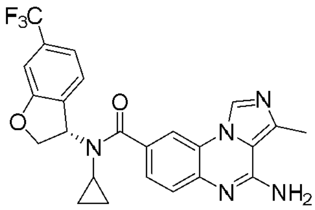 | (S)-4-amino-N-cyclop ropyl-3-methyl-N-(6-( trifluoromethyl)-2,3-d ihydrobenzofuran-3-yl)imidazo[1,5-a]quino xaline-8-carboxamide | Peak2:SFC(column:Phenomenex-Cellul ose-2, (250mm ×30mm,10um), conditions: A for $CO_2$ and B for EtOH (0.1%$NH_3H_2O$), 45%/45%, flow rate: 80 ml/min) |
| 87 | 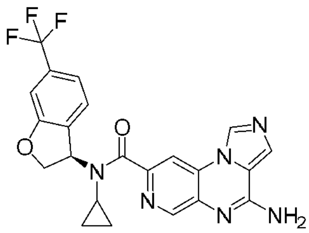 | (R)-4-amino-N-cyclo propyl-N-(6-(trifluoro methyl)-2,3-dihydrob enzofuran-3-yl)imidaz o[1,5-a]pyrido[3,4-e]p yrazine-8-carboxamid e | Peak 1: SFC(column: DAICEL CHIRALCEL OJ, (250mm×30mm, 10um), conditions: A for $CO_2$ and B for EtOH (0.1%$NH_3H_2O$), 35%/35%, flow rate: 80ml/min) |
| 88 | 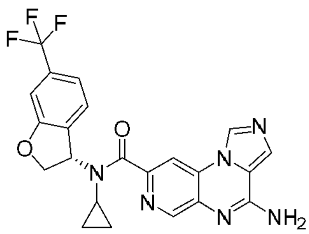 | (S)-4-amino-N-cyclop ropyl-N-(6-(trifluoro methyl)-2,3-dihydrob enzofuran-3-yl)imidaz o[1,5-a]pyrido[3,4-e]p yrazine-8-carboxamid e | Peak 2: SFC(column: DAICEL CHIRALCEL OJ, (250mm×30mm, 10um), conditions: A for $CO_2$ and B for EtOH (0.1%$NH_3H_2O$), 35%/35%, flow rate: 80ml/min) |
| 89 | 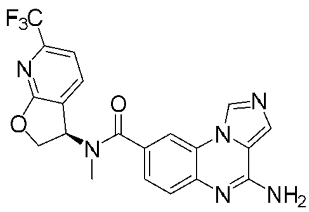 | (R)-4-amino-N-methy 1-N-(6-(trifluoromethy 1)-2,3-dihydrofuro[2,3 -b]pyridin-3-yl) imida zo[1,5-a]quinoxaline-8-carboxamide | Peak 1: SFC( column: DAICEL CHIRALCEL AD, (250mm×30mm, 10um), conditions: A for $CO_2$ and B for EtOH (0.1%$NH_3H_2O$), 45%/45%, flow rate: 150ml/min) |

(continued)

| Ex. | Structure | name | SFC condition |
|---|---|---|---|
| 90 | 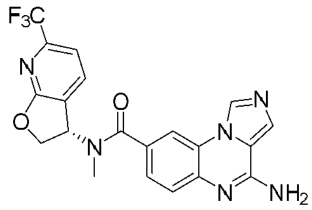 | (S)-4-amino-N-methy 1-N-(6-(trifluoromethy 1)-2,3-dihydrofuro[2,3 -b]pyridin-3-yl) imida zo[1,5-a]quinoxaline-8-carboxamide | Peak 2: SFC( column: DAICEL CHIRALCEL AD, (250mm×30mm, 10um), conditions: A for $CO_2$ and B for EtOH (0.1%$NH_3H_2O$), 45%/45%, flow rate: 150ml/min) |
| 91 | 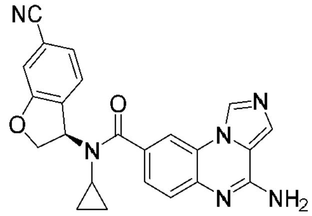 | (R)-4-amino-N-(6-cya no-2,3-dihydrobenzof uran-3-yl)-N-cyclopro pylimidazo[1,5-a]quin oxaline-8-carboxamid e | Peak 1: SFC(column: DAICEL CHIRALPAK AS, (250mm × 30mm, 10um), conditions: A for $CO_2$ and B for EtOH (0.1%$NH_3H_2O$), 45%/45%, flow rate: 100ml/min) |
| 92 | 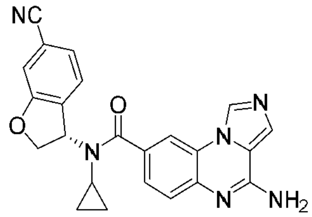 | (S)-4-amino-N-(6-cya no-2,3-dihydrobenzof uran-3-yl)-N-cyclopro pylimidazo[1,5-a]quin oxaline-8-carboxamid e | Peak 2: SFC(column: DAICEL CHIRALPAK AS, (250mm × 30mm, 10um), conditions: A for $CO_2$ and B for EtOH (0.1%$NH_3H_2O$), 45%/45%, flow rate: 100ml/min) |
| 93 | 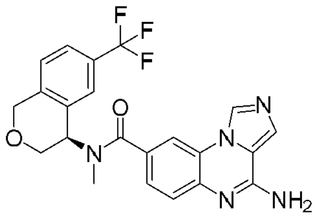 | (R)-4-amino-N-methy 1-N-(6-(trifluoromethy l)isochroman-4-yl)imi dazo[1,5-a]quinoxalin e-8-carboxamide | Peak 1: SFC(column: ChiralCel OJ , (250 × 40mm I.D., 10μm), condition: A for $CO_2$ and B for MeOH (0.1% $NH_3H_2O$), 40%/40%, flow rate: 120ml/min) |
| 94 | 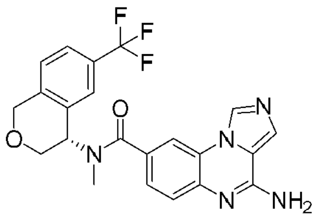 | (S-4-amino-N-methyl -N-(6-(trifluoromethyl )isochroman-4-yl) imi dazo[1,5-a]quinoxalin e-8-carboxamide | Peak 2: SFC(column: ChiralCel OJ , (250 × 40mm I.D., 10μm), condition: A for $CO_2$ and B for MeOH (0.1% $NH_3H_2O$), 40%/40%, flow rate: 120ml/min) |
| 95 | 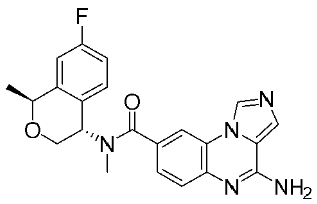 | 4-amino-N-((1S,4S)-7 -fluoro-1-methylisoch roman-4-yl)-N-methyl imidazo[1,5-a]quinox aline-8-carboxamide | Peak1:SFC(column: ChiralCel OJ , (250 × 40mm I.D., 10μm), condition: for $CO_2$ and B for MeOH (0.1% $NH_3H_2O$), 40%/40%, flow rate: 120ml/min) |
| 96 | 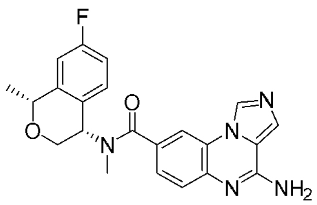 | 4-amino-N-((1R,4S)-7 -fluoro-1-methylisoch roman-4-yl)-N-methylimidazo[1,5-a]quinox aline-8-carboxamide | Peak2:SFC(column: ChiralCel OJ , (250 × 40mm I.D., 10μm), condition: for $CO_2$ and B for MeOH (0.1% $NH_3H_2O$), 40%/40%, flow rate: 120ml/min) |

(continued)

| Ex. | Structure | name | SFC condition |
|---|---|---|---|
| 97 | 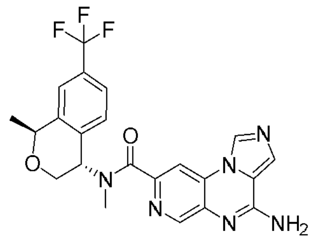 | 4-amino-N-methyl-N-((1S,4S)-1-methyl-7-( trifluoromethyl)isochr oman-4-yl)imidazo[1, 5-a]pyrido[3,4-e]pyra zine-8-carboxamide | Peak1: SFC(column: ChiralCel OJ , (250 × 40mm I.D., 10μm), condition: A for $CO_2$ and B for MeOH (0.1% $NH_3H_2O$), 50%/50%, flow rate: 120ml/min) |
| 98 | 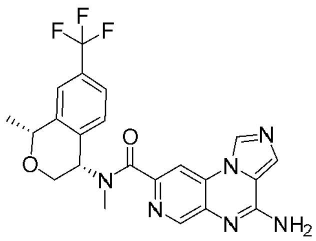 | 4-amino-N-methyl-N-((1R,4S)-1-methyl-7-(trifluoromethyl)isochr oman-4-yl)imidazo[1, 5-a]pyrido[3,4-e]pyra zine-8-carboxamide | Peak2: SFC(column: ChiralCel OJ , (250 × 40mm I.D., 10μm), condition: A for $CO_2$ and B for MeOH (0.1% $NH_3H_2O$), 50%/50%, flow rate: 120ml/min) |
| 99 | 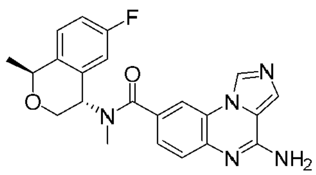 | 4-amino-N-((1S,4S)-6 -fluoro-1-methylisoch roman-4-yl)-N-methyl imidazo[1,5-a]quinox aline-8-carboxamide | Peak1: SFC(column: ChiralCel OJ , (250 × 40mm I.D., 10μm), condition: A for $CO_2$ and B for MeOH (0.1% $NH_3H_2O$), 40%/40%, flow rate: 120ml/min) |
| 100 | 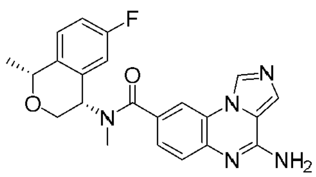 | 4-amino-N-((1R,4S)-6 -fluoro-1-methylisoch roman-4-yl)-N-methyl imidazo[1,5-a]quinox aline-8-carboxamide | Peak2: SFC(column: ChiralCel OJ , (250 × 40mm I.D., 10μm), condition: A for $CO_2$ and B for MeOH (0.1% $NH_3H_2O$), 40%/40%, flow rate: 120ml/min) |
| 101 | 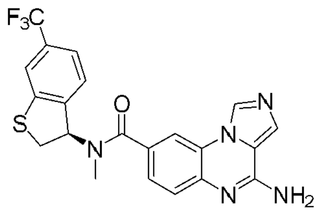 | (R)-4-amino-N-methy 1-N-(6-(trifluoromethy 1)-2,3-dihydrobenzo[b ]thiophen-3-yl) imidaz o[1,5-a]quinoxaline-8 -carboxamide | Peak1: SFC(column:ChiralPak IJ , (250 × 40mm I.D., 10μm), condition: A for $CO_2$ and B for MeOH (0.1% $NH_3H_2O$), 45%/45%, flow rate: 130ml/min) |
| 102 | 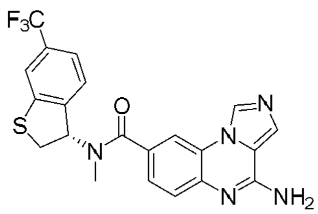 | (S)-4-amino-N-methy 1-N-(6-(trifluoromethy l)-2,3-dihydrobenzo[b ]thiophen-3-yl)imidaz o[1,5-a]quinoxaline-8 -carboxamide | Peak2: SFC(column:ChiralPak IJ , (250 × 40mm I.D., 10μm), condition: A for $CO_2$ and B for MeOH (0.1% $NH_3H_2O$), 45%/45%, flow rate: 130ml/min) |
| 103 | 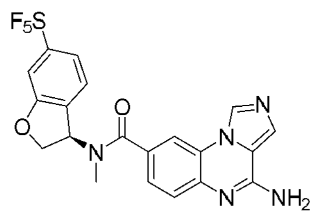 | (R)-4-amino-N-methy 1-N-(6-(pentafluoro-16 -sulfaneyl)-2,3-dihydr obenzofuran-3-yl)imi dazo[1,5-a]quinoxalin e-8-carboxamide | Peak1: SFC(column: Daicel ChiralPak IG , (250 × 40mm I.D., 10μm), condition: A for $CO_2$ and B for MeOH (0.1% $NH_3H_2O$), 50%/50%, flow rate: 140 ml/min) |

(continued)

| Ex. | Structure | name | SFC condition |
|---|---|---|---|
| 104 | 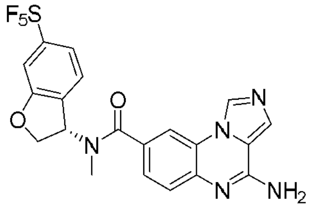 | (S)-4-amino-N-methy 1-N-(6-(pentafluoro-16 -sulfaneyl)-2,3-dihydr obenzofuran-3-yl)imi dazo[1,5-a]quinoxalin e-8-carboxamide | Peak2: SFC(column: Daicel ChiralPak IG , (250 × 40mm I.D., 10μm), condition: A for $CO_2$ and B for MeOH (0.1% $NH_3H_2O$), 50%/50%, flow rate: 140 ml/min) |
| 105 | 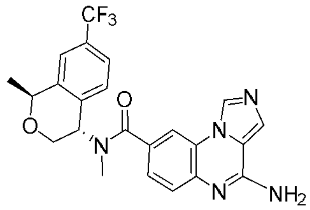 | 4-amino-N-methyl-N-((1S,4S)-1-methyl-7-( trifluoromethyl)isochr oman-4-yl)imidazo[1, 5-a]quinoxaline-8-car boxamide | Peak1: SFC(column: ChiralCel OJ , (250 × 40mm I.D., 10μm), condition: A for $CO_2$ and B for MeOH (0.1% $NH_3H_2O$), 50%/50%, flow rate: 120ml/min) |
| 106 | 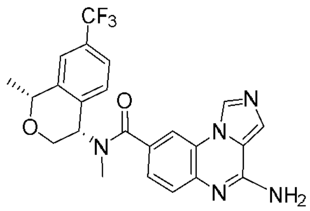 | 4-amino-N-methyl-N-((1R,4S)-1-methyl-7-(trifluoromethyl)isochr oman-4-yl)imidazo[1, 5-a]quinoxaline-8-car boxamide | Peak2: SFC(column: ChiralCel OJ , (250 × 40mm I.D., 10μm), condition: A for $CO_2$ and B for MeOH (0.1% $NH_3H_2O$), 50%/50%, flow rate: 120ml/min) |
| 134 | 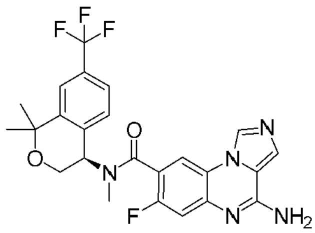 | (R)-4-amino-N-(1,1-d imethyl-7-(trifluorom ethyl)isochroman-4-yl )-7-fluoro-N-methyli midazo[1,5-a]quinoxa line-8-carboxamide | Peak1: SFC(column: ChiralPak IC, (40mm I.D.×250mm, 10μm), condition: A for $CO_2$ and B for MeOH (0.1% $NH_3H_2O$), 50%/50%, flow rate: 140ml/min) |
| 135 | 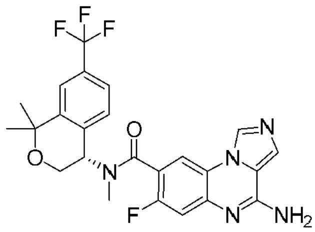 | (S)-4-amino-N-(1,1-di methyl-7-(trifluorome thyl)isochroman-4-yl) -7-fluoro-N-methylim idazo[1,5-a]quinoxali ne-8-carboxamide | Peak2: SFC(column: ChiralPak IC, (40mm I.D.×250mm, 10μm), condition: A for $CO_2$ and B for MeOH (0.1% $NH_3H_2O$), 50%/50%, flow rate: 140ml/min) |

Biology activity testing

I. Tumor cell growth inhibition assay

Test 1: Compound's inhibitory activity on proliferation of HCT-116 MTAP(-/-) deficient cells.

Materials and Cells: HCT-116 MTAP(-/-) deficient cells obtained from Kyinno Bio (China); RPMI-1640 medium, fetal bovine serum, and penicillin-streptomycin purchased from Thermo Fisher Scientific (America); 384-well plates from PerkinElmer (America); Cell-Titer Glo assay kit from Promega (America).

Cell Culture: HCT116 MTAP(-/-) deficient cells were cultured in RPMI 1640 medium containing 10% fetal bovine serum and 1% penicillin-streptomycin at 37°C, 5% $CO_2$. Only cells in logarithmic growth phase were used for experiments.

Cell Proliferation Inhibition Assay: The compound's inhibitory activity on HCT-116 MTAP(-/-) deficient cells was assessed using the Cell-Titer Glo assay kit. Adjusting cell concentration, 40 μL was seeded into a 384-well plate and cultured overnight at 37°C, 5% $CO_2$. Each well received 40 nL of the compound, achieving concentrations from 0 to 10,000 nM (starting concentration 10,000 nM, 3-fold dilution, 10 points), with 0.1% DMSO. The cell plate was incubated for 8 days at 37°C, 5% $CO_2$. Cell-Titer Glo reagent (40 μL) was added to measure cell viability. Results are shown in Table 1.

Test 2: Compound's inhibitory activity on proliferation of HCT-116 MTAP wild-type cells.

Materials and Cells: HCT-116 MTAP wild-type cells obtained from Kyinno Bio (China); RPMI-1640 medium, fetal bovine serum, and penicillin-streptomycin purchased from Thermo Fisher Scientific (America); 384-well plates from PerkinElmer (America); Cell-Titer Glo assay kit from Promega (America).

Cell Culture: HCT116 MTAP wild-type cells were cultured in RPMI 1640 medium containing 10% fetal bovine serum and 1% penicillin-streptomycin at 37°C, 5% $CO_2$. Only cells in logarithmic growth phase were used for experiments.

Cell Proliferation Inhibition Assay: The compound's inhibitory activity on HCT-116 MTAP wild-type cells was assessed using the Cell-Titer Glo assay kit. Adjusting cell concentration, 40 μL was seeded into a 384-well plate and cultured overnight at 37°C, 5% $CO_2$. Each well received 40 nL of the compound, achieving concentrations from 0 to 10,000 nM (starting concentration 10,000 nM, 3-fold dilution, 10 points), with 0.1% DMSO. The cell plate was incubated for 8 days at 37°C, 5% $CO_2$. Cell-Titer Glo reagent (40 μL) was added to measure cell viability. Results are shown in Table 1.

The following Table 1 presents the inhibitory activity of various compound embodiments on the proliferation of HCT116 MTAP(-/-) deficient cells and HCT116 wild-type cells.

Table 1

| Ex. | $IC_{50}$ (nM) | |
|---|---|---|
| | HCT-116 MTAP(-/-) deficient | HCT-116 wild-type |
| 1 | 2306 | >10000 |
| 2 | 61 | 6667.6 |
| 3 | 23.4 | 2251 |
| 4 | 9.9 | 1711 |
| 5 | 4 | 280 |
| 6 | 10.6 | 993 |
| 7 | 141 | >10000 |
| 8 | 106 | 6478.3 |
| 9 | 41.1 | 2144.5 |
| 10 | 31.2 | 3111.3 |
| 11 | 1767.4 | 6410 |
| 12 | 6.1 | 1112.4 |
| 13 | 15.7 | 2511.8 |
| 14 | 14.7 | 1283.1 |
| 15 | 3.5 | 282.3 |
| 16 | 5.6 | 1081 |
| 17 | 4.2 | 243 |
| 18 | 14.5 | 4327.5 |
| 19 | 23.2 | 2233.8 |
| 20 | >3000 | >10000 |
| 21 | 14.8 | 2129.6 |
| 22 | 5.1 | 1688.6 |
| 23 | 119.5 | >10000 |
| 24 | 147.5 | >10000 |
| 25 | 176.9 | >10000 |
| 26 | 67.7 | >10000 |
| 27 | 4.4 | 842.1 |
| 28 | 321.2 | >10000 |

(continued)

| Ex. | $IC_{50}$ (nM) | |
|---|---|---|
| | HCT-116 MTAP(-/-) deficient | HCT-116 wild-type |
| 29 | 7.1 | 934.8 |
| 30 | 2086.4 | >10000 |
| 31 | 296.6 | >10000 |
| 32 | 25.7 | 1411.8 |
| 33 | 15.1 | 2665 |
| 34 | 29.6 | 6572.3 |
| 35 | 23.2 | 2233.8 |
| 36 | 36 | 5126.7 |
| 37 | >3000 | >10000 |
| 38 | 30 | 4063 |
| 39 | 15.9 | 2041.3 |
| 40 | 32.1 | 9203.5 |
| 41 | 11 | 1144 |
| 42 | 659.7 | 5456.9 |
| 43 | 22.4 | 2988 |
| 44 | 310 | >10000 |
| 45 | 10.3 | 947.4 |
| 46 | 52.2 | >10000 |
| 47 | 17.7 | 4096 |
| 48 | >3000 | >3000 |
| 49 | 588.7 | >10000 |
| 50 | 4.2 | 196 |
| 51 | 212.6 | 5864 |
| 52 | 123.4 | >10000 |
| 53 | >3000 | >10000 |
| 54 | 2495 | >10000 |
| 55 | 11.8 | 1221.7 |
| 56 | 15.9 | 1532.1 |
| 57 | 10.5 | 1869.6 |
| 58 | >3000 | >10000 |
| 59 | 710.6 | >10000 |
| 60 | 66.8 | 7074.5 |
| 61 | 7.7 | 882.4 |
| 62 | 19.7 | 3705.7 |
| 63 | 46.8 | 3256.4 |
| 64 | 278.2 | >20000 |
| 65 | 95.3 | 5946.3 |
| 66 | 2257 | >20000 |

(continued)

| Ex. | $IC_{50}$ (nM) | |
|---|---|---|
| | HCT-116 MTAP(-/-) deficient | HCT-116 wild-type |
| 67 | 35.6 | 3639.1 |
| 68 | 381.5 | 8570 |
| 69 | 87.1 | 5019.1 |
| 70 | 54 | 3889 |
| 72 | 117.2 | >20000 |
| 73 | 55.5 | 4622.8 |
| 74 | 148.1 | 5991.4 |
| 75 | 7.3 | 1167.4 |
| 76 | >3000 | >10000 |
| 77 | 101.4 | 4260.8 |
| 78 | 7.1 | 2071.4 |
| 79 | 5.6 | 1302.6 |
| 80 | 39.3 | 9168.6 |
| 81 | 1791.2 | 2359 |
| 82 | 52.9 | 1920.5 |
| 83 | 2635.5 | 4704 |
| 84 | 21.9 | 1470.1 |
| 85 | 790.6 | 780.7 |
| 86 | 61.1 | 1329.3 |
| 87 | 9029.4 | 10755 |
| 88 | 24.1 | 1846.5 |
| 89 | >3000 | >10000 |
| 90 | 36.4 | >10000 |
| 91 | 2179.2 | >20000 |
| 92 | 46.6 | 4753.5 |
| 93 | >3000 | >10000 |
| 94 | 80.8 | 5169.3 |
| 95 | 211.4 | >10000 |
| 96 | 6.6 | 1255.1 |
| 97 | 29.8 | 1547.7 |
| 98 | 1.4 | 114 |
| 99 | 927.7 | 7131 |
| 100 | 68.3 | 3581 |
| 101 | 2187.6 | >10000 |
| 102 | 7.9 | 1993 |
| 103 | 930.6 | 6256.4 |
| 104 | 4.6 | 591.2 |
| 105 | 66.1 | 2193.5 |

(continued)

| Ex. | $IC_{50}$ (nM) | |
|---|---|---|
| | HCT-116 MTAP(-/-) deficient | HCT-116 wild-type |
| 106 | 3.5 | 145.2 |
| 107 | 7.4 | 1727.2 |
| 108 | 120.8 | >10000 |
| 109 | >3000 | 9086 |
| 110 | >3000 | >10000 |
| 111 | 500 | >10000 |
| 112 | 338.1 | >10000 |
| 113 | 88.1 | >10000 |
| 114 | 146.4 | >10000 |
| 115 | 84.3 | >10000 |
| 116 | 109.1 | >10000 |
| 117 | 6.6 | 617.3 |
| 118 | 2933 | >10000 |
| 119 | 13.5 | 1307.8 |
| 120 | 900 | >10000 |
| 121 | 73.7 | >10000 |
| 122 | 50.6 | >10000 |
| 123 | 428.7 | >10000 |
| 124 | 6.5 | 634.4 |
| 125 | 2447.2 | >10000 |
| 126 | 592.1 | >20000 |
| 127 | 100.1 | 9905 |
| 128 | 26 | >10000 |
| 129 | 14.5 | 2676.6 |
| 130 | 5.8 | 1291.6 |
| 131 | >3000 | >10000 |
| 132 | >3000 | >10000 |
| 133 | 71.4 | >10000 |
| 134 | 623 | 5891 |
| 135 | 9.3 | 1959 |

The compounds exhibited potent inhibitory activity against the proliferation of HCT116 MTAP(-/-) cells, with IC50 values ranging from 1.4 nM to 9029.4 nM. In contrast, the inhibitory activity against HCT116 wild-type cells was relatively weak, with IC50 values ranging from 114 nM to >20,000 nM. The selectivity for HCT116 MTAP(-/-) cells over HCT116 wild-type cells ranged from 1.0- to 384.6-fold.

Test 3: Compound's inhibitory activity on proliferation of LU99 MTAP (-/-) cells.

**Materials and Cells:** The LU99 MTAP(-/-) cell line was purchased from Cobioer Biosciences (China). Cell culture media and fetal bovine serum (FBS) were purchased from Thermo Fisher Scientific (USA). 384-well plates were purchased from PerkinElmer (USA). The CellTiter-Glo assay kit was purchased from Promega (USA).

**Cell Culture:** LU99 MTAP(-/-) cells were cultured in a medium supplemented with 10% fetal bovine serum (FBS) at 37°C with 5% CO2. Only cells in the logarithmic growth phase were used for experiments.

**Cell Proliferation Inhibition Assay:** The antiproliferative activity of the compounds against LU99 MTAP(-/-) cells was assessed using the CellTiter-Glo assay kit. Cell suspensions were adjusted to the appropriate density and seeded into 384-well plates at a volume of 40 μL per well, then incubated overnight at 37°C with 5% $CO_2$. Compounds (40 nL) were added to each well to achieve final concentrations ranging from 0 to 1,000 nM (starting concentration 1,000 nM, 2.5-fold serial dilutions, 10 dose points), with a final DMSO concentration of 0.2%. The cell plates were incubated at 37°C with 5% $CO_2$ for 6 days. Subsequently, 40 μL of CellTiter-Glo reagent was added to measure cell viability. The test results are shown in Table 2.

Test 4: Assay for Inhibitory Activity of Compounds against LU99 MTAP-Overexpressing Cell Proliferation

**Materials and Cells:** The LU99 MTAP-overexpressing cell line was purchased from Cobioer Biosciences (China). Cell culture media and fetal bovine serum (FBS) were purchased from Thermo Fisher Scientific (USA). 384-well plates were purchased from PerkinElmer (USA). The CellTiter-Glo assay kit was purchased from Promega (USA).

**Cell Culture:** LU99 MTAP-overexpressing cells were cultured in a medium supplemented with 10% fetal bovine serum (FBS) at 37°C with 5% CO2. Only cells in the logarithmic growth phase were used for experiments.

**Cell Proliferation Inhibition Assay:** The antiproliferative activity of the compounds against LU99 MTAP-overexpressing cells was assessed using the CellTiter-Glo assay kit. Cell suspensions were adjusted to the appropriate density and seeded into 384-well plates at a volume of 40 μL per well, then incubated overnight at 37°C with 5% CO2. Compounds (40 nL) were added to each well to achieve final concentrations ranging from 0 to 10,000 nM (starting concentration 10,000 nM, 2.5-fold serial dilutions, 10 dose points), with a final DMSO concentration of 0.2%. The cell plates were incubated at 37°C with 5% CO2 for 6 days. Subsequently, 40 μL of CellTiter-Glo reagent was added to measure cell viability. The test results are shown in Table 2.

Table 2

| Examples | $IC_{50}$ (nM) | |
|---|---|---|
| | Lu99 MTAP(-/-) | Lu99 MTAP-overexpressing |
| 29 | 10.7 | 1490 |
| 16 | 12.9 | 2484 |
| 107 | 13.2 | 2359 |
| 41 | 9.56 | 1637 |

**Conclusion:** The compounds exhibited potent inhibitory activity against the proliferation of LU99 MTAP(-/-) cells, with $IC_{50}$ values ranging from 9.56 nM to 13.2 nM. In contrast, the inhibitory activity against LU99 MTAP-overexpressing cells was relatively weak, with $IC_{50}$ values ranging from 1,490 nM to 2,484 nM. The selectivity for LU99 MTAP(-/-) cells over LU99 MTAP-overexpressing cells ranged from 139- to 193-fold.

Test 5: Assay for Inhibitory Activity of Compounds against LN18 MTAP(-/-) Cell Proliferation

**Materials and Cells:** The LN18 MTAP(-/-) cell line was provided by Cobioer Biosciences (China). Cell culture media, fetal bovine serum (FBS), and penicillin-streptomycin were purchased from Thermo Fisher Scientific (USA). 384-well plates were purchased from PerkinElmer (USA). The CellTiter-Glo assay kit was purchased from Promega (USA).

**Cell Culture:** LN18 MTAP(-/-) cells were cultured in a medium supplemented with 10% fetal bovine serum (FBS) and 1% penicillin-streptomycin at 37°C with 5% CO2. Only cells in the logarithmic growth phase were used for experiments.

**Cell Proliferation Inhibition Assay:** The antiproliferative activity of the compounds against LN18 MTAP(-/-) cells was assessed using the CellTiter-Glo assay kit. Cell suspensions were adjusted to the appropriate density and seeded into 384-well plates at a volume of 40 μL per well, then incubated overnight at 37°C with 5% CO2. Compounds (40 nL) were added to each well to achieve final concentrations ranging from 0 to 1,000 nM (starting concentration 1,000 nM, 3-fold serial dilutions, 9 dose points), with a final DMSO concentration of 0.25%. The cell plates were incubated at 37°C with 5% CO2 for 7 days. Subsequently, 40 μL of CellTiter-Glo reagent was added to measure cell viability. The test results are shown in Table 3.

Test 6: Assay for Inhibitory Activity of Compounds against LN18 MTAP-Overexpressing Cell Proliferatio

**Materials and Cells:** The LN18 MTAP-overexpressing cell line was purchased from Cobioer Biosciences (China). Cell culture media, fetal bovine serum (FBS), and penicillin-streptomycin were purchased from Thermo Fisher Scientific (USA). 384-well plates were purchased from PerkinElmer (USA). The CellTiter-Glo assay kit was purchased from Promega (USA).

**Cell Culture:** LN18 MTAP-overexpressing cells were cultured in a medium supplemented with 10% fetal bovine serum (FBS) and 1% penicillin-streptomycin at 37°C with 5% CO2. Only cells in the logarithmic growth phase were used for experiments.

**Cell Proliferation Inhibition Assay:** The antiproliferative activity of the compounds against LN18 MTAP-overexpressing cells was assessed using the CellTiter-Glo assay kit. Cell suspensions were adjusted to the appropriate density and seeded into 384-well plates at a volume of 40 μL per well, then incubated overnight at 37°C with 5% CO2. Compounds (40 nL) were added to each well to achieve final concentrations ranging from 0 to 10,000 nM (starting concentration 10,000 nM, 3-fold serial dilutions, 9 dose points), with a final DMSO concentration of 0.25%. The cell plates were incubated at 37°C with 5% CO2 for 7 days. Subsequently, 40 μL of CellTiter-Glo reagent was added to measure cell viability. The test results are shown in Table 3.

Table 3

| Example | $IC_{50}$ (nM) | |
|---|---|---|
| | LN18 MTAP(-/-) | LN18 MTAP-overexpressing |
| 4 | 7.85 | 868 |
| 14 | 5.56 | 898 |
| 107 | 11.1 | 1370 |
| 29 | 5.42 | 812 |
| 16 | 6.57 | 1201 |
| 41 | 14.5 | 1974 |

**Conclusion:** The compounds exhibited potent inhibitory activity against the proliferation of LN18 MTAP(-/-) cells, with $IC_{50}$ values ranging from 5.42 nM to 14.5 nM. In contrast, the inhibitory activity against LN18 MTAP-overexpressing cells was relatively weak, with $IC_{50}$ values ranging from 812 nM to 1,974 nM. The selectivity for LN18 MTAP(-/-) cells over LN18 MTAP-overexpressing cells ranged from 111- to 183-fold.

Test 7: Assay for Inhibitory Activity of Compounds against U87MG MTAP(-/-) Cell Proliferation

**Materials and Cells:** The U87MG MTAP(-/-) cell line was purchased from ATCC (USA). Cell culture media, fetal bovine serum (FBS), and penicillin-streptomycin were purchased from Thermo Fisher Scientific (USA). 384-well plates were purchased from PerkinElmer (USA). The CellTiter-Glo assay kit was purchased from Promega (USA).

**Cell Culture:** U87MG MTAP(-/-) cells were cultured in a medium supplemented with 10% fetal bovine serum (FBS) and 1% penicillin-streptomycin at 37°C with 5% CO2. Only cells in the logarithmic growth phase were used for experiments.

**Cell Proliferation Inhibition Assay:** The antiproliferative activity of the compounds against U87MG MTAP(-/-) cells was assessed using the CellTiter-Glo assay kit. Cell suspensions were adjusted to the appropriate density and seeded into 384-well plates at a volume of 40 μL per well, then incubated overnight at 37°C with 5% CO2. Compounds (40 nL) were added to each well to achieve final concentrations ranging from 0 to 10,000 nM (starting concentration 10,000 nM, 3-fold serial dilutions, 10 dose points), with a final DMSO concentration of 0.5%. The cell plates were incubated at 37°C with 5% CO2 for 6 days. Subsequently, 40 μL of CellTiter-Glo reagent was added to measure cell viability. Paclitaxel was used as a control compound. The test results are shown in Table 4.

Table 4

| Example | $IC_{50}$(nM) U87MG MTAP(-/-) |
|---|---|
| Paclitaxel | 5.55 |
| 4 | 93.1 |

(continued)

| Example | $IC_{50}$(nM) U87MG MTAP(-/-) |
|---|---|
| 14 | 79.5 |
| 107 | 111 |
| 16 | 70.8 |
| 41 | 58.5 |
| 106 | 10 |
| 96 | 91.1 |
| 15 | 19.8 |
| 22 | 81.2 |
| 98 | 14.2 |
| 75 | 72.1 |
| 35 | 34.8 |
| 36 | 343 |
| 38 | 443 |
| 39 | 176 |
| 124 | 64.4 |
| 122 | 777 |
| 55 | 74.9 |
| 33 | 81.7 |
| 40 | 153 |
| 34 | 179 |
| 80 | 376 |
| 56 | 108 |
| 45 | 60.6 |
| 119 | 101 |
| 47 | 475 |
| 117 | 61.3 |
| 79 | 83.3 |
| 78 | 211 |
| 43 | 322 |
| 104 | 28.8 |

**Conclusion:** The compounds exhibited potent inhibitory activity against the proliferation of U87MG MTAP(-/-) cells, with $IC_{50}$ values ranging from 10 nM to 777 nM.

Test 8: Assay for Inhibitory Activity of Compounds against NCI-H838 MTAP(-/-) Cell Proliferation

**Materials and Cells:** The NCI-H838 MTAP(-/-) cell line was purchased from ATCC (USA). Cell culture media, fetal bovine serum (FBS), and penicillin-streptomycin were purchased from Thermo Fisher Scientific (USA). 384-well plates were purchased from PerkinElmer (USA). The CellTiter-Glo assay kit was purchased from Promega (USA).

**Cell Culture:** NCI-H838 MTAP(-/-) cells were cultured in a medium supplemented with 10% fetal bovine serum (FBS) and 1% penicillin-streptomycin at 37°C with 5% CO2. Only cells in the logarithmic growth phase were used for experiments.

**Cell Proliferation Inhibition Assay:** The antiproliferative activity of the compounds against NCI-H838

MTAP(-/-) cells was assessed using the CellTiter-Glo assay kit. Cell suspensions were adjusted to the appropriate density and seeded into 384-well plates at a volume of 40 μL per well, then incubated overnight at 37°C with 5% CO2. Compounds (40 nL) were added to each well to achieve final concentrations ranging from 0 to 3,000 nM (starting concentration 3,000 nM, 3-fold serial dilutions, 9 dose points), with a final DMSO concentration of 0.25%. The cell plates were incubated at 37°C with 5% CO2 for 6 days. Subsequently, 40 μL of CellTiter-Glo reagent was added to measure cell viability. Staurosporine was used as a control compound. The test results are shown in Table 5.

Table 5

| Example | $IC_{50}$(nM) NCI-H838 MTAP(-/-) |
|---|---|
| Staurosporine | 0.8 |
| 107 | 67.8 |
| 16 | 66.8 |
| 41 | 57.6 |
| 29 | 76.8 |

**Conclusion:** The compounds exhibited potent inhibitory activity against the proliferation of NCI-H838 MTAP(-/-) cells, with IC50 values ranging from 57.6 nM to 76.8 nM.

Test 9: Mini Cell Panel: Assay for Inhibitory Activity of Compounds against Proliferation of 20 Tumor Cell Lines

**Materials and Cells:** Fifteen MTAP(-/-) cell lines and five MTAP wild-type cell lines were purchased from ATCC (USA). Cell culture media, fetal bovine serum (FBS), and penicillin-streptomycin were purchased from Thermo Fisher Scientific (USA). 384-well plates were purchased from PerkinElmer (USA). The CellTiter-Glo assay kit was purchased from Promega (USA). Paclitaxel was used as the control compound. The test results are shown in Table 6.

Table 6

| Gene type | Cell line | Example | | | |
|---|---|---|---|---|---|
| | | **107** | **4** | **Paclitaxel** | **29** |
| MTAP (-/-) | SW780 | 210 | 177 | 1.6 | 660 |
| | LN-18 | 9.4 | 7.8 | 20.6 | 5.4 |
| | MDA-MB-231 | 2,061 | 955 | 2.5 | 390 |
| | Ht 1080 | 10.4 | 2.4 | 1.8 | 36.7 |
| | MKN-45 | 43.1 | 17.8 | 1.8 | 39.8 |
| | U87MG | 327 | 148 | 5.2 | 1,037 |
| | ACHN | 2.7 | 4.7 | 4.1 | 2.9 |
| | A549 | 69.1 | 20.2 | 1.9 | 8.5 |
| | LU99 | 39.6 | 21.8 | 1.5 | 37.4 |
| | NCI-H647 | 30.0 | 16.0 | 1.4 | 41.6 |
| | SW1573 | 44.4 | 662 | 2.5 | 47.3 |
| | DOHH-2 | 33.2 | 19.3 | 3.4 | 11.6 |
| | SK-MEL-5 | >1,000 | >1,000 | 7.8 | |
| | BxPc-3 | 32.7 | 15.2 | 0.9 | 57.2 |
| | PSN-1 | 30.9 | 12.6 | 2.9 | 24.1 |

(continued)

| Gene type | Cell line | Example | | | |
|---|---|---|---|---|---|
| | | **107** | **4** | **Paclitaxel** | **29** |
| MTAP Wild type | Calu-1 | >10,000 | 3,370 | 9.8 | 2,303 |
| | SU-DHL-10 | 1,696 | 1,301 | 2.8 | 1,590 |
| | SU-DHL-4 | 3,100 | 1,259 | 1.9 | >10,000 |
| | Aspc-1 | >10,000 | 6,600 | 3.4 | >10,000 |
| | CFPAC-1 | 781 | 407 | 1.1 | 1,064 |

**Conclusion:** Compound 107 exhibited potent inhibitory activity against the proliferation of the 15 MTAP(-/-) cell lines, with $IC_{50}$ values ranging from 2.7 nM to >1,000 nM and a median $IC_{50}$ of 56.6 nM. In contrast, the inhibitory activity of Compound 107 against MTAP wild-type cells was relatively weak, with $IC_{50}$ values ranging from 781 nM to >10,000 nM and a median $IC_{50}$ of 3,331.9 nM. The selectivity, calculated based on the median values, was 58.8-fold.

Compound 4 exhibited potent inhibitory activity against the proliferation of the 15 MTAP(-/-) cell lines, with $IC_{50}$ values ranging from 2.4 nM to >1,000 nM and a median $IC_{50}$ of 38.8 nM. In contrast, the inhibitory activity of Compound 4 against MTAP wild-type cells was relatively weak, with $IC_{50}$ values ranging from 407 nM to 6,600 nM and a median $IC_{50}$ of 1,714.8 nM. The selectivity, calculated based on the median values, was 44.2-fold.

**Conclusion:** Compound 29 exhibited potent inhibitory activity against the proliferation of the 15 MTAP(-/-) cell lines, with $IC_{50}$ values ranging from 2.9 nM to 1,037 nM and a median $IC_{50}$ of 42.4 nM. In contrast, the inhibitory activity of Compound 29 against MTAP wild-type cells was relatively weak, with $IC_{50}$ values ranging from 1,064 nM to >10,000 nM and a median $IC_{50}$ of 3,297.1 nM. The selectivity, calculated based on the median values, was 77.7-fold.

II. Mouse Pharmacokinetic Experiment

1. Tested Compounds

The compounds used in this experiment are specific embodiments of the present invention, with reference to compound AMG473 as an example from Amgen's patent WO 2022/169948A1, identified as example 473.

2. Animals

ICR male mice, N=3/group; sourced from Zhejiang Vital River Laboratory Animal Technology Co., Ltd.

3. Drug Preparation and Administration

Single oral (PO) administration to ICR mice: Compounds were dissolved in dimethyl sulfoxide, added to polyethylene glycol 400 and sterile water, and then adjusted to a clear solution with a small amount of 1 mol/L hydrochloric acid. After overnight fasting, the mice were orally gavaged with a dose of 10 mg/kg.
Single intravenous (IV) administration to ICR mice: Compounds were dissolved in dimethyl sulfoxide, added to polyethylene glycol 400 and sterile water, and then adjusted to a clear solution with a small amount of 1 mol/L hydrochloric acid. After overnight fasting, the mice received tail vein injections with a dose of 3 mg/kg.

4. Sample Collection

Approximately 30 μL/time point blood samples were collected via the tail vein. Potassium ethylenediaminetetraacetate was used as an anticoagulant, and samples were centrifuged at 4000 g/min for 5 minutes at 4°C within 1 hour. Blood sampling time points were 0.0833 (IV), 0.25, 0.5, 1, 2, 4, 6, 8, 24 hours. Samples were stored in a -20°C freezer.

Plasma samples (30μL, 10μL sample + 20μL blank plasma) were mixed with 200μL ice-cold acetonitrile containing internal standards. After vortexing for 30 seconds, samples were centrifuged at 4000g/min for 20 minutes. One hundred microliters of the supernatant were transferred to a 96-well plate, followed by the addition of 200μL ultrapure water. After vortexing for 30 seconds, 5μL or 10μL were injected into LC-MS/MS for analysis.

Table 7: Pharmacokinetic Data

| Ex. | Administration Route (Dosage) | $AUC_{last}$ (hr*ng/ml) | $T_{1/2}$(h) | Bioavailability F (%) |
|---|---|---|---|---|
| **4** | IV(3mg/kg) | 8094 | 4.06 | NA |
| | PO(10mg/kg) | 21020 | 2.67 | 77 |
| **29** | IV(3mg/kg) | 12925 | 4.38 | NA |
| | PO(10mg/kg) | 43906 | 4.16 | 102 |
| **16** | IV(3mg/kg) | 9710 | 3.65 | NA |
| | PO(10mg/kg) | 42546 | 3.31 | 131 |
| **107** | IV(3mg/kg) | 11237 | 4.53 | NA |
| | PO(10mg/kg) | 44065 | 4.56 | 119 |
| **104** | IV(3mg/kg) | 10548 | 6.1 | NA |
| | PO(10mg/kg) | 26536 | 5.02 | 74 |
| **130** | IV(3mg/kg) | 10782 | 4.5 | NA |
| | PO(10mg/kg) | 28276 | 3.34 | 77.5 |
| **41** | IV(3mg/kg) | 5421 | 4.3 | NA |
| | PO(10mg/kg) | 46322 | 4.12 | 257 |
| **AMG473** | PO(10mg/kg) | 16174 | 4.2 | NA |

"NA": absent.

By comparing pharmacokinetic parameters, it can be observed that, compared to AMG473, at equivalent doses and under the same administration route, the embodiments of the present invention consistently exhibit higher plasma exposure. This could reduce the effective dose of the compound, expanding the safety margin.

III. hERG Ion Channel Inhibition Experiment

1. Tested Compounds

The compounds used in this experiment are specific embodiments of the present invention, with reference to compound AMG473 as an example from Amgen's patent WO 2022/169948A1, identified as example 473.

2. Cell Line and Cell Culture

Stably expressing hERG ion channel HEK293 cell line (Catalog: K1236) was purchased from Invitrogen. This cell line was cultured in a medium containing 85% DMEM, 10% dialyzed fetal bovine serum, 0.1 mM non-essential amino acid solution, 100 U/mL penicillin-streptomycin solution, 25 mM HEPES, 5 μg/mL blasticidin, and 400 μg/mL geneticin. When the cell density reached 40%~80% of the culture dish area, digestion was performed using trypsin, and cells were passaged three times per week. Before the experiment, cells were cultured at a density of $5 \times 10^5$ in 3.5 cm culture dishes, induced with 1 μg/mL Doxycycline for 48 hours. Subsequently, cells were digested and seeded onto slides for subsequent manual patch clamp experiments.)

3. Experimental Procedure

1) Place glass slides containing HEK293 cells in the perfusion chamber of the micro-manipulation station.
2) Under an Olympus IX71 or IX73 inverted microscope, position suitable cells in the central field of view using a ×10 objective. Locate the tip of the glass electrode in the central field. Use the micromanipulator to lower the electrode while adjusting the coarse focus spiral to slowly approach the cell.
3) When approaching the cell rapidly, switch to a ×40 objective for observation. Fine-tune the electrode approach using the micromanipulator to gradually reach the cell's surface.
4) Apply negative pressure to establish 1GΩ seal between the electrode tip and the cell membrane.

5) Compensate for the transient capacitance current $C_{fast}$ under voltage clamp mode. Then, repeat the brief negative pressure application for membrane rupture, ultimately reaching the whole-cell recording mode.
6) Under voltage clamp at -60 mV, compensate for slow capacitance current $C_{slow}$, cell membrane capacitance (Cm), and input membrane resistance (Ra).
7) Once the cell is stable, change the clamping voltage to -90 mV, set the sampling frequency to 20 kHz, and the filtering frequency to 10 kHz. Leak current detection conditions involve changing the clamping voltage to -80 mV for a duration of 500 ms.
8) The hERG current testing method is as follows: Apply a 4.8-second depolarization command voltage to depolarize the membrane potential from -80 mV to +30 mV, then instantly apply a 5.2-second repolarization voltage to reduce the membrane potential to -50 mV to remove channel inactivation, allowing observation of the hERG tail current. The peak of the tail current represents the size of the hERG current.
9) The hERG current induced by the test compound is continuously recorded for 120 seconds before administration to assess the stability of the tested cells in producing hERG currents. Only stable cells within an acceptable range are eligible for subsequent compound testing.
10) Testing the inhibitory effect of the test compound on hERG current: First, determine the baseline of hERG current obtained in extracellular fluid containing 0.1% DMSO. After maintaining stable hERG current for at least 5 minutes, sequentially perfuse the solution containing the test compound around the cells from low to high concentrations. After each perfusion, wait for about 5 minutes to allow the compound to act on the cells and simultaneously record hERG currents. Record the last 5 hERG current values after the recorded current stabilizes, and take their average as the final current value at a specific concentration. After testing the compound, add 450 nM dofetilide to the same cell to completely inhibit its current, serving as a positive control for that cell. Meanwhile, positive compound dofetilide is synchronously detected before and after the end of the test drug experiment using the same patch clamp system to ensure the reliability and sensitivity of the entire detection system. The above test steps will be repeated on two separate test cells (n=2). 4. Data Analysis

Data meeting the above criteria will be analyzed using the following steps. Note: Data is output by PatchMaster software.

1) After perfusing blank solvent or compound gradient solution, obtain 5 consecutive stable current values, calculate the average, and use them as "Tail Current Size Blank" and "Tail Current Size Compound," respectively.
The percentage of current inhibition is calculated using the following formula:

Tail current inhibition percentage=(1-(cpd’s tail current-positive control’s tail current)/(blank’s tail current-positive control’s tail current))×100%

2)Dose-response curves were fitted, and $IC_{50}$ values were calculated using GraphPad Prism 8.0 software.
3) The standard deviation range for the three sets of data is less than 15 (SD <15);
4) Widely accepted criteria for assessing the inhibitory potency of compounds in hERG channel detection are as follows:

1) Low inhibitory potency: $IC_{50}$ > 10 μM
2) Moderate inhibitory potency: 1 μM < $IC_{50}$ < 10 μM
3) High inhibitory potency: $IC_{50}$ < 1 μM

Data Quality Control Standards

Only data meeting the following criteria will be subject to further analysis: 1) Initial gigohm seal resistance greater than 1 GΩ; 2) Series resistance less than 15 MΩ and series resistance voltage error less than 5 mV; 3) Leakage current at the detection voltage is less than 50% of the current value under this condition; 4) Tail current greater than the plateau current size before the pre-pulse, with an initial tail current value greater than 250 pA; 5) Rupture resistance Ra less than 15 MΩ; 6) Decay rate of tail current per minute less than 2.5%.
5). The hERG $IC_{50}$ (μM) data are shown in Table 8:

Table 8: hERG $IC_{50}$ data

| Example | hERG $IC_{50}$(μM) |
|---|---|
| **4** | 5.29 |

(continued)

| Example | hERG $IC_{50}$(μM) |
|---|---|
| **29** | 11.4 |
| **16** | 2.3 |
| **107** | 4.5 |
| **104** | 1.51 |
| **130** | 1.36 |
| **41** | 8.16 |
| **AMG473** | <0.37 |

By comparing hERG $IC_{50}$ data, in comparison to AMG473, the embodiments of the present invention demonstrate a lower risk of cardiac toxicity, indicating a larger safety margin.

IV. Mouse Brain Penetration Pharmacokinetics Study

1. Tested Compounds

The test compounds used in this study were the compounds described in the specific examples of the present disclosure.

2. Animals

ICR male mice, N=3/group; sourced from Zhejiang Vital River Laboratory Animal Technology Co., Ltd.

3. Drug Preparation and Administration

Single oral (PO) administration to ICR mice: Compounds were dissolved in dimethyl sulfoxide, added to polyethylene glycol 400 and sterile water, and then adjusted to a clear solution with a small amount of 1 mol/L hydrochloric acid. After overnight fasting, the mice were orally gavaged with a dose of 30 mg/kg or 100 mg/kg.

4. Sample Collection

Blood Collection and Processing: Mice were anesthetized with 5% isoflurane for 3-4 minutes until loss of consciousness. Whole blood (500 μL per time point) was collected via cardiac puncture using dipotassium EDTA (K2EDTA) as the anticoagulant. Plasma was separated by centrifugation (4,000 4000 g/min for 5 minutes at 4°C). Blood samples were collected at 4, 8, 12, 24, and 36 hours. The samples were stored at -80°C.

Brain Tissue Collection: Following complete exsanguination, the thoracic cavity was opened with scissors. The right atrium was incised, and a needle was inserted into the left ventricle. Transcardial perfusion was performed with physiological saline (approximately 10 mL). Upon completion of perfusion, the skull was opened, and brain tissues were harvested and stored at -80°C.

Sample Preparation: A 30 μL plasma aliquot (consisting of 10 μL study sample and 20 μL blank plasma) was mixed with 200 μL of ice-cold acetonitrile containing an internal standard (IS). The mixture was vortexed for 30 seconds and then centrifuged at 4,000 g/min for 20 minutes. A 100 μL aliquot of the supernatant was transferred to a 96-well plate and mixed with 200 μL of ultrapure water. After vortexing for 30 seconds, a volume of 5 μL or 10 μL was injected into the LC-MS/MS system for analysis.

Brain Homogenate Preparation: Brain tissue samples were weighed and homogenized in phosphate-buffered saline (PBS) at a 1:4 ratio (tissue weight [g] to PBS volume [mL]). A 30 μL aliquot of brain homogenate (consisting of 10 μL study sample and 20 μL blank brain homogenate) was mixed with 200 μL of ice-cold acetonitrile containing an internal standard. The mixture was vortexed for 30 seconds and centrifuged at 4,000 g/min for 20 minutes. A 100 μL aliquot of the supernatant was transferred to a 96-well plate and mixed with 200 μL of ultrapure water. After vortexing for 30 seconds, a volume of 2 μL or 10 μL was injected into the LC-MS/MS system for analysis.

Table 9: PK data

| Example | Route (Dose) | Sampling Time (h) | Brain concentration (ng/g) | Plasma concentration (ng/ml) | B/P ratio |
|---|---|---|---|---|---|
| **107** | PO(30mg/kg) | 4 | 885 | 7520 | 0.118 |
| | | | 1290 | 10600 | 0.122 |
| | | | 1780 | 11700 | 0.152 |
| | | 8 | 1825 | 6370 | 0.286 |
| | | | 1700 | 7500 | 0.227 |
| | | | 2080 | 6810 | 0.305 |
| | | 12 | 1005 | 2750 | 0.365 |
| | | | 1040 | 2390 | 0.435 |
| | | | 855 | 2670 | 0.320 |
| | | 24 | 47.6 | 201 | 0.237 |
| | | | 65.0 | 349 | 0.186 |
| | | | 142 | 631 | 0.225 |
| | | 36 | 30.0 | 49.6 | 0.605 |
| | | | 25.0 | 38.3 | 0.653 |
| | | | 19.1 | 27.1 | 0.705 |
| | PO(100mg/kg) | 2 | 6650 | 38800 | 0.171 |
| | | | 4740 | 41500 | 0.114 |
| | | | 10800 | 55400 | 0.195 |
| | | 4 | 5550 | 33100 | 0.168 |
| | | | 4560 | 32000 | 0.143 |
| | | | 6100 | 36400 | 0.168 |
| | | 8 | 2460 | 12300 | 0.200 |
| | | | 1455 | 9620 | 0.151 |
| | | | 2480 | 17500 | 0.142 |
| | | 24 | 427 | 1330 | 0.321 |
| | | | 356 | 1280 | 0.278 |
| | | | 434 | 1950 | 0.222 |

Conclusion: As indicated by the results in the table above, Compound 107 exhibits favorable brain penetration in rodents.

V. In Vivo Efficacy of Compounds of the Present Disclosure in U87 Orthotopic Xenograft Models

Cell Culture: Human glioma U87-luc cells (Caliper-124577) were cultured as monolayers *in vitro.* The cells were maintained in EMEM supplemented with 10% fetal bovine serum (FBS), 100 U/mL penicillin, and 100 μg/mL streptomycin in an incubator at 37°C with 5% CO2. Routine subculturing was performed twice weekly using trypsin-EDTA. When the cells reached 80-90% confluence and the required cell number was achieved, the cells were harvested, counted, and prepared for inoculation.

Animals: Female BALB/c nude mice, weighing 18-20 g. A total of 50 mice were obtained (target study size of 24 mice, with an ~88% surplus). The animals were provided by Beijing Vital River Laboratory Animal Technology Co., Ltd.

Tumor Inoculation: Prior to cell inoculation, each nude mouse was anesthetized via intraperitoneal injection of 1.25% avertin at a dose of 20 μL/g. The anesthetized mouse was fixed onto a stereotaxic instrument. The scalp was disinfected with 70% ethanol and covered with a sterile drape. A midline longitudinal incision (approximately 1 cm) was

made to expose the skull. A small hole was drilled using a cranial drill at the coordinates 2 mm right and 0.5 mm anterior to the bregma. Using a microsyringe, 3 μL of U87-luc cell suspension ($3.0\times10^5$ cells in PBS containing 20% Matrigel) was injected into the hole at a depth of 3.5 mm from the skull surface. The burr hole was sealed with tissue adhesive (or bone wax), the incision was sutured, and the skin was disinfected with povidone-iodine solution. The animals were kept warm on a heating pad during the recovery period and monitored until fully awake before being returned to their cages.

Analgesia: For analgesia, Meloxicam (3 mg/kg) was injected subcutaneously 30 minutes prior to surgery and 24 hours post-surgery.

Bioluminescence Imaging: Mice were weighed and administered D-luciferin (150 mg/kg) via intraperitoneal injection. Ten minutes post-injection, the mice were anesthetized using a mixture of oxygen and isoflurane. Once fully anesthetized, the animals were transferred to the IVIS Lumina III in vivo imaging system for detection. Bioluminescence measurements were acquired for all mice at 13 minutes post-injection. Whole-body bioluminescence, including metastatic tumors, was measured and images were captured. Imaging was performed on Day 7 post-inoculation and weekly thereafter following grouping and dosing (5 time points in total). The data are presented as bioluminescence signal values.

Animal Grouping: Seven days after cell inoculation, 24 mice were selected from the total of 50 mice based on their bioluminescence values. Using an Excel-based stratified randomization procedure, the mice were divided into 3 groups (8 mice per group): the Vehicle group, the 15 mg/kg BID group, and the 30 mg/kg QD group.

Animal Husbandry: Upon arrival, animals were acclimated to the laboratory environment for 3-7 days prior to the start of the experiment. The animals were housed in a Specific Pathogen Free (SPF) grade facility using Individually Ventilated Cages (IVC) (4 mice per cage). All cages, bedding, and water were sterilized prior to use. All experimental personnel wore protective clothing and latex gloves when working in the animal facility. Cage cards were labeled with the number of animals per cage, sex, strain, date of receipt, dosing regimen, experiment number, group assignment, and experiment start date. Cages, feed, and water were replaced twice weekly.

The housing environment and lighting conditions were as follows:

- Temperature: 20-26°C
- Humidity: 40-70%
- Light Cycle: 12-hour light / 12-hour dark cycle

Diet Composition: The diet conformed to certified standards for laboratory animal feed. Maximum contaminant levels were within controlled limits, with routine testing performed by the manufacturer. Animals were provided with autoclaved drinking water. Both the diet and water were free of substances that could influence tumor growth.

Observations and Ethical Compliance: The preparation of this study protocol and any subsequent amendments shall be reviewed and approved by the Institutional Animal Care and Use Committee (IACUC) of WuXi AppTec (Shanghai) prior to implementation. Animal care and use will be conducted in compliance with the guidelines of the Association for Assessment and Accreditation of Laboratory Animal Care (AAALAC).

Animal health status and mortality will be monitored daily. Routine examinations will include observations of tumor growth and the effects of drug treatment on daily behavior and clinical signs, such as activity levels, food and water consumption, body weight changes (measured twice weekly), physical appearance, and any other abnormalities. Mortality and adverse effects will be recorded for each group.

Experimental Endpoint: The experimental endpoint is animal survival.

Here is the translation, utilizing standard terminology for survival studies and humane endpoints.

Monitoring and Data Analysis: Animals were monitored daily, and the time of death was recorded. Body weight was measured twice weekly, or daily if animals showed obvious signs of illness. If an animal's health deteriorated or it became moribund (characterized by significant weight loss >20%, inability to eat or drink normally, difficulty moving, or paralysis), it was considered a mortality event and immediately euthanized via CO2 inhalation.

The Median Survival Time (MST, days) was calculated for each group. The Increase in Life-Span (ILS) was calculated by comparing the MST of the treatment group to that of the model control group, expressed as a percentage increase over the control group. The calculation is as follows:

$$\text{ILS } (\%) = (\text{MSTt} - \text{MSTv})/\text{MSTv} \times 100$$

Where:

- MSTt: Median Survival Time of the treatment group
- $MST_V$: Median Survival Time of the Vehicle group

Tumor Growth Monitoring: Bioluminescence signal intensity was used to monitor intracranial tumor growth.

Bioluminescence imaging was performed once or twice weekly. The measured signal values were used to calculate the relative value T/C (%), where T and C represent the mean bioluminescence of the treatment group and the control group, respectively, on a given day. The survival time (in days) was recorded for all mice.

Tumor Growth Inhibition (TGI) Calculation: The Tumor Growth Inhibition (TGI) rate was calculated using the following formula: TGI(%)=[1-(Ti-T0)/ (Vi-V0)] ×100%
Where:

Ti: Mean bioluminescence signal of the treatment group at the end of treatment.
T0: Mean bioluminescence signal of the treatment group at the start of treatment.
Vi: Mean bioluminescence signal of the control (vehicle) group at the end of treatment.
V0: Mean bioluminescence signal of the control (vehicle) group at the start of treatment.

Termination of Experiment (Humane Endpoints): Animals shall be euthanized if their health condition continues to deteriorate or if any of the following conditions are observed:

Body weight loss > 20%; Paralysis; Dehydration; Piloerection (ruffled fur); Hunched posture (kyphosis); Pallor (whitening) of the ears, nose, eyes, or feet; Labored breathing (dyspnea); Convulsions; Persistent diarrhea; Lethargy (sluggishness or reduced activity); Vocalization

Statistical Analysis: Animal survival time was analyzed using the Kaplan-Meier method. Survival time was defined as the duration from tumor inoculation to the time of death. The analysis provided the Median Survival Time (MST) and the corresponding 95% Confidence Interval (CI) for each group. Kaplan-Meier survival curves were generated for each group and compared using the Log-rank test.

Experimental Results: After grouping, bioluminescence imaging was performed once weekly. The data are presented in Table 10.

Table 10. Summary of Bioluminescence Data

| Groups | Bioluminescence Signal (photons/sec) | | | | | |
|---|---|---|---|---|---|---|
| | days | 0 | 7 | 14 | 21 | 28 |
| Vehicle Control Group (PO, QD) | Mean | 5.838E+06 | 3.862E+06 | 1.201E+07 | 7.158E+07 | 5.021E+08 |
| | SEM | 7.011E+05 | 1.079E+06 | 3.374E+06 | 1.866E+07 | 1.347E+08 |
| Example 107 (30 mg/kg, PO, QD) | Mean | 5.837E+06 | 1.419E+06 | 1.930E+06 | 1.968E+06 | 2.412E+06 |
| | SEM | 7.161E+05 | 3.346E+05 | 5.916E+05 | 6.347E+05 | 7.529E+05 |

Statistical Analysis: Comparisons of bioluminescence data among three or more groups were performed using one-way ANOVA. If the F-value was statistically significant, post-hoc multiple comparisons were conducted following the ANOVA. All data analyses were performed using SPSS 17.0 software. A value of $p < 0.05$ was considered statistically significant. The experimental results are shown in Figure 1.

**Results:** As shown in Table 10 and Figure 1, in the U87MG orthotopic mouse xenograft model, the compound of Example 107 of the present disclosure demonstrated significant inhibition of tumor growth following administration at 30 mg/kg once daily (QD) for 28 days, with a Tumor Growth Inhibition (TGI) rate exceeding 100%.

While preferred embodiments have been described above, it will be apparent to those skilled in the art that modifications may be made without departing from the present disclosure. Such modifications are considered to be variations included within the scope of the present disclosure.

## Claims

1. A method for treating a tumor or cancer, comprising administering to a subject in need thereof a compound represented by Formula (I), or a pharmaceutically acceptable salt, ester, prodrug, stereoisomer, or isotopic derivative thereof,

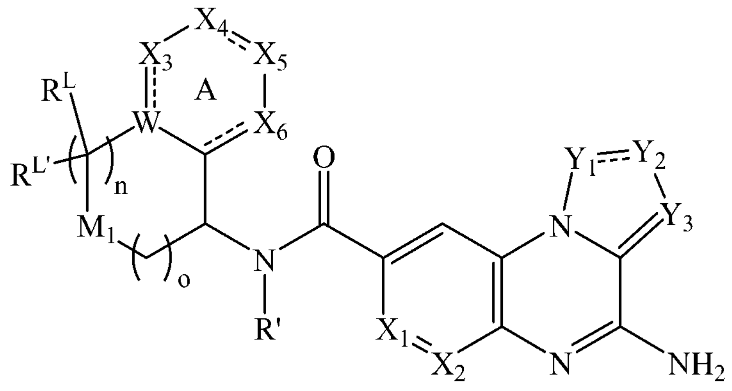

Formula (I)

wherein, W is C;

wherein, $X_3$ is N or $CR^{X3}$; $X_4$ is N or $CR^{X4}$; $X_5$ is N or $CR^{X5}$; $X_6$ is N or $CR^{X6}$;

wherein, when $X_3$ is $CR^{X3}$, $R^{X3}$ is hydrogen, deuterium, $C_1$-$C_6$ alkyl, $C_1$-C6 haloalkyl, halogen, -$OR^a$, -$SR^a$, -P(O)$R^aR^b$, -CN, -$S(O)_2R^a$, -S(O)$R^a$, -$SF_5$, -$NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxyl($C_1$-$C_6$ alkyl), $NR^aR^b$, -CN, $C_1$-$C_6$ haloalkyl, $C_1$-$C_6$ haloalkoxy, -$SO_3R^a$, -$SR^a$, -$SF_5$, -C(O)$R^a$, -C(O)$OR^a$, -OC(O)$R^a$, -OC(O)$NR^aR^b$, -$NR^aCOR^b$ or -$CONR^aR^b$;

wherein, when $X_4$ is $CR^{X4}$, $R^{X4}$ is hydrogen, deuterium, $C_1$-$C_6$ alkyl, $C_1$-$C_6$ haloalkyl, halogen, -$OR^a$, -$SR^a$, -P(O)$R^aR^b$, -CN, -$S(O)_2R^a$, -S(O)$R^a$, -$SF_5$, -$NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxyl($C_1$-$C_6$ alkyl), $NR^aR^b$, -CN, $C_1$-$C_6$ haloalkyl, $C_1$-$C_6$ haloalkoxy, -$SO_3R^a$, -$SR^a$, -$SF_5$, -C(O)$R^a$, -C(O)$OR^a$, -OC(O)$R^a$, -OC(O)$NR^aR^b$, -$NR^aCOR^b$ or -$CONR^aR^b$;

wherein, when $X_5$ is $CR^{X5}$, $R^{X5}$ is hydrogen, deuterium, $C_1$-$C_6$ alkyl, $C_1$-C6 haloalkyl, halogen, -$OR^a$, -$SR^a$, -P(O)$R^aR^b$, -CN, -$S(O)_2R^a$, -S(O)$R^a$, -$SF_5$, -$NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxyl($C_1$-$C_6$ alkyl), $NR^aR^b$, -CN, $C_1$-$C_6$ haloalkyl, $C_1$-$C_6$ haloalkoxy, -$SO_3R^a$, -$SR^a$, -$SF_5$, -C(O)$R^a$, -C(O)$OR^a$, -OC(O)$R^a$, -OC(O)$NR^aR^b$, -$NR^aCOR^b$ or -$CONR^aR^b$;

wherein, when $X_6$ is $CR^{X6}$, $R^{X6}$ is hydrogen, deuterium, $C_1$-$C_6$ alkyl, $C_1$-$C_6$ haloalkyl, halogen, -$OR^a$, -$SR^a$, -P(O)$R^aR^b$, -CN, -$S(O)_2R^a$, -S(O)$R^a$, -$SF_5$, -$NR^aR^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxyl($C_1$-$C_6$ alkyl), $NR^aR^b$, -CN, $C_1$-$C_6$ haloalkyl, $C_1$-$C_6$ haloalkoxy, -$SO_3R^a$, -$SR^a$, -$SF_5$, -C(O)$R^a$, -C(O)$OR^a$, -OC(O)$R^a$, -OC(O)$NR^aR^b$, -$NR^aCOR^b$ or -$CONR^aR^b$;

wherein, the ring A can optionally fuse at the chemical bond between $X_3$ and $X_4$ with a 5-6 membered saturated or unsaturated ring, which can contain 0-3 heteroatoms selected from O, N, S;

wherein, the ring A can optionally fuse at the chemical bond between $X_4$ and $X_5$ with a 5-6 membered saturated or unsaturated ring, which can contain 0-3 heteroatoms selected from O, N, S;

wherein, the ring A can optionally fuse at the chemical bond between $X_5$ and $X_6$ with a 5-6 membered saturated or unsaturated ring, which can contain 0-3 heteroatoms selected from O, N, S;

wherein, the ring A also can be optionally substituted with 0, 1, 2, 3 groups selected from hydrogen, $C_1$-$C_6$ alkyl, deuterated $C_1$-$C_6$ alkyl, halogen, -$OR^a$, -$SR^a$, -P(O)$R^aR^b$, -CN, -$S(O)_2R^a$, -S(O)$R^a$, -$SF_5$, -$NR^aR^b$, $C_1$-$C_6$ haloalkyl, $C_1$-$C_6$ haloalkoxy, hydroxy($C_1$-$C_6$) alkyl or substituted groups which is optionally substituted with 0-4 substituted groups selected from deuterium, halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxy ($C_1$-$C_6$ alkyl), $NR^aR^b$, -CN, $C_1$-$C_6$ haloalkyl, $C_1$-$C_6$ haloalkoxy, -$SO_3R^a$, -$SR^a$, -$SF_5$,

-C(O)$R^a$, -C(O)O$R^a$, -OC(O)$R^a$, -OC(O)N$R^aR^b$, -N$R^a$CO$R^b$, or -CON$R^aR^b$;

wherein, R' is $C_1$-$C_6$ alkyl, $C_3$-$C_{10}$ cycloalkyl, 4-10 membered heterocyclyl, $C_6$-$C_{10}$ aryl, 5-10 membered heteroaryl, which are independently optionally substituted with 0-3 groups selected from deuterium, halogen, $C_1$-$C_6$ alkyl, $C_2$-$C_6$ alkenyl, $C_2$-$C_6$ alkynyl, $C_3$-$C_6$ cycloalkyl, 3-6 membered saturated or unsaturated monocyclic heterocyclyl, -O$R^a$, oxo, hydroxyl($C_1$-$C_6$ alkyl), N$R^aR^b$, -CN, $C_1$-$C_6$ haloalkyl, $C_1$-$C_6$ haloalkoxy, -$SO_3R^a$, -S$R^a$, -S(O)$_2R^a$, -S(O)$R^a$, -$SF_5$, -C(O)$R^a$, -C(O)O$R^a$, -OC(O)$R^a$, -OC(O)N$R^aR^b$, -N$R^a$CO$R^b$ or -CON$R^aR^b$;

preferably, R' is -CH$R^2R^3$ or -CD$R^2R^3$;

wherein, $R^2$ and $R^3$ are independently hydrogen, deuterium, -O$R^a$, halogen, -CN, -$C_1$-$C_6$ alkyl, $C_1$-$C_6$ haloalkyl, $C_1$-$C_6$ haloalkoxy, hydroxy($C_1$-$C_6$ alkyl), -$C_3$-$C_{10}$ cycloalkyl or 4-10 membered heterocyclyl, $C_6$-$C_{10}$ aryl and 5-10 membered heteroaryl, the 4-10 membered heterocyclyl, $C_6$-$C_{10}$ aryl and 5-10 membered heteroaryl can be optionally substituted with 0-3 groups selected from deuterium, halogen, $C_1$-$C_6$ alkyl, $C_2$-$C_6$ alkenyl, $C_2$-$C_6$ alkynyl, $C_3$-$C_6$ cycloalkyl, -O$R^a$, oxo, hydroxyl($C_1$-$C_6$ alkyl), N$R^aR^b$, -CN, $C_1$-$C_6$ haloalkyl, $C_1$-$C_6$ haloalkoxy, -$SO_3R^a$, -S$R^a$, -S(O)$_2R^a$, -S(O)$R^a$, -$SF_5$, -C(O)$R^a$, -C(O)O$R^a$, -OC(O)$R^a$, -OC(O)N$R^aR^b$, -N$R^a$CO$R^b$ or -CON-$R^aR^b$;

wherein, $M^1$ is C$R^aR^b$, N$R^a$, O, S or Se;

wherein, $R^L$ and $R^{L'}$ are independently hydrogen, deuterium, $C_1$-$C_6$ alkyl, or $R^L$ and $R^{L'}$ can be taken together with the atoms to which they are attached to form a $C_3$-$C_6$ carbocyclyl or heterocyclyl;

wherein, n and o are independently 0, 1 or 2.

wherein, $X_1$ is N or C$R^{X1}$;

wherein, $X_2$ is N or C$R^{X2}$;

wherein, $Y_1$ is C$R^{Y1}R^{Y1'}$, N$R^{Y1}$, O, S, Se;

wherein, $Y_2$ is C$R^{Y2}$R$Y^{2'}$, N$R^{Y2}$, O, S, Se;

wherein, $Y_3$ is C$R^{Y3}$R$Y^{3'}$, N$R^{Y3}$, O, S, Se;

wherein, $R^{X1}$ and $R^{X2}$ are independently hydrogen, deuterium, $C_1$-$C_6$ alkyl, deuterated $C_1$-$C_6$ alkyl, $C_1$-$C_6$ haloalkyl, $C_1$-$C_6$ haloalkoxy, hydroxy($C_1$-$C_6$) alkyl, $C_2$-$C_6$ alkenyl, $C_2$-$C_6$ alkynl, -O$R^a$, -S$R^a$, -S(O)$_2R^a$, -S(O) $R^a$, -CN, -OC(O)$R^a$, -OCON$R^aR^b$, halogen, -OS$O_3R^a$, -N$R^aR^b$, -$SF_5$;

wherein, $R^{Y1}$, $R^{Y1'}$, $R^{Y2}$, $R^{Y2'}$, $R^{Y3}$ and $R^{Y3'}$ are independtly absent, hydrogen, deuterium, $C_1$-$C_6$ alkyl, deuterated $C_1$-$C_6$ alkyl, $C_1$-$C_6$ haloalkyl, $C_1$-$C_6$ haloalkoxy, hydroxyl($C_1$-$C_6$) alkyl, $C_2$-$C_6$ alkenyl, $C_2$-$C_6$ alkynyl, $C_3$-$C_6$ cycloalkyl, -O$R^a$, -S$R^a$, -S(O)$_2R^a$, -S(O)$R^a$, -CN, -OC(O)$R^a$, -OCON$R^aR^b$, halogen, -OS$O_3R^a$, -N$R^aR^b$, -$SF_5$;

wherein, - - - is single bond or double bond;

wherein, $R^a$ and $R^b$ are independently hydrogen, deuterium, halogen, $C_1$-$C_6$ alkyl, $C_3$-$C_6$ cyclopropyl, $C_1$-$C_6$ haloalkyl, or $R^a$ and $R^b$ can be taken together with the atoms to which they are attached to form a 3-14 membered saturated or unsaturated cycle, which can optionally contain 0-2 heteroatoms selected from O, S and N.

2. The method according to claim 1, wherein the compound, or a pharmaceutically acceptable salt, ester, prodrug, stereoisomer, or isotopic derivative thereof, has the structure of the following Formula (II):

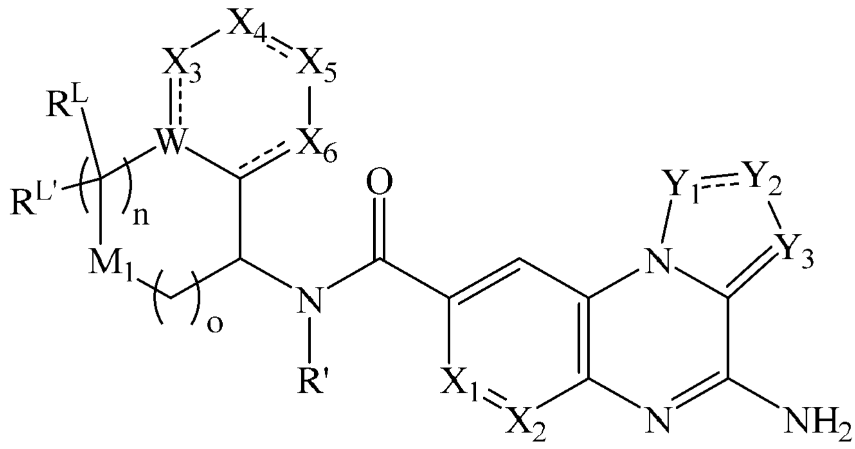

Formula (II)

wherein, $X_3$ is N or C$R^{X3}$; $X_4$ is N or C$R^{X4}$; $X_5$ is N or C$R^{X5}$; $X_6$ is N or C$R^{X6}$;

wherein, when $X_3$ is C$R^{X3}$, $R^{X3}$ is hydrogen, deuterium, $C_1$-$C_6$ alkyl, $C_1$-C6 haloalkyl, halogen, -O$R^a$, -S$R^a$, -P(O)

$R^aR^b$, -CN, -S(O)$_2$R$^a$, -S(O)R$^a$, -SF$_5$, -NR$^a$R$^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -OR$^a$, oxo, hydroxyl($C_1$-$C_6$ alkyl), NR$^a$R$^b$, -CN, $C_1$-$C_6$ haloalkyl, $C_1$-$C_6$ haloalkoxy, -SO$_3$R$^a$, -SR$^a$, -SF$_5$, -C(O)R$^a$, -C(O)OR$^a$, -OC(O)R$^a$, -OC(O)NR$^a$R$^b$, -NR$^a$COR$^b$ or -CONR$^a$R$^b$;

wherein, when $X_4$ is CR$^{X4}$, R$^{X4}$ is hydrogen, deuterium, $C_1$-$C_6$ alkyl, $C_1$-C6 haloalkyl, halogen, -OR$^a$, -SR$^a$, -P(O)R$^a$R$^b$, -CN, -S(O)$_2$R$^a$, -S(O)R$^a$, -SF$_5$, -NR$^a$R$^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -OR$^a$, oxo, hydroxyl($C_1$-$C_6$ alkyl), NR$^a$R$^b$, -CN, $C_1$-$C_6$ haloalkyl, $C_1$-$C_6$ haloalkoxy, -SO$_3$R$^a$, -SR$^a$, -SF$_5$, -C(O)R$^a$, -C(O)OR$^a$, -OC(O)R$^a$, -OC(O)NR$^a$R$^b$, -NR$^a$COR$^b$ or -CONR$^a$R$^b$;

wherein, when $X_5$ is CR$^{X5}$, R$^{X5}$ is hydrogen, deuterium, $C_1$-$C_6$ alkyl, $C_1$-C6 haloalkyl, halogen, -OR$^a$, -SR$^a$, -P(O)R$^a$R$^b$, -CN, -S(O)$_2$R$^a$, -S(O)R$^a$, -SF$_5$, -NR$^a$R$^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -OR$^a$, oxo, hydroxyl($C_1$-$C_6$ alkyl), NR$^a$R$^b$, -CN, $C_1$-$C_6$ haloalkyl, $C_1$-$C_6$ haloalkoxy, -SO$_3$R$^a$, -SR$^a$, -SF$_5$, -C(O)R$^a$, -C(O)OR$^a$, -OC(O)R$^a$, -OC(O)NR$^a$R$^b$, -NR$^a$COR$^b$ or -CONR$^a$R$^b$;

wherein, when $X_6$ is CR$^{X6}$, R$^{X6}$ is hydrogen, deuterium, $C_1$-$C_6$ alkyl, $C_1$-C6 haloalkyl, halogen, -OR$^a$, -SR$^a$, -P(O)R$^a$R$^b$, -CN, -S(O)$_2$R$^a$, -S(O)R$^a$, -SF$_5$, -NR$^a$R$^b$, $C_1$-$C_6$ haloalkyl, $C_1$-$C_6$ haloalkoxy, $C_1$-$C_6$ alkylthio, hydroxy($C_1$-$C_6$ alkyl) or $C_3$-$C_{10}$ cycloalkyl, $C_6$-$C_{10}$ cycloalkenyl, 4-10 membered heterocyclyl, 6-10 membered heterocycloalkenyl, $C_6$-$C_{10}$ aryl, 5-10 membered heteroaryl, wherein each ring is optionally substituted with 0-4 deuterium, halogen, $C_1$-$C_6$ alkyl, $C_2$-$C_6$ alkenyl, $C_2$-$C_6$ alkynyl, $C_3$-$C_6$ cycloalkyl, -OR$^a$, oxo, hydroxyl($C_1$-$C_6$ alkyl), NR$^a$R$^b$, -CN, $C_1$-$C_6$ haloalkyl, $C_1$-$C_6$ haloalkoxy, -SO$_3$R$^a$, -SR$^a$, -SF$_5$, -C(O)R$^a$, -C(O)OR$^a$, -OC(O)R$^a$, -OC(O)NR$^a$R$^b$, -NR$^a$COR$^b$ or -CONR$^a$R$^b$;

wherein, R' is $C_1$-$C_6$ alkyl, $C_3$-$C_{10}$ cycloalkyl, 4-10 membered heterocyclyl, $C_6$-$C_{10}$ aryl, 5-10 membered heteroaryl, which are independently optionally substituted with 0-3 groups selected from deuterium, halogen, $C_1$-$C_6$ alkyl, $C_2$-$C_6$ alkenyl, $C_2$-$C_6$ alkynyl, $C_3$-$C_6$ cycloalkyl, 3-6 membered saturated or unsaturated monocyclic heterocyclyl, -OR$^a$, oxo, hydroxyl($C_1$-$C_6$ alkyl), NR$^a$R$^b$, -CN, $C_1$-$C_6$ haloalkyl, $C_1$-$C_6$ haloalkoxy, -SO$_3$R$^a$, -SR$^a$, -S(O)$_2$R$^a$, -S(O)R$^a$, -SF$_5$, -C(O)R$^a$, -C(O)OR$^a$, -OC(O)R$^a$, -OC(O)NR$^a$R$^b$, -NR$^a$COR$^b$ or -CONR$^a$R$^b$;

preferably, R' is -CHR$^2$R$^3$ or -CDR$^2$R$^3$;

wherein, $R^2$ and $R^3$ are independently hydrogen, deuterium, -OR$^a$, halogen, -CN, -$C_1$-$C_6$ alkyl, $C_1$-$C_6$ haloalkyl, $C_1$-$C_6$ haloalkoxy, hydroxy($C_1$-$C_6$ alkyl), -$C_3$-$C_{10}$ cycloalkyl or 4-10 membered heterocyclyl, $C_6$-$C_{10}$ aryl and 5-10 membered heteroaryl, the 4-10 membered heterocyclyl, $C_6$-$C_{10}$ aryl and 5-10 membered heteroaryl can be optionally substituted with 0-3 groups selected from deuterium, halogen, $C_1$-$C_6$ alkyl, $C_2$-$C_6$ alkenyl, $C_2$-$C_6$ alkynyl, $C_3$-$C_6$ cycloalkyl, -OR$^a$, oxo, hydroxyl($C_1$-$C_6$ alkyl), NR$^a$R$^b$, -CN, $C_1$-$C_6$ haloalkyl, $C_1$-$C_6$ haloalkoxy, -SO$_3$R$^a$, -SR$^a$, -S(O)$_2$R$^a$, -S(O)R$^a$, -SF$_5$, -C(O)R$^a$, -C(O)OR$^a$, -OC(O)R$^a$, -OC(O)NR$^a$R$^b$, -NR$^a$COR$^b$ or -CONR$^a$R$^b$;

wherein, $M^1$ is CR$^a$R$^b$, NR$^a$, O, S or Se;

wherein, $R^L$ and $R^{L'}$ are independently hydrogen, deuterium, $C_1$-$C_6$ alkyl, or $R^L$ and $R^{L'}$ can be taken together with the atoms to which they are attached to form a $C_3$-$C_6$ carbocyclyl or heterocyclyl;

wherein, n and o are independently 0, 1 or 2;

wherein, $X_1$ is N or CR$^{X1}$;

wherein, $X_2$ is N or CR$^{X2}$;

wherein, $Y_1$ is CR$^{Y1}$RY$^{1'}$, NR$^{Y1}$, O, S, Se;

wherein, $Y_2$ is CR$^{Y2}$RY$^{2'}$, NR$^{Y2}$, O, S, Se;

wherein, $Y_3$ is CR$^{Y3}$RY$^{3'}$, NR$^{Y3}$, O, S, Se;

wherein, R$^{X1}$ and R$^{X2}$ are independently hydrogen, deuterium, $C_1$-$C_6$ alkyl, deuterated $C_1$-$C_6$ alkyl, $C_1$-$C_6$ haloalkyl, $C_1$-$C_6$ haloalkoxy, hydroxy($C_1$-$C_6$) alkyl, $C_2$-$C_6$ alkenyl, $C_2$-$C_6$ alkynl, -OR$^a$, -SR$^a$, -S(O)$_2$R$^a$, -S(O)R$^a$, -CN, -OC(O)R$^a$, -OCONR$^a$R$^b$, halogen, -OSP$_3$R$^a$, -NR$^a$R$^b$, -SF$_5$;

wherein, R$^{Y1}$, R$^{Y1'}$, R$^{Y2}$, R$^{Y2'}$, R$^{Y3}$ and R$^{Y3'}$ are independtly absent, hydrogen, deuterium, $C_1$-$C_6$ alkyl, deuterated $C_1$-$C_6$ alkyl, $C_1$-$C_6$ haloalkyl, $C_1$-$C_6$ haloalkoxy, hydroxyl($C_1$-$C_6$) alkyl, $C_2$-$C_6$ alkenyl, $C_2$-$C_6$ alkynyl, $C_3$-$C_6$ cycloalkyl, -OR$^a$, -SR$^a$, -S(O)$_2$R$^a$, -S(O)R$^a$, -CN, -OC(O)R$^a$, -OCONR$^a$R$^b$, halogen, -OSO$_3$R$^a$, -NR$^a$R$^b$, -SF$_5$;

wherein, - - - is single bond or double bond;

wherein, R$^a$ and R$^b$ are independently hydrogen, deuterium, halogen, $C_1$-$C_6$ alkyl, $C_3$-$C_6$ cyclopropyl, $C_1$-$C_6$ haloalkyl, or R$^a$ and R$^b$ can be taken together with the atoms to which they are attached to form a 3-14 membered

saturated or unsaturated cycle, which can optionally contain 0-2 heteroatoms selected from O, S and N.

3. The method according to claim 1 or claim 2, wherein, $\overline{- - -}$ is single bond or double bond.

4. The method according to claim 1 or claim 2, wherein, $X_1$ is $CR^{X1}$ or N, wherein, $R^{X1}$ is hydrogen, deuterium, halogen, -CN, $C_1$-$C_6$ alkyl, deuterated $C_1$-$C_6$ alkyl, $C_1$-$C_6$ alkoxy, $C_1$-$C_6$ haloalkyl.

5. The method according to claim 4, wherein, $X_1$ is CH, CF or N.

6. The method according to any one of claims 1 to 5, wherein, $X_2$ is CH or CD.

7. The method according to any one of claims 1 to 6, wherein, $X_2$ is CH.

8. The method according to any one of claims 1 to 4, wherein, $X_3$ is CH, CD or N.

9. The method according to claim 8, wherein, $X_3$ is CH.

10. The method according to any one of claims 1 to 9, wherein, $X_4$ is $CR^{X4}$ or N, wherein, $R^{X4}$ is hydrogen, deuterium, $C_1$-$C_6$ alkyl, deuterated $C_1$-$C_6$ alkyl, $C_1$-$C_6$ alkoxy, $C_1$-$C_6$ alkylthio, $C_1$-$C_6$ haloalkyl, $C_1$-$C_6$ haloalkoxy, $C_1$-$C_6$ haloalkylthio, halogen, $SF_5$, $-S(O)_2R^a$, -P(O) $R^aR^b$, CN or $C_3$-$C_{10}$ cycloalkyl, $C_6$-$C_{10}$ cycloalkenyl, 4-10 membered heterocyclyl, $C_6$-$C_{10}$ aryl and 5-10 membered heteroaryl, wherein, each ring is optionally substituted with 0-4 groups independently selected from deuterium, halogen, $C_1$-$C_6$ alkyl, $C_2$-$C_6$ alkenyl, $C_2$-$C_6$ alkynyl, $C_3$-$C_6$ cycloalkyl, $-OR^a$, oxo, hydroxy($C_1$-$C_6$) alkyl, $NR^aR^b$, -CN, $C_1$-$C_6$ haloalkyl, $C_1$-$C_6$ haloalkoxy, $-S(O)_2R^a$, $-SR^a$, $-SF_5$, $-C(O)R^a$, -C(O) $OR^a$, $-OC(O)R^a$, $-OC(O)NR^aR^b$, $-NR^aCOR^b$ or $-CONR^aR^b$.

11. The method according to claim 10, wherein, $X_4$ is $CR^{X4}$; wherein, $R^{X4}$ is $C_1$-$C_6$ alkoxy, $C_1$-$C_6$ alkylthio, $C_1$-$C_6$ haloalkoxy, $C_1$-$C_6$ haloalkylthio, $C_1$-$C_6$ haloalkyl, $-SF_5$.

12. The method according to claim 11, wherein, $X_4$ is $CR^{X4}$; wherein, $R^{X4}$ is $C_1$-$C_6$ haloalkoxy, $C_1$-$C_6$ haloalkylthio, $C_1$-$C_6$ haloalkyl, $-SF_5$.

13. The method according to any one of claims 1 to 12, wherein, $X_5$ is $CR^{X5}$ or N, wherein, $R^{X5}$ is hydrogen, deuterium, $C_1$-$C_6$ alkyl, $C_1$-$C_6$ alkoxy, $C_1$-$C_6$ haloalkyl, $C_1$-$C_6$ haloalkoxy, halogen, $-SF_5$ or CN.

14. The method according to any one of claims 1 to 13, wherein, $X_5$ is CH.

15. The method according to any one of claims 1 to 14, wherein, $X_6$ is CH, CD or N.

16. The method according to any one of claims 1 to 15, wherein, $X_6$ is CH.

17. The method according to any one of claims 1 to 16, wherein, the chemical bond between $Y_1$ and $Y_2$ is double bond.

18. The method according to claim 17, wherein, $Y_1$ is CH, CD or $CCH_3$.

19. The method according to claim 17 or 18, wherein, $Y_2$ is N.

20. The method according to any one of claims 1 to 19, wherein, $Y_3$ is CH, CD, $CCH_3$ or $CCH_2OH$.

21. The method according to any one of claims 1 to 20, wherein, R' is $-CHR^2R^3$ or $-CDR^2R^3$, wherein, $R^2$ and $R^3$ are independently hydrogen, deuterium, $C_1$-$C_6$ alkyl or $C_3$-$C_{10}$ cycloalkyl, 4-10 membered heterocyclyl, $C_6$-$C_{10}$ aryl, 5-10 membered heteroaryl, wherein, each ring is independently optionally substituted with 0-3 groups selected from halogen, $C_1$-$C_6$ alkyl, $C_2$-$C_6$ alkenyl, $C_2$-$C_6$ alkynyl, $C_3$-$C_6$ cycloalkyl, $-OR^a$, oxo, hydroxy($C_1$-$C_6$) alkyl, $NR^aR^b$, -CN, $C_1$-$C_6$ haloalkyl, $C_1$-$C_6$ haloalkoxy, $-SO_3R^a$, $-SR^a$, $-S(O)_2R^a$, $-S(O)R^a$, $-SF_5$, $-C(O)R^a$, $-C(O)OR^a$, $-OC(O)R^a$, -OC(O) $NR^aR^b$, $-NR^aCOR^b$ or $-CONR^aR^b$.

22. The method according to any one of claims 1 to 21, wherein, R' is $-CHR^2R^3$ or $-CDR^2R^3$, wherein, $R^2$ is hydrogen, deuterium, $C_1$-$C_6$ alkyl; $R^3$ is hydrogen, deuterium, $C_1$-$C_6$ alkyl or $C_3$-$C_{10}$ cycloalkyl, 4-10 membered heterocyclyl, $C_6$-$C_{10}$ aryl, 5-10 membered heteroaryl, wherein, each ring is independently optionally substituted with 0-3 groups

selected from halogen, $C_1$-$C_6$ alkyl, $C_2$-$C_6$ alkenyl, $C_2$-$C_6$ alkynyl, $C_3$-$C_6$ cycloalkyl, -$OR^a$, oxo, hydroxy($C_1$-$C_6$) alkyl, $NR^aR^b$, -CN, $C_1$-$C_6$ haloalkyl, $C_1$-$C_6$ haloalkoxy, -$SO_3R^a$, -$SR^a$, -$S(O)_2R^a$, -$S(O)R^a$, -$SF_5$, -$C(O)R^a$, -$C(O)OR^a$, -OC(O)$R^a$, -$OC(O)NR^aR^b$, -$NR^aCOR^b$ or -$CONR^aR^b$.

**23.** The method according to any one of claims 1 to 22, wherein, R' is $C_3$-$C_{10}$ cycloalkyl which is optionally substituted with 0-3 groups independently selected from deuterium, halogen, $C_1$-$C_6$ alkyl, hydroxy($C_1$-$C_6$) alkyl, -$OR^a$, -CN, $NR^aR^b$, $C_1$-$C_6$ haloalkyl, $C_1$-$C_6$ haloalkoxy.

**24.** The method according to any one of claims 1 to 23, wherein, R' is $C_1$-$C_6$ alkyl (preferably methyl, ethyl) or deuterated $C_1$-$C_6$ alkyl (preferably deuterated methyl, deuterated ethyl) or $C_3$-$C_6$ cycloalkyl (preferably cyclopropyl).

**25.** The method according to any one of claims 1 to 24, wherein, $M_1$ is O or S.

**26.** The method according to any one of claims 1 to 25, wherein, o is 1 or 2.

**27.** The method according to claim 26, wherein, o is 1.

**28.** The method according to any one of claims 1 to 27, wherein, n is 0 or 1.

**29.** The method according to claim 28, wherein, n is 0.

**30.** The method according to claim 28, wherein, n is 1.

**31.** The method according to claim 30, wherein, $R^L$ and $R^{L'}$ are independently hydrogen or $C_1$-$C_6$ alkyl.

**32.** A method for treating a tumor or cancer, comprising administering to a subject in need thereof following compound, or a pharmaceutically acceptable salt, ester, prodrug, stereoisomer, or isotopic derivative thereof,

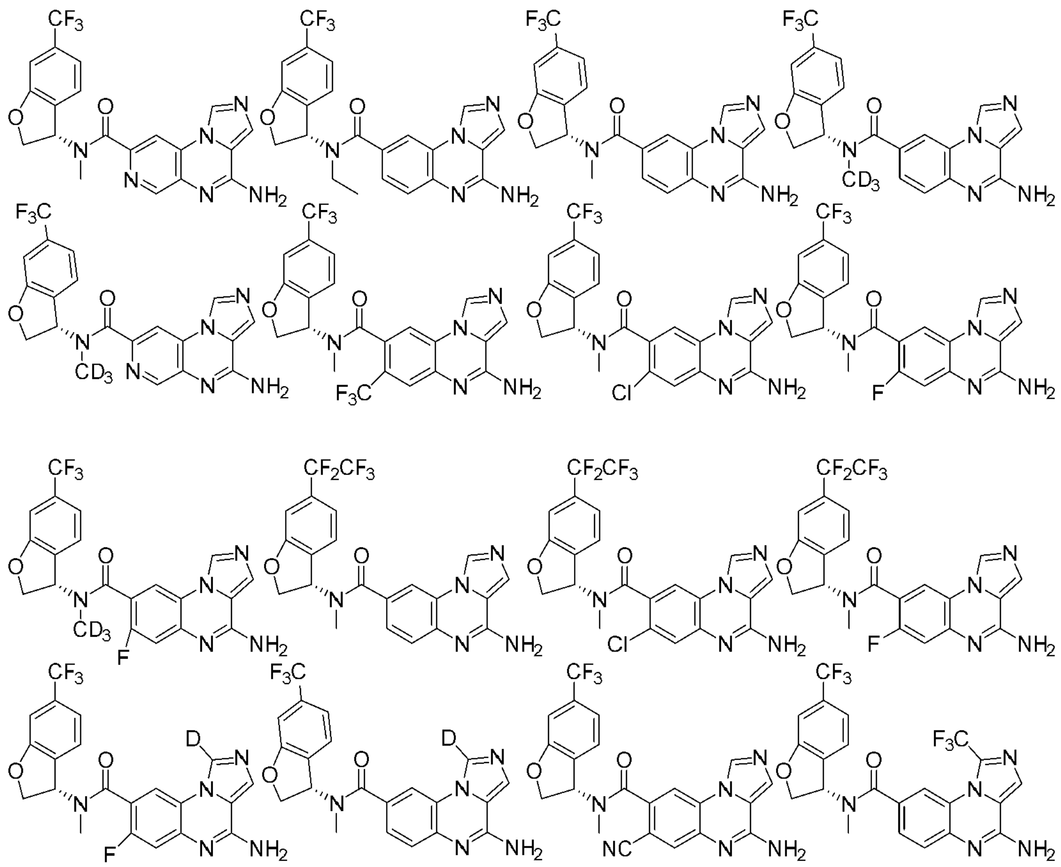

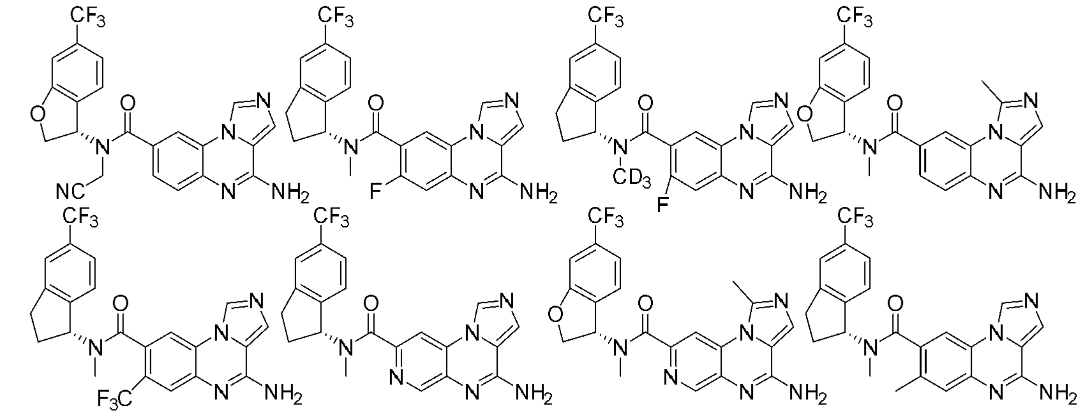

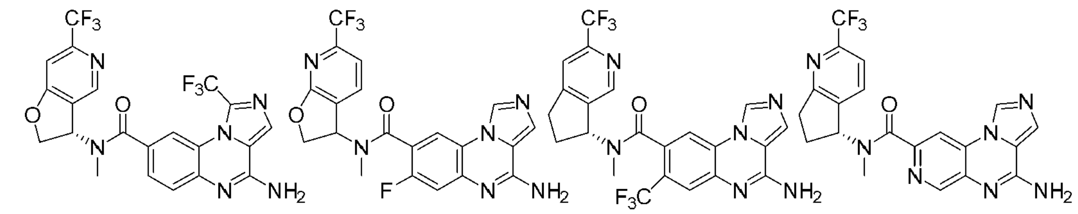

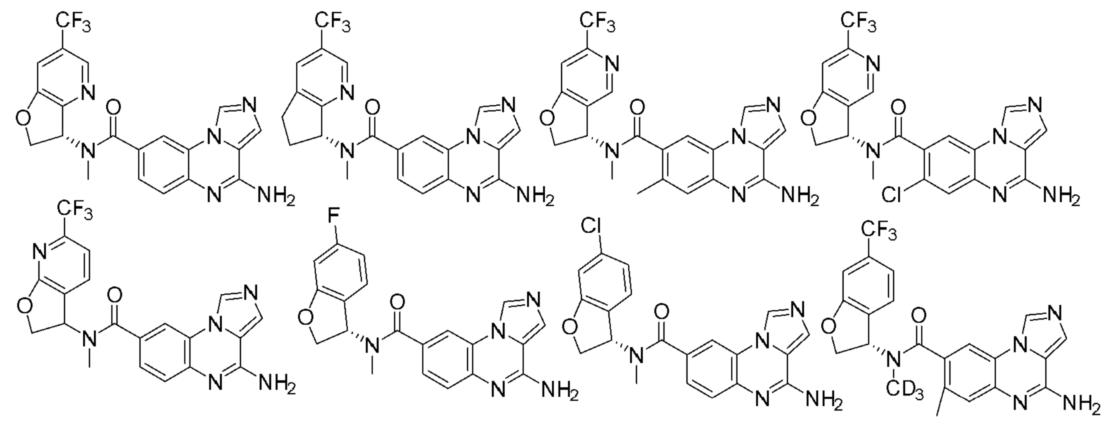

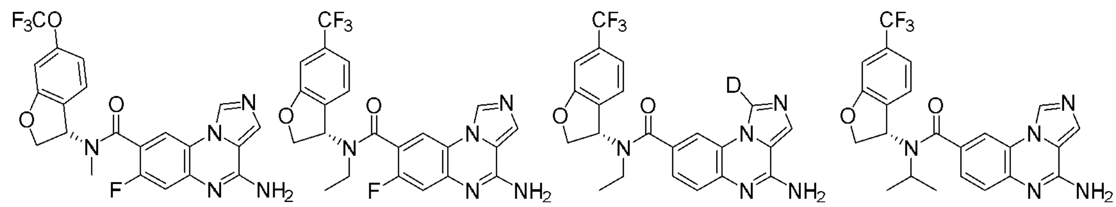

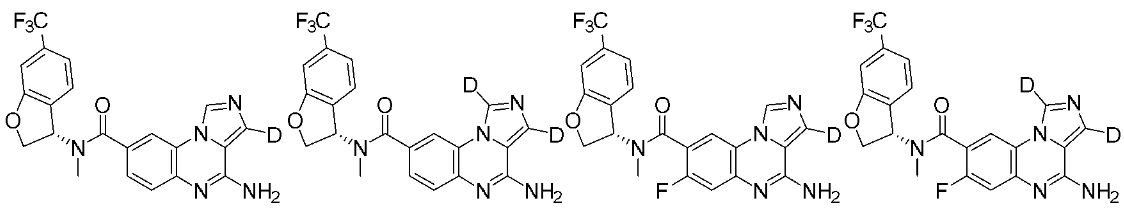

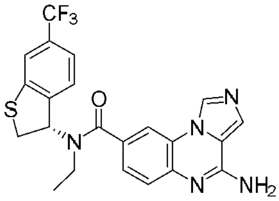 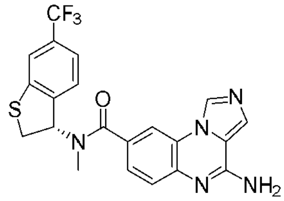 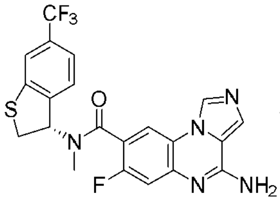 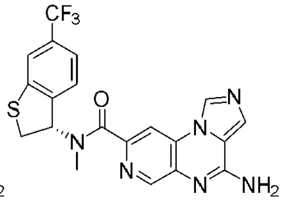

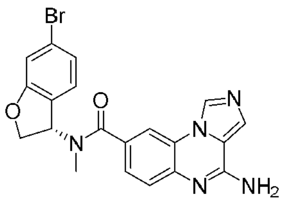 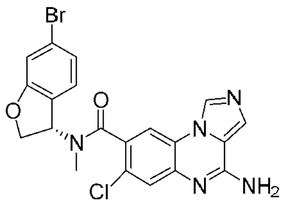 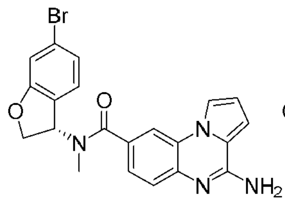 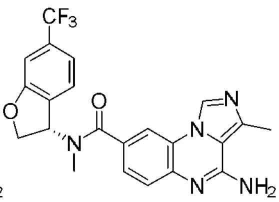

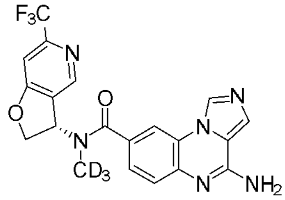 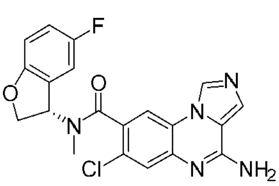 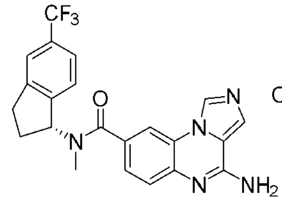 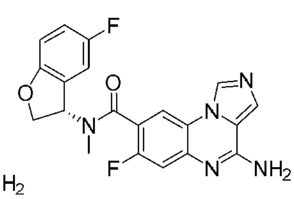

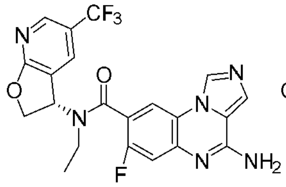 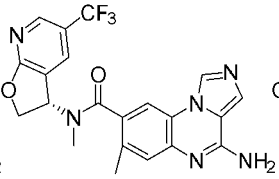 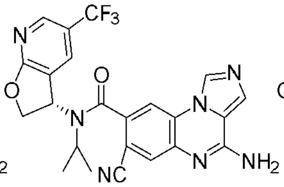 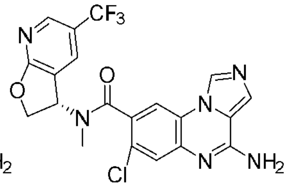

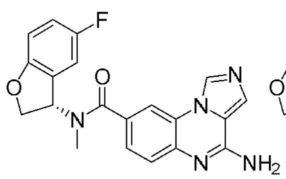 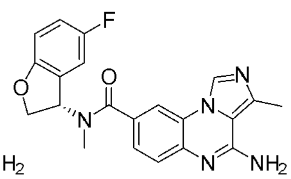 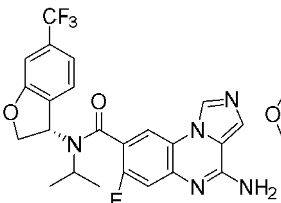 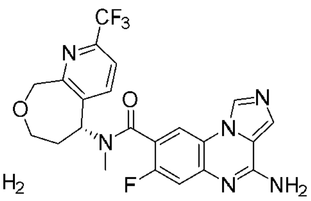

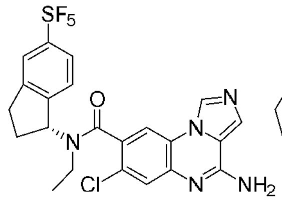 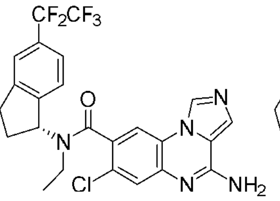 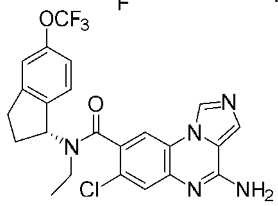 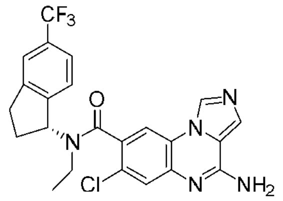

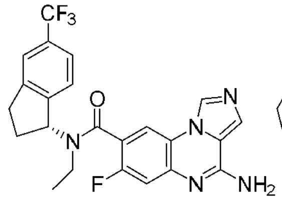 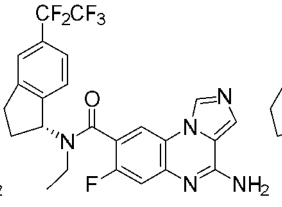 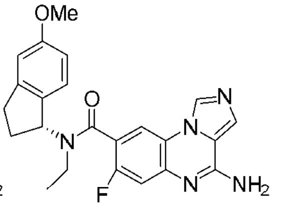 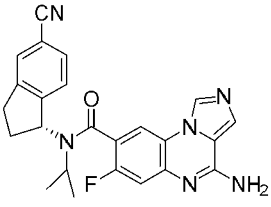

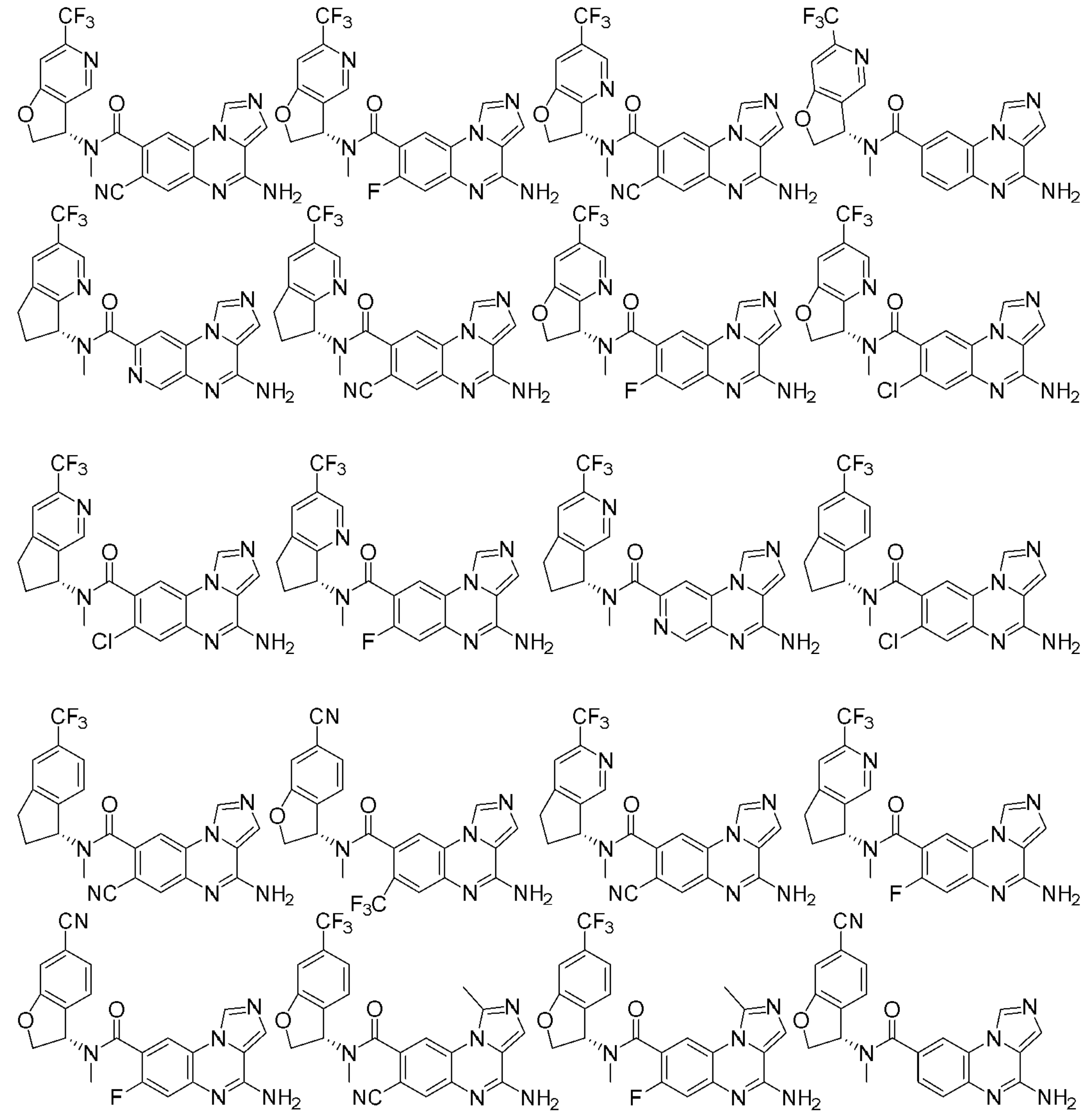

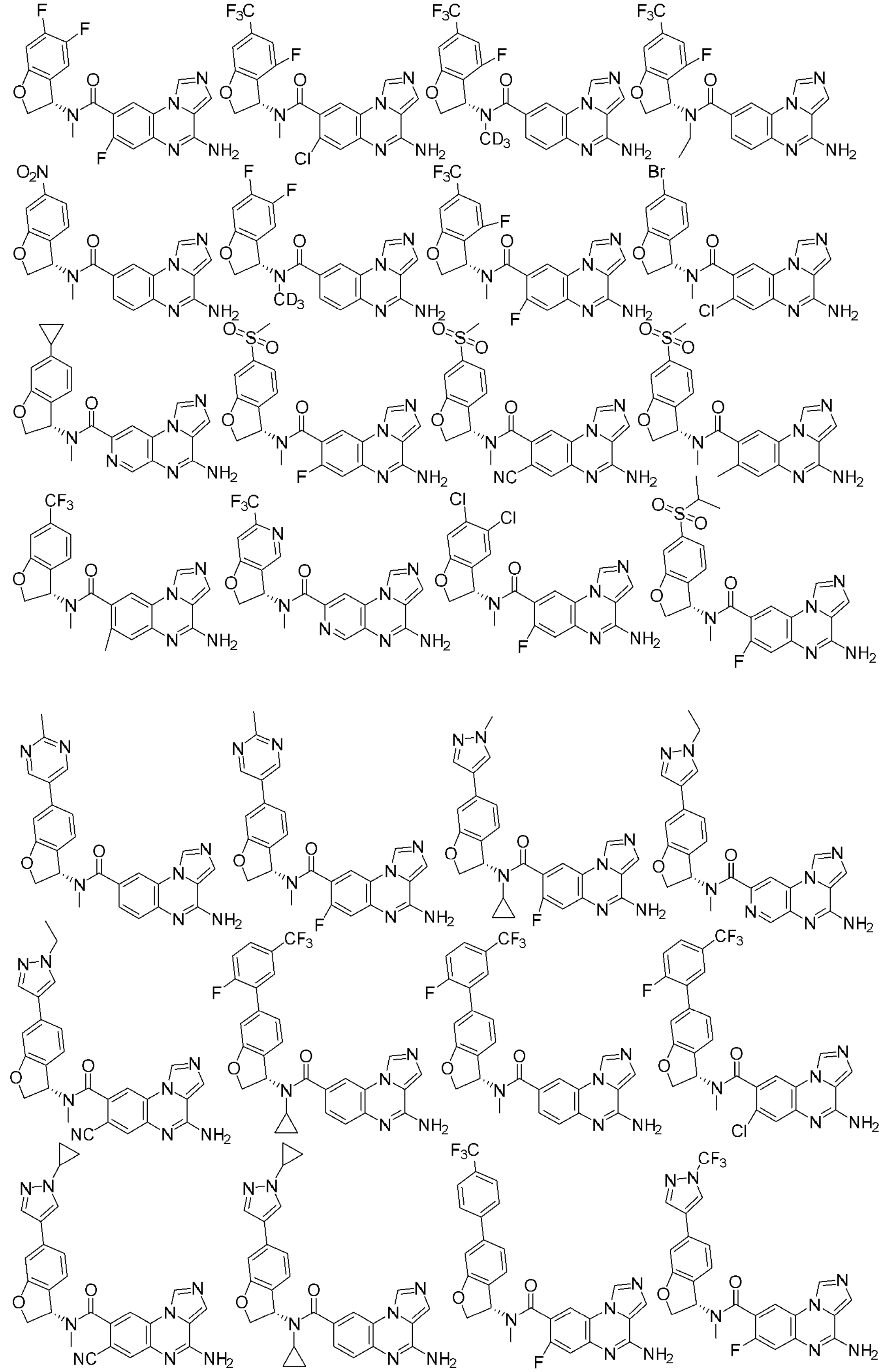

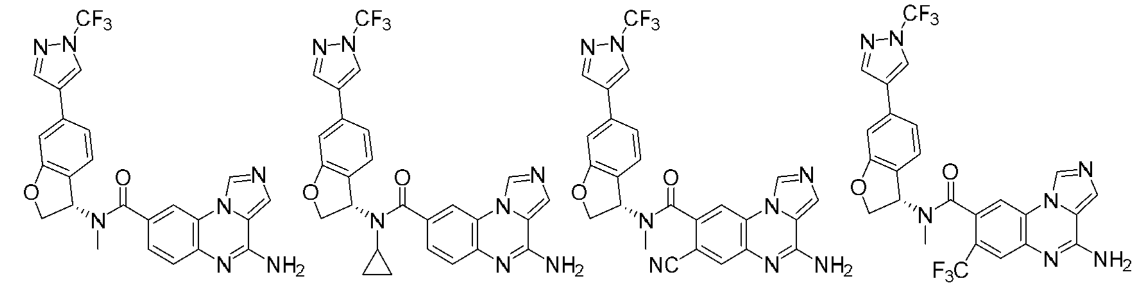

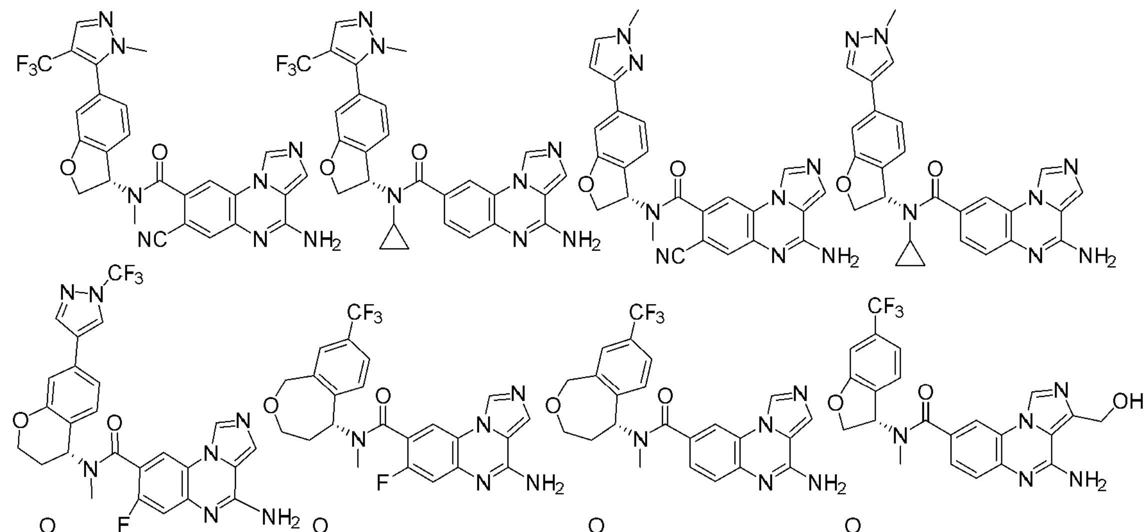

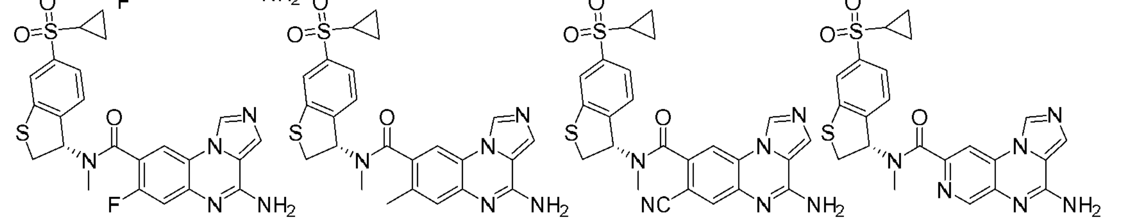

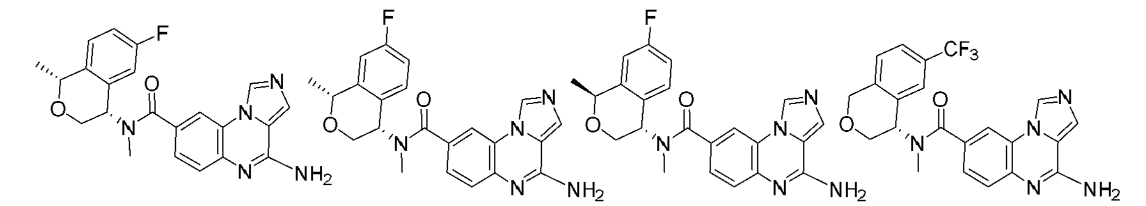

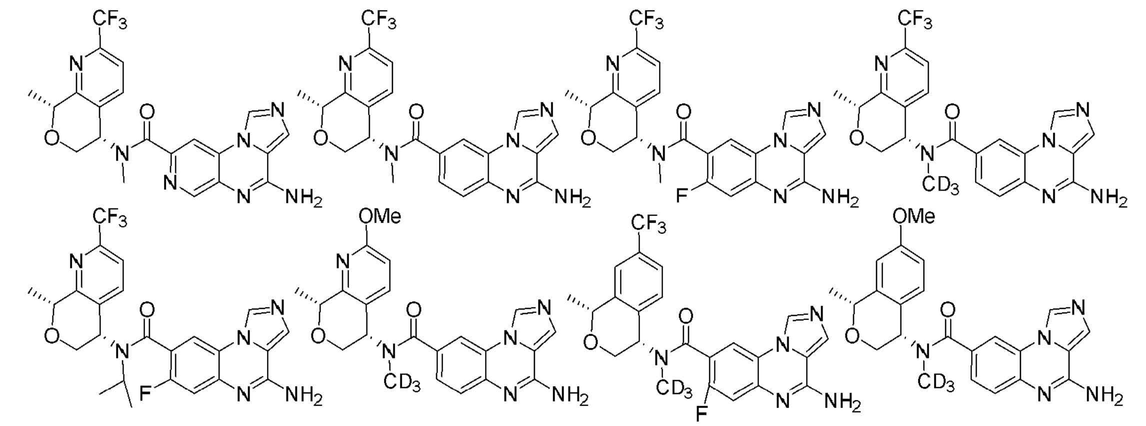

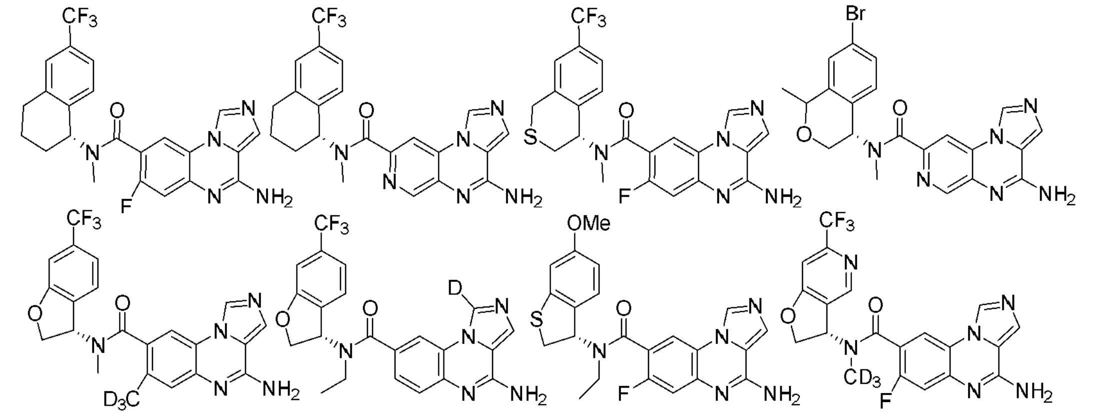

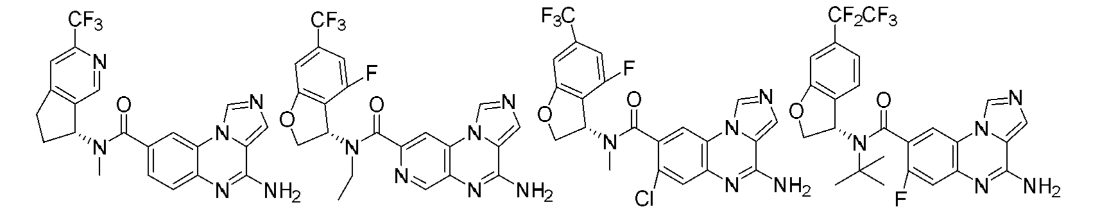

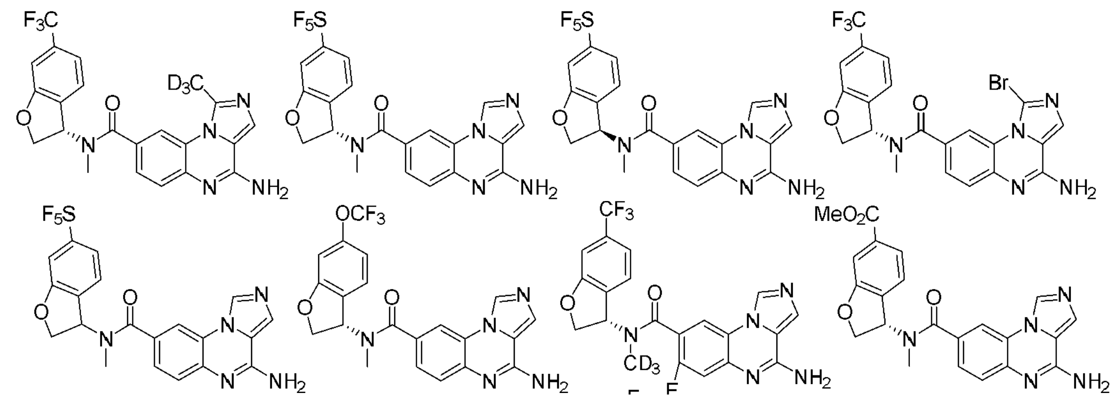

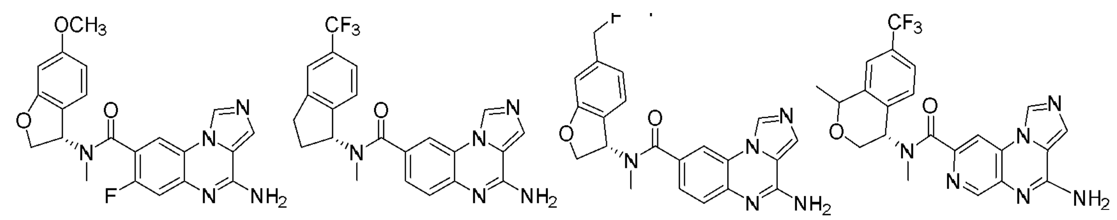

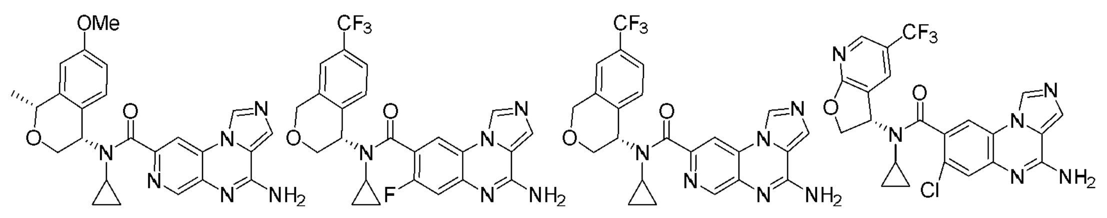

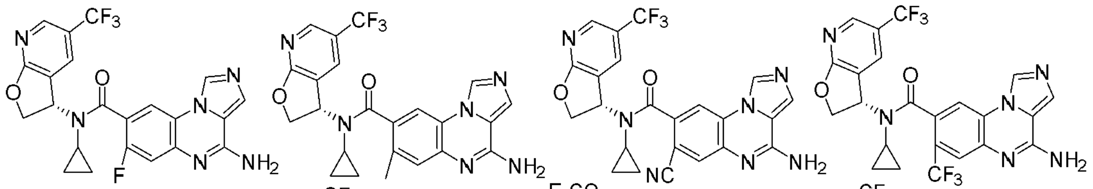

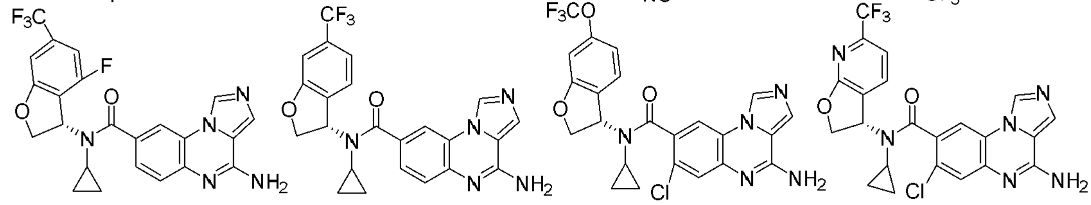

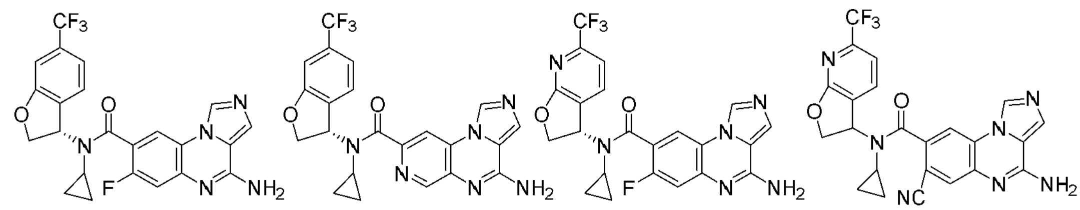

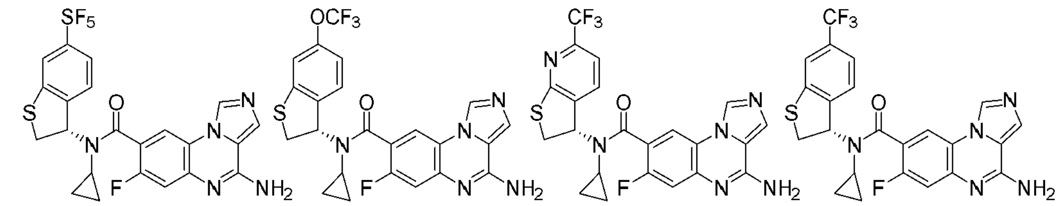

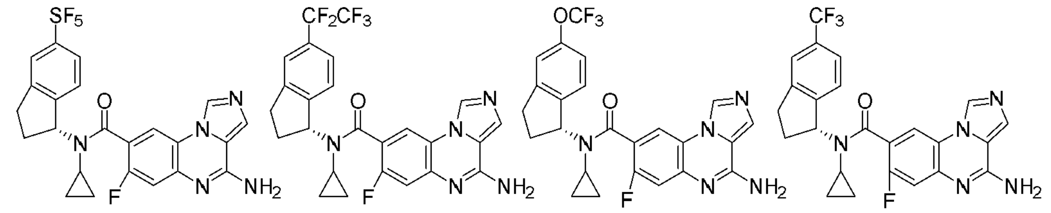

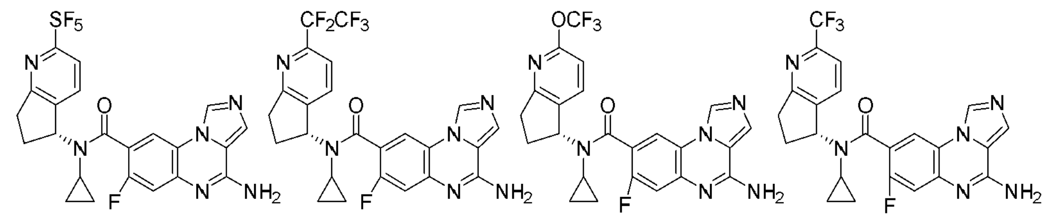

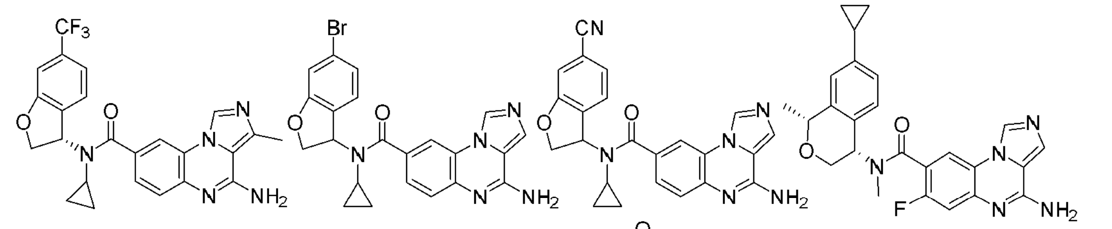

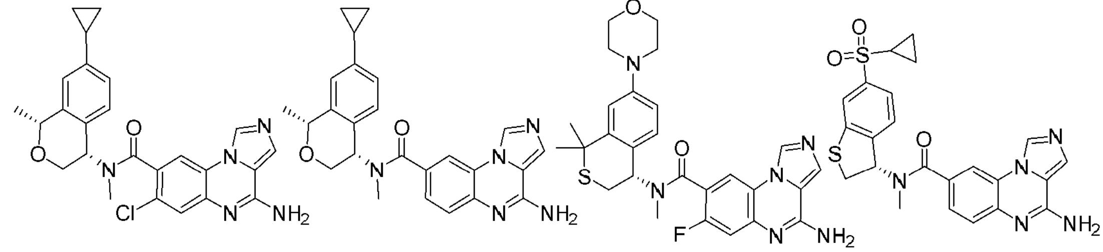

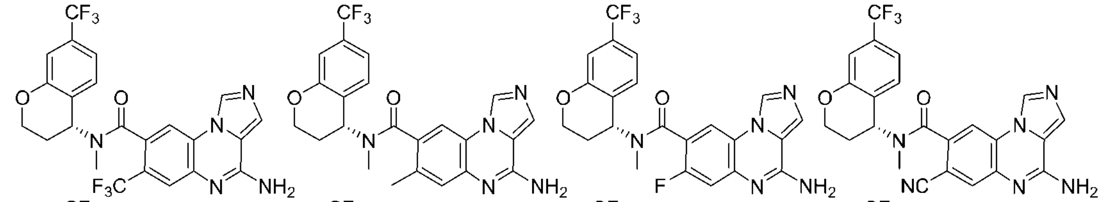

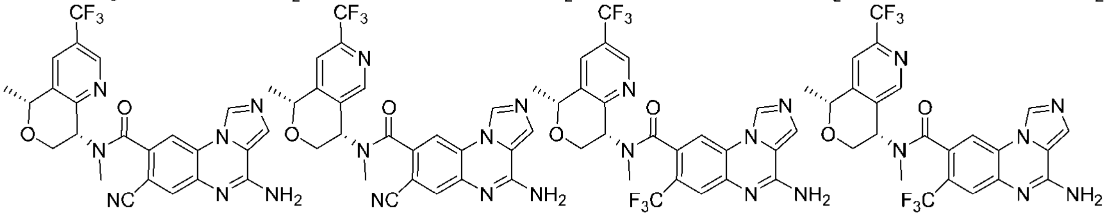

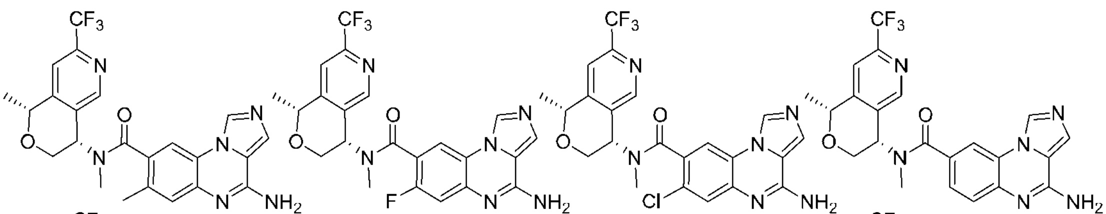

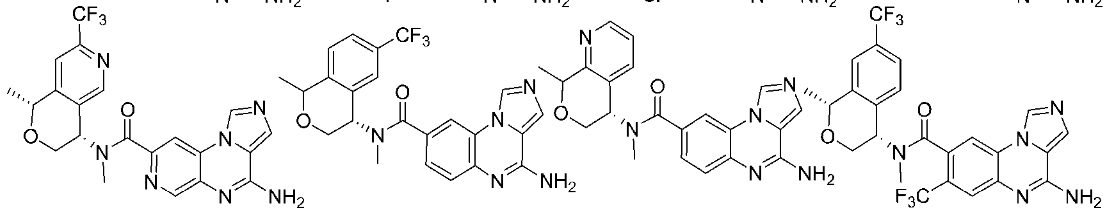

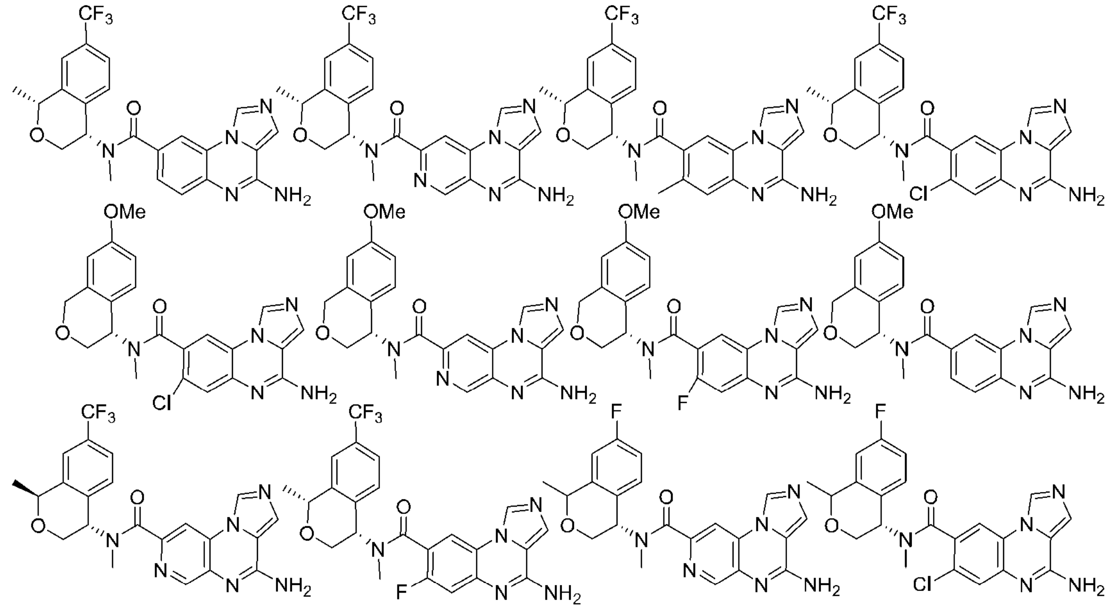

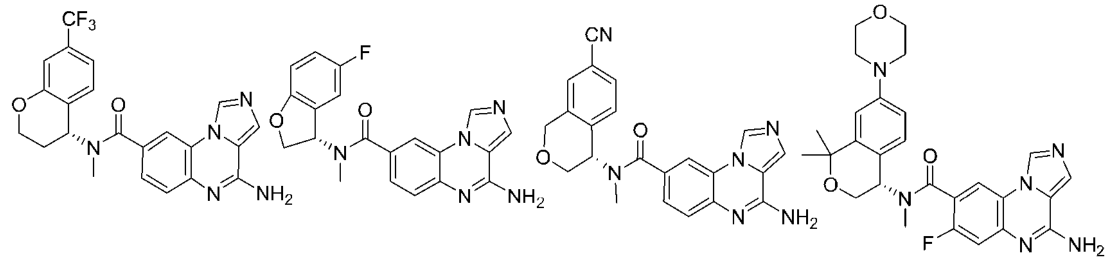

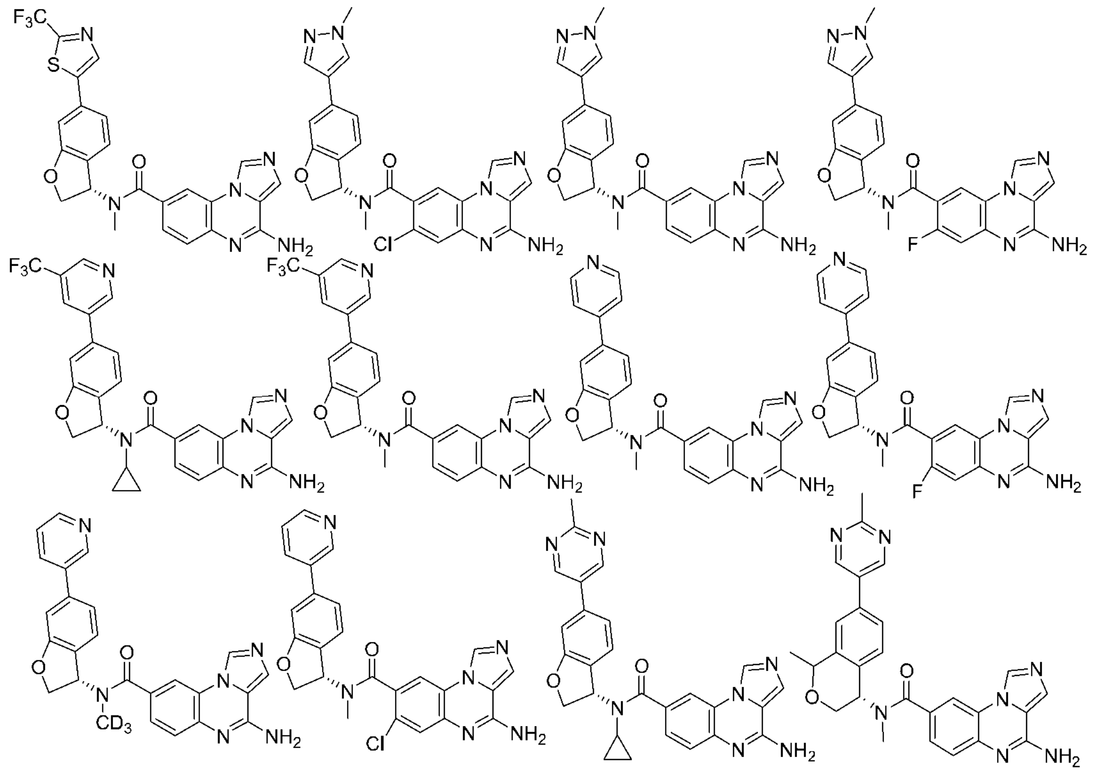

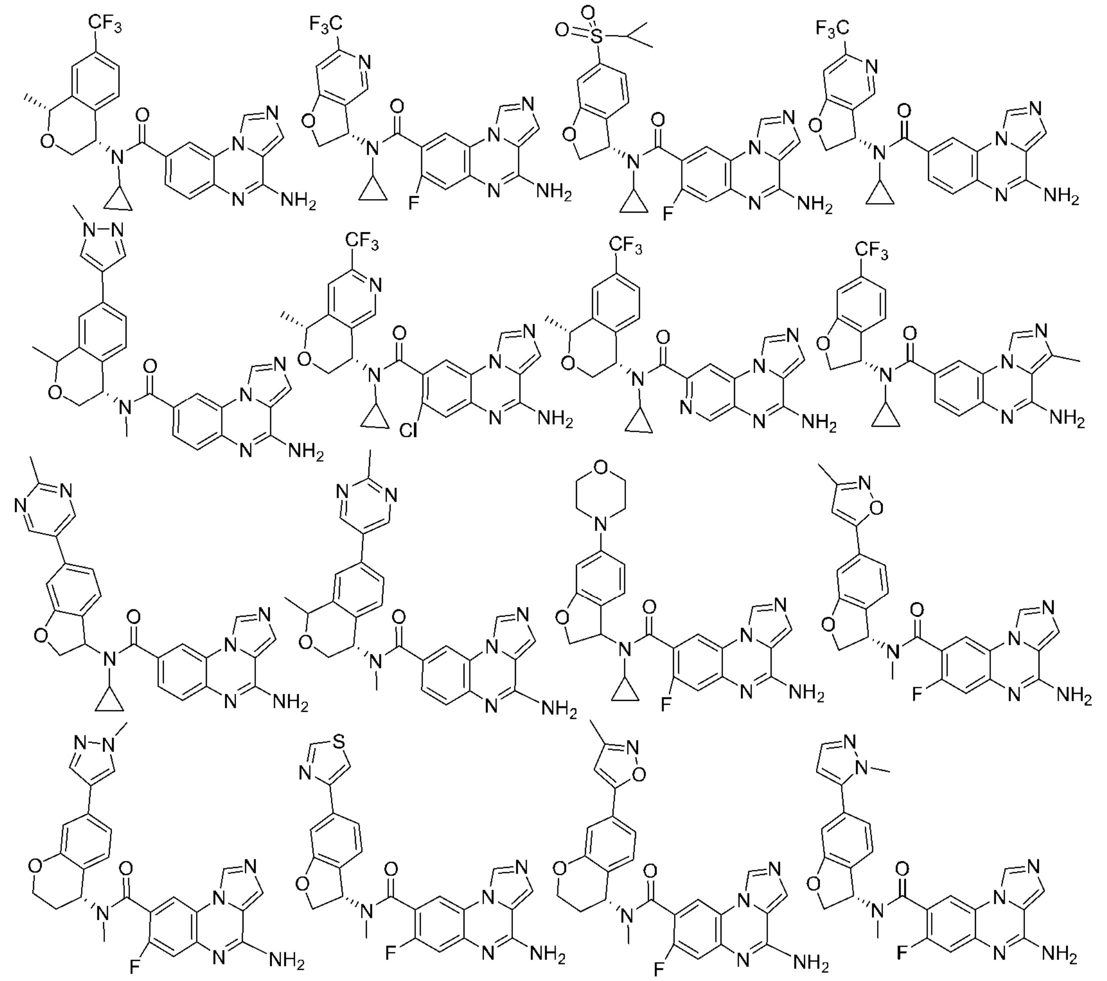

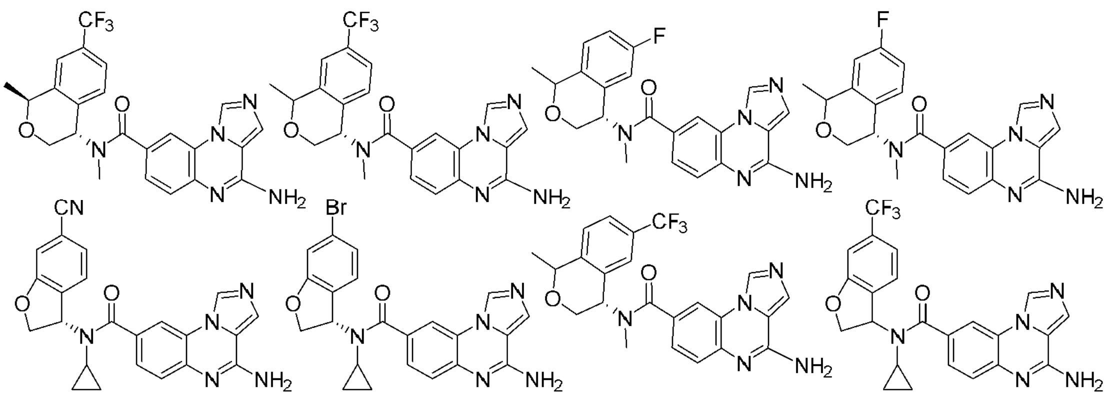

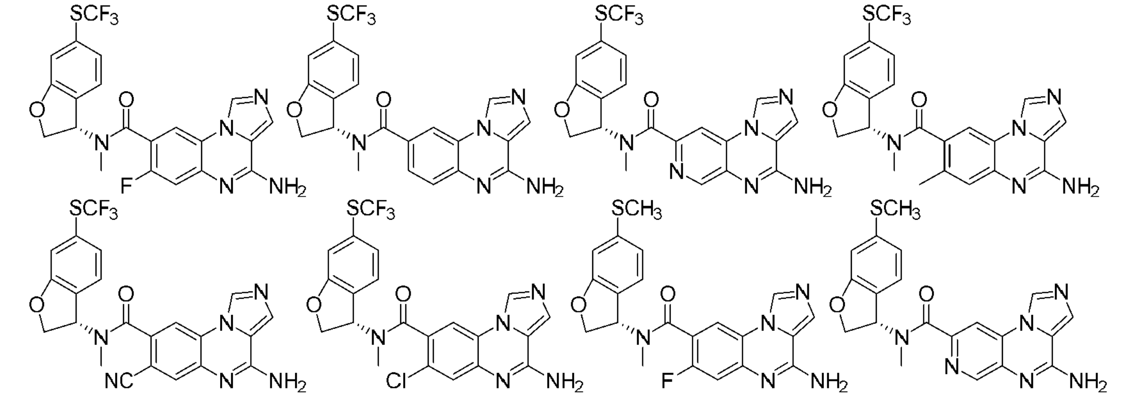

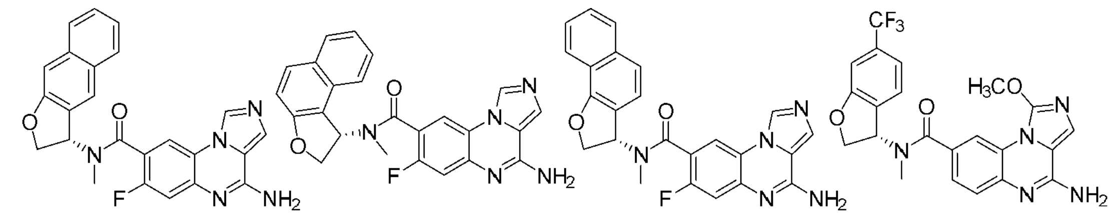

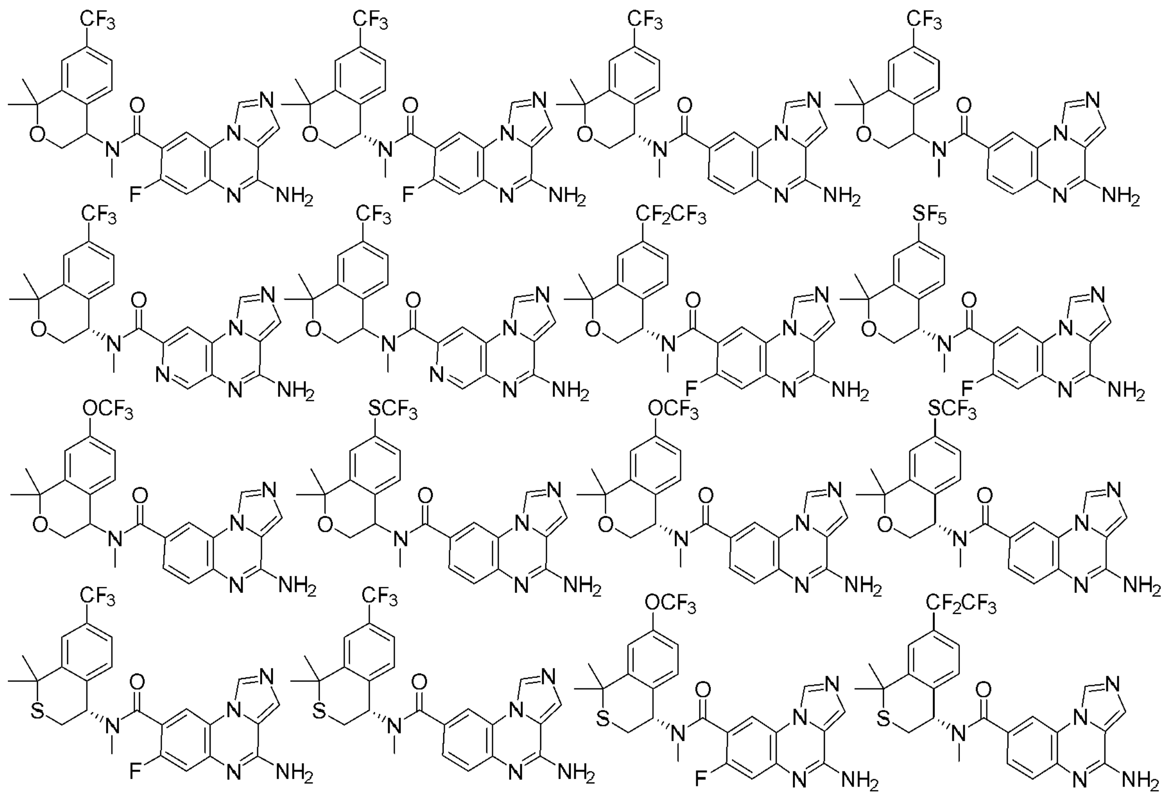

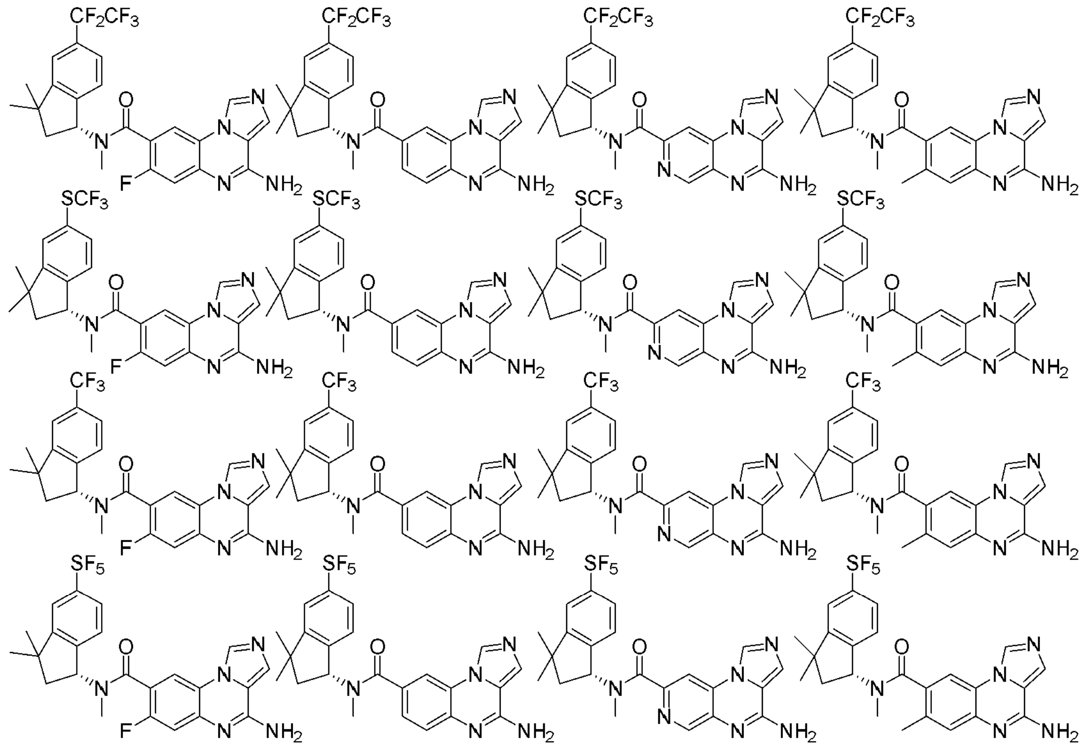

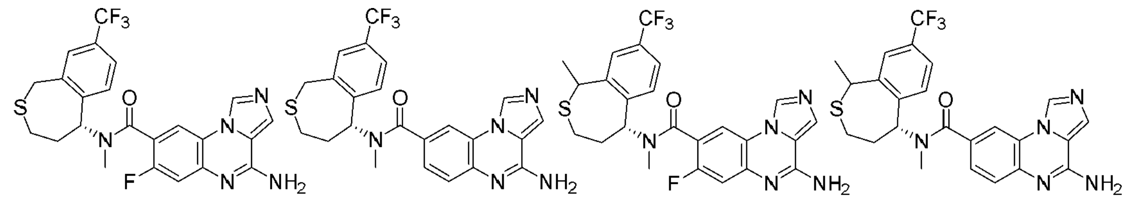

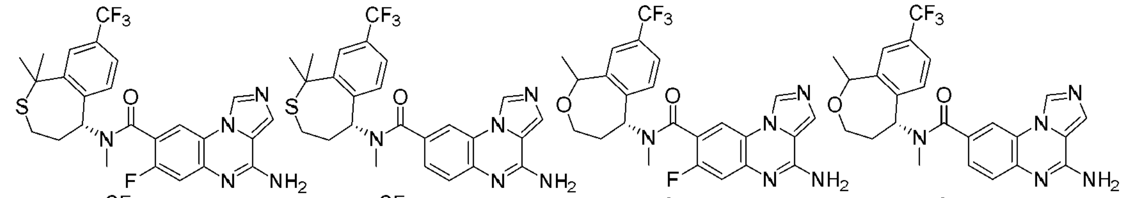

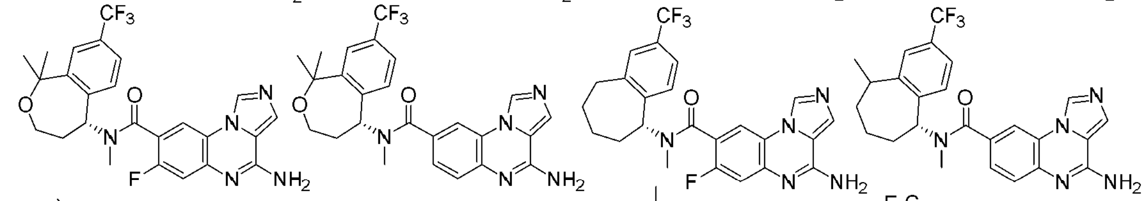

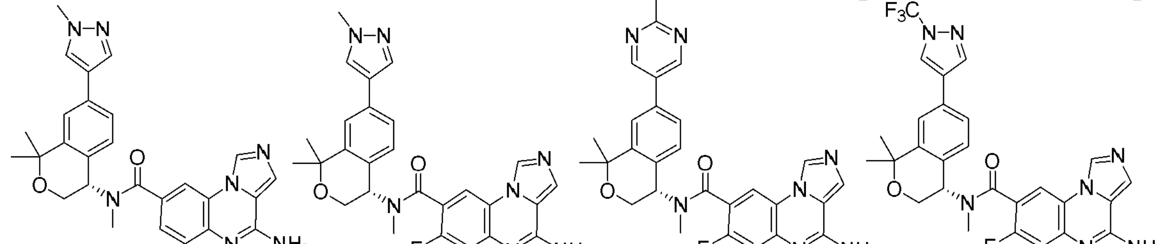

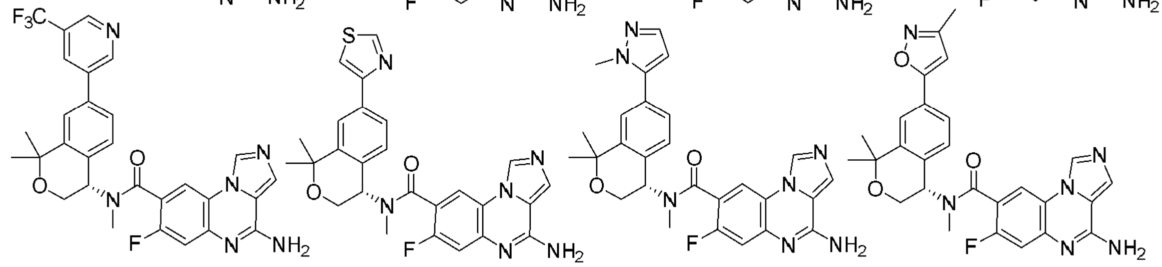

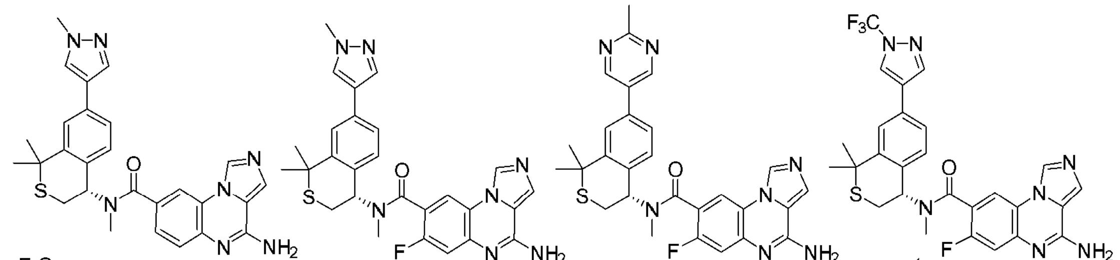

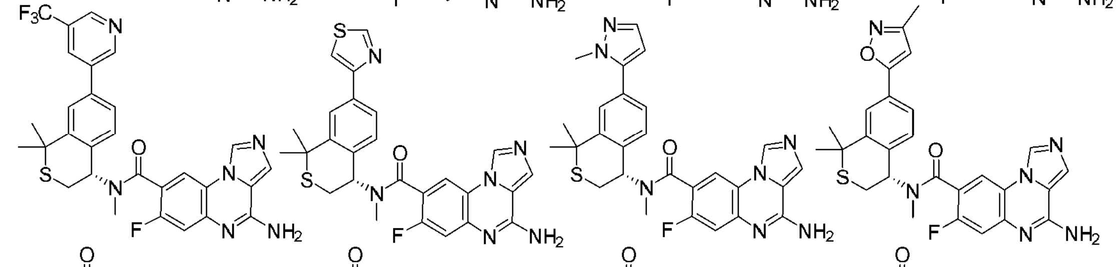

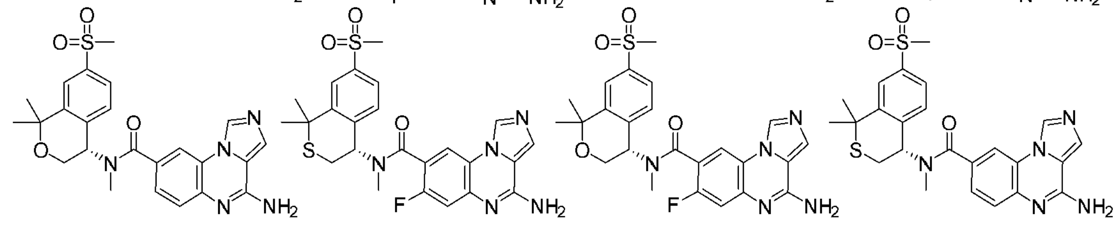

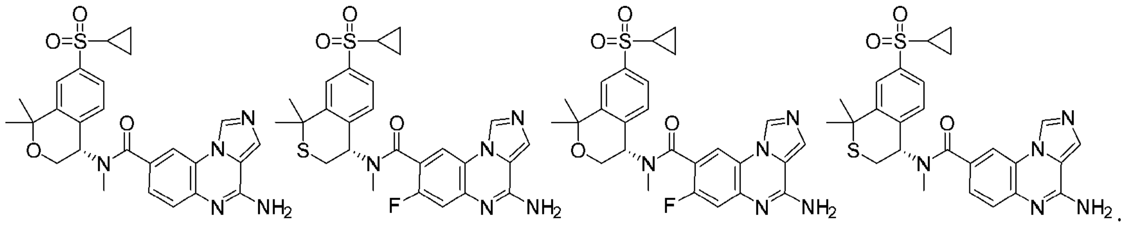

**33.** The method according to any one of claims 1 to 32, wherein, the compound is selected from the group consisting of:

(S)-4-amino-N-methyl-N-(6-(trifluoromethyl)-2,3-dihydrobenzofuran-3-yl)imidazo[1,5-a]quinoxaline-8-carboxamide
(S)-4-amino-N-methyl-N-(6-(trifluoromethyl)-2,3-dihydrobenzofuran-3-yl)imidazo[1,5-a]pyrido[3,4-e]pyrazine-8-carboxamide
(S)-4-amino-7-fluoro-N-methyl-N-(6-(trifluoromethyl)-2,3-dihydrobenzofuran-3-yl)imidazo[1,5-a]quinoxaline-8-carboxamide
(S)-4-amino-N-(methyl-d3)-N-(6-(trifluoromethyl)-2,3-dihydrobenzofuran-3-yl)imidazo[1,5-a]quinoxaline-8-carboxamide
(S)-4-amino-N-methyl-N-(6-(pentafluoro-$\lambda^6$-sulfaneyl)-2,3-dihydrobenzofuran-3-yl)imidazo[1,5-a]quinoxaline-8-carboxamide
(S)-4-amino-N-methyl-N-(6-(perfluoroethyl)-2,3-dihydrobenzofuran-3-yl)imidazo[1,5-a]quinoxaline-8-carboxamide
(S)-4-amino-7-fluoro-N-methyl-N-(6-(trifluoromethyl)-2,3-dihydrobenzofuran-3-yl)imidazo[1,5-a]quinoxaline-8-carboxamide.

**34.** The method according to any one of claims 1 to 33, wherein, the compound is selected from: (S)-4-amino-N-methyl-N-(6-(trifluoromethyl)-2,3-dihydrobenzofuran-3-yl)imidazo[1,5-a]quinoxaline-8-carboxamide.

**35.** The method according to any one of claims 1 to 34, wherein the tumor or cancer comprises a homozygous deletion of the methylthioadenosine phosphorylase (MTAP) gene.

**36.** The method according to any one of claims 1 to 35, wherein the tumor or cancer further comprises a homozygous deletion of the cyclin-dependent kinase inhibitor 2A (CDKN2A) gene.

**37.** The method according to any one of claims 1 to 36, wherein the tumor or cancer is selected from: lung cancer (non-small cell lung cancer (including adenocarcinoma and squamous cell carcinoma) or small cell lung cancer), pancreatic cancer, head and neck cancer, bladder cancer, esophageal cancer, mesothelioma, prostate cancer, breast cancer, brain cancer (e.g., glioblastoma multiforme and astrocytoma), skin cancer, cervical cancer, testicular cancer, colorectal cancer, endometrial cancer, gastric cancer, liver cancer (e.g., hepatoblastoma and hepatocellular adenoma), laryngeal cancer, oral cancer, ovarian cancer, thyroid cancer, cholangiocarcinoma, angiosarcoma, hemangioma, gallbladder cancer, papillary carcinoma, colon cancer, renal cancer, melanoma, multiple myeloma, chronic myeloid leukemia, hematological tumor, lymphoid tumor (e.g., diffuse large B-cell lymphoma), bone cancer, adrenal cancer, thymoma, malignant peripheral nerve sheath tumor, papillary renal cell carcinoma, and clear cell renal cell carcinoma.

**38.** The method according to any one of claims 1 to 37, wherein the tumor or cancer is selected from: brain cancer (e.g., glioblastoma multiforme and astrocytoma), lung cancer, colorectal cancer, bone cancer, gastric cancer, esophageal cancer, cholangiocarcinoma, liver cancer, ovarian cancer, breast cancer, hematological tumor, lymphoid tumor, malignant peripheral nerve sheath tumor, pancreatic cancer, bladder cancer, prostate cancer, and head and neck cancer.

**39.** The method according to any one of claims 1 to 38, wherein the tumor or cancer comprises metastatic lesions in tissues or organs distant from the primary tumor site.

**40.** The method for treating a tumor or cancer according to claim 38, wherein the tumor or cancer is selected from: glioma, lung adenocarcinoma, lung squamous cell carcinoma, bone cancer, gastric cancer, esophageal cancer, cholangiocarcinoma, liver cancer, ovarian cancer, malignant peripheral nerve sheath tumor, and lymphoid tumor.

**41.** The method according to 39, wherein the tumor or cancer is a cancer associated with brain metastasis.

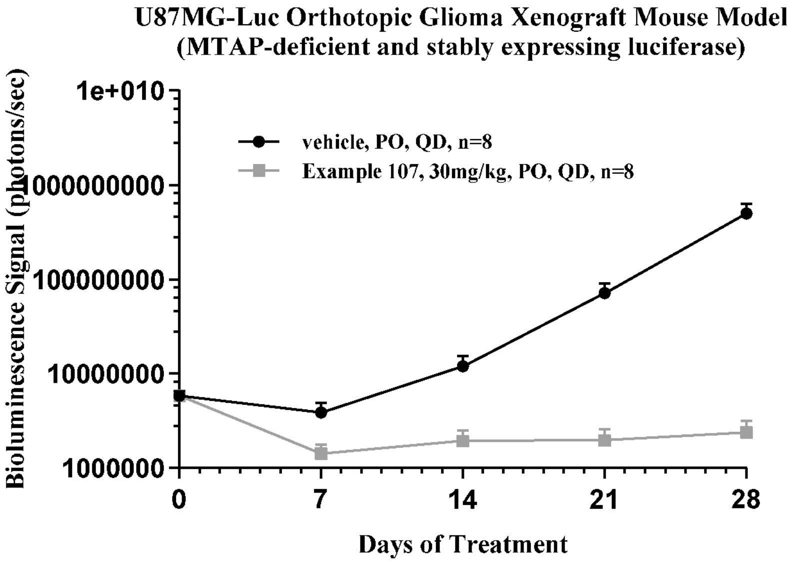
U87MG-Luc Orthotopic Glioma Xenograft Mouse Model
(MTAP-deficient and stably expressing luciferase)
Bioluminescence Signal (photons/sec)
1e+010
1000000000
100000000
10000000
1000000
vehicle, PO, QD, n=8
Example 107, 30mg/kg, PO, QD, n=8
0
7
14
21
28
Days of Treatment

Figure 1

**INTERNATIONAL SEARCH REPORT**

International application No. **PCT/CN2024/124325**

**A. CLASSIFICATION OF SUBJECT MATTER**

A61K31/519(2006.01)i; A61K31/536(2006.01)i; C07D471/14(2006.01)i; C07D487/04(2006.01)i; C07D519/00(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K 31/-; C07D 471/-; C07D 487/-; C07D 519/-; A61P 35/-

Documentation searched other than minimum documentation to the extent that such documents are included in the fields searched

Electronic data base consulted during the international search (name of data base and, where practicable, search terms used)

CNTXT, ENTXTC, DWPI, REGISTRY, CAPLUS, MARPAT, CNKI: 湃隆, PRMT5, 蛋白质精氨酸甲基转移酶, 瘤, 癌, cancer, tumor, oncology, carcinoma, protein arginine methyltransferase, 式I结构式检索, structural formula search for formula I

**C. DOCUMENTS CONSIDERED TO BE RELEVANT**

| Category* | Citation of document, with indication, where appropriate, of the relevant passages | Relevant to claim No. |
|---|---|---|
| PX | WO 2024067433 A1 (SHANGHAI APEIRON THERAPEUTICS COMPANY LIMITED) 04 April 2024 (2024-04-04)<br>description, page 1, and claims 1-32 | 1-41 |
| A | WO 2004085439 A1 (PFIZER PRODUCTS INC.) 07 October 2004 (2004-10-07)<br>abstract, and claims 1-14 | 1-41 |
| A | WO 9909845 A1 (BRISTOL-MYERS SQUIBB COMPANY) 04 March 1999 (1999-03-04)<br>abstract, and claims 1-44 | 1-41 |
| A | 王廷良等 (WANG, Tingliang et al.). "基于结构虚拟筛选发现蛋白质精氨酸甲基转移酶5抑制剂 (Discovery of PRMT5 Inhibitors via Structure-Based Virtual Screening)" *化学通报 (Chemistry),* Vol. 86, No. (9), 12 September 2023 (2023-09-12), 1137-1145<br>ISSN: 0441-3776,<br>page 1137, abstract, and page 1139, schemes 2 and 3 | 1-41 |

☑ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **15 January 2025** | **26 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2024/124325**

**C. DOCUMENTS CONSIDERED TO BE RELEVANT**

| Category* | Citation of document, with indication, where appropriate, of the relevant passages | Relevant to claim No. |
|---|---|---|
| A | Vittoria Colotta et al. "Synthesis of 4-Amino-6-(hetero)arylalkylamino-1,2,4-triazolo[4,3-a]quinoxalin-1-one Derivatives as Potent A2A Adenosine Receptor Antagonists" *Bioorganic & Medicinal Chemistry,* Vol. 11, No. (24), 30 October 2003 (2003-10-30), 5509-5518<br>ISSN: 0968-0896,<br>page 5509, abstract | 1-41 |

Form PCT/ISA/210 (second sheet) (July 2022)

**INTERNATIONAL SEARCH REPORT**

International application No.
**PCT/CN2024/124325**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-41**
because they relate to subject matter not required to be searched by this Authority, namely:

Claims 1-41 set forth a method for treating tumors or cancers, which falls within subject matter for which no search is required by the International Searching Authority as defined in PCT Rule 39.1(iv). However, in the present international search report, a search is still carried out with regard to a corresponding pharmaceutical use subject matter.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/124325**

| Patent document cited in search report | | | Publication date (day/month/year) | Patent family member(s) | | | Publication date (day/month/year) |
|---|---|---|---|---|---|---|---|
| WO | 2024067433 | A1 | 04 April 2024 | TW | 202413362 | A | 01 April 2024 |
| | | | | US | 2024294528 | A1 | 05 September 2024 |
| | | | | CN | 118613482 | A | 06 September 2024 |
| WO | 2004085439 | A1 | 07 October 2004 | JP | 2006521343 | A | 21 September 2006 |
| | | | | CA | 2520251 | A1 | 07 October 2004 |
| | | | | BRPI | 0407926 | A | 21 February 2006 |
| | | | | MXPA | 05010293 | A | 17 November 2005 |
| | | | | EP | 1613629 | A1 | 11 January 2006 |
| | | | | US | 2004192698 | A1 | 30 September 2004 |
| | | | | US | 7202245 | B2 | 10 April 2007 |
| WO | 9909845 | A1 | 04 March 1999 | AR | 013441 | A1 | 27 December 2000 |
| | | | | US | 6235740 | B1 | 22 May 2001 |
| | | | | AU | 8681798 | A | 16 March 1999 |
| | | | | PE | 106300 | A1 | 23 January 2000 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022169948 A1